# EUROPEAN PATENT SPECIFICATION

(11) **EP 1 671 578 B1**
(45) Date of publication and mention of the grant of the patent: **21.08.2013**
(21) Application number: 04792115.0
(22) Date of filing: 06.10.2004
(51) Int. Cl.: A61B 5/02, A61B 5/00

(54) **SPHYGMOMANOMETER**
SPHYGMOMANOMETER
SPHYGMOMANOMETRE

(30) Priority: 09.10.2003 JP 2003350932; 09.10.2003 JP 2003350933; 05.01.2004 JP 2004000660; 20.04.2004 JP 2004124168; 27.07.2004 JP 2004218616
(43) Date of publication of application: 21.06.2006
(73) Proprietor: NIPPON TELEGRAPH AND TELEPHONE CORPORATION, Tokyo 100-8116 (JP)
(72) Inventor: Uenishi, Yuji, c/o NTT Intellectual Property Center, Musashino-shi, Tokyo 1808585 (JP); Higurashi, Eiji, c/o NTT Intellectual Property Center, Musashino-shi, Tokyo 1808585 (JP); Naganuma, Kazunori, c/o NTT Intellectual Property Cent, Musashino-shi, Tokyo 1808585 (JP); Sudo, Shouichi, c/o NTT Intellectual Property Center, Musashino-shi, Tokyo 1808585 (JP); Shimada, Junichi, c/o NTT Intellectual Property Center, Musashino-shi, Tokyo 1808585 (JP); Aihara, Kimihisa, c/o NTT Intellectual Property Center, Musashino-shi, Tokyo 1808585 (JP); Koizumi, Hiroshi, c/o NTT Intellectual Property Center, Musashino-shi, Tokyo 1808585 (JP); Tatara, Naoe, c/o NTT Intellectual Property Center, Musashino-shi, Tokyo 1808585 (JP); Hayashida, Shoichi, c/o NTT Intellectual Property Cent, Musashino-shi, Tokyo 1808585 (JP); Mino, Shinji, c/o NTT Intellectual Property Center, Musashino-shi, Tokyo 1808585 (JP); Oguchi, Taisuke, c/o NTT Intellectual Property Center, Musashino-shi, Tokyo 1808585 (JP); Tochikubo, Osamu, Yokohama-shi, Kanagawa 2400024 (JP)
(74) Representative: Maury, Richard Philip
(86) International application number: PCT/JP2004/014759
(87) International publication number: WO 2005/034742

(56) References cited:
- EP-A1- 0 678 277
- WO-A1-02/089663
- JP-A- 4 256 727
- JP-A- 7 241 279
- JP-A- 9 122 083
- JP-A- 2003 052 652
- JP-B2- 2 796 540
- JP-B2- 3 270 916
- JP-B2- 4 017 651
- JP-U- 6 055 602
- JP-U- 53 034 292
- JP-U- 63 111 103
- US-A- 3 051 165
- US-A- 3 412 729
- US-A- 5 267 566

## Description

### TECHNICAL FIELD

The present invention relates to an apparatus for detecting living body information at an ear part.

### BACKGROUND ART

As the population is aging, response to lifestyle-related diseases of adults is becoming a large public problem. Especially, as to diseases related to high blood pressure, it is recognized that collecting blood pressure data for the long term is very important. From this viewpoint, various measurement apparatuses for measuring living body information such as the blood pressure are being developed.

As a conventional technology for measuring living body information at an external ear part, there is a patient monitoring apparatus that is inserted into an external auditory meatus or other parts of the external ear for wearing continuously (refer to patent document 1, for example). This apparatus calculates pulse, pulse wave, electrocardiogram, body temperature, arterial oxygen saturation, blood pressure and the like based on received light amount of scattered light of infrared light or visible light that is radiated into the living body. However, this apparatus does not have any means for fixing to the ear so that living body information cannot be measured stably. In addition, any concrete measurement method of blood pressure is not disclosed.

Although the shape of the ear is complicated (refer to non-patent document 1, for example), the conventional apparatus is for being worn in the external auditory meatus or on an earlobe. Therefore, the apparatus is difficult to be fixed to the ear.

In addition, as an apparatus to be worn in the external auditory meatus or on the earlobe, there is an emergency information apparatus that includes a wireless communication means, an arterial oxygen saturation sensor, a body temperature sensor, an electrocardiogram sensor and a pulse wave sensor (refer to patent document 2, for example). The sensor part of this apparatus is inserted into the external auditory meatus and the data communication part also serves as a fixing means to the ear. But, this apparatus cannot be necessarily worn stably.

On the other hand, as to measurement of blood pressure, there is a research result that, a blood pressure measurement apparatus using a pulsation waveform of a blood vessel (refer to non-patent document 2, for example) enables high-precision measurement of blood pressure as compared with blood pressure measurement apparatuses of other schemes such as a cuff vibration method and a volume compensation method (refer to non-patent document 3, for example).

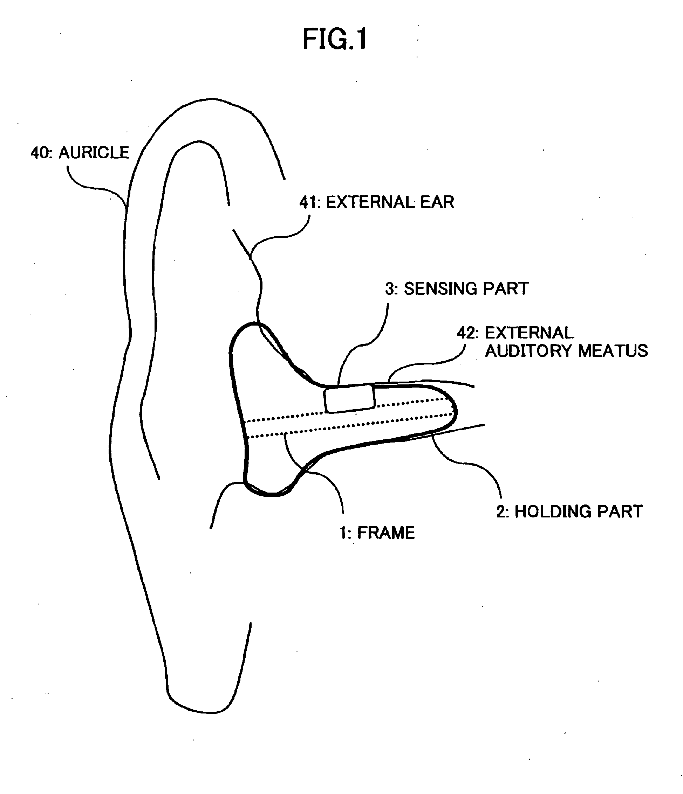

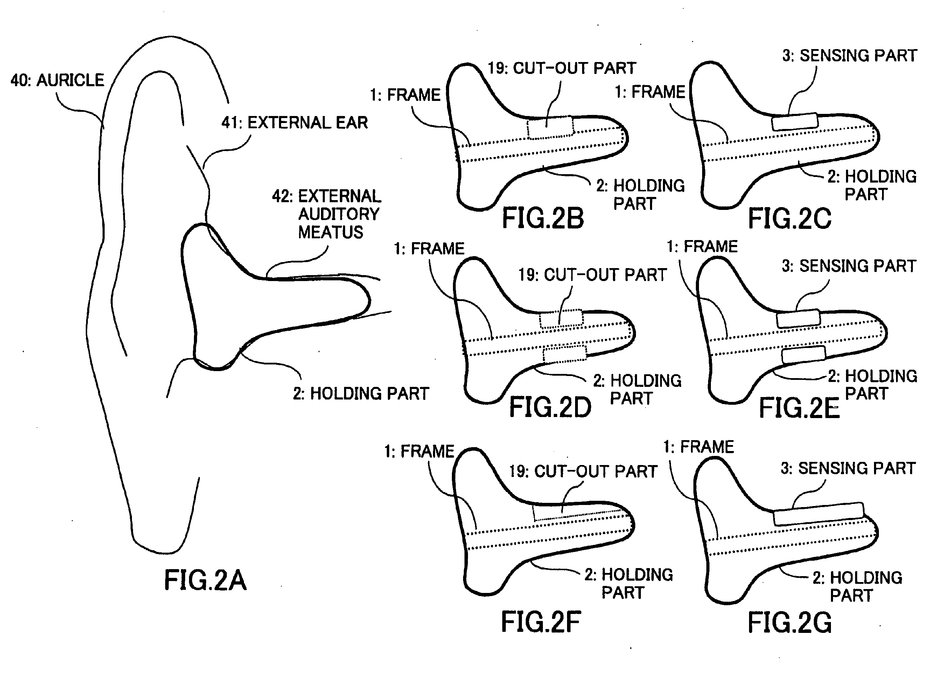

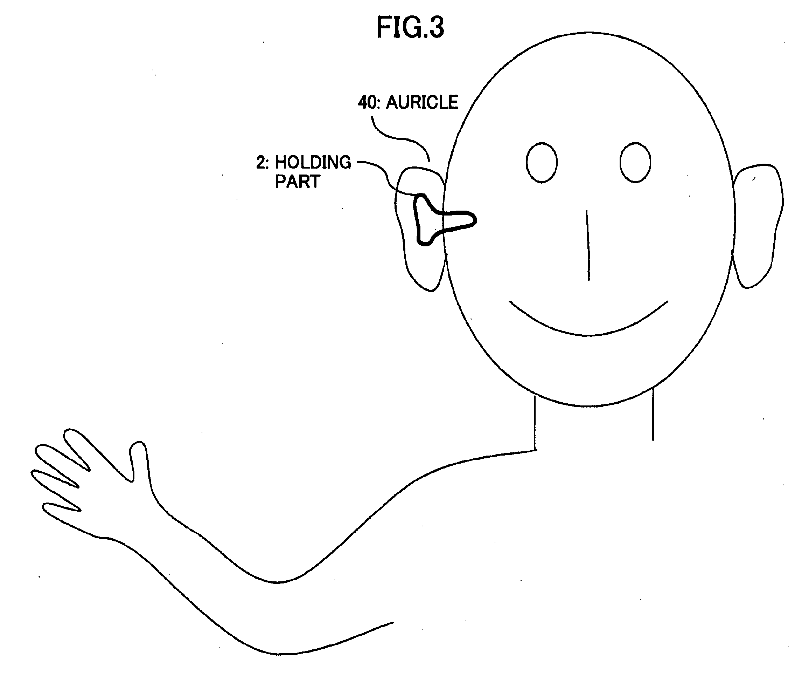

In this application, names of parts of the auricle are mainly based on the non-patent document 1, and names of cartilage of the auricle are based on the non-patent document 4. In addition, the patent document 3 can be taken as an example of a document related to an apparatus for measuring blood pressure.
[Patent document 1] Japanese Laid-Open Patent Application No.9-122083
[Patent document 2] Japanese Laid-Open Patent Application No.11-128174
[Patent document 3] Japanese Patent No.3531386
[Non-patent document 1] Sobotta, Atlas of Human Anatomy, vol.1 (translation supervisor: Michio Okamoto), p.126-p.127, Igaku Shoin
[Non-patent document 2] Osamu Tochikubo,Yoshiyuki Kawaso,Eiji Miyajima,Masao Ishii:A new poto-oscillometric method employing the delta-algorithm for accurate blood pressure measurement. Journal of Hypertension 1997,Vol2 pp.148-pp.151,Fig.1,Fig.3
[Non-patent document 3] K. Yamakoshi, T.Togawa, "Living body sensor and Measurement apparatus", Japan Society of Medical Electronics and Biological Engineering / ME text book series, A-1, pp.39-52
[Non-patent document 4] Sobotta, Atlas of Human Anatomy, vol.1 (translation supervisor: Michio Okamoto), p.127, Igaku Shoin, October 1, 1996
[Non-patent document 5] L.A. GEDDES ┌The DIRECT and INDIRECT MEASURMENT of BLOOD PRESSURE┐, YEAR BOOK MEDIAL PUBLISHERS, INC. p. 97, Figs.2-22
US 3412729, US3 051165, JP 53 034292 U and JP4 017651 each disclose devices for measuring blood pressure at the ear.

### DISCLOSURE OF THE INVENTION

### PROBLEM TO BE SOLVED BY THE INVENTION

As to measurement such as blood pressure measurement, in which pressurization to a living body tissue is necessary, it is difficult to accurately measure the pulse wave and the blood pressure since noise is apt to be mixed due to vibration. Thus, it is a problem to measure a blood pressure stably. In addition, since it is difficult to measure the blood pressure at constant intervals or continuously in daily activities or in a state in which a blood pressure meter is always worn. Thus, it is a problem to realize a method of holding an apparatus for detecting living body information.

The present invention is contrived for solving the above-mentioned problems, and an object of the present invention is to provide an apparatus for measuring living body information at an ear part of a human body.

### MEANS FOR SOLVING THE PROBLEM

The solution is achieved by a blood-pressure meter according to claim 1.

All embodiments disclosed herein which do not fall under the scope of claim 1 are merely examples useful for understanding the claimed invention

### EFFECT OF THE INVENTION

According to the present invention, an apparatus that measures living body information and that is suitable for measurement at an ear part of a human body can be provided. In addition, by adopting a configuration including the pressure applying part, an apparatus especially suitable for measuring a blood pressure can be provided.

### BRTEF DESCRIPTION OF THF DRAWINGS

Fig.1 is a figure showing a configuration of a living body information collecting apparatus of an embodiment 1-1 of the present invention;
Fig.2 is a figure for explaining a manufacturing method of a holding part of the living body information collecting apparatus of the embodiment 1-1 of the present invention;
Fig.3 is a figure for explaining an example in which the living body information collecting apparatus of the embodiment 1-1 of the present invention is worn to a living body;
Fig.4 is a figure showing another configuration of the living body information collecting apparatus of the embodiment 1 of the present invention;
Fig.5 is a figure showing a configuration of the living body information collecting apparatus of an embodiment 1-2 of the present invention;
Fig.6 is a figure showing a configuration of the living body information collecting apparatus of an embodiment 1-3 of the present invention;
Fig.7 is a figure showing a configuration of the living body information collecting apparatus of the embodiment 1-3 of the present invention;
Fig.8 is a figure for explaining an example in which the living body information collecting apparatus of the embodiment 1-3 of the present invention is worn to a living body;
Fig.9 is a figure showing a configuration of the living body information collecting apparatus of an embodiment 1-4 of the present invention;
Fig.10 is a figure for explaining an example in which the living body information collecting apparatus of the embodiment 1-4 of the present invention is worn to a living body;
Fig.11 is a figure showing a configuration of the living body information collecting apparatus of an embodiment 1-5 of the present invention;
Fig.12 is a figure showing a configuration of the living body information collecting apparatus of an embodiment 1-6 of the present invention;
Fig.13 is a figure showing a configuration of the living body information collecting apparatus of an embodiment 1-7 of the present invention;
Fig.14 is a figure for explaining principle 1 of blood pressure measurement;
Fig.15 is a figure for explaining principle 1 of blood pressure measurement;
Fig.16 is a block diagram of a conventional blood pressure measurement apparatus;
Fig.17 is a figure for explaining principle 2 of blood pressure measurement;
Fig.18 is a figure showing another example of the living body information collection.
Fig.19 is a figure showing a configuration of the living body information collecting system of an embodiment 1-8 of the present invention;
Fig.20 is a figure showing a configuration of the living body information collecting system of an embodiment 1-9 of the present invention;
Fig.21 is a figure showing a configuration of the living body information collecting system of an embodiment 1-10 and an embodiment 11 of the present invention;
Fig.22 is a figure showing a configuration of the living body information collecting system of an embodiment 1-12 of the present invention;
Fig.23 is a figure showing a configuration of the living body information collecting system of an embodiment 1-13 of the present invention;
Fig.24 is a figure showing an implementation example and an example of wearing to the living body for the living body information collecting system of the embodiment 1-13 of the present invention;
Fig.25 is a figure showing an implementation example of the holding part of the living body information collecting apparatus of the embodiment of the present invention;
Fig.26 is a figure showing a configuration of a blood-pressure meter of an embodiment 2-1 of the present invention;
Fig.27 is a figure for explaining blood pressure measurement using the principle 1 of blood pressure measurement in the embodiment. 2-1 of the present invention in detail;
Fig.28 is a figure showing a configuration of a blood-pressure meter of an embodiment 2-2 of the present invention;
Fig.29 is a figure showing a configuration of a blood-pressure meter of an embodiment 2-3 of the present invention;
Fig. 30 is a figure showing a configuration of a blood-pressure meter of an embodiment 2-4 of the present invention;
Fig.31 is a figure showing a configuration of a blood-pressure meter of the embodiment 2-4 of the present invention;
Fig.32 is a figure showing a configuration of a blood-pressure meter of the embodiment 2-4 of the present invention;
Fig.33 is a figure showing a configuration of a blood-pressure meter of an embodiment 2-5 of the present invention;
Fig.34 is a figure showing a configuration of a blood-pressure meter of an embodiment 2-6 of the present invention;
Fig.35 is a figure showing a configuration of a blood-pressure meter of an embodiment 2-6 of the present invention;
Fig.37 is a figure showing a configuration of a blood-pressure meter of an embodiment 2-8 of the present invention;
Fig.38 is a figure showing a configuration of a blood-pressure meter of an embodiment 2-9 of the present invention;
Fig.39 is a figure showing a configuration of a blood-pressure meter of an embodiment 2-10 of the present invention;
Fig.40 is a figure showing a configuration of a blood-pressure meter of the embodiment 2-10 of the present invention;
Fig.41 is a figure showing a configuration of a blood-pressure meter of an embodiment 2-11 of the present invention;
Fig.42 is a figure showing a configuration of a blood-pressure meter of the embodiment 2-11 of the present invention;
Fig.43 is a figure showing a configuration of a blood-pressure meter of an embodiment 2-12 of the present invention;
Fig.44 is a figure showing a configuration of a blood-pressure meter of the embodiment 2-12 of the present invention;
Fig.45 is a figure showing a configuration of a blood-pressure meter of an embodiment 2-13 of the present invention;
Fig.46 is a figure showing a configuration of a blood-pressure meter of the embodiment 2-13 of the present invention;
Fig.47 is a figure showing a configuration of a blood-pressure meter of the embodiment 2-13 of the present invention;
Fig. 48 is a figure showing a configuration in which a fixing part 4 and a fixing adjustment part 5 are added to the blood-pressure meter of the embodiment 2-9;
Fig.49 is a figure showing a configuration, in which the fixing part 4 and the fixing adjustment part 5 are added to the blood-pressure meter of the embodiment 2-12;
Fig.50 is a figure showing a configuration in which the fixing part 4 and the fixing adjustment part 5 are added to the blood-pressure meter of the embodiment 2-12;
Fig.51 is a figure showing a configuration of a blood-pressure meter of an embodiment 2-15 of the present invention;
Fig.52 is a figure showing a configuration of a blood-pressure meter of an embodiment 2-16 of the present invention;
Fig.53 is a figure showing a state in which the blood-pressure meter of the embodiment 2-16 is worn to the ear;
Fig.54 is a figure showing a configuration of a blood-pressure meter of an embodiment 2-17 of the present invention;
Fig.55 is a figure showing an example in which a suspension mechanism 61 is attached to a temple 62 of the eyeglasses;
Fig.56 is a figure showing an example in which the suspension mechanism 61 is attached to the top part of the temple 62 of the eyeglasses;
Fig.57 is a figure showing structure of cartilage in the auricle and names of each part;
Fig.58 is a figure showing structure of of the auricle and names of each part;
Fig.59 is a figure for explaining the external ear;
Fig.60 is a figure for explaining periphery of the external ear;
Fig.61 is a figure showing a configuration example of a living body information detection apparatus of a third embodiment;
Fig.62 is a figure showing a configuration example of a living body information detection apparatus of the third embodiment;
Fig.63 is a figure showing a configuration example of a living body information detection apparatus of the third embodiment;
Fig.64 is a figure showing a configuration example of a living body information detection apparatus of the third embodiment;
Fig.65 is a figure showing a configuration example of a living body information detection apparatus of the third embodiment;
Fig.66 is a figure showing a configuration example of a living body information detection apparatus of the third embodiment;
Fig.67 is a figure for explaining principle for detecting a pulse wave using a light-emitting element and a light-receiving element;
Fig.68 is a figure showing a configuration example of a living body information detection apparatus that can measure blood pressure in the third embodiment;
Fig.69 is a figure showing a configuration example of a living body information detection apparatus that can measure blood pressure in the third embodiment;
Fig.70 is a figure showing a configuration example of a living body information detection apparatus that can measure blood pressure in the third embodiment;
Fig.71 is a figure showing a configuration example of a living body information detection apparatus that can measure blood pressure in the third embodiment;
Fig.72 is a figure showing a configuration example of a living body information detection apparatus that can measure blood pressure in the third embodiment;
Fig.73 is a figure showing a configuration example of a living body information detection apparatus that can measure blood pressure in the third embodiment;
Fig.74 is a figure showing a configuration example of a living body information detection apparatus that can measure blood pressure in the third embodiment;
Fig.75 is a figure showing a configuration example of a living body information detection apparatus that can measure blood pressure in the third embodiment;
Fig.76 is a figure showing a configuration example of a living body information detection apparatus that can measure blood pressure in the third embodiment;
Fig.77 is a figure showing a configuration example of a living body information detection apparatus that can measure blood pressure in the third embodiment;
Fig.78 is a figure showing a configuration example of a living body information detection apparatus that can measure blood pressure in the third embodiment;
Fig.79 is a figure showing a configuration example of a living body information detection apparatus that can measure blood pressure in the third embodiment;
Fig.80 is a figure showing a configuration example of a living body information detection apparatus that can measure blood pressure in the third embodiment;
Fig.81 is a figure showing a configuration example of a living body information detection apparatus that can measure blood pressure in the third embodiment;
Fig.82 is an explanation figure showing a structure example of a living body information detection apparatus of a fourth embodiment;
Fig.83 is an explanation figure showing a structure example of a living body information detection apparatus of the fourth embodiment;
Fig.84 is an explanation figure showing a structure example of a living body information detection apparatus of the fourth embodiment;
Fig.85 is an explanation figure showing a state in which the living body information detection apparatus of the fourth embodiment is worn to the auricle;
Fig.86 is an explanation figure showing a structure example of a living body information detection apparatus of the fourth embodiment;
Fig.87 is an explanation figure showing a structure example of a living body information detection apparatus of the fourth embodiment;
Fig. 88 is an explanation figure showing a structure example of a living body information detection apparatus of the fourth embodiment;
Fig.89 is an explanation figure showing a state in which the living body information detection apparatus of the fourth embodiment is worn to the auricle;
Fig.90 is an explanation figure showing a structure example of a living body information detection apparatus of the fourth embodiment;
Fig.91 is an explanation figure showing a structure example of a living body information detection apparatus of the fourth embodiment and a state in which the living body information detection apparatus is worn to the auricle;
Fig.92 is an explanation figure showing a structure example of a living body information detection apparatus of the fourth embodiment and a state in which the living body information detection apparatus is worn to the auricle;
Fig.93 is an explanation figure showing a structure example of a living body information detection apparatus of the fourth embodiment and a state in which the living body information detection apparatus is worn to the auricle;
Fig.94 is an explanation figure showing a structure example of a living body information detection apparatus of the fourth embodiment;
Fig.95 is an explanation figure showing a structure example of a living body information detection apparatus of the fourth embodiment and a state in which the living body information detection apparatus is worn to the auricle;
Fig.96 is an explanation figure showing a structure example of a living body information detection apparatus of the fourth embodiment;
Fig.97 is an explanation figure for explaining principle for detecting a pulse wave using a light-emitting element and a light-receiving element;
Fig.98 is an explanation figure showing a structure example of a living body information detection apparatus of the fourth embodiment and a state in which the living body information detection apparatus is worn to the auricle;
Fig.99 is an explanation figure showing a state in which a sensor part of the living body information detection apparatus of the fourth embodiment is worn to the auricle;
Fig.100 is an explanation figure showing a state in which a sensor part of the living body information detection apparatus of the fourth embodiment is worn to the auricle;
Fig.101 is an explanation figure showing a state in which a sensor part of the living body information detection apparatus of the fourth embodiment is worn to the auricle;
Fig.102 is an explanation figure showing a state in which a sensor part of the living body information detection apparatus of the fourth embodiment is worn to the auricle;
Fig.103 is an explanation figure showing a state in which a sensor part of the living body information detection apparatus of the fourth embodiment is worn to the auricle;
Fig.104 is an explanation figure showing a state in which a sensor part of the living body information detection apparatus of the fourth embodiment is worn to the auricle;
Fig.105 is an explanation figure showing a state in which a sensor part of the living body information detection apparatus of the fourth embodiment is worn to the auricle;
Fig.106 is an explanation figure showing a state in which a sensor part of the living body information detection apparatus of the fourth embodiment is worn to the auricle;
Fig.107 is an explanation figure showing a state in which a sensor part of the living body information detection apparatus of the fourth embodiment is worn to the auricle;
Fig.108 is an explanation figure showing a state in which a sensor part of the living body information detection apparatus of the fourth embodiment is worn to the auricle;
Fig.109 is an explanation figure showing a state in which a sensor part of the living body information detection apparatus of the fourth embodiment is worn to the auricle;
Fig.110 is a figure showing a configuration example of the living body information detection apparatus of the fourth embodiment;
Fig.111 is a figure showing a configuration example of the living body information detection apparatus of the fourth embodiment;
Fig.112 is a schematic section view showing a configuration example of a cuff of the fifth embodiment;
Fig.113 is a schematic diagram showing a configuration example of the cuff of the fifth embodiment, and Fig.113A a top view, Fig.113B is a section view at A-A' in the top view;
Fig.114 is a schematic diagram showing a configuration example of the cuff of the fifth embodiment, and Fig.113A a top view, Fig.113B is a section view at A-A' in the top view;
Fig.115 is a schematic section view showing a configuration example of the cuff of the fifth embodiment, and a process in which the cuff presses the living body;
Fig.116 is a schematic section view showing a configuration example of the cuff of the fifth embodiment, and a process in which the cuff presses the living body;
Fig.117 is a schematic section view showing a configuration example of the cuff of the fifth embodiment, and a process in which the cuff presses the living body;
Fig.118 is a schematic section view showing a configuration of the cuff of the fifth embodiment;
Fig.119 is a schematic section view showing a configuration of the cuff of the fifth embodiment;
Fig.120 is a schematic section view showing a configuration of the cuff of the fifth embodiment;
Fig.121 is a schematic section view showing a configuration of the cuff of the fifth embodiment;
Fig.122 is a schematic section view showing a configuration of the cuff of the fifth embodiment;
Fig.123 is a schematic section view showing a configuration of the cuff of the fifth embodiment;
Fig.124 is a schematic section view showing a configuration of the cuff of the fifth embodiment;
Fig.125 is a schematic section view showing a configuration of the cuff of the fifth embodiment;
Fig.126 is a schematic section view showing a configuration of the cuff of the fifth embodiment;
Fig.127 is a schematic section view showing a configuration of the cuff of the fifth embodiment;
Fig.128 is a schematic section view showing a configuration of the cuff of the fifth embodiment;
Fig.129 is a schematic section view showing a configuration of the cuff of the fifth embodiment;
Fig.130 is a schematic section view showing a configuration of the cuff of the fifth embodiment;
Fig.131 is a schematic section view showing a configuration of the cuff of the fifth embodiment;
Fig.132 is a schematic section view showing a configuration of the cuff of the fifth embodiment;
Fig.133 is a schematic section view showing a configuration of the cuff of the fifth embodiment;
Fig.134 is an explanation figure of a living body information detection circuit and a cuff of the sixth embodiment;
Fig.135 is an explanation figure of a living body information detection circuit and a cuff of the sixth embodiment;
Fig.136 is an explanation figure of principle of blood pressure measurement;
Fig.137 is an explanation figure for explaining examples for detecting a pulsation waveform by a living body information detection circuit of the sixth embodiment and a conventional living body information detection circuit;
Fig.138 is an explanation figure of a living body information detection circuit and a cuff of the sixth embodiment;
Fig.139 is an explanation figure of a living body information detection circuit and a cuff of the sixth embodiment;
Fig.140 is an explanation figure of a living body information detection circuit and a cuff of the sixth embodiment;
Fig.141 is an explanation figure of a living body information detection circuit and a cuff of the sixth embodiment;
Fig.142 is an explanation figure of a living body information detection circuit and a cuff of the sixth embodiment;
Fig.143 is an explanation figure of a living body information detection circuit and a cuff of the sixth embodiment;
Fig.144 is an explanation figure of a living body information detection circuit and a cuff of the sixth embodiment;
Fig.145 is an explanation figure of a living body information detection circuit and a cuff of the sixth embodiment;
Fig.146 is an explanation figure of a living body information detection circuit and a cuff of the sixth embodiment;
Fig.147 is an explanation figure of a living body information detection circuit and a cuff of the sixth embodiment;
Fig.148 is an explanation figure of a living body information detection circuit and a cuff of the sixth embodiment;
Fig.149 is an explanation figure of a living body information detection circuit and a cuff of the sixth embodiment;
Fig.150 is a figure for explaining blood pressure measurement in the sixth embodiment;
Fig.151 is a figure for explaining blood pressure measurement in the sixth embodiment;
Fig.152 is a figure for explaining blood pressure measurement in the sixth embodiment;
Fig.153 is a figure for explaining blood pressure measurement in the sixth embodiment;
Fig.154 is a figure of a configuration of a main body part of a living body information measurement apparatus in a seventh embodiment.

### Explanation of reference signs

### (First embodiment)

1 frame, 2 holding part, 3 sensing part, 4 drive control part, 5 transmission part, 6 power supply part, 7 suspension part, 8 portable terminal, 9 terminal receiving part, 10 display part, 11 communication part, 12 terminal receiving part, 13 receiving part, 14 acoustic part, 15 transmit and receive part, 16 signal line, 17 pressure supplying pipe, 18 acoustic part suspension part, 19 cut-out part, 20 light-emitting element, 21 light-receiving element, 22 pressure generation mechanism, 23 pressure detection mechanism, 30 blood pressure sensor, 31 body temperature sensor, 32 pulse sensor, 33 posture sensor, 34 acceleration sensor, 35 blood oxygen levels sensor, 36 electroencephalogram sensor, 37 signal line, 40 auricle, 41 external ear, 42 external auditory meatus, 50 information processing apparatus, 51 communication network, 52 antenna

### (Second embodiment)

1 first arm, 2 second arm, 3 holding frame part, 4 fixing part, 5 fixing adjustment part, 6 control part, 7 display part, 10 light-emitting element, 11 first light-emitting element, 12 second light-emitting element, 15 driving circuit, 16 first driving circuit, 17 second driving circuit, 20 light-receiving element 21 first light-receiving element, 22 second light-receiving element, 25 signal processing circuit, 30 pressure applying part, 31 first pressure applying part, 32 second pressure applying part, 35 pressure control part, 36 first pressure control part, 37 second pressure control part, 40 pressure sensor, 45 pump, 50, a part of auricle, 60 fixing mechanism, 61 suspension mechanism, 62 temple of eyeglasses, 70 blood-pressure meter, 80 auricle

### (Third embodiment)

1 tragus, 2 antitragus, 3 concha auriculae, 4 antihelix, 5 helix, 6 crus anthelicis, 7 crus helicis, 8 cavum conchae, 11 lamina of tragus, 12 cartilage of acoustic meatus, 13 antihelix, 14 helix, 15 pina helices, 16 squamous part of temporal bone, 17 incisura cartilaginis meatus acustici externi, 18 tympanic portion of the temporal bone, 20 living body tissue, 30 living body information detection apparatus, 31 hollow, 32 fixing mechanism, 41 light-emitting element, 42 light-receiving element, 43 incident light, 44 scattered light, 45 cuff, 46 air pipe, 47 cuff, 48 cuff, 61 air pipe, 62 air pipe

### (Forth embodiment)

1 tragus, 2 antitragus, 3 concha auriculae, 4 antihelix, 5 helix, 6 crus anthelicis, 7 crus helicis, 8 cavum conchae, 11 lamina of tragus, 12 cartilage of acoustic meatus, 13 antihelix, 14 helix, 15 pina helices, 16 squamous part of temporal bone, 17 incisura cartilaginis meatus acustici externi, 18 tympanic portion of the temporal bone, 30 living body information detection apparatus, 31 first arm, 32 second arm, 33 sensor, 34 sensor, 35 spindle, 36 air pipe, 37 signal line, 38 pinching part, 40 distance variable mechanism, 41 rotation mechanism, 42 position variable mechanism, 43 length variable mechanism, 44 length variable mechanism, 45 cushion, 46 ear suspension mechanism, 47 magnet, 48 magnet, 49 light shielding cover, 50 light shielding cover, 51 light shielding cover, 52 light shielding cover base, 53 speaker, 55 cuff, 56 cuff, 57 support, 58 support, 61 light-emitting element, 62 light-receiving element, 65 incident light, 66 scattered light

### (Fifth embodiment)

1 living body, 12 case, 13 elastic member, 14 pressing surface, 15 side part, 16 air supplying pipe, 17 fixing part, 18, 19 slack, 21 light-emitting element, 22 irradiating light, 23 light-receiving element, 24 scattered light, 50-62 cuff

### (Sixth embodiment)

1 living body, 2 tragus, 11 living body information detection circuit, 12 case, 13 living body pressing surface, 14 air pipe, 15 cuff, 16 air pipe, 17 U-shaped arms, 21 light-emitting element, 22 irradiating light, 23 light-receiving element, 31 light shielding structure, 32 hood, 33 light shielding structure, 34 lens, 43 lens, 51 applied pressure, 61 pressure in artery, 62 maximum blood pressure, 63 average blood pressure, 71 pulsation waveform, 72 flat part, 75 pulsation waveform, 76 pulsation waveform

### PREFERRED EMBODIMENTS FOR CARRYING OUT THE INVENTION

In the following, first to seventh embodiments of the present invention are described.

### (First embodiment)

First, the first embodiment is described.

### [Embodiment 1-1]

Fig.1 shows a configuration of a living body information collecting apparatus in the embodiment 1-1 of the present invention. As shown in Fig.1, the living body information collecting apparatus of this embodiment includes a hollow frame 1, a holding part 2 for holding the hollow frame 1 in the external auditory meatus, and a sensing part 3 that is attached to the hollow frame 1. Fig.1 shows a state in which the holding part 2 is worn in the external ear 41. Reference signs in figures in each embodiment in this application are assigned independently for each embodiment unless otherwise stated.

In the following, an example of a method for manufacturing the living body information collecting apparatus is described with reference to Figs.2A-2G each showing a section view of the living body information collecting apparatus. For manufacturing the living body information collecting apparatus of this embodiment, a shape of the external ear 41 and the external auditory meatus 42 of a subject is made with polymer resin impression material and the like. Of course, a shape applicable for any external ear and external auditory meatus of any person may be made. Next, based on this model, a whole shape of the holding part 2 is made with silicone resin and the like, for example. Further, a part is hollowed for keeping a route of sound to from the frame 1 as shown in Fig.2B. Further, a part 19 is cut out so as to be removed as shown in Fig.2B to place the sensing part 3 as shown in Fig.2C.

When the sensing part 3 is a cylinder, the cylindrical cut-out part 19 is cut out to be removed as shown in Fig.2D, so as to place the sensing part 3 as shown in Fig.2E. In addition, when it is necessary that the sensing part 3 applies a pressure to the external auditory meatus 42, a cut-out part 19 shown in Fig.2F is cut out such that the sensing part 3 efficiently touches the external auditory meatus 42, and the sensing part 3 is attached to the holding part 2 as shown in Fig.2G. An example of a state in which the holding part 2 is attached to the auricle 40 is as shown in Fig.2A.

It is needless to say that the living body information collecting apparatus is not limited to one manufactured in the manufacturing method described in this embodiment.

The operation of the living body information collecting apparatus of this embodiment is described with reference to Fig.1. A driving circuit (not shown in the figure) for driving the sensing part 3 and a signal processing circuit (not shown in the figure) for processing a signal of a measurement result of the sensing part 3 are connected to the sensing part 3 shown in Fig.1. The driving circuit sends a driving signal to the sensing part 3, the sensing part 3 measures living body information and sends a measurement result to the signal processing circuit. According to the living body information collecting apparatus of this configuration, living body information can be collected without affecting the sense of hearing.

Fig.3 shows an example of a state in which the living body information collecting apparatus of this embodiment is worn to a living body. According to the living body information collecting apparatus that can be worn as shown in Fig.3, living body information can be continuously collected even in daily life, while performing work or in sleeping.

In addition, in the living body information collecting apparatus of this embodiment, since the sensing part 3 is placed in the external auditory meatus 42 to measure living body information, the living body information collecting apparatus is hard to be affected by disturbance such as change of external temperature. Further, when a sensor related to blood is placed in the sensing part 3, for example, there is a merit that reproducibility of a measurement value is good since position relationship with the heart can be always kept constant.

The shape of the living body information collecting apparatus may be configured such that a part of the living body information collecting apparatus may include a shape formed by a cylinder, a cone, a prism, a pyramid, a truncated cone or a truncated pyramid, and include a hollow part that is a route of sound in the axial orientation of the cylinder, the cone, the prism, the pyramid, the truncated cone or the truncated pyramid, and the sensing part for collecting living body information.

The orientation of the axis of the cylinder, the prism, the truncated cone or the truncated pyramid is an orientation of a line connecting between a top surface and a bottom surface that are opposite to each other. The orientation of the axis of the cone or the pyramid is an orientation of a line connecting an apex and a bottom surface that is opposed to the apex. The hollow part dose not necessarily pass through the apex.

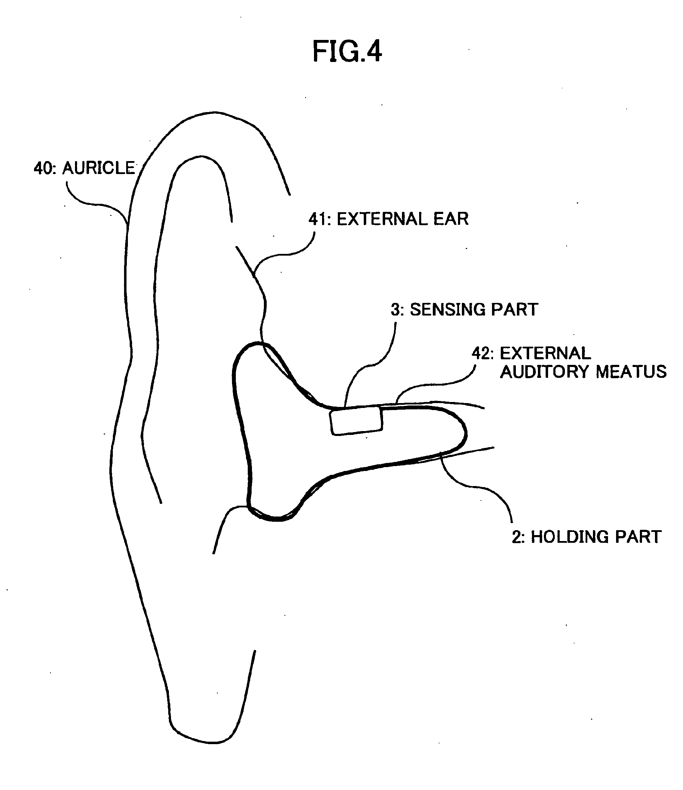

In addition, as shown in Fig.4, the living body information collecting apparatus of the first embodiment may be configured without the hollow part.

According to this living body information collecting apparatus, since the part of the shape formed by the cylinder, the cone, the prism, the pyramid, the truncated cone or the truncated pyramid can be inserted into the external auditory meatus, living body information can be collected while the apparatus is inserted in the external auditory meatus. In addition, since the hollow part is provided, even though the living body information collecting apparatus of the present invention is inserted into the external auditory meatus, living body information can be continuously collected without impeding hearing. Also in the living body information collecting apparatus of this shape, configurations of embodiments described below can be applied.

### [Embodiment 1-2]

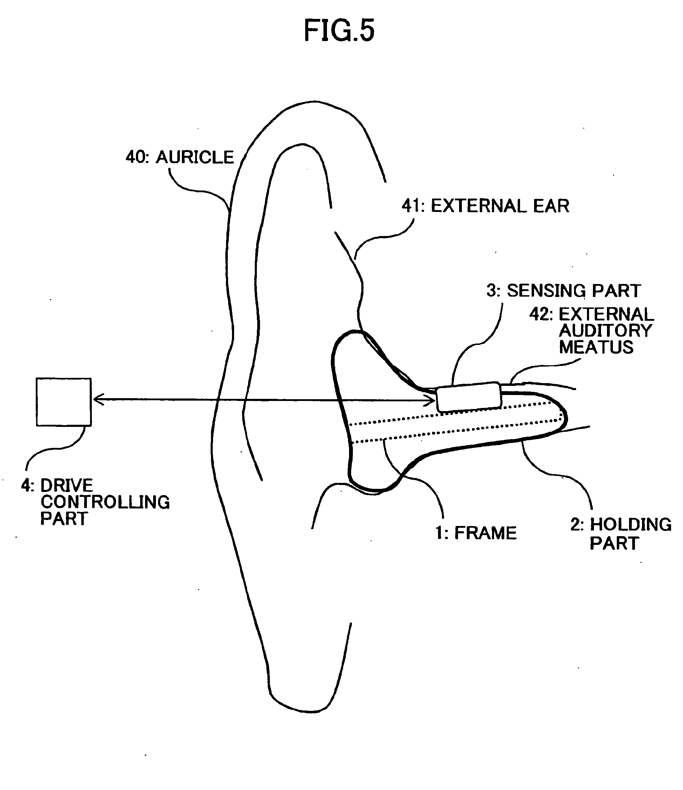

In the following, this embodiment is described with reference to Fig.5. Fig.5 shows a configuration of the living body information collecting apparatus of this embodiment. As shown in Fig.5, the living body information collecting apparatus of this embodiment includes a hollow frame 1, a holding part 2 for holding the hollow frame 1 to the external auditory meatus, a sensing part 3 attached to the hollow frame 1, and a drive controlling part 4 for performing drive control for the sensing part 3 and processing a signal from the sensing part. The drive controlling part 4 is connected to the sensing part 3 via the signal line.

Next, operation of the living body information collecting apparatus of this embodiment is described. The configuration including the hollow frame 1, the holding part 2 and the sensing part 3 is the same as that of the before-mentioned living body information collecting apparatus. A display part (not shown in the figure) for displaying a measurement result can be connected to the drive controlling part 4 shown in Fig.5. A drive signal is sent to the sensing part 3 via the drive controlling part 4, so that the sensing part 3 measures living body information and sends a measurement result to the drive controlling part 4. The drive controlling part 4 processes the signal of the measurement result of the sensing part 3, and displays the result on the display part (not shown in the figure) provided in the outside. In Fig.5, although the drive controlling part 4 is shown in the outside of the holding part 2, this is for the sake of explanation of the configuration and operation. The drive controlling part 4 can be downsized very much as an LSI so that it can be installed in the holding part 2. As mentioned above, the living body information collecting apparatus of this embodiment can easily measure and collect living body information.

According to the living body information collecting apparatus that can be worn in the way as shown in Fit..5, a connection line between the sensing part 3 and the drive controlling part 4 is not necessary. Thus, the living body information can be continuously collected while performing daily life or work and while sleeping. When the sensing part includes a plurality of sensors, the effect obtained by decreasing the connection line between the sensing part 3 and the drive controlling part 4 further increases.

### [Embodiment 1-3]

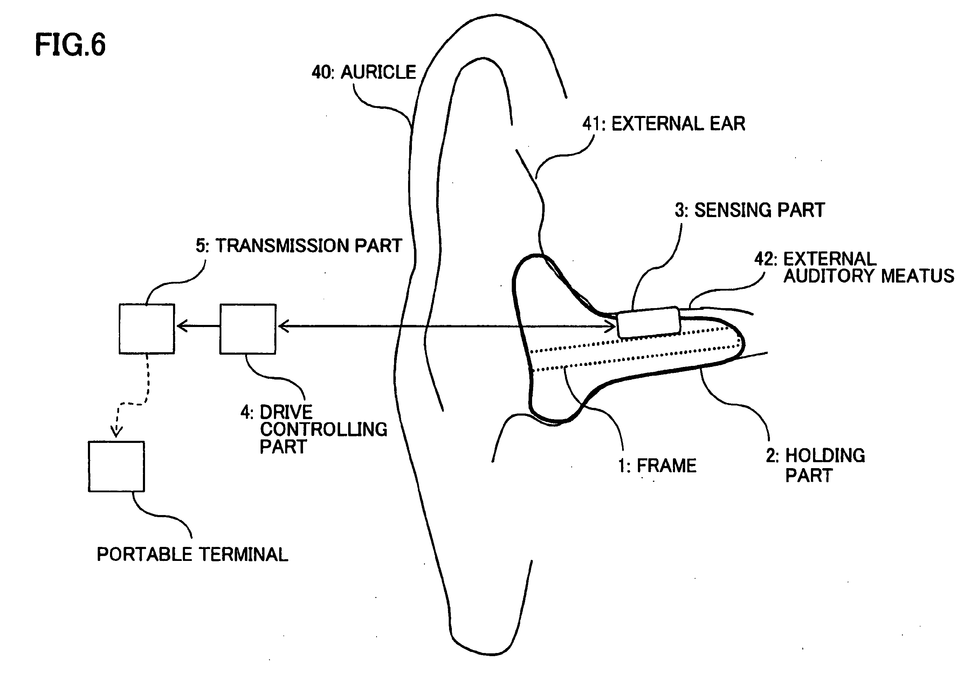

In the following, the embodiment 1-3 of the present invention is described with reference to Fig.6. Fig.6 shows a configuration of this embodiment of the living body information collecting apparatus. As shown in Fig.6, the living body information collecting apparatus of this embodiment includes a hollow frame 1, a holding part 2 for holding the hollow frame 1 to the external auditory meatus, a sensing part 3 attached to the hollow frame 1, a drive controlling part 4 for performing drive control for the sensing part 3 and processing a signal from the sensing part, and a transmission part 5 for transmitting information processed by the drive controlling part. Configurations and operations of the hollow frame 1, the holding part 2, the sensing part 3, and the drive controlling part 4 are the same as those of before-mentioned embodiments, and the sensing part 3 and the drive controlling part 4 are connected via a signal line and the drive controlling part 4 and the transmission part 5 are connected via a signal line.

Operation of the living body information collecting apparatus of this embodiment is described. A power source circuit is connected for providing power source to the sensing part 3, the drive controlling part 4 and the transmission part 5. When the transmission part 5 transmits living body information measured by the sensing part 3 by a wireless signal, optical signal or via the signal line, a portable terminal, for example, having a function for receiving the transmitted signal is provided in the outside. A drive signal is sent to the sensing part 3 via the drive controlling part 4, so that the sensing part 3 measures living body information and sends a measurement result to the drive controlling part 4. The drive controlling part 4 processes the signal of the measurement result sent from the sensing part 3, and sends the process result to the transmission part 5. The transmission part 5 transmits the process result obtained by processing the measurement result of the living body information to the portable terminal by a wireless signal or an optical signal or via a signal line.

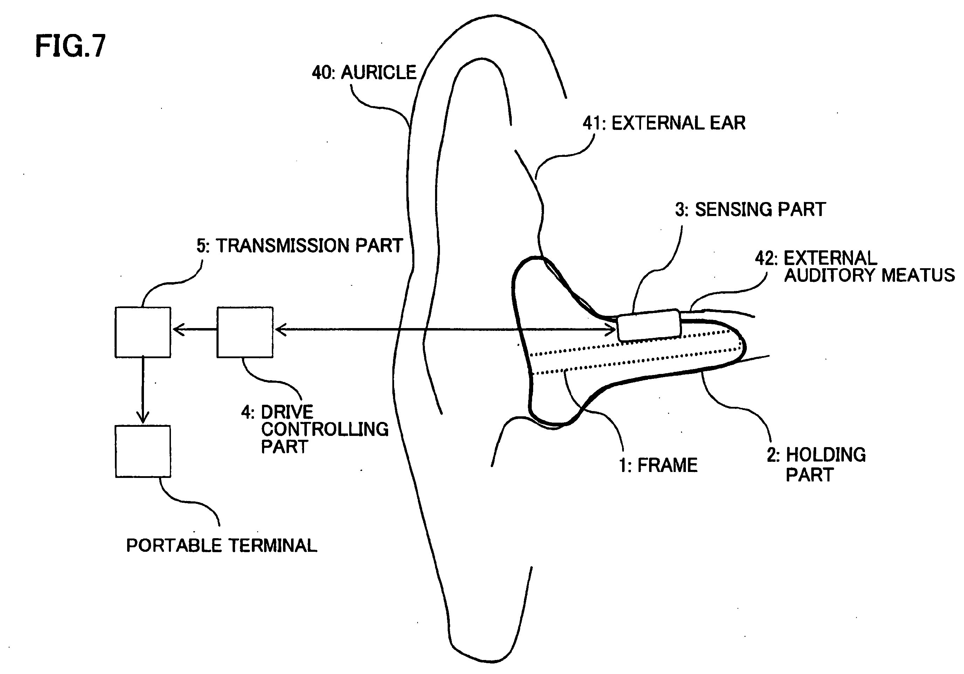

Fig.6 shows a case in which the transmission part 5 and the portable terminal transmit the wireless signal, and Fig.7 shows a case in which the transmission part 5 and the portable terminal are connected via a signal line. Although the drive controlling part 4 and the transmission part 5 are shown in the outside of the holding part 2 in Figs.6 and 7, this configuration is only for the sake of convenience of explanation for the configuration and operation of the living body information collecting apparatus. The drive controlling part 4 and the transmission part 5 ca be downsized very much using a LSI, and can be installed in the holding part 2. By transmitting the living body information to the portable terminal provided in the outside, the living body information can be displayed, for example.

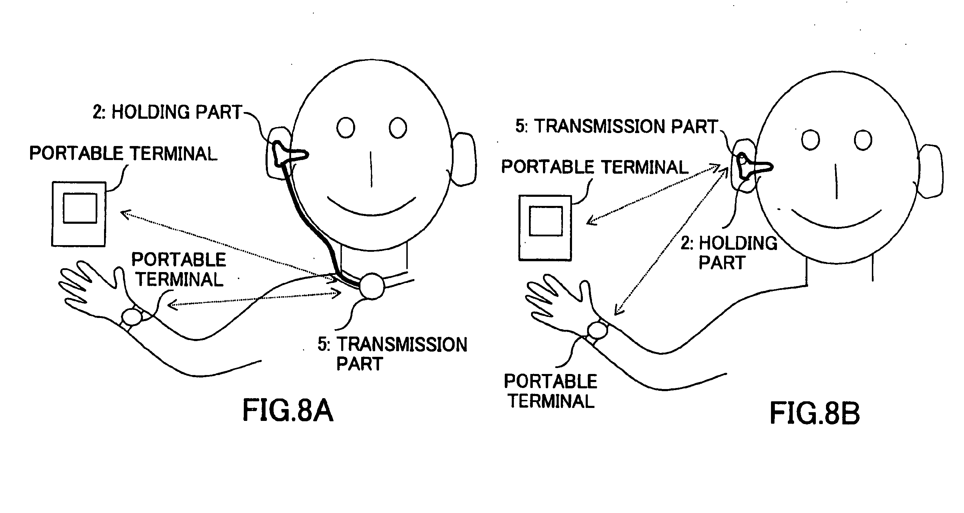

Fig.8 shows an example of a state for wearing the living body information collecting apparatus of this embodiment to a living body. Fig.8A shows a case where the transmission part 5 is not installed in the holding part 5, and is worn on the neck like a necklace. Fig.8B shows a case where the transmission part 5 is installed in the holding part 2. In Figs.8A and 8B, both of PDA type and wristwatch type are shown as the portable terminal, any one of them can be used as the portable terminal. By wearing the transmission part 5 on the neck, load for the holding part can be decreased so that wearing feeling of the living body information collecting apparatus can be improved. When the transmission part 5 can be downsized, the number of connection lines can be decreased by integrating the transmission part 5 with the holding part.

### [Embodiment 1-4]

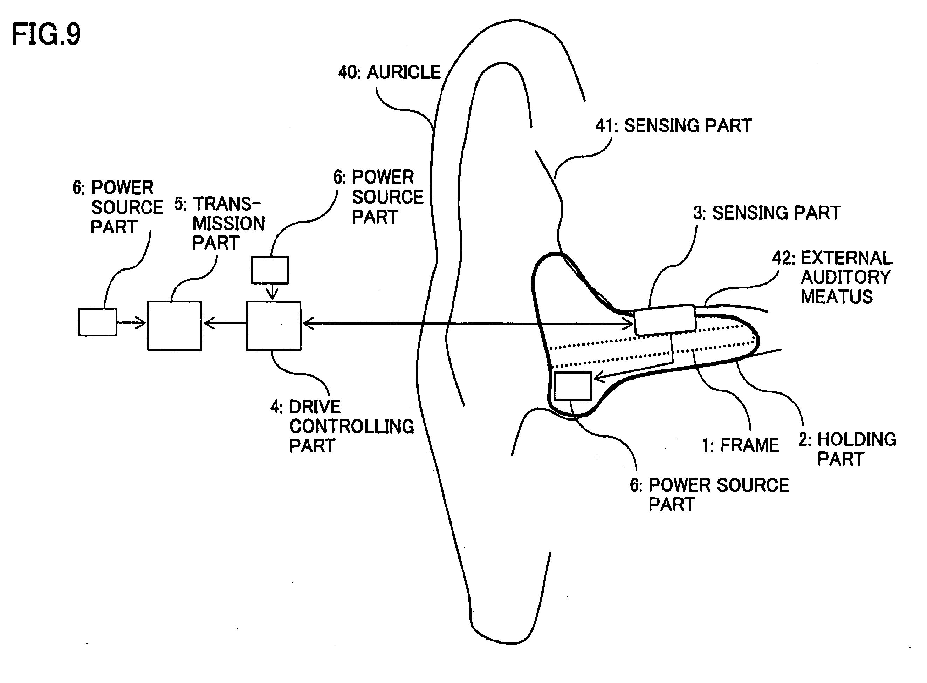

In the following, the embodiment 1-4 of the present invention is described with reference to Fig.9. This embodiment includes the following three cases.

In the first case, the power source part 6 is further provided in the sensing part 3 of the living body information collecting apparatus of the embodiment shown in Fig.1. In the second case, the power source part 6 is further provided in the sensing part 3 or the drive controlling part 4 of the living body information collecting apparatus of the embodiment shown in Fig.5, and the sensing part 3 and the drive controlling part 4 are connected by a signal line and a power source line. In the third case, the power source part 6 is further provided in any one of the sensing part 3, the drive controlling part 4 and the transmission part 5 of the living body information collecting apparatus of the embodiment shown in Fig.5, and the sensing part 3 and the drive controlling part 4 are connected by a signal line and the transmission part 5 and the power source part 6 are connected by a power source line. Since these cases are similar, the third case that represents these cases is described with reference to Fig.9.

Fig.9 shows a configuration of the living body information collecting apparatus of this embodiment. As shown in Fig.9, the living body information collecting apparatus includes a hollow frame 1, a holding part 2 for holding the hollow frame 1 to the external auditory meatus, a sensing part 3 attached to the hollow frame 1, a drive controlling part 4 for performing drive control for the sensing part 3 and processing a signal from the sensing part, a transmission part 5 for transmitting information processed by the drive controlling part, and a power source part 9 for providing power to at least one of the sensing part 3, the drive controlling part 4 and the transmission part 5.

In Fig.9, the power source part 6 is connected to each of the sensing part 3, the drive controlling part 4 and the transmission part 5. However, the power source part 6 can be connected to any one of the sensing part 3, the drive controlling part 4 and the transmission part 5. In addition, although the sensing part 3 and the drive controlling part 4 are connected via a signal line, and the drive controlling part 4 and the power source part 6 are connected via a power source line, Fig.9 shows only the signal line to avoid complexity.

Although the drive controlling part 4, the transmission part 5 and the power source part 6 are shown in the outside of the holding part 2 in Fig.9, the drive controlling part 4, the transmission part 5 and the power source part 6 can be downsized very much using a LSI, and can be installed in the holding part 2.

Operation of the living body information collecting apparatus of this embodiment is described. The operation of the living body information collecting apparatus of this embodiment is the same as that of the previously described embodiment except that the power source 6 is provided in any one of the sensing part 3, the drive controlling part 4 and the transmission part 5 to supply power to other parts, in which a power source circuit is connected to each of sensing part 3, the drive controlling part 4 and the transmission part 5 to supply power from the outside in the operation of the living body information collecting apparatus in the previous embodiment.

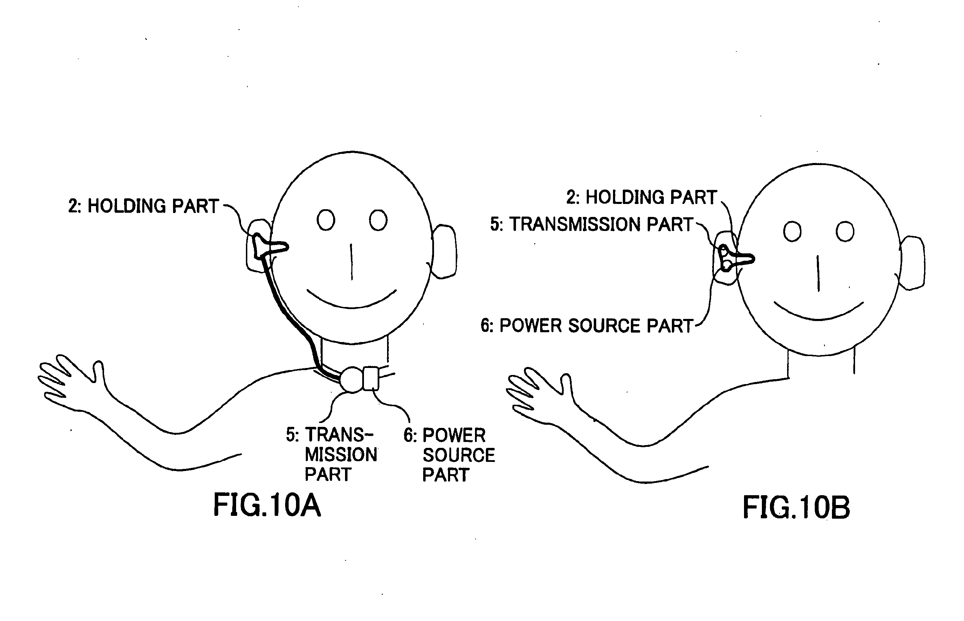

Fig.10 shows an example for wearing the living body information collecting apparatus to a living body. Fig.10A shows a case where the transmission part 5 is provided with the power source part 6, and the transmission part 5 and the power source part 6 are worn on the neck like a necklace. Fig.10B shows a case where the transmission part 5 and the power source part 6 are installed in the holding part 2. It is desirable that the power source part includes a battery to enable the living body information collecting apparatus to be portable.

As mentioned above, the living body information collecting apparatus can be carried easily, and the living body information can be measured and collected continuously or continually.

### [Embodiment 1-5]

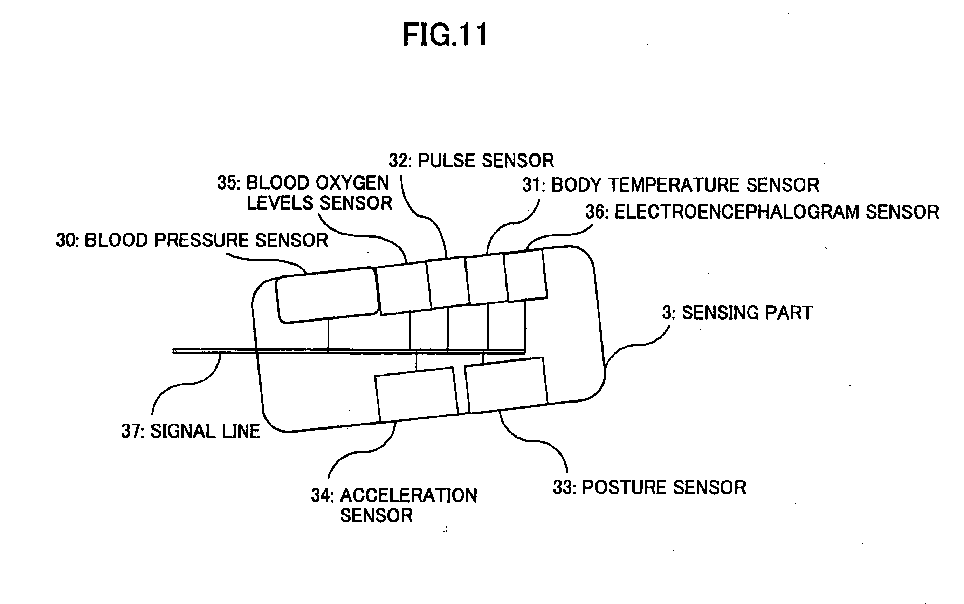

The embodiment 1-5 of the present invention is described with reference to Fig.11. Fig.11 shows the configuration of the living body information collecting apparatus of this embodiment. Fig.11 shows an enlarged view of the sensing part 3.

In Fig.11, the sensing part 3 includes at least one of a blood pressure sensor 30, a body temperature sensor 31, a pulse sensor 32, a posture sensor 33, an acceleration sensor 34, a blood oxygen levels sensor 35, and an electroencephalogram sensor 36. In addition, in Fig.11, a signal line 37 for extracting the measurement result to the outside of the sensing part 3 is connected to at least one sensor of the blood pressure sensor 30, the body temperature sensor 31, the pulse sensor 32, the posture sensor 33, the acceleration sensor 34, the blood oxygen levels sensor 35, and the electroencephalogram sensor 36 included in the sensing part 3. Fig.11 shows one line as the signal line 37. But, this is for the sake of convenience for avoiding complexity of the figure, and Fig.11 means that there may be a case where plural signal lines of plural sensors included in the sensing part 3 are included in the signal line 37.

A concrete example of the sensors of the sensing part 3 of Fig.11 is described. The blood pressure sensor 30 can be configured by a sensor for applying a pressure to the external auditory meatus 42, emitting a laser beam by a light-emitting element to a part to which the pressure is applied in the external auditory meatus 42, receiving a reflected light from the external auditory meatus 42 by a photoreceptor, measuring a pulse waveform of a blood-vessel in the external auditory meatus 42 by the reflected light, and measuring a blood pressure from the pulse waveform. The body temperature sensor 31 may be formed by a thermometer using a thermistor, for example. The pulse sensor 32 may measure the pulse based on pulsation of the external auditory meatus 42 using a vibration meter or may measure the pulse at the same time from pulsation waveform when the blood pressure sensor measures a blood pressure based on the pulsation waveform. The posture sensor 33 may be a sensor for measuring the amount of tilt in each of three axis orientations of back and forth, right and left, and up and down by attaching a weight to a spring material and by measuring amount of movement in each of three axis orientations of back and forth, right and left, and up and down by gravity. The blood oxygen levels sensor 35 may be configured by a sensor that emmits laser beams of two wavelengths of 850nm and 1200nm to the external auditory meatus 42, measures each of reflected light amounts to obtain blood oxygen levels using difference of absorption amounts of laser beams due to hemoglobin in the blood between the two wavelengths. The electroencephalogram sensor 36 may be configured by a sensor that detects change of potential of external auditory meatus 42, or detects change of an electric field.

The blood pressure sensor 30, the body temperature sensor 31, the pulse sensor 32, the posture sensor 33, the acceleration sensor 34, the blood oxygen levels sensor 35, and the electroencephalogram sensor 36 can be downsized using micromachine technology and LSI technology, so that these can be placed in the sensing part 3. The sensing part 3 may install at least one of the various sensors or may install plural sensors.

Operation of the living body information collecting apparatus of this embodiment is the same as that of the before-mentioned living body information collecting apparatus. As mentioned above, the living body information collecting apparatus of this embodiment can measure and collect various living body information.

### [Embodiment 1-6]

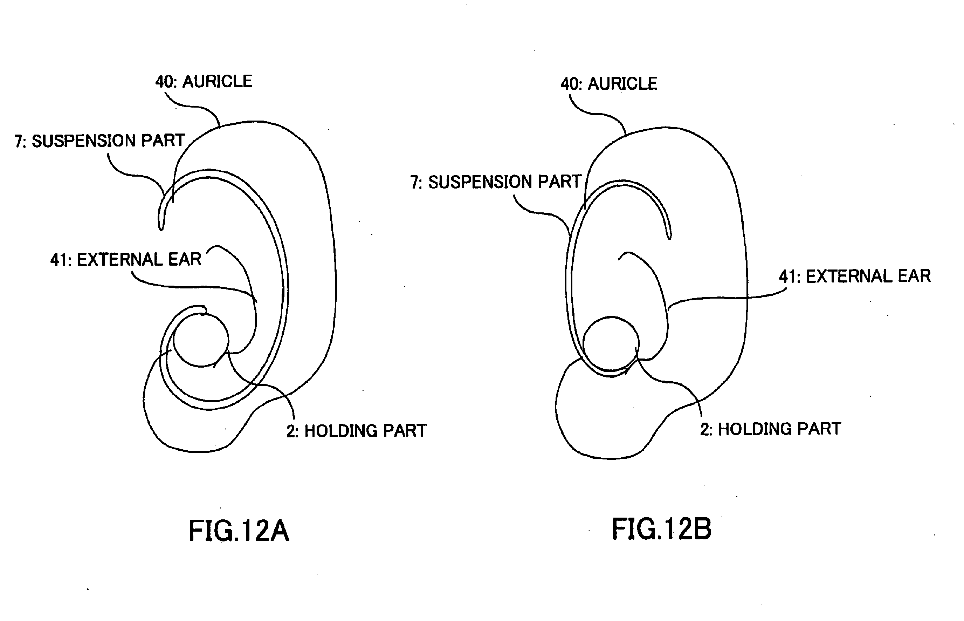

In the following, the embodiment 1-6 of the present invention is described with reference to Fig.12. Fig.12 shows a configuration of the living body information collecting apparatus of the this embodiment. The living body information collecting apparatus of this embodiment further includes a suspension part 7 for suspending the holding part 2 from the external ear 40 with respect to the living body information collecting apparatus described in the embodiments 1-1 - 1-5. This embodiment can be applied similarly to each living body information collecting apparatus, a common example shown in Fig.12 is described.

In Fig.12, the holding part 2 is suspended from the auricle 40 by the suspension part 7. In addition, in Fig.12, the auricle 40 is drawn as a transparent image for clearly showing the shape of the suspension part 7. The shape of the suspension part 7 may be one that surrounds the auricle 40 to the occipital side as shown in Fig.12A. Alternatively, the shape may be one that surrounds the auricle 40 to the face side as shown in Fig.12B, or may be a circle-like shape or a linear shape.

Operation of the living body information collecting apparatus of this embodiment is the same as the living body information collecting apparatuses described in the before-mentioned embodiments 1-1 - 1-5. Since the living body information collecting apparatus of this embodiment is stably fixed to the auricle 40, weight load to the holding part can be decreased.

### [Embodiment 1-7]

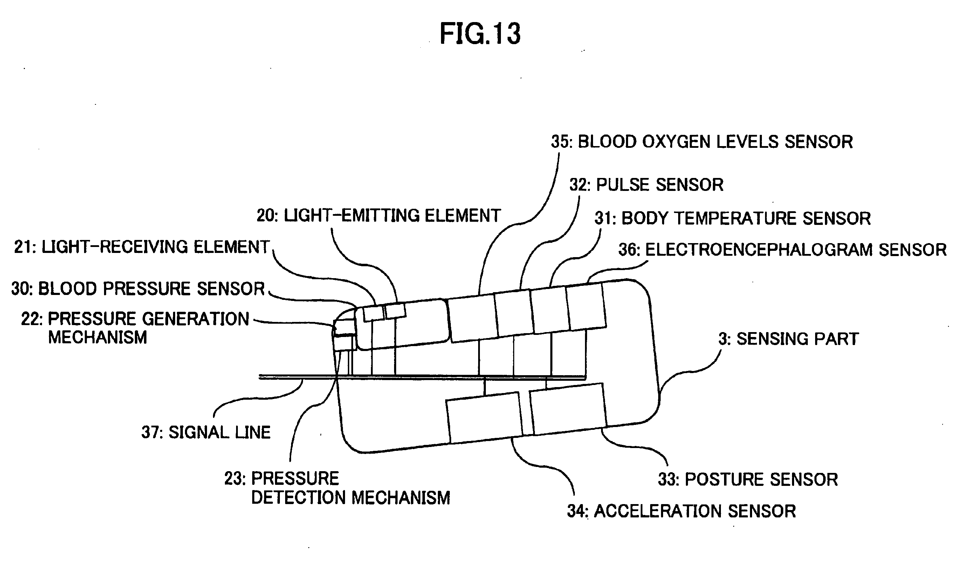

Fig.13 is a figure showing a configuration of the sensing part 3 in the embodiment 1-7. As shown in the figure, in the embodiment 1-7, the blood pressure sensor 30 includes at least a pair of a light-emitting element 20 and a light-receiving element 21, a pressure generation mechanism 22 and a pressure detection mechanism 23 to measure a blood pressure using these elements. Before describing the blood-pressure meter of the embodiment 1-7, principles 1 and 2 for measuring a blood pressure used here are described.

### [Principle 1 of blood pressure measurement]

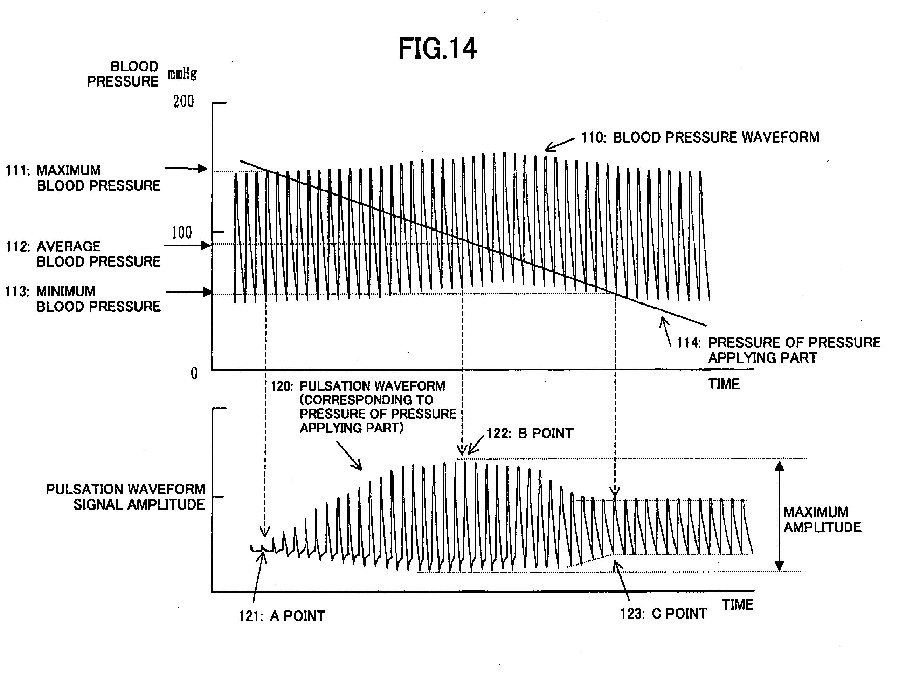

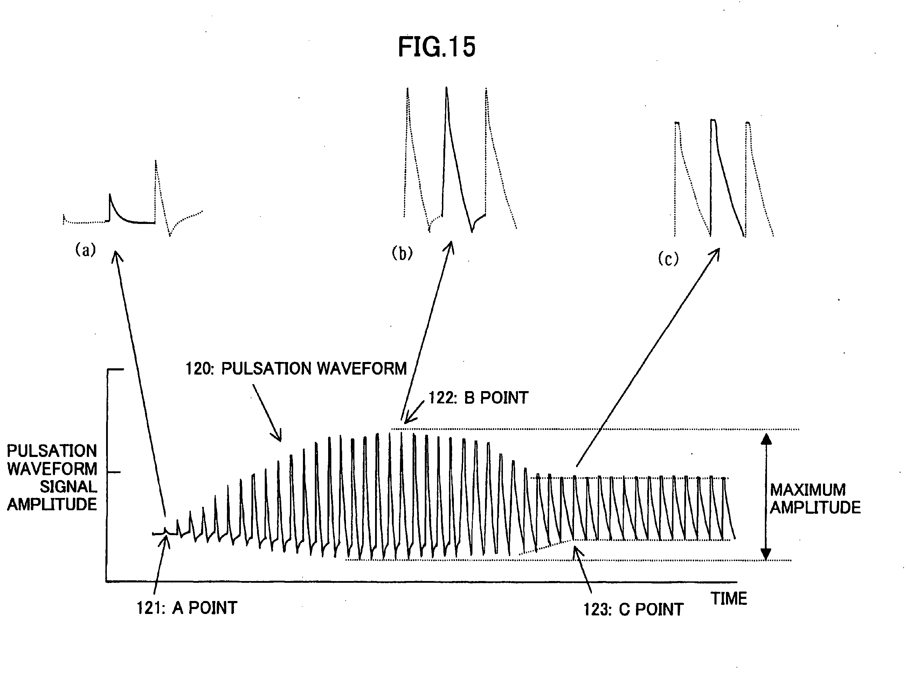

First, the principle 1 for measuring the blood pressure is described with reference to Figs.14 and 15.

Fig.14 shows relationship among a blood pressure waveform 110, a pressure 114 of a pressure applying part when applying a pressure to a part of a human body, and a pulsation waveform 120 at the pressure applying part.

As shown in the blood pressure waveform 110, the blood pressure changes like gentle undulation in whole while showing a sawtooth waveform due to heart action. This blood pressure waveform 110 is shown for the sake of explanation of the principle of blood pressure measurement, and can be measured by a precision blood pressure measuring device inserted into a blood vessel. But, this blood pressure waveform 110 is not one measured by a conventional blood pressure measuring device that performs measurement from the outside of the human body.

First, when the pressure of the pressure applying part is gradually decreased from a state in which blood flow is stopped by applying adequately high pressure to the part of the human body, the pressure decreases as time passes.

The pulsation waveform 120 shown in Fig.14 is a pulsation waveform of a blood vessel at the part of the human body measured in the above-mentioned pressure decreasing step. When the pressure 114 of the pressure applying part is adequately high, the blood flow stops so that the pulsation waveform 120 of the blood vessel scarcely appears. But, as the pressure 114 of the pressure applying part decreases, a small triangle-like pulsation waveform appears. A time point when the pulsation waveform 120 of the blood vessel appears is shown as A point 121 in Fig.14. Further, as the pressure 114 of the pressure applying part decreases, the amplitude of the pulsation waveform 120 increases so that it becomes the maximum value at B point 122. As the pressure 114 of the pressure applying part further decreases, after the amplitude of the pulsation waveform 120 gradually decreases, the top part of the pulsation waveform 120 becomes constant to show flat state. After the top part of the pulsation waveform 120 becomes the constant value, the bottom part of the pulsation waveform 120 also changes to a constant value from a decreasing state. A time point when the value of the bottom part of the pulsation waveform 120 changes to the constant value is shown as C point 123. In addition, the maximum blood pressure 111, the average blood pressure 112 and the minimum blood pressure 113 that are explained next are shown in Fig.14. In the step of decrease of the pressure 114 of the pressure applying part, a value of the pressure 114 of the pressure applying part corresponding to the A point 121 that is the change point appearing in the pulsation waveform 120 is the maximum blood pressure 111, the value of the pressure 114 of the pressure applying part corresponding to B point 122 is the average blood pressure 112, and the value of the pressure 114 of the pressure applying part corresponding to the C point 123 is the minimum blood pressure 113.

Fig.15 is one showing only the pulsation waveform 120 of Fig.14 again for explaining the feature of the pulsation waveform 120. (a), (b) and (c) in Fig.15 are enlarged views of the pulsation waveform 120 of the A point 121, B point 122 and C point 123 respectively. More specifically, each of (a), (b) and (c) in Fig.15 shows, by a solid line, a period of pulse-like waveform forming the pulsation waveform corresponding to one of the A point 121, B point 122 and C point 123 of Fig.14, and shows an adjacent pulse-like waveform by a dotted line.

When viewing each of the pulse-like waveforms forming the pulsation waveform 120, near the A point 121 corresponding to the maximum blood pressure, the greater part is flat and there is a small triangle-like pulse having a small amplitude as the pulse-like waveform indicated as (a). As the time becomes closer to the B point 122 corresponding to the average blood presser, the top part of the triangle becomes sharp and the flat part decreases. At the B point 122, as shown in (b), time occupations of the flat part and the triangle are approximately the same, and the pulse-like waveform can be said to be a shape obtained by cutting out lower half part of a triangular wave that vibrates up and down. Further, as the time becomes closer to the C point 123 corresponding to the minimum blood pressure 113, the pulse-like waveform forming the pulsation waveform 120 is resembling a triangular wave in shape, and at the C point 123, the rising part of the pulse-like waveform comes close to vertical and the falling part becomes gentle as shown in (c). Accordingly, each of the pulse-like waveforms forming the pulsation waveform 120 shows a shape having a very remarkable feature within the range from the A point 121 corresponding to the maximum blood pressure to the C point 123 corresponding to the minimum blood pressure.

It is known that, when the blood pressure changes, only the amplitude of the pulsation waveform 120 changes but the shape does not change. That is, in Fig.14, when the blood pressure as a whole changes to higher blood pressure side so that the blood pressure waveform 110 moves to higher side as a whole, the amplitude of the pulsation waveform 120 increases. On the other hand, when the blood pressure as a whole changes to lower blood pressure side so that the blood pressure waveform 110 moves to lower side as a whole, the amplitude of the pulsation waveform 120 decreases. However, the shape of the waveform is kept similar. Therefore, by comparing a waveform of one period of the pulse-like waveform forming the pulsation waveform measured at an arbitrary time point with each pulse-like waveform forming the pulsation waveform 120 shown in Fig.15, it can be determined which level the measured waveform corresponds to between the maximum blood pressure and the minimum blood pressure.

Blood pressure measurement when decreasing the pressure is described as mentioned above with reference to Figs.14 and 15. By the way, change of the pulsation waveform for the pressure when gradually increasing the pressure can be also explained based on the same principle, and blood pressure measurement can be performed in the same way. This can be applied to all embodiments of the specification of this application.

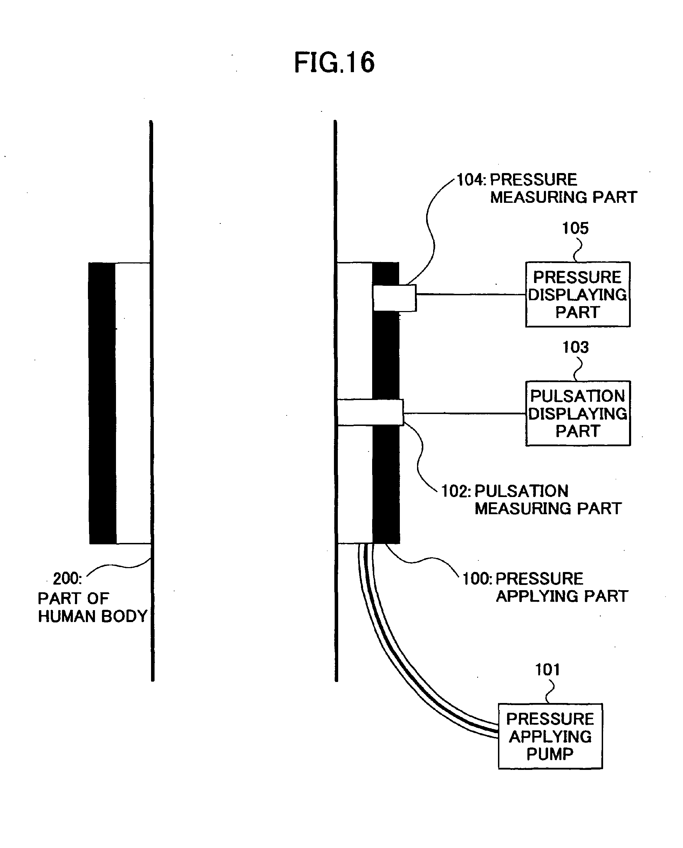

In the following, for reference purposes, a conventional blood pressure measurement method using a blood pressure measurement apparatus described in the non-patent document 2 shown in Fig.16 is described. This blood pressure measurement apparatus is configured by a pressure applying part 100, a pressure applying pump 101, a pulsation measuring part 102 for measuring the pulsation waveform of a blood vessel, a pulsation displaying part 103 for displaying the pulsation waveform of a blood vessel, a pressure measuring part 104, and a pressure displaying part 105. In Fig.16, the pressure applying part 100 attached to a part 200 of the human body applies pressure to the part 200 of the human body using a pressure supplied from the pressure applying pump 101. The pressure measuring part 104 measures the pressure applied to the part 200 of the human body by the pressure applying part 100, and the value of the pressure is displayed on the pressure displaying part 105. The pulsation measuring part 102 measures the pulsation waveform of the blood vessel of the part 200 of the human body that is pressurized, and displays the pulsation waveform on the pulsation displaying part 103.

In the conventional technology, the size of the pulsation waveform 120 that changes in a step to gradually decrease the pressure 114 of the pressure applying part from a pressure adequately high for stopping the blood flow, that is, an amount corresponding to pulsation waveform signal amplitude of the pulsation waveform 120 is determined as loudness of sound by hearing with an ear using a stethoscope. Or the pulsation waveform signal amplitude of the pulsation waveform 120 is measured by electronically detecting it and displaying it. By these methods and the like, the A point 121 corresponding to the maximum blood pressure 111 and the C point 123 corresponding to the minimum blood pressure 113 are determined, and by measuring the pressure applied to the part of the human body at the time points, and the maximum blood pressure 111 and the minimum blood pressure 113 are measured.

### [Principle 2 of blood pressure measurement]

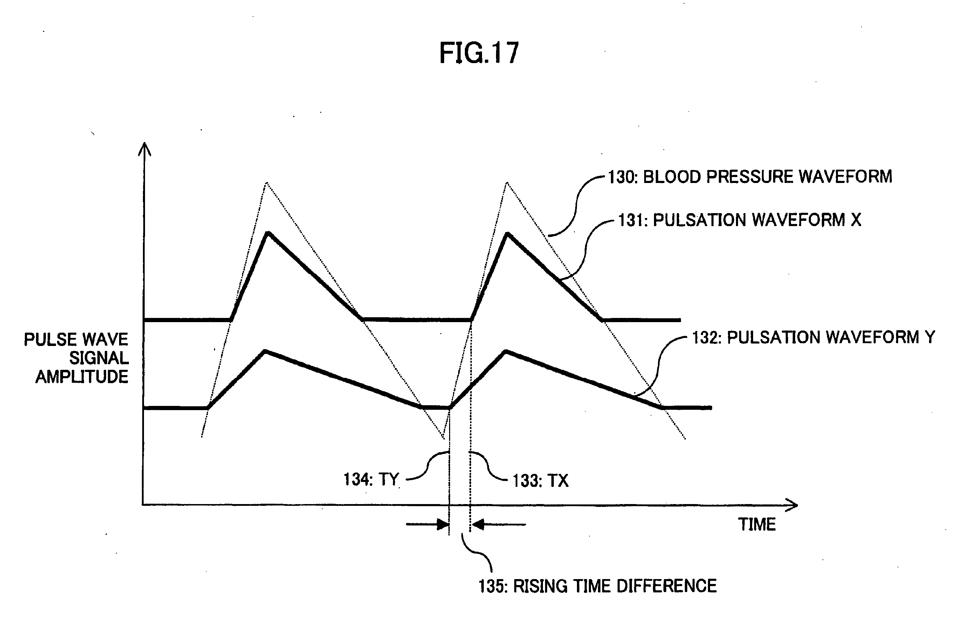

Next, the principle 2 of the blood pressure measurement is described with reference to Fig.17.

Fig.17 is a figure showing change of the pulsation waveform when applying different pressures respectively to a part and another part of the human body. In Fig.17, the pulsation waveform X 131 shows a waveform of a part pressurized by a relatively high pressure, and a pulsation waveform Y 132 shows a waveform of another part pressurized by a relatively low pressure. The blood pressure changes as shown as a blood pressure waveform 130. A time point TX 133 shows a time point when the waveform of the pulsation waveform X 131 rises, the time point TY 134 shows a time point when the waveform of the pulsation waveform Y 132 rises, and the rising time difference 135 shows a difference between the time point TX 133 and a time point TY 134.

As shown in Fig.17, the pulsation when the pressure of the pressure applying part is high forms a triangle having a short base, and the pulsation when the pressure of the pressure applying part is low becomes a triangle having a long base. In addition, the time point at which the pulsation waveform rises when the pressure of the pressure applying part is high delays with respect to the time point at which the pulsation waveform rises when the pressure of the pressure applying part is low. There is correspondence relationship between a difference between the rising time points, that is, the rising time difference 135 and a difference between the pressure of the pressure applying part at the time when the pulsation waveform X 131 is measured and the pressure of the pressure applying part when the pulsation waveform Y 132 is measured. Therefore, for example, by measuring the pressure of the pressure applying part at the time when the pulsation waveform X 131 is measured and the rising time difference 135, the pressure of the pressure applying part at the time when the pulsation waveform Y 132 is measured, that is, the blood pressure at the time can be measured. By measuring a pulsation waveform at a referred part of the human body based on the above-principle, another part of the human body can be measured.

That is, with respect to the pulsation waveform at the part of the human body when a predetermined pressure is applied to the part of the human body, each rising time difference of the pulsation waveform when various pressures (plural pressures from the maximum blood pressure level to the minimum blood pressure level shown in Fig.14, for example) are applied at another part of the human body is held by associating the time difference with the pressure (or relative blood pressure level assuming that the maximum blood pressure is 100 and the minimum blood pressure is 0) applied to the another part of the human body. Such data are held for various references. Accordingly, by measuring the pulsation waveform at the referred part of the human body, a blood pressure level of the blood pressure of the another part of the human body can be measured from the pulsation waveform of the another part of the human body.

### [Explanation of embodiment 1-7]

In the following, the embodiment 1-7 of the present invention is described with reference to Fig.13. In Fig.13, when the sensing part 3 of the living body information collecting apparatus is the blood pressure sensor 30 in this embodiment, the blood pressure sensor 30 includes at least a pair of a light-emitting element 20 and a light-receiving element 21, a pressure generation mechanism 22 and a pressure detection mechanism 23.

Although Fig.13 shows the blood pressure sensor 30, the body temperature sensor 31, the pulse sensor 32, the posture sensor 33, the acceleration sensor 34, the blood oxygen levels sensor 35, and the electroencephalogram sensor 36 that are placed in the sensing part 3 of the living body information collecting apparatus of this embodiment, all of these sensors are not necessarily placed as mentioned before.

In a configuration example of the blood pressure sensor 30 that may be placed in the sensing part 3 of the living body information collecting apparatus shown in Fig.13, the blood pressure sensor 30 includes a pressure applying function for applying a pressure on the external auditory meatus 42, and the light-emitting element 20 and the light-receiving element 21 are placed at the external auditory meatus 42 side of the part on which the pressure is applied. The light-emitting element 20 and the light-receiving element 21 are placed adjacent to each other such that, each of the light-emitting surface of the light-emitting element 20 and the light-receiving surface of the light receiving element 21 is directed to the external auditory meatus 42 side, so that, when the light-emitting element 20 emits a laser beam and the like and the emitted light is reflected by the external auditory meatus 42, the reflected light is received by the light-receiving element 21.

Fig.13 shows an example where one pair of the light-emitting element 20 and the light-receiving element 21 are placed. Also when more than one pairs of light-emitting element and light-receiving element are placed, they are placed on the external auditory meatus 42 side in the part on which a pressure is applied by the blood pressure sensor 30 while position relationship similar to that of the light-emitting element 20 and the light-receiving element 21 is kept. The pressure generation mechanism 22 and the pressure detection mechanism 23 are placed in the outside of the pressure applying part, and each of the pressure detection mechanism 22 and the detection mechanism 23 are connected to the outside of the holding part 2 via a signal line. When the pressure generation mechanism 22 receives an instruction signal via the signal line, the pressure generation mechanism 22 generates an instructed pressure and supplies the pressure to a pressure applying part of the blood pressure sensor 30. The pressure detection mechanism 23 has a function for measuring the pressure generated by the pressure generation mechanism 22 and sending the result via the signal line.

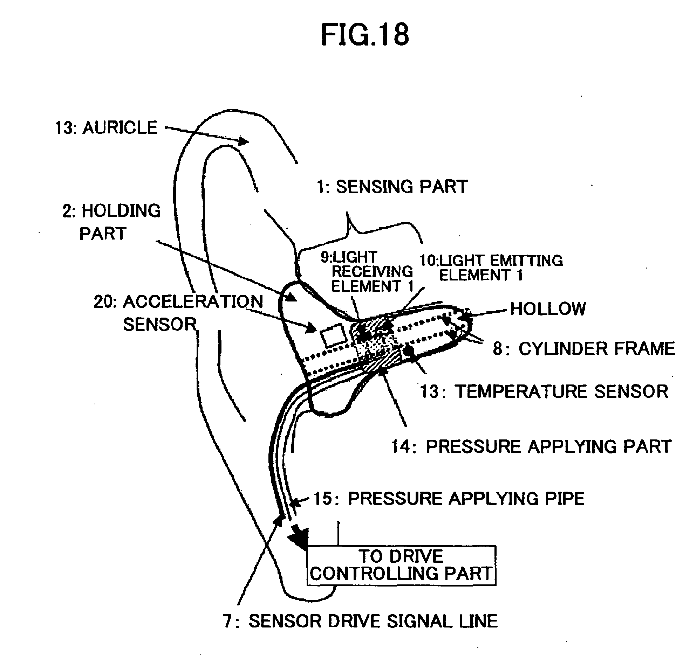

Fig.18 shows another structure example of the living body information collecting apparatus including the blood pressure sensor. This living body information collecting apparatus includes a hollow cylinder frame 8 having a holding part 2 at the back part, and a sensing part 1 having a pressure applying part 14 and light receiving and emitting parts 9 and 10 in the frame part that touches the meatal.

In the pressure applying part 14, a concave part formed like a concentric circle with respect to the frame axis around the frame 8 and an air receiver composed of elastic member placed at the concave part are formed. When air is supplied and released via the pressure applying pipe, the elastic member is displaced to the outside in the diameter direction of the frame so that the member evenly pressurizes the meatal wall. For the pressure applying part, a structure of covering the opening of the concave part formed in the periphery part of the frame with the elastic member, or a structure of fixing a doughnut-like air belt at the concave part can be adopted. In addition, the pressure applying part can be realized without using such air system by placing a micro actuator such as piezo-actuator, shape memory alloy and the like in the concave part. In addition, as the actuator, a mechanical one using oil pressure or water pressure can be used.

In addition, the shape of the frame 8 is not limited to the hollow cylinder shape. It is adequate that the frame can be inserted into the meatal (column, cone, pyramid, prism, truncated cone, truncated pyramid and the like, for example). In addition, the direction in which the pressure applying part expands is not necessarily concentric and all-around. The blood pressure can be measured if the pressure applying part expands to at least one direction to the outside from the center.

Operation when a pair of the light-emitting element 20 and the light-receiving element 21 is placed in the living body information collecting apparatus of this embodiment shown in Fig.13 is described. The operation also applies to the structure shown in Fig.18. The signal lines of Fig.13 are connected to a driving circuit of the light-emitting element 20, a signal processing circuit for processing the receiving signal of the receiving element 21 and for displaying the waveform, a control circuit of the pressure generation mechanism 22, a display circuit of the measurement result of the pressure detection mechanism 23. By the way, the driving circuit, the signal processing circuit and the control circuit can be included in the drive control part 4 shown in Fig.5 and the like.

The control circuit controls the pressure generation mechanism 22 to cause it to generate an arbitrary pressure so that the pressure applying part of the blood pressure sensor 30 applies a pressure. The pressure detection mechanism 23 measures the pressure generated by the pressure generation mechanism 22, sends the result to the display circuit, and the display circuit displays the measurement value of the pressure. The driving circuit drives the light-emitting element 20. The light-emitting element 20 emits a laser beam and the like to the external auditory meatus 42, and the light-receiving element 21 receives reflected light reflected from the external auditory meatus 42.

The amount or frequency of the reflected light reflected from blood vessel on the surface or in the inside of the external auditory meatus 42 changes due to pulsation of the blood vessel on the surface or in the inside of the external auditory meatus 42. The light-receiving element 21 converts the change of the received reflected light into an electrical signal, and sends the signal to the signal processing circuit via the signal line. The signal processing circuit measures the pulsation waveform of the external auditory meatus 42 based on the change of the received reflected light, and displays the pulsation waveform.

From the principle 1 of the blood pressure measurement, it can be determined which level the displayed pulsation waveform corresponds to between the maximum blood pressure and the minimum blood pressure, and a pressure measured by the pressure detection mechanism 23 at the time and displayed by the display circuit is the blood pressure corresponding to the level. In addition, the signal processing circuit may store relationship between reference pulsation waveform and blood pressure level so that the blood pressure level can be displayed by comparing measured pulsation waveform and reference waveform. Further, by changing the pressure generated by the pressure generation mechanism 22 by the control circuit, blood pressure of arbitrary level between the maximum blood pressure and the minimum blood pressure can be measured. In addition, by using the principle 2 of the blood pressure measurement, when two pairs of light-emitting element and light-receiving element are placed, blood pressure measurement is available using difference between rising time points of waveforms measured by each pair.

Further, when placing many light-emitting elements and light-receiving elements, by statistically processing pulsation waveforms measured by each pair of light-emitting element and light-receiving element, measurement accuracy can be improved by decreasing noise. Accordingly, the living body information collecting apparatus of the embodiment of the present invention can easily measure and collect living body information.

### [Embodiment 1-8]

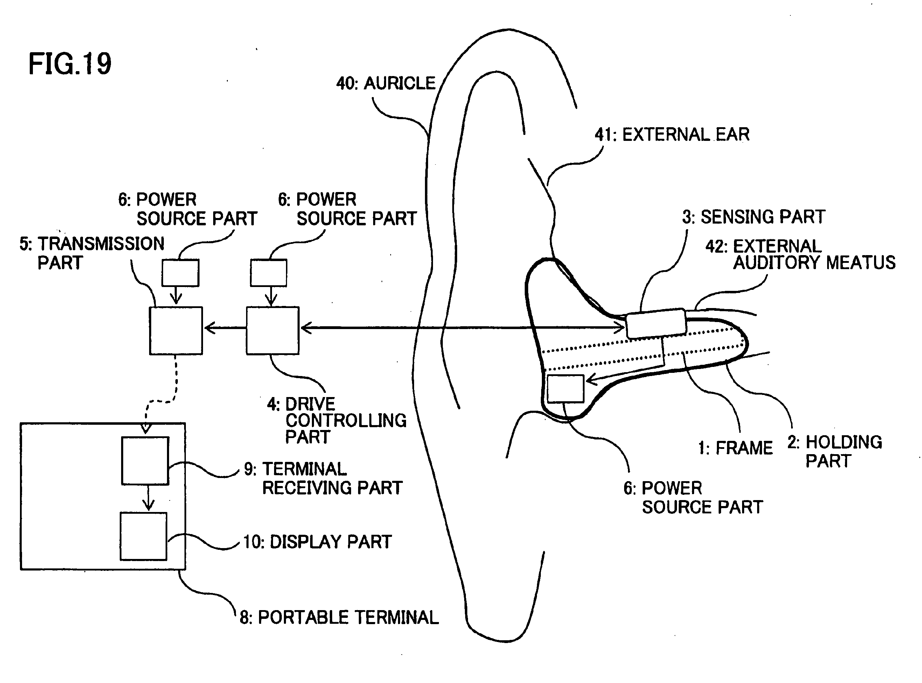

In the following, the embodiment 1-8 is described with reference to Fig.19. Fig.19 shows a configuration of the living body information collecting system of this embodiment. The living body information collecting system of this embodiment is a living body information collecting system including a portable terminal 8 and the before-mentioned living body information collecting apparatus. The portable terminal 8 includes a terminal receiving part 9 for performing receive processing on information from the transmission part 5, and a display part 10 for displaying information from the terminal receiving part 9.

In Fig.19, the living body information collecting apparatus is the same as the living body information collecting apparatus described with reference to Fig.9. Although the power source part 6 is connected to each of the sensing part 3, the drive control part 4 and the transmission part 5, this is for the sake of explanation and Fig.9 means that the power source 6 is connected to any one of the sensing part 3, the drive control part 4 and the transmission part 5 in the same way as the living body information collecting apparatus shown in Fig.9. By the way, as the living body information collecting apparatus, each apparatus for measuring living body information described in other embodiments in this specification can be applied.

In the mobile terminal 8, the terminal receiving part 9 and the display part 10 are connected via a signal line. Each of the transmission part 5 of the living body information collecting apparatus and the terminal receiving part 9 included in the mobile terminal 8 has means for performing communications using a wireless signal or an optical signal, or they are connected by a signal line.

Operation of the living body information collecting system of this embodiment is described. The living body information collecting system of this embodiment measures living body information similarly to the before-mentioned living body information collecting apparatus, and the transmission part 5 sends the measurement result using a wireless signal or an optical signal, or via a signal line to the potable terminal 8. The potable terminal 8 receives this signal by the included terminal receiving part 9, and performs processing and displays data on the display part 10.

As mentioned above, the living body information collecting system of this embodiment can display collected living body information on the portable terminal.

### [Embodiment 1-9]

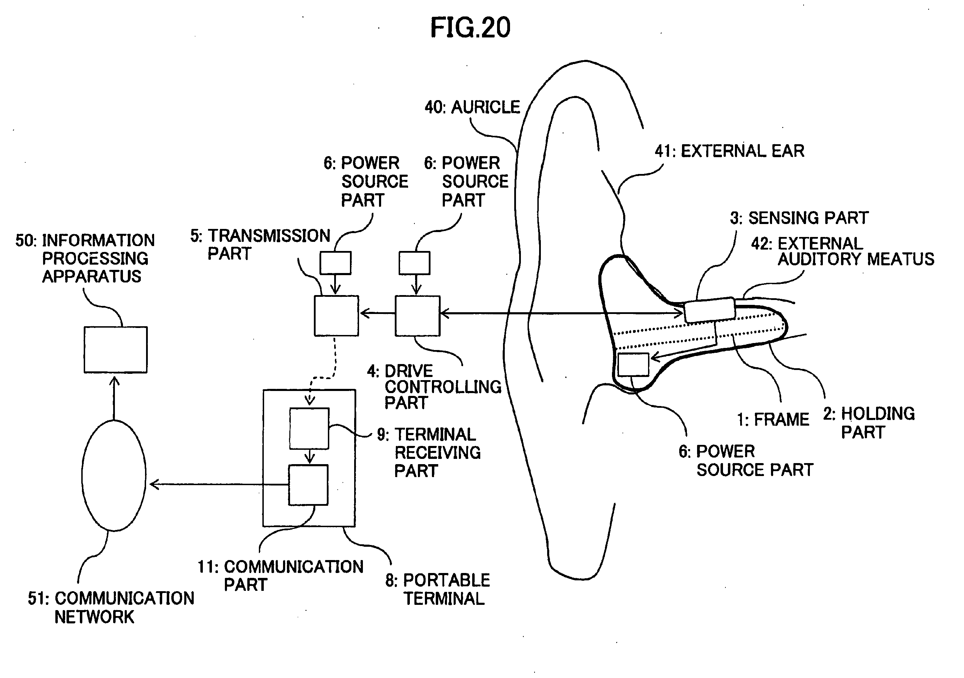

In the following, the embodiment 1-9 of the present invention is described with reference to Fig.20. Fig.20 shows a configuration of the living body information collecting system of this embodiment. The living body information collecting system of this embodiment is a living body information collecting system including a portable terminal 8 and the before-mentioned living body information collecting apparatus. The portable terminal 8 includes a terminal receiving part 9 for performing receive processing on information from the transmission part 5, and a communication part 11 for transmitting a signal from the terminal receiving part 9 to an information processing apparatus 50 via a communication network 51.

In Fig.20, although the power source part 6 is connected to each of the sensing part 3, the drive control part 4 and the transmission part 5, this is for the sake of explanation and Fig.9 means that the power source 6 is connected to any one of the sensing part 3, the drive control part 4 and the transmission part 5 in the same way as the living body information collecting apparatus shown in Fig.9. In the portable terminal 8, the terminal receiving part 9 and the communication part 11 is connected via a signal line. Each of the transmission part 5 of the living body information collecting apparatus and the terminal receiving part 9 included in the mobile terminal 8, and each of communication part 11 in the portable terminal 8 and the communication network 51 has means for performing communication using a wireless signal or an optical signal, or they are connected by a signal line.

The information processing apparatus 50 is connected to the communication network 51. The communication network 51 may be a relatively small-scale communication network in a clinic, or may be a large-scale communication network such as the Internet. Further, the information processing apparatus 50 may be a small-scale personal computer or may be a large-scale information processing apparatus. The information processing apparatus 50 includes a function for collecting living body information.

Operation of the living body information collecting system of this embodiment is described. The living body information collecting system of this embodiment measures living body information in the same way as the before-mentioned living body information collecting apparatus. The transmission part 5 sends the measurement result to the portable terminal 8 via a wireless signal or an optical signal or via a signal line. The portable terminal 8 performs receive processing for the information using the included terminal receiving part 9, and sends the information to the information processing apparatus via the communication network 51 by the communication part 11, so that the information processing apparatus 50 can collect the receiving living body information. As described above, the living body information collecting system of this embodiment can send collected living body information to a remote information processing apparatus.

As mentioned above, by sending the measurement result of the living body information to the remote information processing apparatus via the communication network so as to collect the living body information, the storing apparatus of the portable terminal can be downsized so that customer convenience improves. Further, for example, it becomes possible for an expert to observe change of health state by collectively collecting past measurement data, and it becomes possible to perform analysis such as comparison with standard data of healthy persons.

### [Embodiment 1-10]

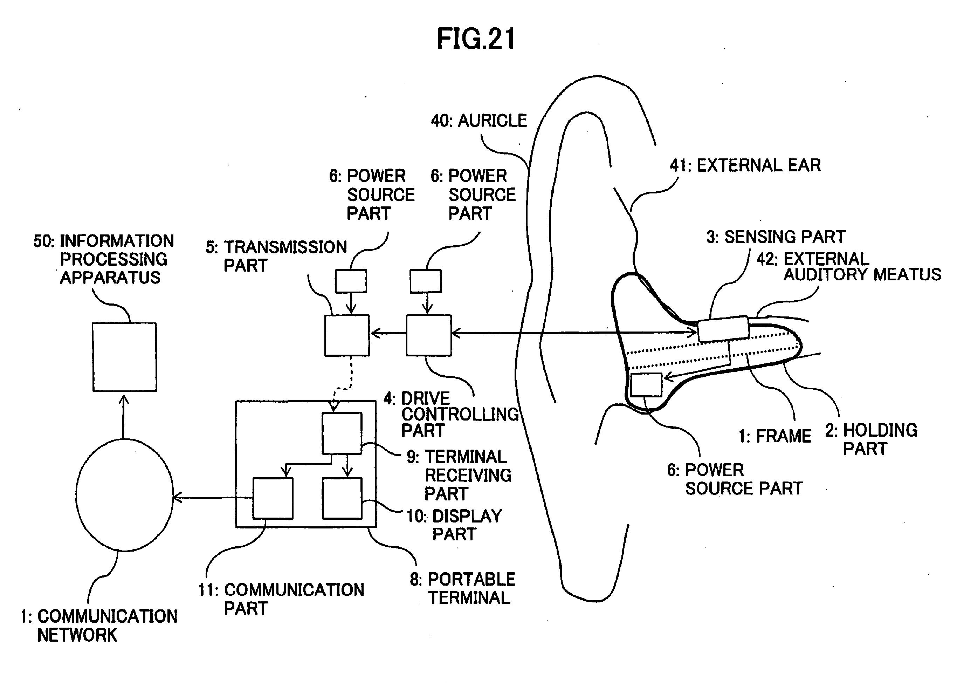

In the following, the embodiment 1-10 of the present invention is described with reference to Fig.21. Fig.21 shows a configuration of the living body information collecting system of this embodiment. The living body information collecting system of this embodiment is a living body information collecting system including a portable terminal 8 and the before-mentioned living body information collecting apparatus. The portable terminal 8 includes a terminal receiving part 9 for performing receive processing on information from the transmission part 5, a communication part 11 for transmitting a signal from the terminal receiving part 9 to an information processing apparatus 50 via a communication network 51, and a display part 10 for displaying information from the terminal receiving part 9.

In Fig.21, although the power source part 6 is connected to each of the sensing part 3, the drive control part 4 and the transmission part 5, this is for the sake of explanation and Fig.9 means that the power source 6 is connected to any one of the sensing part 3, the drive control part 4 and the transmission part 5 in the same way as the living body information collecting apparatus shown in Fig.9. In the portable terminal 8, the terminal receiving part 9 is connected to the communication part 11 and the display part 10 via signal lines. Each of the transmission part 5 of the living body information collecting apparatus and the terminal receiving part 9 included in the mobile terminal 8, and each of communication part 11 in the portable terminal 8 and the communication network 51 has means for performing communication using a wireless signal or an optical signal, or they are connected by a signal line.

The information processing apparatus 50 is connected to the communication network 51. The communication network 51 may be a relatively small-scale communication network in a clinic, or may be a large-scale communication network such as the Internet. Further, the information processing apparatus 50 may be a small-scale personal computer or may be a large-scale information processing apparatus. The information processing apparatus 50 includes a function for collecting living body information.

Operation of the living body information collecting system of this embodiment is described. The living body information collecting system of this embodiment measures living body information in the same way as the before-mentioned living body information collecting apparatus. The transmission 5 sends the measurement result to the portable terminal 8 via a wireless signal or an optical signal or via a signal line. The portable terminal 8 performs receive processing for the information using the included terminal receiving part 9, and sends the information to the information processing apparatus 50 via the communication network 51 by the communication part 11. At the same time, information from the terminal receiving part 9 is displayed on the display part 10.

As described above, the living body information collecting system of this embodiment can send collected living body information to a remote information processing apparatus, and the portable terminal can display the living body information.

As described above, by sending the measurement result of the living body information to the remote information processing apparatus via the communication network so as to collect the living body information, and at the same time, displaying the information on the portable terminal, the measurement result of the current living body information can be ascertained instantly, and if the result shows abnormal value, it can be cope with promptly, so that customer convenience further improves.

### [Embodiment 1-11]

In the following, the embodiment 1-11 of the present invention is described with reference to Fig.21. Configuration of the living body information collecting system of this embodiment is the same as the living body information collecting system shown in Fig.21.

Operation of the living body information collecting system of this embodiment is described. The living body information collecting system of this embodiment measures living body information in the same way as the before-mentioned living body information collecting apparatus. The transmission 5 sends the measurement result to the portable terminal 8 via a wireless signal or an optical signal or via a signal line. The portable terminal 8 performs receive processing for the information using the included terminal receiving part 9, and sends the information to the information processing apparatus 50 via the communication network 51 by the communication part 11. At the same time, information from the terminal receiving part 9 is displayed on the display part 10. Further the communication part 11 included in the portable terminal 8 performs receiving processing for information sent from the information processing apparatus 50 via the communication network 51. Examples of information sent from the information processing apparatus 50 are a range of healthy status of various living body information, an instruction to measure additional other living body information based on a result of analysis for the current measured value, or an instruction to further perform work-up.

As described above, the living body information collecting system of this embodiment can further receive instruction from the information processing apparatus via the communication network. As mentioned above, according to the living body information collecting system, since advanced knowledge stored in the information processing apparatus can be used by providing the function of receiving and processing information from the information processing apparatus by the portable terminal, further advanced living body information can be measured so that convenience further improves.

### [Embodiment 1-12]

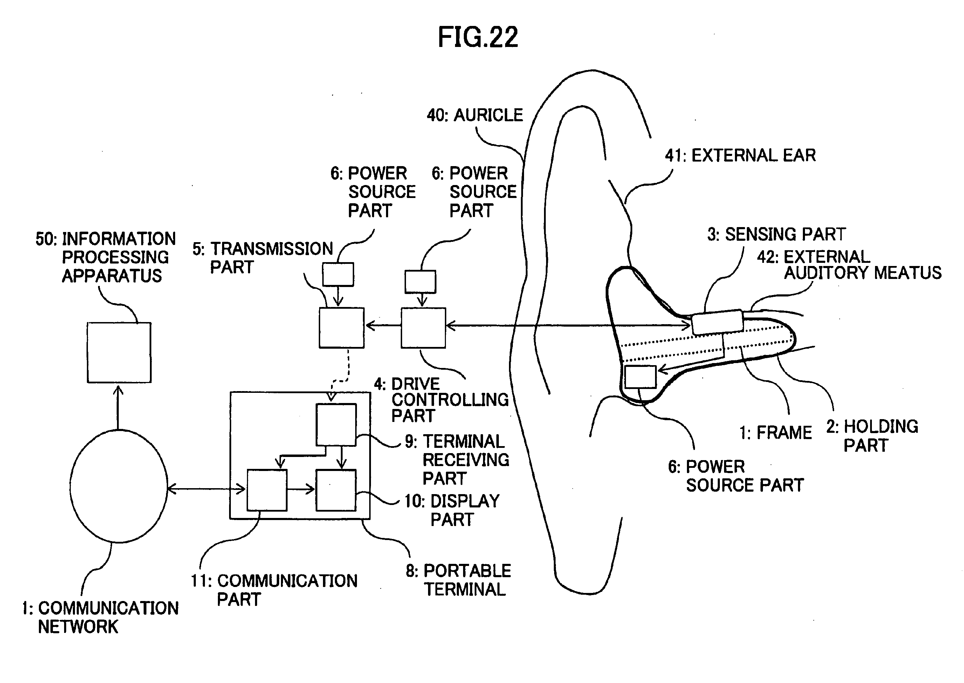

In the following, the embodiment 1-12 of the present invention is described with reference to Fig.22. Configuration of the living body information collecting system of this embodiment is the same as the living body information collecting system shown in Fig.21, and the display part 10 further includes a function for displaying information from the information processing apparatus 50.

Operation of the living body information collecting system of this embodiment is described. In the operation of the living body information, in addition to the operation of the before-described living body information collecting system, the display part 10 included in the portable terminal 8 displays information sent from the information processing apparatus 50 via the communication network 51. Examples of information to be displayed are a range of healthy status of various living body information, an instruction to measure additional other living body information based on a result of analysis for the current measured value, or an instruction to further perform work-up.

As described above, the living body information collecting system of this embodiment can display information from the information processing apparatus. As mentioned above, since the living body information collecting system includes the function for displaying information from the information processing apparatus on the portable terminal, an instruction from the information processing apparatus can be promptly ascertained and the instruction can be promptly cope with, so that convenience further improves.

### [Embodiment 1-13]

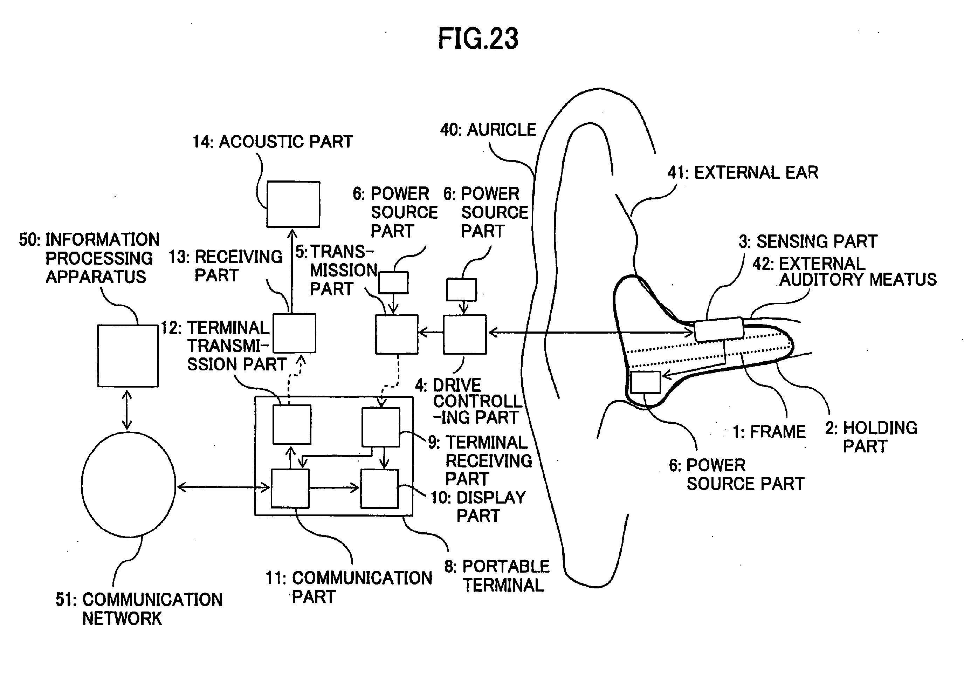

In the following, the embodiment 1-13 of the present invention is described with reference to Fig.23. Fig.23 shows configuration of the living body information collecting system of this embodiment. Compared with the before-mentioned living body information collecting system, in the living body information collecting system of this embodiment, the portable terminal 8 further includes a terminal transmission part 12 for transmitting information from the information processing apparatus 50 to the living body information collecting apparatus. The living body information collecting apparatus further includes a receiving part 13 for performing receiving processing on information from the terminal transmission part 12 and an acoustic part 14 for transmitting information received from the receiving part 13 by sound. In Fig.23, the portable terminal 8 is formed by the terminal receiving part 9, the display part 10, the communication part 11 and the terminal transmission part 12.

Each of the pair of the terminal receiving part 9 of the portable terminal 8 and the transmission part 5 of the living body information collecting apparatus, the pair of the terminal transmission part 12 of the portable terminal 8 and the receiving part 13 of the living body information collecting apparatus, and the pair of the communication part 11 of the portable terminal 8 and the communication network 51 includes function for performing communication using a wireless signal, an optical signal or via a signal line. The terminal receiving part 9 of the portable terminal 8 is connected to each of the display part 10 and the communication part 11 via a signal line. The communication part 11 is connected to each of the display part 10 and the terminal transmission part 12 via a signal line. The receiving part 13 and the acoustic part 14 in the living body information collecting apparatus are connected via a signal line.

Operation of the living body information collecting system of this embodiment is described. The living body information collecting system of this embodiment measures living body information in the same way as the before-mentioned living body information collecting apparatus. The result of measurement is sent from the transmission part 5 to the portable terminal 8. The portable terminal 8 receives living body measured information transmitted from the transmission part 5 of the living body information collecting apparatus by the terminal receiving part 9, displays the living body information on the display part 10 and sends the living body information to the communication part 11. The communication part 11 transmits the living body information to the information processing apparatus 50 via the communication network 51. The information processing apparatus 50 processes the received measurement result, and sends the result of processing of the measurement result or information for instructing next measurement to the communication part 11 of the portable terminal 8 via the communication network 51. The communication part 11 receives the information from the information processing apparatus 50, displays the information on the display part 10, and sends the information to the terminal transmission part 12. The terminal transmission part 12 sends the information to the receiving part 13 of the living body information collecting apparatus. The receiving part 13 receives this information and sends it to the acoustic part 14. The acoustic part 14 receives this information and outputs as sound.

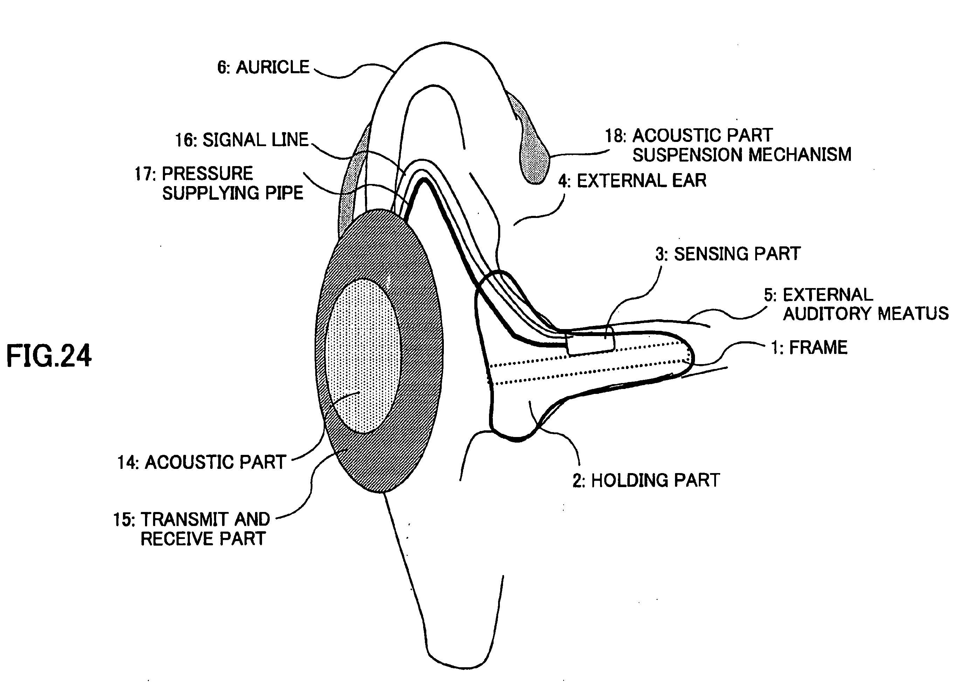

Fig.24 shows an example of implementation and wearing to the living body of the living body information collecting apparatus forming the living body information collecting system of this embodiment. In Fig.24, the living body information collecting apparatus forming the living body information collecting system of this embodiment is formed by an acoustic part 14, a transmit and receive part 15, an acoustic part suspension mechanism 18, a signal line, a pressure supplying pipe 17, a holding part 2, and a sensing part 3. The transmit and receive part 15 implements, in its inside, the drive controlling part 4, the transmission part 5, the receiving part 3 and the power source part 6 shown in Fig.23. Further, the pressure generation mechanism 22 described in the before-mentioned embodiment can be implemented in its inside. In this case, the sensing part 3 and the transmit and receive part 15 are connected by the signal line 16 and the pressure supplying pipe 17. The acoustic part 14 and the transmit and receive part 15 are connected by the signal line and they are integrated, and they are suspended from the auricle 40 by the acoustic part suspension mechanism 18.

The living body information collecting system of this embodiment can transmits information from the information processing apparatus to a human by sound. The apparatus can be used as a conventional headphone for music. As mentioned above, since the living body information collecting system transmits information from the information processing apparatus by sound, a subject can easily ascertain information from the information processing apparatus.

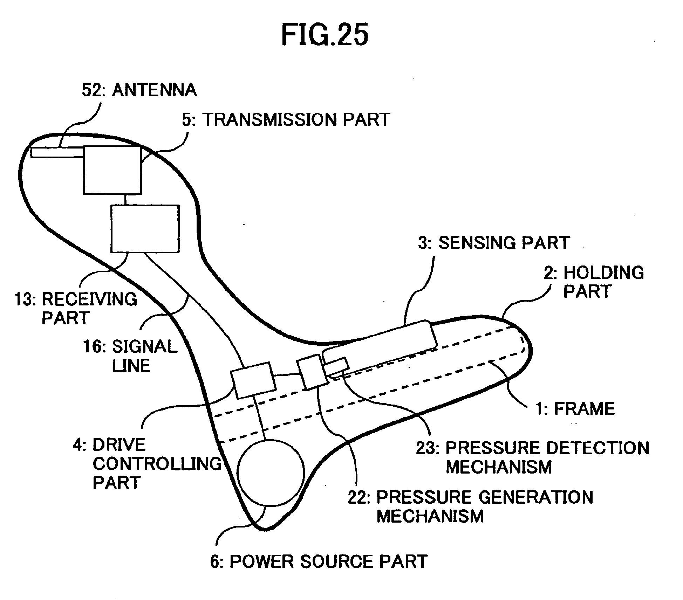

Further, Fig.25 shows an implementation example of the holding part 2 of the living body information collecting apparatus of the living body information collecting system of the before-mentioned embodiments 1-1 - 1-13. In Fig.25, the holding part 2 includes a sensing part 3, a drive controlling part 4, a transmission part 5, a receiving part 13, an antenna 52, a power source part 6, a pressure generation mechanism 22 and a pressure detection mechanism 23. In addition, the power source part 6 supplies power to the drive controlling part 4, the receiving part 13, the transmission part 5, the pressure generation mechanism 22 and the sensing part 3. The drive controlling part 4 is connected to the receiving part 13, the transmission part 5, the pressure generation mechanism 22, the pressure detection mechanism 23, and the sensing part 3 by the signal lines 16. The antenna is necessary when the receiving part 13 or the transmission part 5 communicates with the portable terminal 8 using a wireless signal, for example.

In Fig.25, although the holding part 2 includes a sensing part 3, a drive controlling part 4, a transmission part 5, a receiving part 13, an antenna 52, a power source part 6, a pressure generation mechanism 22 and a pressure detection mechanism 23, it does not mean that all of these are implemented. Only necessary components for each of the living body information collecting apparatuses of the living body information collecting systems of each embodiment are implemented.

By adopting the above implementation, the holding part 2 can be downsized very much and much weight reduction can be available, so that long time stable measurement of living body information can be realized and convenience improves.

As mentioned above, according to the first embodiment, the living body information can be collected while the apparatus is inserted into the external auditory meatus 42. In addition, since a hollow part is provided, living body information can be continuously collected without affecting hearing. The shape can be formed based on the shape of the eternal ear and the external auditory meatus.

In addition, by providing the drive controlling part and the transmission part to the living body information collecting apparatus, a living body information collecting apparatus that can measure living body information easily and quickly and that is easy to carry can be provided.

In addition, according to the first embodiment, it becomes possible to measure blood pressure, pulse, body temperature, posture, acceleration, blood oxygen levels and electroencephalogram continuously or continually, and it becomes possible to collect the measurement result remotely, analyze the result based on advanced knowledge, and to realize various measurement with high accuracy and with reliability by using remote instructions.

### (Second embodiment)

Next, the second embodiment of the present invention is described.

### [Embodiment 2-1]

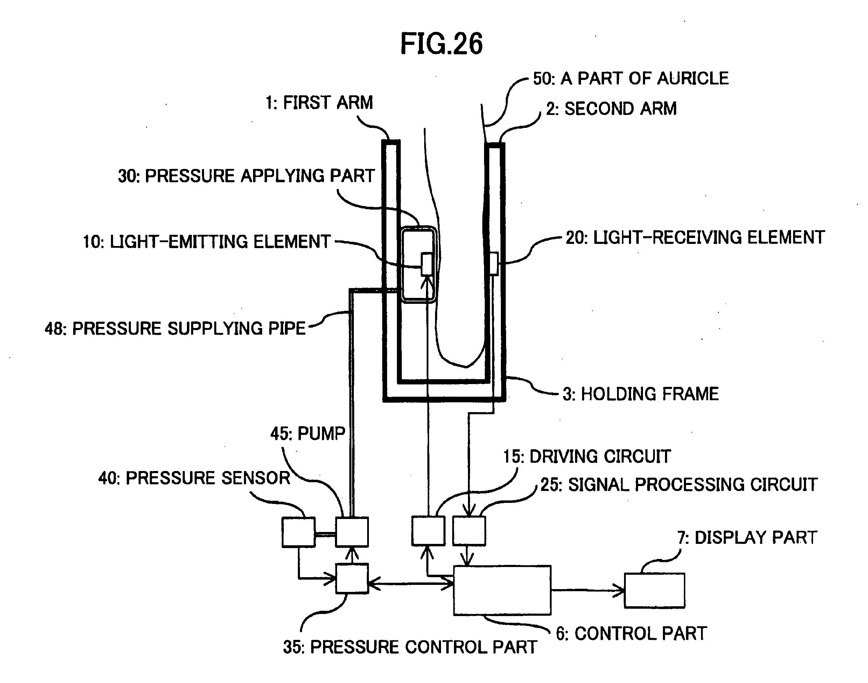

Fig.26 is a block diagram of a blood-pressure meter that is the embodiment 2-1 of the present invention. The blood-pressure meter of the embodiment 2-1 is formed by a holding frame part 3 for pinching a part 50 of an auricle using a pushing pressure between a first arm 1 and a second arm 2, a pressure applying part 30 that is provided in the inside of the first arm and that is pressure-variable, a pair of light-emitting element 10 and a light-receiving element 20 for measuring light transmittance between the pressure applying part 30 and the second arm 2, a control part 6, a display part 7, a pressure sensor 40, a pressure control part 35, a pump 45, a driving circuit 15, and a signal processing circuit 25. The pressure applying part 30 and the pump 45 are connected by a pressure supplying pipe 48. The pump 45 and the pressure sensor 40 is connected by a pipe. The light-emitting element 10 and the driving circuit 15, and, the light receiving element 20 and the signal processing circuit 25 are respectively connected by a signal line. The holding frame part 3 is formed by elastic-deformable metal or plastic or the like such that the holding frame part 3 can be worn on the auricle by widening the interval between the first arm 1 and the second arm 2, which is similar to each holding frame 3 of other embodiments.

The control part 6 is connected to each of the pressure control part 35, the driving circuit 15, the signal processing circuit 25 and the display part 7 by a signal line. The pressure control part is connected to each of the pressure sensor 40 and the pump by a signal line. The pressure-variable pressure applying part 30 placed in the inside of the first arm 1 and the second arm 2 are placed so as to pinch the part 50 of the auricle. One of the pair of the light-emitting element 10 and the light-receiving element 20 is placed in the inside of the pressure applying part 30, and another is placed in the inside of the second arm 2. In Fig.26, although the light-emitting element 10 is placed in the pressure applying part 30 and the light-receiving element 20 is placed in the second arm 2, inversely, the light-emitting element 10 may be placed in the second arm 2 and the light-receiving element 20 may be placed in the pressure applying part 30. The light-emitting element 10 and the light-receiving element 20 are placed on a line such that they are opposed to each other. That is, they are placed such that emitted light of the light-emitting element 10 can be received by the light-receiving element 2.

Next, operation of the blood-pressure meter of the embodiment 2-1 is described. The control part 6 has a function for performing controls of the whole blood-pressure meter such as measurement start or end of the blood-pressure meter. The control part 6 sends a signal to the pressure control part 35 so as to instruct the pressure control part 35 to drive the pump 45 to apply a pressure to the pressure applying part 30. The pressure control part 35 sends a signal to the pump 45 so as to instruct the pump 45 to supply a pressure, instructed by the control part 6, to the pressure applying part 30 via the pressure supplying pipe 48. The pressure sensor 40 measures the pressure supplied by the pump 45 to the pressure applying part 30 via the pressure supplying pipe 48, and transmits the measured result to the pressure controlling part 35 by the signal line. The pressure control part 35 controls the pump 45 such that the pressure supplied by the pump 45 that is measured by the pressure sensor 40 is the same as the pressure instructed by the control part 6.

On the other hand, the control part 6 sends a signal to the driving circuit 15 to instruct the driving circuit 15 to cause the light-emitting element 10 to illuminate. The driving circuit 15 receives this signal, drives the light-emitting element 10. The light-emitting element 10 emits laser light and the like to the part 50 of the auricle. The emitted light passes through the part 50 of the auricle, and the light-receiving element 20 receives the transmitted light. The light-receiving element 20 converts the received transmitted light into an electrical signal and sends the signal to the signal processing circuit 25 via the signal line.

The signal processing circuit 25 stores relationship between pulsation waveform and (level of) blood pressure described in "principle 1 of blood pressure measurement". The signal processing circuit 25 processes the electrical signal corresponding to the waveform of the transmitted light received by the light-receiving element 20, and sends the result to the control part 6. The control part 6 displays the measurement result on the display part 7.

A blood pressure is measured using the blood-pressure meter of this embodiment in the following way. The light-emitting element 10 emits a light beam such as a laser light beam to the part 50 of the auricle. When the emitted light passes through the inside of the part of the auricle, the emitted light receives change of attenuation or frequency corresponding to pulsation of the part of the auricle that repeats expand and contraction due to pulsation of a blood vessel. The light-receiving element 20 measures a pulsation waveform based on the change of the amount of the transmitted light or the change of frequency, converts the pulsation waveform to the electrical signal and sends the signal to the signal processing circuit 25.

The signal processing circuit 25 compares the pulsation waveform measured by the light-receiving element 20 with pulsation waveforms that are stored beforehand to determine which level the blood pressure at this time corresponds to between the maximum blood pressure and the minimum blood pressure, and sends the result to the control part 6.

The control part 6 displays a value of the blood pressure at this time and the level of the blood pressure between the maximum blood pressure and the minimum blood pressure on the display part 7 based on the result received from the signal processing circuit 25 and the pressure measured by the pressure sensor 40 at the same time. According to the above-mentioned operation, the blood-pressure meter of this embodiment measures the blood pressure. Further, by changing the pressure applied by the pressure applying part 30 via the pressure control part 35 by operating the control part 6, a blood pressure corresponding to any level between the maximum blood pressure and the minimum blood pressure can be measured.

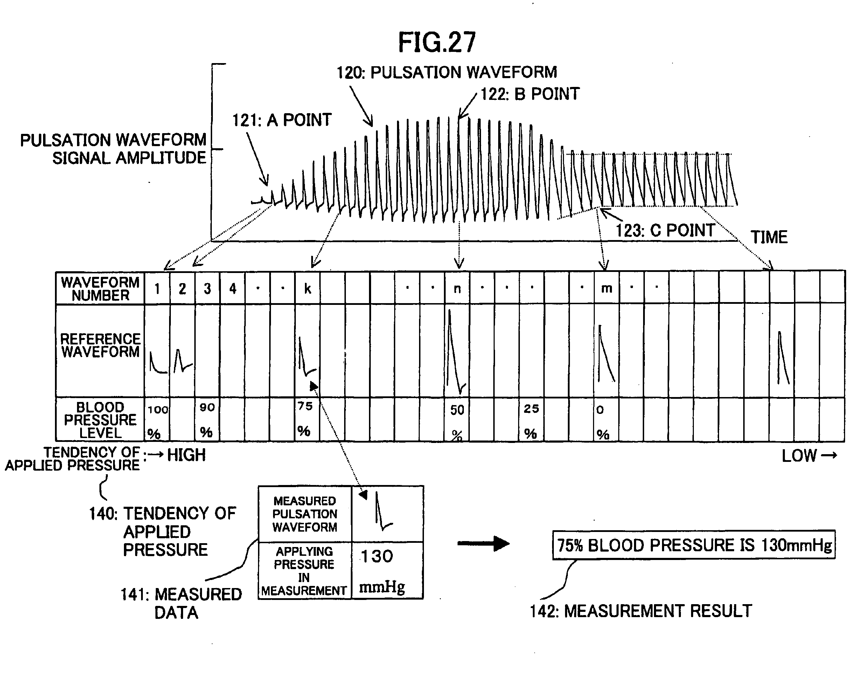

Blood pressure measurement in this embodiment is described more concretely with reference to Fig.27.

Fig.27 shows again the pulsation waveform 120, the A point 121 corresponding to the maximum blood pressure, the B 122 point corresponding to the average blood pressure, the C point 123 corresponding to the minimum blood pressure shown in Fig.14. In the table in Fig.27, the upper row indicates waveform numbers, the middle row indicates reference waveforms, and the bottom row indicates blood pressure levels. The reference waveforms in the middle row in the table are obtained by dividing pulse-like waveforms forming the pulsation waveform 120 for each period, and arranging the divided waveforms from the maximum blood pressure side to the minimum blood pressure side. The waveform numbers of the upper row are numbers "1, 2, 3, ... " each assigned to the corresponding reference waveform in the middle row from the maximum blood pressure side to the minimum blood pressure side. The blood pressure levels in the bottom row are numbers each proportionally allocated for a blood pressure level corresponding to a reference waveform between the maximum blood pressure and the minimum blood pressure assuming that the waveform corresponding to the maximum blood pressure, that is, a waveform of number 1 is 100% and the minimum blood pressure is 0%. These waveform numbers, reference waveforms and blood pressure levels are stored in the signal processing circuit 25 shown in Fig.26.

The tendency of applied pressure 140 indicates that the waveform number "1" in the table corresponds to a case when the applied pressure 114 shown in Fig.14 is high, and that, the larger the waveform number is, the lower the applied pressure 114 shown in Fig.14 is low.

The signal processing circuit 25 searches the table of Fig.27 to determine what number of reference waveform the pulsation waveform measured by the light-receiving element 20 corresponds to.

The calculation for the search can be performed in the following way. Each of "measured pulsation waveform" in the measured data 141 and the reference pulsation waveform are divided to 1000 equal parts on the time axis, for example, and an amplitude value corresponding to each time is represented by a digital signal. First, the "measured pulsation waveform" and the reference waveform of number 1 are compared. In this case, after making maximum values of both waveforms to be the same, amplitudes of the both waveforms are compared for each corresponding time. The reason for comparing the amplitudes of the both waveforms for each corresponding time after making maximum values of both waveforms to be the same is that, it is necessary to compare them using information of shape of pulsation waveform since the amplitude of the pulsation waveform changes due to blood pressure. As a result of the comparison, when a difference is obtained, the difference is stored. Next, comparison between "measured pulsation waveform" and reference waveform of number 2 is performed in the same procedure. By repeating such operation from the reference waveform of number 1 to the last number, a number of a reference waveform having a waveform nearest to the "measured pulsation waveform" can be searched for.

In the measurement example of Fig.27, the waveform nearest to the "measured pulsation waveform" is the waveform number k in the table of Fig.27, and it is determined that the blood pressure level corresponding to this waveform is 75% between the maximum blood pressure and the minimum blood pressure. In addition, the measurement example of Fig.27 shows a case in which the applied pressure in measurement in the measurement data 141 is measured as 130mmHg by the pressure sensor 40 in Fig.26. Therefore, the result of the blood pressure measurement becomes "75% blood pressure is 130mmHg" as in the measurement result 142 shown in Fig.27.

Another configuration can be adopted by removing the signal processing circuit 25 from the blood-pressure meter shown in Fig.26. In this case, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship between pulsation waveform and blood pressure being prepared separately to the blood-pressure meter beforehand. Based on the pressure measured by the pressure sensor 40, the control part 6 displays the blood pressure at this time on the display part 7. Accordingly, the blood pressure can be measured. Further, by changing the pressure applied by the pressure applying part 30 via the pressure control part 35 by operating the control part 6, a blood pressure corresponding to any level between the maximum blood pressure and the minimum blood pressure can be measured.

Further, a configuration obtained by removing the pressure control part 35, the pressure sensor 40, the pump 45, the driving circuit 15, the signal processing circuit 25, the control part 6 and the display part 7 from the blood-pressure meter of Fig.26 can be adopted.

In this blood-pressure meter, a pressure is supplied to the pressure applying part 30 by a pump and the like that exists in the outside of the blood-pressure meter, and power and a driving signal are supplied to the light-emitting element 10 from the outside. In addition, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship among pulsation waveform, the amplitude value and blood pressure being prepared separately to the blood-pressure meter beforehand. By changing the pressure applied by the pressure applying part 30 using an external pump and the like, a blood pressure corresponding to any level between the maximum blood pressure and the minimum blood pressure can be measured.

### [Embodiment 2-2]

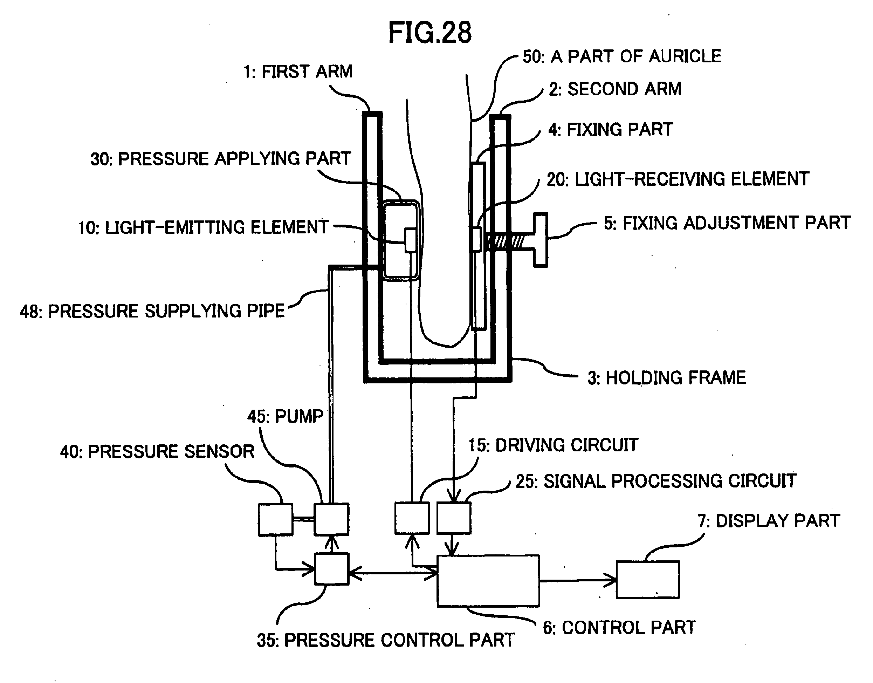

Next, the embodiment 2-2 of this invention is described. Fig.28 is a block diagram of a blood-pressure meter in the embodiment 2-2 of the present invention.

The blood-pressure meter of the embodiment 2-2 is formed by a holding frame part 3 for pinching a part 50 of an auricle using a pushing pressure between a first arm 1 and a second arm 2, a pressure applying part 30 that is provided in the inside of the first arm 1 and that is pressure-variable, a fixing part 4 that is provided in the inside of the second arm 2 and that is fixed on a part of the auricle, a fixing adjustment part 5 that is provided with the fixing part at the top and that pushes the fixing part to the part of the auricle, a pair of light-emitting element 10 and a light-receiving element 20 for measuring light transmittance between the pressure applying part 30 and the fixing part 4, a control part 6, a display part 7, a pressure sensor 40, a pressure control part 35, a pump 45, a driving circuit 15, and a signal processing circuit 25. The pressure applying part 30 and the pump 45 are connected by a pressure supplying pipe 48. The pump 45 and the pressure sensor 40 is connected by a pipe. The light-emitting element 10 and the driving circuit 15, and, the light receiving element 20 and the signal processing circuit 25 are respectively connected by a signal line. The control part 6 is connected to each of the pressure control part 35, the driving circuit 15, the signal processing circuit 25 and the display part 7 by a signal line. The pressure control part 35 is connected to each of the pressure sensor 40 and the pump 45 by a signal line. The pressure-variable pressure applying part 30 placed in the inside of the first arm 1 and the fixing part 4 are placed so as to pinch the part 50 of the auricle. The fixing adjustment part 5 has a function for adjusting an interval between the pressure applying part 30 and the fixing part 4. When the pressure applying part 30 and the fixing part 4 are placed to pinch the part 50 of the auricle, the fixing adjustment part 5 adjusts the fixing part 4 such that the fixing part 4 pushes the part 50 of the auricle and the pressure applying part 30 and the fixing part 4 pinches the part 50 of the auricle with a proper interval. One of the pair of the light-emitting element 10 and the light-receiving element 20 is placed in the inside of the pressure applying part 30, and another is placed in the inside of the fixing part 4.

In Fig.28, although the light-emitting element 10 is placed in the pressure applying part 30 and the light-receiving element 20 is placed in the fixing part 4, inversely, the light-emitting element 10 may be placed in the fixing part 4 and the light-receiving element 20 may be placed in the pressure applying part 30. The light-emitting element 10 and the light-receiving element 20 are placed on a line such that they are opposed to each other. That is, they are placed such that emitted light of the light-emitting element 10 can be received by the light-receiving element 20.

Next, operation of the blood-pressure meter of this embodiment is described. The control part 6 has a function for performing controls of the whole blood-pressure meter such as measurement start or end of the blood-pressure meter. The control part 6 sends a signal to the pressure control part 35 so as to instruct the pressure control part 35 to drive the pump 45 to apply a pressure to the pressure applying part 30. The pressure control part 35 sends a signal to the pump 45 so as to instruct the pump 45 to supply a pressure, instructed by the control part 6, to the pressure applying part 30 via the pressure supplying pipe 48. The pressure sensor 40 measures the pressure supplied by the pump 45 to the pressure applying part 30 via the pressure supplying pipe 48, and transmits the measured result to the pressure controlling part 35 by the signal line. The pressure control part 35 controls the pump 45 such that the pressure supplied by the pump 45 that is measured by the pressure sensor 40 is the same as the pressure instructed by the control part 6. On the other hand, the control part 6 sends a signal to the driving circuit 15 to instruct the driving circuit to cause the light-emitting element 10 to illuminate. The driving circuit 15 receives this signal, drives the light-emitting element 10. The light-emitting element 10 emits laser light and the like to a part 50 of the pump 45. The emitted light passes through the part 50 of the pump 45, and the light-receiving element 20 receives the transmitted light. The light-receiving element 20 converts the received transmitted light into an electrical signal and sends the signal to the signal processing circuit 25 via the signal line. The signal processing circuit 25 stores relationship between pulsation waveform and blood pressure as described in the embodiment 2-1. The signal processing circuit 25 processes the electrical signal corresponding to the waveform of the transmitted light received by the light-receiving element 20, and sends the result to the control part 6. The control part 6 displays the measurement result on the display part 7.

A blood pressure is measured using the blood-pressure meter of this embodiment in the following way. The light-emitting element 10 emits a light beam such as a laser light beam to the part 50 of the pump 45. When the emitted light passes through the inside of the part of the pump 45, the emitted light receives change of attenuation or frequency corresponding to pulsation of the part of the pump 45 that repeats expand and contraction due to pulsation of a blood vessel. The light-receiving element 20 measures the pulsation waveform based on the change of the amount of the transmitted light or the change of frequency, converts the pulsation waveform to the electrical signal and sends the signal to the signal processing circuit 25. The signal processing circuit 25 compares the pulsation waveform measured by the light-receiving element 20 with pulsation waveforms that are stored beforehand to determine which level the blood pressure at this time corresponds to between the maximum blood pressure and the minimum blood pressure, and sends the result to the control part 6. The control part 6 displays a value of the blood pressure at this time and the level of the blood pressure between the maximum blood pressure and the minimum blood pressure on the display part 7 based on the result received from the signal processing circuit 25 and the pressure measured by the pressure sensor 40 at the same time.

According to the above-mentioned operation, the blood-pressure meter of this embodiment measures the blood pressure. Further, by changing the pressure applied by the pressure applying part 30 via the pressure control part 35 by operating the control part 6, a blood pressure corresponding to any level between the maximum blood pressure and the minimum blood pressure can be measured.

As mentioned above, according to this embodiment, the fixing adjustment part 5 adjusts the interval between the pressure applying part 30 and the fixing part 4 according to individual variation of thickness of the part 50 of the auricle. Therefore, useless operation of the pump 45 can be eliminated so that there is a merit that the capacity of the pump 45 can be decreased.

Another configuration can be adopted by removing the signal processing circuit 25 from the blood-pressure meter shown in Fig.28. In this case, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship between pulsation waveform and blood pressure being prepared separately to the blood-pressure meter beforehand. Based on pressure measured by the pressure sensor 40, the control part 6 displays the blood pressure at this time on the display part 7. Accordingly, the blood pressure can be measured. Further, by changing the pressure applied by the pressure applying part 30 via the pressure control part 35 by operating the control part 6, a blood pressure corresponding to any level between the maximum blood pressure and the minimum blood pressure can be measured.

Further, a configuration obtained by removing the pressure control part 35, the pressure sensor 40, the pump 45, the driving circuit 15, the signal processing circuit 25, the control part 6 and the display part 7 from the blood-pressure meter of Fig.28 can be adopted.

In this blood-pressure meter, a pressure is supplied to the pressure applying part 30 by a pump and the like that exists in the outside of the blood-pressure meter, and power and a driving signal are supplied to the light-emitting element 10 from the outside. In addition, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship among pulsation waveform, the amplitude value and blood pressure being prepared separately to the blood-pressure meter beforehand. By changing the pressure applied by the pressure applying part 30 using an external pump and the like, a blood pressure corresponding to any level between the maximum blood pressure and the minimum blood pressure can be measured. Also in this case, the fixing adjustment part 5 adjusts the interval between the pressure applying part 30 and the fixing part 4 according to individual variation of thickness of the part 50 of the auricle. Therefore, useless operation of the external pump can be eliminated so that there is a merit that the capacity of the external pump can be decreased.

### [Embodiment 2-3]

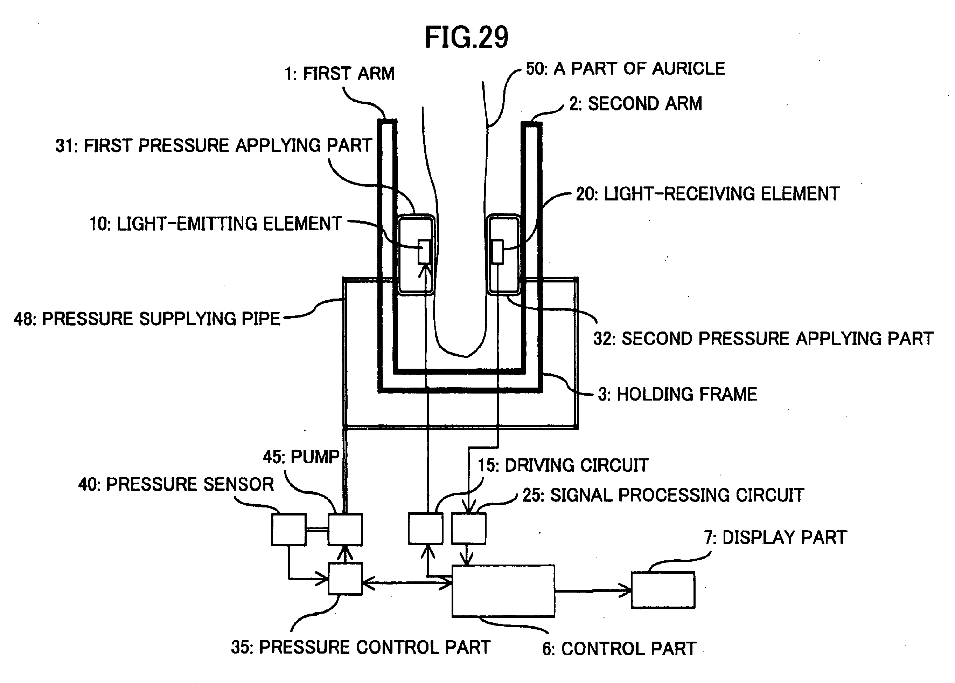

Next, the embodiment 2-3 of the present invention is described. Fig.29 is a block diagram of a blood-pressure meter that is the embodiment 2-3 of the present invention.

The blood-pressure meter of the embodiment 2-3 is formed by a holding frame part 3 for pinching a part 50 of an auricle using a pushing pressure between a first arm 1 and a second arm 2, a first pressure applying part 31 that is provided in the inside of the first arm 1 and that is pressure-variable, a second pressure applying part 32 that is provided in the inside of the second arm 1 and that is pressure-variable, a pair of light-emitting element 10 and a light-receiving element 20 for measuring light transmittance between the first pressure applying part 31 and the second pressure applying part 32, a control part 6, a display part 7, a pressure sensor 40, a pressure control part 35, a pump 45, a driving circuit 15, and a signal processing circuit 25.

The first pressure applying part 31 and the second pressure applying part 32 are connected to the pump 45 by a pressure supplying pipe 48. The pump 45 and the pressure sensor 40 is connected by a pipe. The light-emitting element 15 and the driving circuit 15, and the light receiving element 20 and the signal processing circuit 25 are respectively connected by a signal line. The control part 6 is connected to each of the pressure control part 35, the driving circuit 15, the signal processing circuit 25 and the display part 7 by a signal line. The pressure control part 35 is connected to each of the pressure sensor 40 and the pump by a signal line. The first pressure applying part 31 and the second pressure applying part 32 are placed so as to pinch the part 50 of the auricle. One of the pair of the light-emitting element 10 and the light-receiving element 20 is placed in the inside of the first pressure applying part 31, and another is placed in the inside of the second pressure applying part 32. In Fig.29, although the light-emitting element 10 is placed in the first pressure applying part 31 and the light-receiving element 20 is placed in the second pressure applying part 32, inversely, the light-emitting element 10 may be placed in the second pressure applying part 32 and the light-receiving element 20 may be placed in the first pressure applying part 31. The light-emitting element 10 and the light-receiving element 20 are placed on a line such that they are opposed to each other. That is, they are placed such that emitted light of the light-emitting element 10 can be received by the light-receiving element 2.

Next, operation of the blood-pressure meter of the embodiment 2-3 is described. The control part 6 has a function for performing controls of the whole blood-pressure meter such as measurement start or end of the blood-pressure meter. The control part 6 sends a signal to the pressure control part 35 so as to instruct the pressure control part 35 to drive the pump 45 to apply pressure to the pressure applying part 30. The pressure control part 35 sends a signal to the pump 45 so as to instruct the pump 45 to supply a pressure, instructed by the control part 6, to the first pressure applying part 31 and the second pressure applying part 32 via the pressure supplying pipe 48. The pressure sensor 40 measures the pressure supplied by the pump 45 to the first pressure applying part 31 and the second pressure applying part 32 via the pressure supplying pipe 48, and transmits the measured result to the pressure controlling part 35 by the signal line. The pressure control part 35 controls the pump 45 such that the pressure supplied by the pump 45 that is measured by the pressure sensor 40 is the same as the pressure instructed by the control part 6.

On the other hand, the control part 6 sends a signal to the driving circuit 15 to instruct the driving circuit 15 to cause the light-emitting element 10 to illuminate. The driving circuit 15 receives this signal, drives the light-emitting element 10. The light-emitting element 10 emits laser light and the like to a part 50 of the auricle. The emitted light passes through the part 50 of the auricle, and the light-receiving element 20 receives the transmitted light. The light-receiving element 20 converts the received transmitted light into an electrical signal and sends the signal to the signal processing circuit 25 via the signal line. The signal processing circuit 25 stores relationship between pulsation waveform and blood pressure. The signal processing circuit 25 processes the electrical signal corresponding to the waveform of the transmitted light received by the light-receiving element 20, and sends the result to the control part 6. The control part 6 displays the measurement result on the display part 7.

A blood pressure is measured using the blood-pressure meter of this embodiment in the following way. The light-emitting element 10 emits a light beam such as a laser light beam to the part 50 of the auricle. When the emitted light passes through the inside of the part of the ear, the emitted light receives change of attenuation or frequency corresponding to pulsation of the part of the auricle that repeats expand and contraction due to pulsation of a blood vessel. The light-receiving element 20 measures the pulsation waveform based on the change of the amount of the transmitted light or the change of frequency, converts the pulsation waveform to the electrical signal and sends the signal to the signal processing circuit 25. The signal processing circuit 25 compares the pulsation waveform measured by the light-receiving element 20 with pulsation waveforms that are stored beforehand to determine which level the blood pressure at this time corresponds to between the maximum blood pressure and the minimum blood pressure, and sends the result to the control part 6. The control part 6 displays a value of the blood pressure at this time and the level of the blood pressure between the maximum blood pressure and the minimum blood pressure on the display part 7 based on the result received from the signal processing circuit 25 and the pressure measured by the pressure sensor 40 at the same time.

According to the above-mentioned operation, the blood-pressure meter of this embodiment measures the blood pressure. Further, by changing the pressure applied by the first pressure applying part 31 and the second pressure applying part 32 via the pressure control part 35 by operating the control part 6, a blood pressure corresponding to any level between the maximum blood pressure and the minimum blood pressure can be measured.

Another configuration can be adopted by removing the signal processing circuit 25 from the blood-pressure meter shown in Fig.29. In this case, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship between pulsation waveform and blood pressure being prepared separately to the blood-pressure meter beforehand. Based on pressure measured by the pressure sensor 40, the control part 6 displays the blood pressure at this time on the display part 7. Accordingly, the blood pressure can be measured. Further, by changing the pressure applied by the first pressure applying part 31 and the second pressure applying part 32 via the pressure control part 35 by operating the control part 6, a blood pressure corresponding to any level between the maximum blood pressure and the minimum blood pressure can be measured.

Further, a configuration obtained by removing the pressure control part 35, the pressure sensor 40, the pump 45, the driving circuit 15, the signal processing circuit 25, the control part 6 and the display part 7 from the blood-pressure meter of Fig.29 can be adopted.

In this blood-pressure meter, a pressure is supplied to the pressure applying part 30 by a pump and the like that exists in the outside of the blood-pressure meter, and power and a driving signal are supplied to the light-emitting element 10 from the outside. In addition, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship among pulsation waveform, the amplitude value and blood pressure being prepared separately to the blood-pressure meter beforehand. By changing the pressure applied by the first pressure applying part 31 and the second pressure applying part 32 using an external pump and the like, a blood pressure corresponding to any level between the maximum blood pressure and the minimum blood pressure can be measured.

### [Embodiment 2-4]

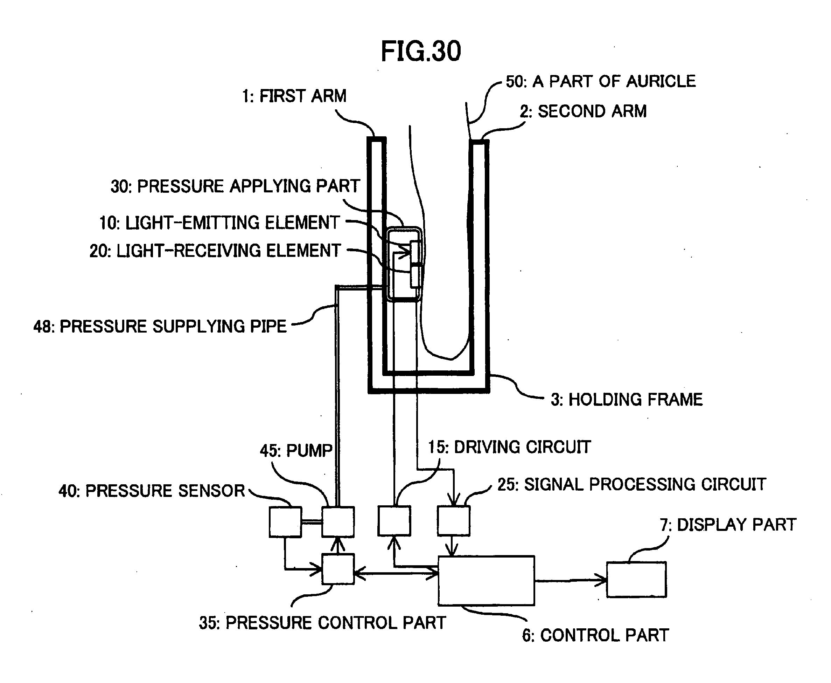

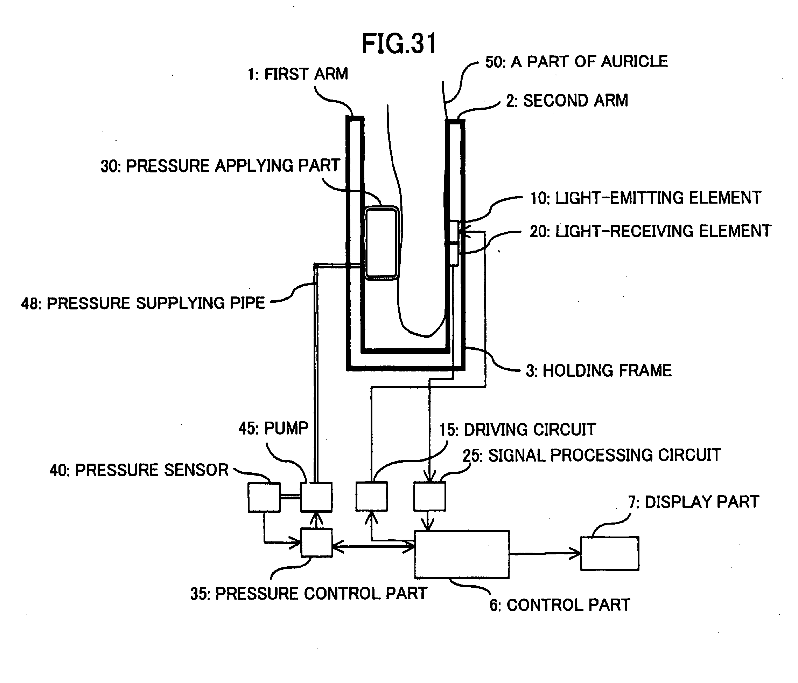

Next; the embodiment 2-4 of the present invention is described. Each of Figs.30 and 31 is a block diagram of a blood-pressure meter in the embodiment 2-4 of the present invention.

The blood-pressure meter of the embodiment 2-4 is formed by a holding frame part 3 for pinching a part 50 of an auricle using a pushing pressure between a first arm 1 and a second arm 2, a pressure applying part 30 that is provided in the inside of the first arm 1 and that is pressure-variable, a pair of light-emitting element 10 and a light-receiving element 20 for measuring light reflectance between the pressure applying part 30 and the second arm 2, a control part 6, a display part 7, a pressure sensor 40, a pressure control part 35, a pump 45, a driving circuit 15, and a signal processing circuit 25. The pressure applying part 30 and the pump 45 are connected by a pressure supplying pipe 48. The pump 45 and the pressure sensor 40 is connected by a pipe. The light-emitting element 10 and the driving circuit 15, and the light receiving element 20 and the signal processing circuit 25 are respectively connected by a signal line. The control part 6 is connected to each of the pressure control part 35, the driving circuit 15, the signal processing circuit 25 and the display part 7 by a signal line. The pressure control part 35 is connected to each of the pressure sensor 40 and the pump 45 by a signal line. The pressure-variable pressure applying part 30 placed in the inside of the first arm 1 and the second arm 2 are placed so as to pinch the part 50 of the auricle. The pair of the light-emitting element 10 and the light-receiving element 20 is placed in the inside of the pressure applying part 30 or in the inside of the second arm 2. In Fig.30, although the pair of the light-emitting element 10 and the light-receiving element 20 is placed in the pressure applying part 30, the pair of the light-emitting element 10 and the light-receiving element 20 may be placed in the inside of the second arm 2 as shown in Fig.31. The light-emitting element 10 and the light-receiving element 20 are placed adjacent to each other such that a light-emitting surface of the light emitting element 10 and a light-receiving surface of the light-receiving element 20 are directed to the inside of the first arm 1 or the second arm 2. That is, they are placed such that, when the emitted light of the light-emitting element 10 is reflected from an outside part, the reflected light can be received by the light-receiving element 2.

Next, operation of the blood-pressure meter of this embodiment is described with reference to Fig.30. As to Fig.30 and Fig.31, only the placement positions of the pair of the light-emitting element 10 and the light-receiving element 20 are different, and the operations are the same. Therefore, explanations are given with reference to Fig. 30.

The control part 6 has a function for performing controls of the whole blood-pressure meter such as measurement start or end of the blood-pressure meter. The control part 6 sends a signal to the pressure control part 35 so as to instruct the pressure control part 35 to drive the pump 45 to apply a pressure to the pressure applying part 30. The pressure control part 35 sends a signal to the pump 45 so as to instruct the pump 45 to supply a pressure, instructed by the control part 6, to the pressure applying part 30 via the pressure supplying pipe 48. The pressure sensor 40 measures the pressure supplied by the pump 45 to the pressure applying part 30 via the pressure supplying pipe 48, and transmits the measured result to the pressure control part 35 by the signal line. The pressure control part 35 controls the pump 45 such that the pressure supplied by the pump 45 that is measured by the pressure sensor 40 is the same as the pressure instructed by the control part 6. On the other hand, the control part 6 sends a signal to the driving circuit 15 to instruct the driving circuit to cause the light-emitting element 10 to illuminate. The driving circuit 15 receives this signal, drives the light-emitting element 10. The light-emitting element 10 emits laser light and the like to a part 50 of the auricle. The emitted light is reflected from an blood vessel and the like in the surface or in the inside of the part 50 of the auricle, and the light-receiving element 20 receives the reflected light. The light-receiving element 20 converts the reflected light into an electrical signal and sends the signal to the signal processing circuit 25 via the signal line.. The signal processing circuit 25 stores relationship between pulsation waveform and blood pressure. The signal processing circuit 25 processes the electrical signal corresponding to the waveform of the reflected light received by the light-receiving element 20, and sends the result to the control part 6. The control part 6 displays the measurement result on the display part 7.

A blood pressure is measured using the blood-pressure meter of this embodiment in the following way. The light-emitting element 10 emits a light beam such as a laser light beam to the part 50 of the auricle. When the emitted light is reflected from the blood vessel and the like in the surface or the inside of the part 50 of the auricle, the reflected light receives change of the light amount or change of frequency corresponding to pulsation of the part 50 of the auricle that repeats expand and contraction due to pulsation of the blood vessel. The light-receiving element 20 measures the pulsation waveform based on the change of the amount of the reflected light or the change of frequency, converts the pulsation waveform to the electrical signal and sends the signal to the signal processing circuit 25.

The signal processing circuit 25 compares the pulsation waveform measured by the light-receiving element 20 with pulsation waveforms that are stored beforehand to determine which level the blood pressure at this time corresponds to between the maximum blood pressure and the minimum blood pressure, and sends the result to the control part 6. The control part 6 displays a value of the blood pressure at this time and the level of the blood pressure between the maximum blood pressure and the minimum blood pressure on the display part 7 based on the result received from the signal processing circuit 25 and the pressure measured by the pressure sensor 40 at the same time.

According to the above-mentioned operation, the blood-pressure meter of this embodiment measures the blood pressure. Further, by changing the pressure applied by the pressure applying part 30 via the pressure control part 35 by operating the control part 6, a blood pressure corresponding to any level between the maximum blood pressure and the minimum blood pressure can be measured.

Another configuration can be adopted by removing the signal processing circuit 25 from the blood-pressure meter shown in Fig.30 or Fig.31. In this case, a pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship between pulsation waveform and blood pressure being prepared separately to the blood-pressure meter beforehand. Based on the pressure measured by the pressure sensor 40, the control part 6 displays the blood pressure at this time on the display part 7. Accordingly, the blood pressure can be measured. Further, by changing the pressure applied by the pressure applying part 30 via the pressure control part 35 by operating the control part 6, a blood pressure corresponding to any level between the maximum blood pressure and the minimum blood pressure can be measured.

Further, a configuration obtained by removing the pressure control part 35, the pressure sensor 40, the pump 45, the driving circuit 15, the signal processing circuit 25, the control part 6 and the display part 7 from the blood-pressure meter of Fig.30 or Fig.31 can be adopted.

In this blood-pressure meter, a pressure is supplied to the pressure applying part 30 by a pump and the like that exists in the outside of the blood-pressure meter, and power and a driving signal are supplied to the light-emitting element 10 from the outside. In addition, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship among pulsation waveform, the amplitude value and blood pressure being prepared separately to the blood-pressure meter beforehand. By changing the pressure applied by the pressure applying part 30 using an external pump and the like, a blood pressure corresponding to any level between the maximum blood pressure and the minimum blood pressure can be measured.

### [Embodiment 2-5]

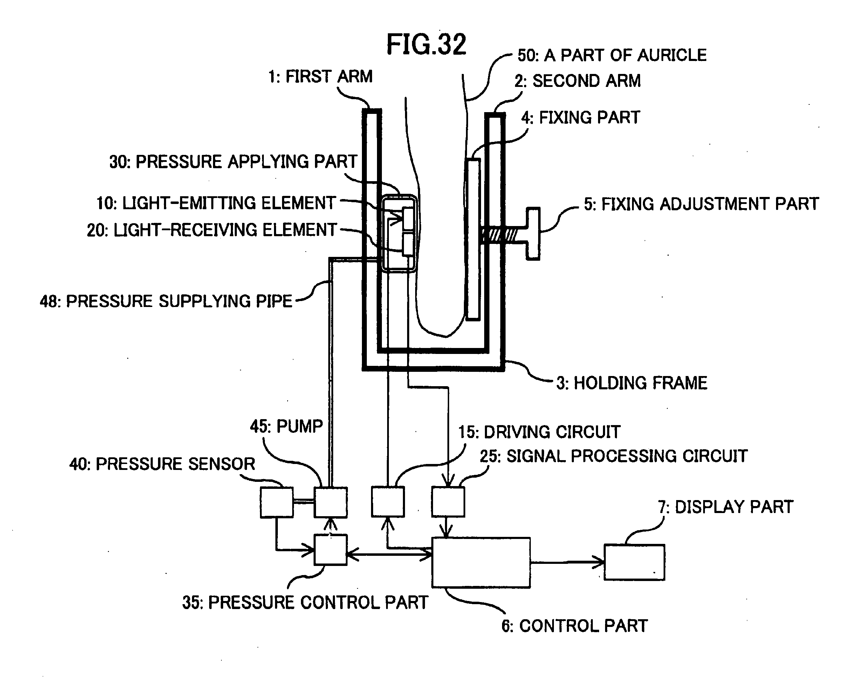

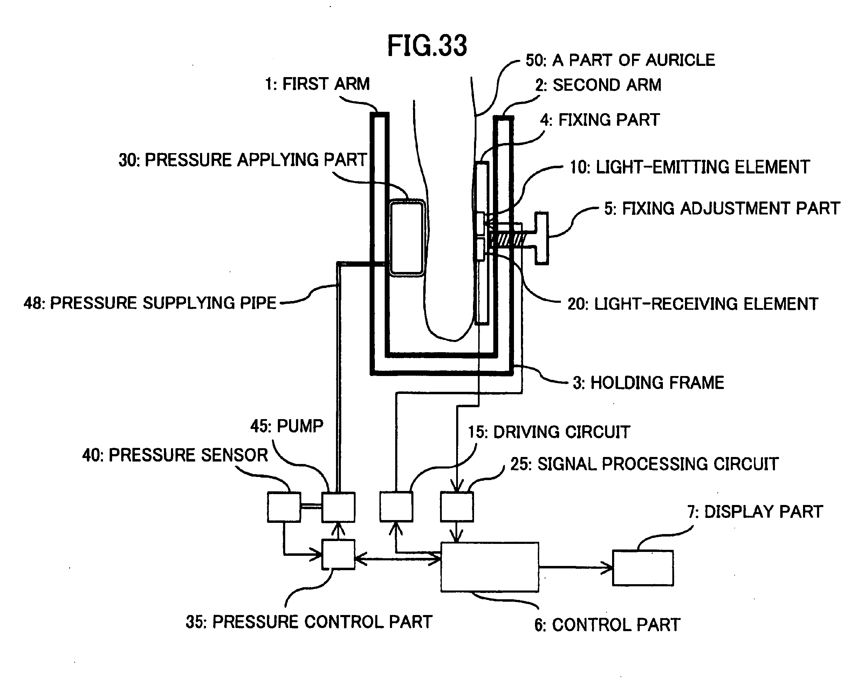

Next, the embodiment 2-5 of this invention is described. Each of Figs.32 and 33 is a block diagram of a blood-pressure meter in the embodiment 2-5.

The blood-pressure meter of the embodiment 2-5 is formed by a holding frame part 3 for pinching a part 50 of an auricle using a pushing pressure between a first arm 1 and a second arm 2, a pressure applying part 30 that is provided in the inside of the first arm 1 and that is pressure-variable, a fixing part 4 that is provided in the inside of the second arm 2 and that is fixed on a part of the auricle, a fixing adjustment part 5 that is provided with the fixing part 4 at the top and that pushes the fixing part 4 to the part of the auricle, a pair of light-emitting element 10 and a light-receiving element 20 that measures light reflectance and that is provided in the pressure applying part 30 or the fixing part 4, a control part 6, a display part 7, a pressure sensor 40, a pressure control part 35, a pump 45, a driving circuit 15, and a signal processing circuit 25. The pressure applying part 30 and the pump 45 are connected by a pressure supplying pipe 48. The pump 45 and the pressure sensor 40 is connected by a pipe. The light-emitting element 10 and the driving circuit 15, and the light receiving element 20 and the signal processing circuit 25 are respectively connected by a signal line. The control part 6 is connected to each of the pressure control part 35, the driving circuit 15, the signal processing circuit 25 and the display part 7 by a signal line. The pressure control part 35 is connected to each of the pressure sensor 40 and the pump 45 by a signal line. The pressure-variable pressure applying part 30 placed in the inside of the first arm 1 and the fixing part 4 are placed so as to pinch the part 50 of the auricle. The fixing adjustment part 5 has a function for adjusting an interval between the pressure applying part 30 and the fixing part 4. When the pressure applying part 30 and the fixing part 4 are placed to pinch the part 50 of the auricle, the fixing adjustment part 5 adjusts the fixing part 4 such that the fixing part 4 pushes the part 50 of the auricle and that the pressure applying part 30 and the fixing part 4 pinches the part 50 of the auricle with a proper interval. The pair of the light-emitting element 10 and the light-receiving element 20 is placed in the inside of the pressure applying part 30 or the inside of the fixing part 4.

In Fig.32, although the pair of the light-emitting element 10 and the light-receiving element 20 is placed in the pressure applying part 30, the pair of the light-emitting element 10 and the light-receiving element 20 may be placed in inside of the fixing part 4 as shown in Fig.33. The light-emitting element 10 and the light-receiving element 20 are placed adjacent to each other such that each of the light-emitting surface of the light-emitting element 10 and the light-receiving surface of the light receiving element 20 is directed to the inside of the first arm 1 or the second arm 2. That is, when the emitted light of the light-emitting element 10 is reflected from an outside part, the reflected light is received by the light-receiving element 20.

Next, operation of the blood-pressure meter of this embodiment is described with reference to Fig.32. As to Fig.32 and Fig.33, only the placement positions of the pair of the light-emitting element 10 and the light-receiving element 20 are different, and the operations are the same. Therefore, explanations are given with reference to Fig.32.

The control part 6 has a function for performing controls of the whole blood-pressure meter such as measurement start or end of the blood-pressure meter. The control part 6 sends a signal to the pressure control part 35 so as to instruct the pressure control part 35 to drive the pump 45 to apply a pressure to the pressure applying part 30. The pressure control part 35 sends a signal to the pump 45 so as to instruct the pump 45 to supply a pressure, instructed by the control part 6, to the pressure applying part 30 via the pressure supplying pipe 48. The pressure sensor 40 measures the pressure supplied by the pump 45 to the pressure applying part 30 via the pressure supplying pipe 48, and transmits the measured result to the pressure controlling part 35 by the signal line. The pressure control part 35 controls the pump 45 such that the pressure supplied by the pump 45 that is measured by the pressure sensor 40 is the same as the pressure instructed by the control part 6. On the other hand, the control part 6 sends a signal to the driving circuit 15 to instruct the driving circuit to cause the light-emitting element 10 to illuminate. The driving circuit 15 receives this signal, drives the light-emitting element 10. The light-emitting element 10 emits laser light and the like to a part 50 of the auricle. The emitted light is reflected from a blood vessel and the like in the surface or the inside of the part 50 of the auricle, and the light-receiving element 20 receives the reflected light. The light-receiving element 20 converts the reflected light into an electrical signal and sends the signal to the signal processing circuit 25 via the signal line. The signal processing circuit 25 stores relationship between pulsation waveform and blood pressure. The signal processing circuit 25 processes the electrical signal corresponding to the waveform of the reflected light received by the light-receiving element 20, and sends the result to the control part 6. The control part 6 displays the measurement result on the display part 7.

A blood pressure is measured using the blood-pressure meter of this embodiment in the following way. The light-emitting element 10 emits a light beam such as a laser light beam to the part 50 of the auricle. When the emitted light is reflected from the surface or inside blood vessel of the part 50 of the auricle, the reflected light receives change of the light amount or change of frequency corresponding to pulsation of the part 50 of the auricle that repeats expand and contraction due to pulsation of the blood vessel. The light-receiving element 20 measures the pulsation waveform based on the change of the amount of the reflected light or the change of frequency, converts the pulsation waveform to the electrical signal and sends the signal to the signal processing circuit 25. The signal processing circuit 25 compares the pulsation waveform measured by the light-receiving element 20 with pulsation waveforms that are stored beforehand to determine which level the blood pressure at this time corresponds to between the maximum blood pressure and the minimum blood pressure, and sends the result to the control part 6. The control part 6 displays a value of the blood pressure at this time and the level of the blood pressure between the maximum blood pressure and the minimum blood pressure on the display part 7 based on the result received from the signal processing circuit 25 and the pressure measured by the pressure sensor 40 at the same time. According to the above-mentioned operation, the blood pressure is measured. By changing the pressure applied by the pressure applying part 30 via the pressure control part 35 by operating the control part 6, a blood pressure corresponding to any level between the maximum blood pressure and the minimum blood pressure can be measured. As mentioned above, when the blood-pressure meter of this embodiment is attached on the part 50 of the auricle, the fixing adjustment part 5 adjusts the interval of the pressure applying part 30 and the fixing part 4 according to individual variation of thickness of the part 50 of the auricle. Therefore, useless operation of the pump 45 can be eliminated so that there is a merit that the capacity of the pump 45 can be decreased.

Another configuration can be adopted by removing the signal processing circuit 25 from the blood-pressure meter shown in Fig.32 or Fig.33. In this case, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship between pulsation waveform and blood pressure being prepared separately to the blood-pressure meter beforehand. Based on pressure measured by the pressure sensor 40, the control part 6 displays the blood pressure at this time on the display part 7. Accordingly, the blood pressure can be measured. Further, by changing the pressure applied by the pressure applying part 30 via the pressure control part 35 by operating the control part 6, a blood pressure corresponding to any level between the maximum blood pressure and the minimum blood pressure can be measured.

Further, a configuration obtained by removing the pressure control part 35, the pressure sensor 40, the pump 45, the driving circuit 15, the signal processing circuit 25, the control part 6 and the display part 7 from the blood-pressure meter of Fig.32 or 33 can be adopted.

In this blood-pressure meter, a pressure is supplied to the pressure applying part 30 by a pump and the like that exists in the outside of the blood-pressure meter, and power and a driving signal are supplied to the light-emitting element 10 from the outside. In addition, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship among pulsation waveform, the amplitude value and blood pressure being prepared separately to the blood-pressure meter beforehand. By changing the pressure applied by the pressure applying part 30 using an external pump and the like, a blood pressure corresponding to any level between the maximum blood pressure and the minimum blood pressure can be measured. Also in this case, the fixing adjustment part 5 adjusts the interval of the pressure applying part 30 and the fixing part 4 according to individual variation of thickness of the part 50 of the auricle. Therefore, useless operation of the pump can be eliminated so that there is a merit that the capacity of the external pump can be decreased.

### [Embodiment 2-6]

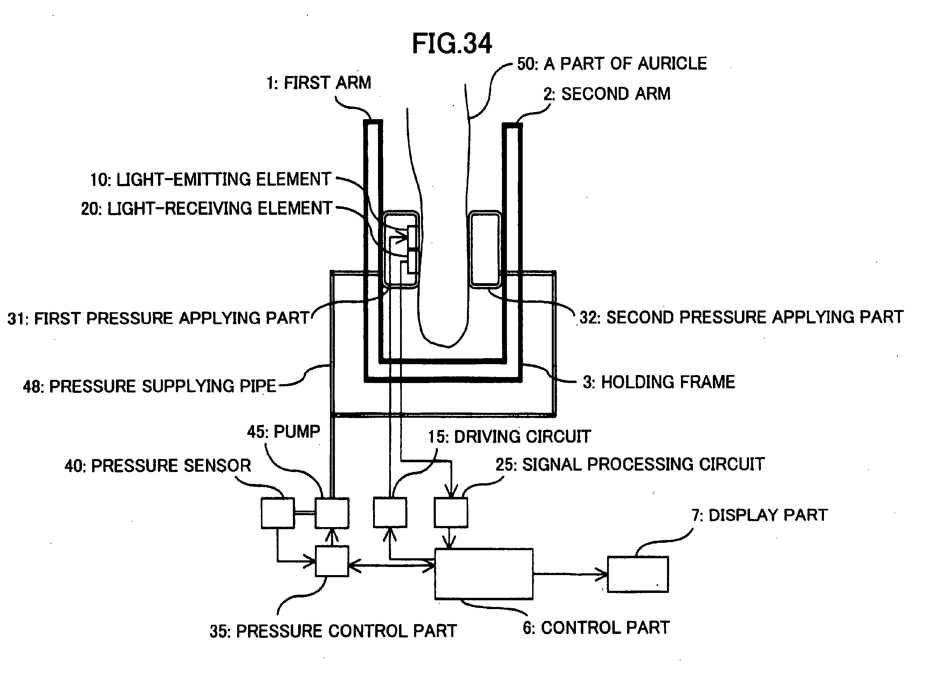

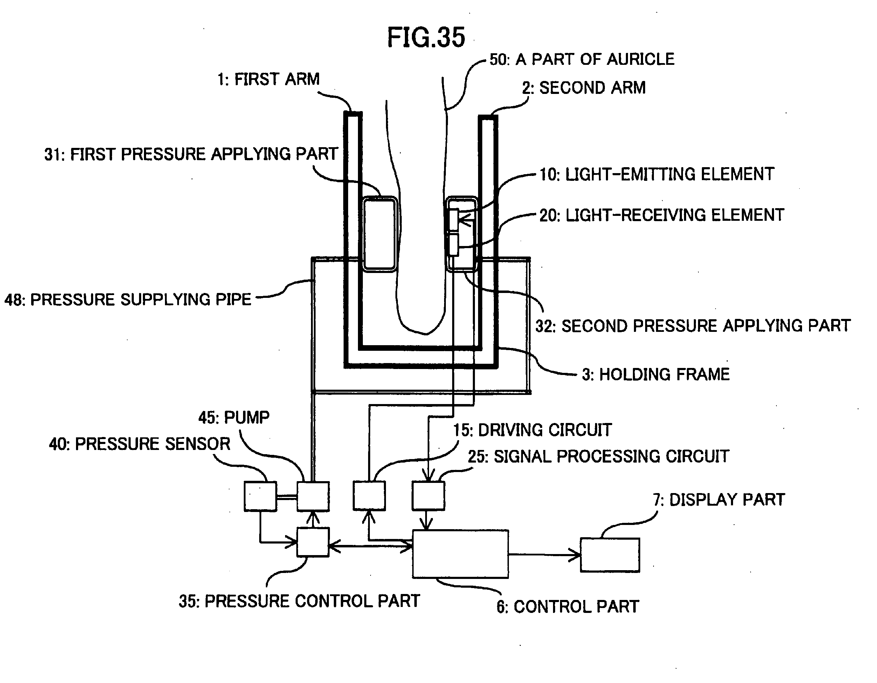

Next, the embodiment 2-6 of the present invention is described. Each of Figs.34 and 35 is a block diagram of a blood-pressure meter in the embodiment 2-4 of the present invention.

The blood-pressure meter of the embodiment 2-6 is formed by a holding frame part 3 for pinching a part 50 of an auricle using a pushing pressure between a first arm 1 and a second arm 2, a first pressure applying part 31 that is provided in the inside of the first arm 1 and that is pressure-variable, a second pressure applying part 32 that is provided in the inside of the second arm 2 and that is pressure-variable, a pair of light-emitting element 10 and a light-receiving element 20 that measures light reflectance and that is provided in the first pressure applying part 31 of the first arm 1 or in the second pressure applying part 32 of the second arm 2, a control part 6, a display part 7, a pressure sensor 40, a pressure control part 35, a pump 45, a driving circuit 15, and a signal processing circuit 25. The first pressure applying part 31 and the second pressure applying part 32 are connected to the pump 45 by a pressure supplying pipe 48. The pump 45 and the pressure sensor 40 is connected by a pipe. The light-emitting element 10 and the driving circuit 15, and the light receiving element 20 and the signal processing circuit 25 are respectively connected by a signal line. The control part 6 is connected to each of the pressure control part 35, the driving circuit 15, the signal processing circuit 25 and the display part 7 by a signal line. The pressure control part 35 is connected to each of the pressure sensor 40 and the pump 45 by a signal line. The first pressure applying part 31 and the second pressure applying part 32 are placed so as to pinch the part 50 of the auricle. The pair of the light-emitting element 10 and the light-receiving element 20 is placed in the inside of the first pressure applying part 31 or in the inside of the second pressure applying part 32. In Fig.34, although the pair of the light-emitting element 10 and the light-receiving element 20 is placed in the inside of the first pressure applying part 31, the pair of the light-emitting element 10 and the light-receiving element 20 may be placed in the inside of the second pressure applying part 32 as shown in Fig.35. The light-emitting element 10 and the light-receiving element 20 are placed adjacent to each other such that a light-emitting surface of the light emitting element 10 and a light-receiving surface of the light-receiving element 20 are directed to the inside of the first arm 1 or the second arm 2. That is, they are placed such that, when the emitted light of the light-emitting element 10 is reflected from an outside part, the reflected light can be received by the light-receiving element 2.

Next, operation of the blood-pressure meter of this embodiment is described. As to Fig.34 and Fig.35, only the placement positions of the pair of the light-emitting element 10 and the light-receiving element 20 are different, and the operations are the same. Therefore, explanations are given with reference to Fig.34.

The control part 6 has a function for performing controls of the whole blood-pressure meter such as measurement start or end of the blood-pressure meter. The control part 6 sends a signal to the pressure control part 35 so as to instruct the pressure control part 35 to drive the pump 45 to apply a pressure to the pressure applying parts 31 and 32. The pressure control part 35 sends a signal to the pump 45 so as to instruct the pump 45 to supply a pressure, instructed by the control part 6, to the pressure applying parts 31 and 32 via the pressure supplying pipe 48. The pressure sensor 40 measures the pressure supplied by the pump 45 to the pressure applying parts 31 and 32 via the pressure supplying pipe 48, and transmits the measured result to the pressure controlling part 35 by the signal line. The pressure control part 35 controls the pump 45 such that the pressure supplied by the pump 45 that is measured by the pressure sensor 40 is the same as the pressure instructed by the control part 6. On the other hand, the control part 6 sends a signal to the driving circuit 15 to instruct the driving circuit to cause the light-emitting element 10 to illuminate. The driving circuit 15 receives this signal, drives the light-emitting element 10. The light-emitting element 10 emits laser light and the like to a part 50 of the auricle. The emitted light is reflected from the surface or inside blood vessel of the part 50 of the ear, and the light-receiving element 20 receives the reflected light. The light-receiving element 20 converts the reflected light into an electrical signal and sends the signal to the signal processing circuit 25 via the signal line. The signal processing circuit 25 stores relationship between pulsation waveform and blood pressure. The signal processing circuit 25 processes the electrical signal corresponding to the waveform of the reflected light received by the light-receiving element 20, and sends the result to the control part 6. The control part 6 displays the measurement result on the display part 7.

The blood-pressure meter measures a blood pressure in the following way. The light-emitting element 10 emits a light beam such as a laser light beam to the part 50 of the auricle. When the emitted light is reflected from the surface or inside blood vessel and the like of the part 50 of the auricle, the reflected light receives change of the light amount or change of frequency corresponding to pulsation of the part 50 of the auricle that repeats expand and contraction due to pulsation of a blood vessel. The light-receiving element 20 measures the pulsation waveform based on the change of the amount of the reflected light or the change of frequency, converts the pulsation waveform to the electrical signal and sends the signal to the signal processing circuit 25. The signal processing circuit 25 compares the pulsation waveform measured by the light-receiving element 20 with pulsation waveforms that are stored beforehand to determine which level the blood pressure at this time corresponds to between the maximum blood pressure and the minimum blood pressure, and sends the result to the control part 6. The control part 6 displays a value of the blood pressure at this time and the level of the blood pressure between the maximum blood pressure and the minimum blood pressure on the display part 7 based on the result received from the signal processing circuit 25 and the pressure measured by the pressure sensor 40 at the same time. According to the above-mentioned operation, the blood-pressure meter of this embodiment measures the blood pressure. Further, by changing the pressure applied by the pressure applying part 30 via the pressure control part 35 by operating the control part 6, a blood pressure corresponding to any level between the maximum blood pressure and the minimum blood pressure can be measured.

Another configuration can be adopted by removing the signal processing circuit 25 from the blood-pressure meter shown in Fig.34 or Fig.35. In this case, a pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship between pulsation waveform and blood pressure being prepared separately to the blood-pressure meter beforehand. Based on the pressure measured by the pressure sensor 40, the control part 6 displays the blood pressure at this time on the display part 7. Accordingly, the blood pressure can be measured. Further, by changing the pressure applied by the first pressure applying part 31 and the second pressure applying part 32 via the pressure control part 35 by operating the control part 6, a blood pressure corresponding to any level between the maximum blood pressure and the minimum blood pressure can be measured.

Further, a configuration obtained by removing the pressure control part 35, the pressure sensor 40, the pump 45, the driving circuit 15, the signal processing circuit 25, the control part 6 and the display part 7 from the blood-pressure meter of Fig.34 or Fig.35 can be adopted.

In this blood-pressure meter, a pressure is supplied to the pressure applying part 30 by a pump and the like that exists in the outside of the blood-pressure meter, and power and a driving signal are supplied to the light-emitting element 10 from the outside. In addition, the pulsation waveform measured by the light-receiving element 20 is observed by connecting an oscilloscope, for example, to the light-receiving element 20, so that an external apparatus or a human determines which level the pulsation waveform corresponds to between the maximum blood pressure and the minimum blood pressure based on data indicating relationship among pulsation waveform, the amplitude value and blood pressure being prepared separately to the blood-pressure meter beforehand. By changing the pressure applied by the first pressure applying part 31 and the second pressure applying part 32 using an external pump and the like, a blood pressure corresponding to any level between the maximum blood pressure and the minimum blood pressure can be measured.

### [Embodiment 2-7]

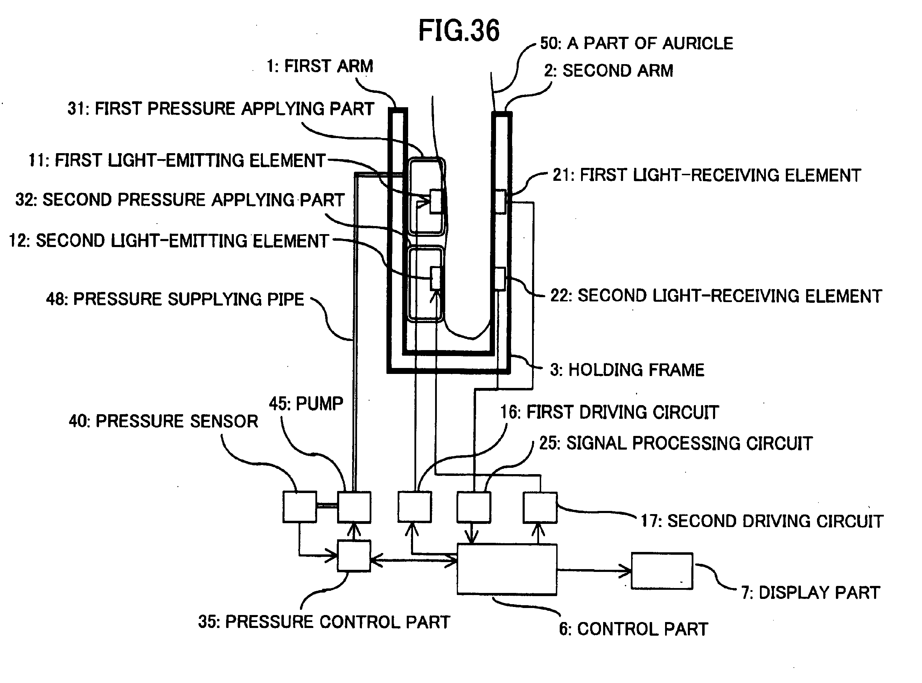

Next, the embodiment 2-7 of the present invention is described with reference to Fig.36.

The blood-pressure meter of a first example of the embodiment 2-7 is formed by a holding frame part 3 for pinching a part 50 of an auricle using a pushing pressure between a first arm 1 and a second arm 2, a first pressure applying part 31 that is provided in the inside of the first arm 1 and that is pressure-variable, a second pressure applying part 32.that is provided in the inside of the second arm 1 and that is pressure-variable, a first pair of light-emitting element 11 and a light-receiving element 21 for measuring light transmittance between the first pressure applying part 31 and the second arm 2, and a second pair of light-emitting element 12 and a light-receiving element 22 for measuring light transmittance between the second pressure applying part 32 and the second arm 2. The first pressure applying part 31 and the second pressure applying part 32 that are provided in the inside of the first arm 1 and the second arm 2 are placed such that they pinch the part 50 of the auricle. One of the light-emitting element 11 and the light-receiving element 21 of the first pair is placed in the first pressure applying part 31, and another is placed in the second arm 2. In addition, one of the light-emitting element 12 and the light-receiving element 22 of the second pair is placed in the second pressure applying part 32, and another is placed in the second arm 2. The first light-emitting element 11 and the first light-receiving element 21 are placed on a line such that they are opposed to each other, and the second light-emitting element 12 and the second light-receiving element 22 are placed on a line such that they are opposed to each other. That is, they are placed such that emitted light of each of the first light-emitting element 11 and the second light-emitting element 12 can be received by each of the first light-receiving element 21 and the second light-receiving element 22.

Operation of the blood-pressure meter is described. Different pressures are applied to the first pressure applying part 31 and the second pressure applying part 32 from the outside using a pump and the like. In this example, the pressure of the second pressure applying part 32 is set to be a very small pressure. Power is supplied to the first light-emitting element 11, the second light-emitting element 12, the first light-receiving element 21 and the second light-receiving element 22 from the outside. A driving signal is supplied from the outside to cause each of the first light-emitting element 11 and the second light-receiving element 12 to illuminate. A pressure sensor for measuring the applied pressure is attached to the first pressure applying part 31. Each of the first light-emitting element 11 and the second light-emitting element 12 emits a light beam such as a laser light beam to the part 50 of the auricle. Each emitted light passes through the part 50 of the auricle, and is received by each of the first light-receiving element 21 and the second light-receiving element 22. When the emitted light passes through the inside of the part 50 of the auricle, the emitted light receives change of attenuation or frequency corresponding to pulsation of the part of the auricle that repeats expand and contraction due to pulsation of a blood vessel. Each of the first light-receiving element 21 and the second light-receiving element 22 measures the pulsation waveform based on the change of the amount of the transmitted light or the change of frequency, converts the pulsation waveform to the electrical signal. Then, an oscilloscope and the like is connected to each of the first light-receiving element 21 and the second light-receiving element 22, for example, so as to measure a time difference between a rising point of a pulsation waveform measured by the first light-receiving element 21 and a rising point of a pulsation waveform measured by the second light-receiving element 22. Since the very small pressure is applied to the second pressure applying part 32, the pulsation waveform measured by the second light-receiving element 22 corresponds to the minimum blood pressure.

According to the principle 2 of the blood pressure measurement, based on the time difference between the rising point of the pulsation waveform measured by the first light-receiving element 21 and the rising point of the pulsation waveform measured by the second light-receiving element 22, it can be determined which level the pulsation waveform measured by the first light-receiving element 21 corresponds to between the maximum blood pressure and the minimum blood pressure with respect to the minimum blood pressure. In addition, at the same time, by measuring the pressure applied by the first pressure applying part 31 using a pressure sensor, a value of the blood pressure at the time can be measured. By changing the applying pressure of the first pressure applying part 31, a blood pressure of any level between the maximum blood pressure and the minimum blood pressure can be measured.

As shown in Fig.36, a configuration obtained by adding a control part 6, a display part 7, a pressure sensor 40, a pressure control part 35, a pump 45, a first driving circuit 16 and a second driving circuit 17 to the above-mentioned blood-pressure meter can be adopted.

In this case, the first pressure applying part 31 and the pump 45 are connected by a pressure supplying pipe 48. The pump 45 and the pressure sensor 40 is connected by a pipe. The first light-emitting element 11 and the first driving circuit 16 are connected by a signal line, and the second light-emitting element 12 and the second driving circuit 17 are connected by a signal line. The control part 6 is connected to each of the pressure control part 35, the first driving circuit 16, the second driving circuit 17 and the display part 7 by a signal line. The pressure control part 35 is connected to each of the pump 45 and the pressure sensor 40 by a signal line.

The control part 6 has a function for performing controls of the whole blood-pressure meter such as measurement start or end of the blood-pressure meter. The control part 6 sends a signal to the pressure control part 35 so as to instruct the pressure control part 35 to drive the pump 45 to apply an arbitrary pressure to the first pressure applying part 31. The pressure control part 35 sends a signal to the pump 45 so as to instruct the pump 45 to supply a pressure, instructed by the control part 6, to the first pressure applying part 31 via the pressure supplying pipe 48. The pressure sensor 40 measures the pressure supplied by the pump 45 to the first pressure applying part 31 via the pressure supplying pipe 48, and transmits the measured result to the pressure controlling part 35 by the signal line. The pressure control part 35 controls the pump 45 such that the pressure supplied by the pump 45 that is measured by the pressure sensor 40 is the same as the pressure instructed by the control part 6. The very small pressure, for example, is applied to the second pressure applying part 32. On the other hand, the control part 6 sends a signal to each of the first driving circuit 16 and the second driving circuit 17 to instruct the driving circuits to cause the first light-emitting element 16 and the second light-emitting element 17 to illuminate. Each of the first driving circuit 16 and the second driving circuit 17 receives this signal, drives each of the first light-emitting element 11 and the second light-emitting element 12. Each of the first light-emitting element 11 and the second light-emitting element 12 emits laser light and the like to a part 50 of the auricle. The emitted light passes through the part 50 of the auricle, and each of the first light-receiving element 21 and the second light-receiving element 22 receives the transmitted light. Power is supplied to the first light-receiving element 21 and the second light-receiving element 22 from the outside. Each of the first light-receiving element 21 and the second light-receiving element 22 converts the received transmitted light into an electrical signal.

This blood-pressure meter measures the blood pressure in the following way. Each of the first light-emitting element 11 and the second light-emitting element 12 emits a light beam such as a laser light beam to the part 50 of the auricle. When the emitted light passes through the inside of the part 50 of the auricle, the emitted light receives change of attenuation or frequency corresponding to pulsation of the part of the auricle that repeats expand and contraction due to pulsation of a blood vessel. Each of the first light-receiving element 21 and the second light-receiving element 22 measures the pulsation waveform based on the change of the amount of the transmitted light or the change of frequency, converts the pulsation waveform to the electrical signal. Then, an oscilloscope and the like is connected to each of the first light-receiving element 21 and the second light-receiving element 22, for example, so as to measure a time difference between a rising point of a pulsation waveform measured by the first light-receiving element 21 and a rising point of a pulsation waveform measured by the second light-receiving element 22. Since the very small pressure is applied to the second pressure applying part 32, the pulsation waveform measured by the second light-receiving element 22 corresponds to the minimum blood pressure. According to the principle 2 of the blood pressure measurement, based on the time difference between the rising point of the pulsation waveform measured by the first light-receiving element 21 and the rising point of the pulsation waveform measured by the second light-receiving element 22, it can be determined which level the pulsation waveform measured by the first light-receiving element 21 corresponds to between the maximum blood pressure and the minimum blood pressure with respect to the minimum blood pressure. In addition, at the same time, by measuring the pressure applied by the first pressure applying part 31 using a pressure sensor, a value of the blood pressure at the time can be measured. By changing the applying pressure of the first pressure applying part 31, a blood pressure of any level between the maximum blood pressure and the minimum blood pressure can be measured. Accordingly, in the embodiment 2-7, the blood pressure can be measured more easily than the blood pressure meter described first.

As shown in Fig.36, a configuration obtained by further adding a signal processing circuit 25 to the above-mentioned configuration can be adopted. As a result of adding the signal processing circuit 25, signal lines connecting between the first light-receiving element 21 and the signal processing circuit 25 and between the second light-receiving element 22 and the signal processing circuit 25, and a signal line connecting between the control part 6 and the signal processing circuit 25 are added.

By adopting this configuration, the signal processing circuit 25 stores relationship between time differences and blood pressure levels with respect to a blood pressure, measures a time difference between a rising point of a pulsation waveform measured by the first light-receiving element 21 and a rising point of a pulsation waveform measured by the second light-receiving element 22, and determines the blood pressure level based on the time difference. Therefore, the blood pressure can be measured more easily.

### [Embodiment 2-8]

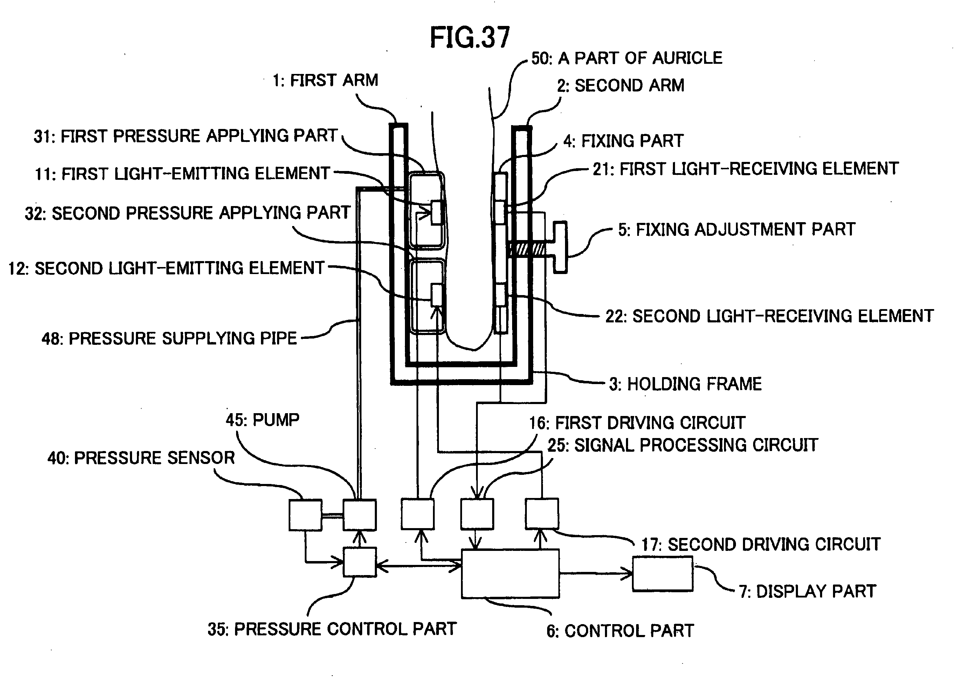

Next, the embodiment 2-8 of the present embodiment is described with reference to Fig. 37.

The blood-pressure meter of the embodiment 2-8 is one obtained by adding a fixing part 4 and a fixing adjustment part 5 to the second arm 2 of a blood-pressure meter shown in Fig.36 like the embodiment 2-2. By adding the fixing part 4 and the fixing adjustment part 5, when the blood-pressure meter is attached to the part 50 of the auricle, the fixing adjustment part 5 adjusts the interval of the pressure applying part 30 and the fixing part 4 according to individual variation of thickness of the part 50 of the auricle. Therefore, useless operation of the pump 45 can be eliminated so that there is a merit that the capacity of the pump 45 can be decreased, compared with the configuration of Fig.36.

### [Embodiment 2-9]

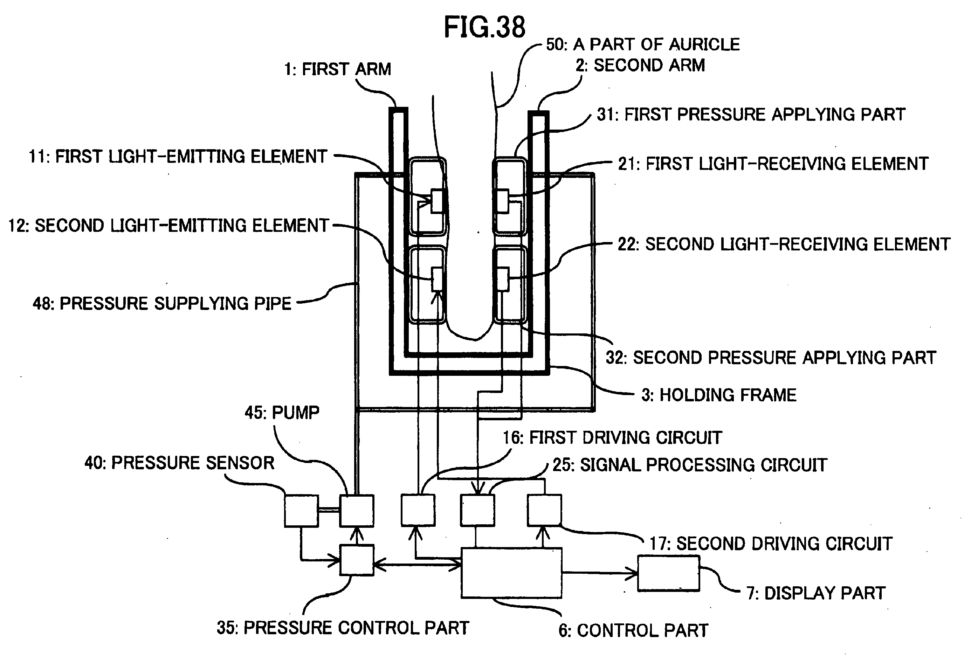

Next, the embodiment 2-9 of the present embodiment is described with reference to Fig.38.

The blood-pressure meter of the embodiment 2-9 is different from the configuration shown in Fig.36 in that the pressure applying part 31 is placed in the inside of each of the arm 1 and the arm 2. Each of the light-receiving element 21 and the light-emitting element 11 is provided in the inside of the pressure applying part 31. The pressure applying part 32, the light-receiving element 22 and the light-emitting element 12 are placed similarly. Other configuration and the measurement method of blood pressure are the same as those of the blood-pressure meter of the embodiment 2-7.

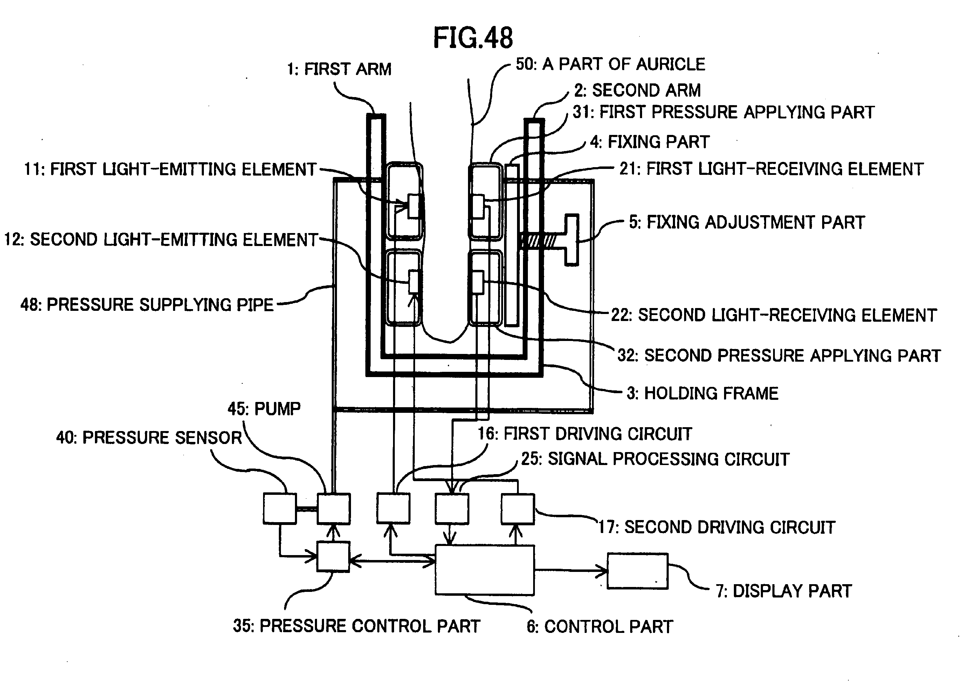

Further, as shown in Fig.48, a configuration obtained by adding the fixing part 4 and the fixing adjustment part 5 to the configuration of Fig.38 can be applied.

### [Embodiment 2-10]

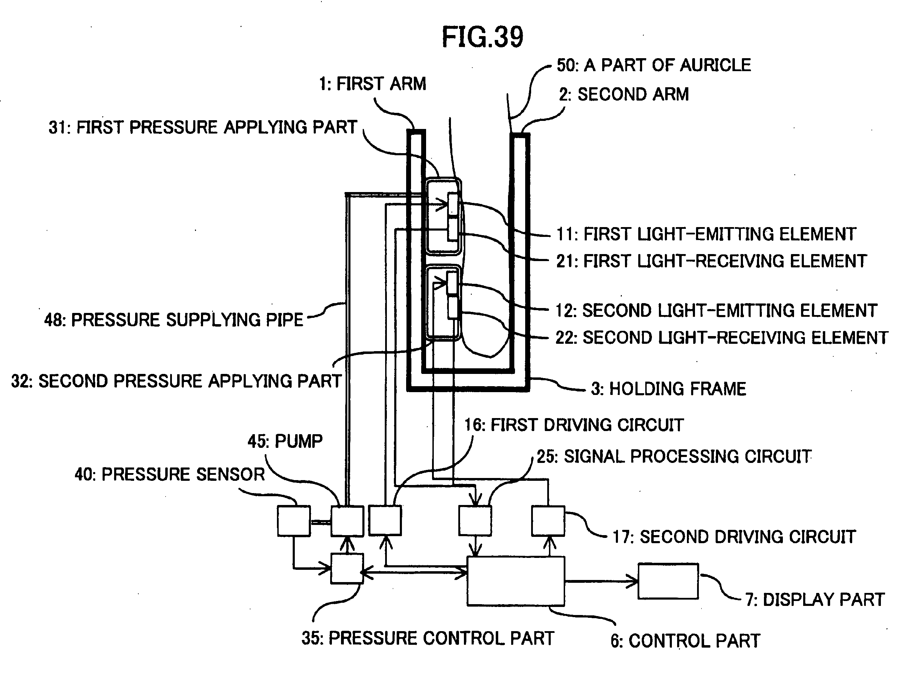

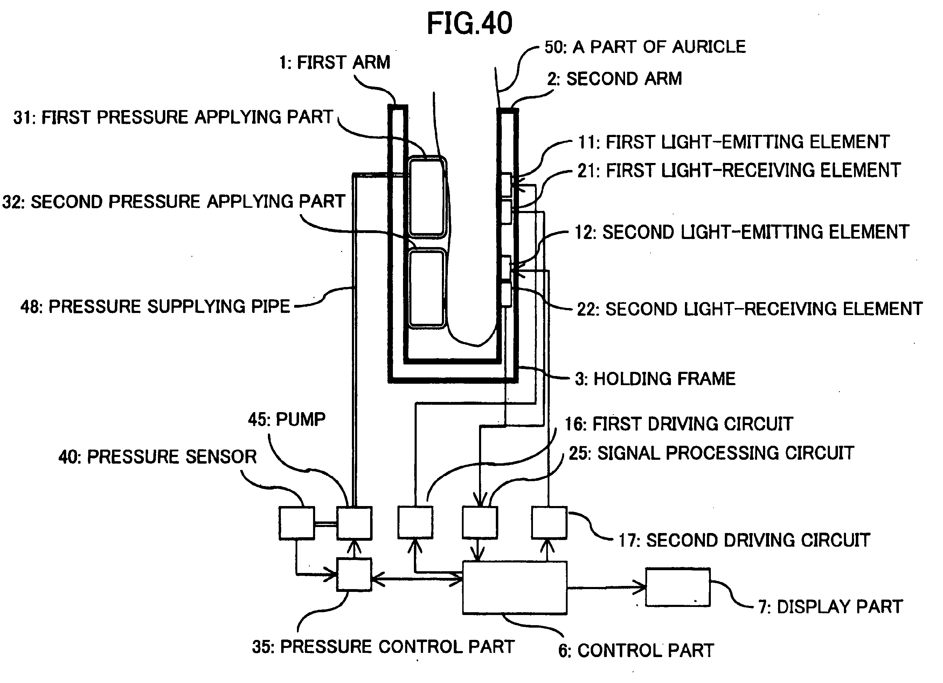

Next, the embodiment 2-10 of the present invention is described with reference to Fig.39 and Fig.40.

The blood-pressure meter of a first example of the embodiment 2-10 is formed by a holding frame part 3 for pinching a part of an auricle using a pushing pressure between a first arm 1 and a second arm 2, a first pressure applying part 31 that is provided in the inside of the first arm 1 and that is pressure-variable, a second pressure applying part 32 that is provided in the inside of the first arm 1 and that is pressure-variable, a first pair of light-emitting element 11 and a light-receiving element 21 for measuring light reflectance provided in the first pressure applying part 31 or the second arm 2, and a second pair of light-emitting element 12 and a light-receiving element 22 for measuring light reflectance provided in the second pressure applying part 32 or the second arm 2. The first pressure-variable pressure applying part 31 and the second pressure applying part 32 that are provided in the inside of the first arm 1, and the second arm 2 are placed such that they pinch the part 50 of the auricle. Each of the first pair of the light-emitting element 11 and the light-receiving element 21, and the second pair of the light-emitting element 12 and the light-receiving element 22 is placed in the first pressure applying part 31 or the second pressure applying part 32 respectively, or in the inside of the second arm 2. In Fig.39, although the first pair of the first light-emitting element 11 and the first light-receiving element 21 is placed in the first pressure applying part 31, and the second pair of the second light-emitting element 12 and the second light-receiving element 22 is placed in the second pressure applying part 32, each of the first pair of the first light-emitting element 11 and the first light-receiving element 21, and the second pair of the second light-emitting element 12 and the second light-receiving element 22 can be placed in the inside of the second arm 2 as shown in Fig.40. The first light-emitting element 11 and the first light-receiving element 21 are placed adjacent to each other, and the second light-emitting element 12 and the second light-receiving element 22 are placed adjacent to each other such that, the light-emitting surfaces of the first light-emitting element 11 and the second light-emitting element 12, and the light-receiving surfaces of the first light receiving element 21 and the second light-receiving element 22 are directed to the inside of the first arm 1 or the second arm 2, so that, when emitted lights emitted from the first light-emitting element 11 and the second light-emitting element 12 are reflected by an outside part, the reflected lights are received by the first light-receiving element 21 and the second light-receiving element 22 respectively.

Operation of the blood-pressure meter is described. Different pressures are applied to the first pressure applying part 31 and the second pressure applying part 32 from the outside using a pump and the like. In this example, the pressure of the second pressure applying part 32 is set to be a very small pressure. Power is supplied to the first light-emitting element 11, the second light-emitting element 12, the first light-receiving element 21 and the second light-receiving element 22 from the outside. A driving signal is supplied from the outside to cause each of the first light-emitting element 11 and the second light-emitting element 12 to illuminate. A pressure sensor for measuring the applied pressure of the second pressure applying part 32 is attached to the second pressure applying part 32. Each of the first light-emitting element 11 and the second light-emitting element 12 emits a light beam such as a laser light beam to the part 50 of the auricle. Each emitted light is reflected from a blood vessel and the like in the surface or in the inside of the part 50 of the auricle, the reflected light receives change of attenuation of light or frequency corresponding to pulsation of the part 50 of the auricle that repeats expand and contraction due to pulsation of a blood vessel. Each of the first light-receiving element 21 and the second light-receiving element 22 measures a pulsation waveform based on the change of the amount of the reflected light or the change of frequency, converts the pulsation waveform to the electrical signal. Then, an oscilloscope and the like is connected to each of the first light-receiving element 21 and the second light-receiving element 22, for example, so as to measure a time difference between a rising point of a pulsation waveform measured by the first light-receiving element 21 and a rising point of a pulsation waveform measured by the second light-receiving element 22. Since the very small pressure is applied to the second pressure applying part 32, the pulsation waveform measured by the second light-receiving element 22 corresponds to the minimum blood pressure. According to the principle 2 of the blood pressure measurement, based on the time difference between the rising point of the pulsation waveform measured by the first light-receiving element 21 and the rising point of the pulsation waveform measured by the second light-receiving element 22, it can be determined which level the pulsation waveform measured by the first light-receiving element 21 corresponds to between the maximum blood pressure and the minimum blood pressure with respect to the minimum blood pressure. In addition, at the same time, by measuring the pressure applied by the first pressure applying part 31 using a pressure sensor, a value of the blood pressure at the time can be measured. By changing the applying pressure of the first pressure applying part 31, a blood pressure of any level between the maximum blood pressure and the minimum blood pressure can be measured.

A configuration obtained by adding a control part 6, a display part 7, a pressure sensor 40, a pressure control part 35, a pump 45, a first driving circuit 16 and a second driving circuit 17 to the above-mentioned blood-pressure meter can be also adopted. In this case, the first pressure applying part 31 and the pump 45 are connected by a pressure supplying pipe 48. The pump 45 and the pressure sensor 40 is connected by a pipe. The first light-emitting element 11 and the first driving circuit 16 are connected by a signal line, and the second light-emitting element 12 and the second driving circuit 17 are connected by a signal line. The control part 6 is connected to each of the pressure control part 35, the first driving circuit 16, the second driving circuit 17 and the display part 7 by a signal line. The pressure control part 35 is connected to each of the pump 45 and the pressure sensor 40 by a signal line. The control part 6 has a function for performing controls of the whole blood-pressure meter such as measurement start or end of the blood-pressure meter. The control part 6 sends a signal to the pressure control part 35 so as to instruct the pressure control part 35 to drive the pump 45 to apply an arbitrary pressure to the first pressure applying part 31. The pressure control part 35 sends a signal to the pump 45 so as to instruct the pump 45 to supply a pressure, instructed by the control part 6, to the first pressure applying part 31 via the pressure supplying pipe 48. The pressure sensor 40 measures the pressure supplied by the pump 45 to the first pressure applying part 31 via the pressure supplying pipe 48, and transmits the measured result to the pressure controlling part 35 by the signal line. The pressure control part 35 controls the pump 45 such that the pressure supplied by the pump 45 that is measured by the pressure sensor 40 is the same as the pressure instructed by the control part 6. The very small pressure, for example, is applied to the second pressure applying part 32. On the other hand, the control part 6 sends a signal to each of the first driving circuit 16 and the second driving circuit 17 to instruct the driving circuits to cause the first light-emitting element 11 and the second light-emitting element 12 to illuminate. Each of the first driving circuit 16 and the second driving circuit 17 receives this signal, drives each of the first light-emitting element 11 and the second light-emitting element 12. Each of the first light-emitting element 11 and the second light-emitting element 12 emits laser light and the like to a part 50 of the auricle. Each emitted light is reflected from the surface or inside blood vessel of the part 50 of the auricle, the reflected light receives change of attenuation of light or frequency corresponding to pulsation of the part 50 of the auricle that repeats expand and contraction due to pulsation of a blood vessel. Each of the first light-receiving element 21 and the second light-receiving element 22 measures a pulsation waveform based on the change of the amount of the reflected light or the change of frequency, converts the pulsation waveform to the electrical signal. Then, an oscilloscope and the like is connected to each of the first light-receiving element 21 and the second light-receiving element 22, for example, so as to measure a time difference between a rising point of a pulsation waveform measured by the first light-receiving element 21 and a rising point of a pulsation waveform measured by the second light-receiving element 22. Since the very small pressure is applied to the second pressure applying part 32, the pulsation waveform measured by the second light-receiving element 22 corresponds to the minimum blood pressure. According to the principle 2 of the blood pressure measurement, based on the time difference between the rising point of the pulsation waveform measured by the first light-receiving element 21 and the rising point of the pulsation waveform measured by the second light-receiving element 22, it can be determined which level the pulsation waveform measured by the first light-receiving element 21 corresponds to between the maximum blood pressure and the minimum blood pressure with respect to the minimum blood pressure. In addition, at the same time, by measuring the pressure applied by the first pressure applying part 31 using a pressure sensor, a value of the blood pressure at the time can be measured. By changing the applying pressure of the first pressure applying part 31, a blood pressure of any level between the maximum blood pressure and the minimum blood pressure can be measured.

A configuration obtained by further adding a signal processing circuit 25 to the above-mentioned configuration can be adopted. As a result of adding the signal processing circuit 25, signal lines connecting between the first light-receiving element 21 and the signal processing circuit 25 and between the second light-receiving element 22 and the signal processing circuit 25, and a signal line connecting between the control part 6 and the signal processing circuit 25 are added.

By adopting this configuration, the signal processing circuit 25 stores relationship between time differences and blood pressure levels with respect to a blood pressure, measures a time difference between a rising point of a pulsation waveform measured by the first light-receiving element 21 and a rising point of a pulsation waveform measured by the second light-receiving element 22, and determines the blood pressure level based on the time difference. Therefore, the blood pressure can be measured more easily.

### [Embodiment 2-11]

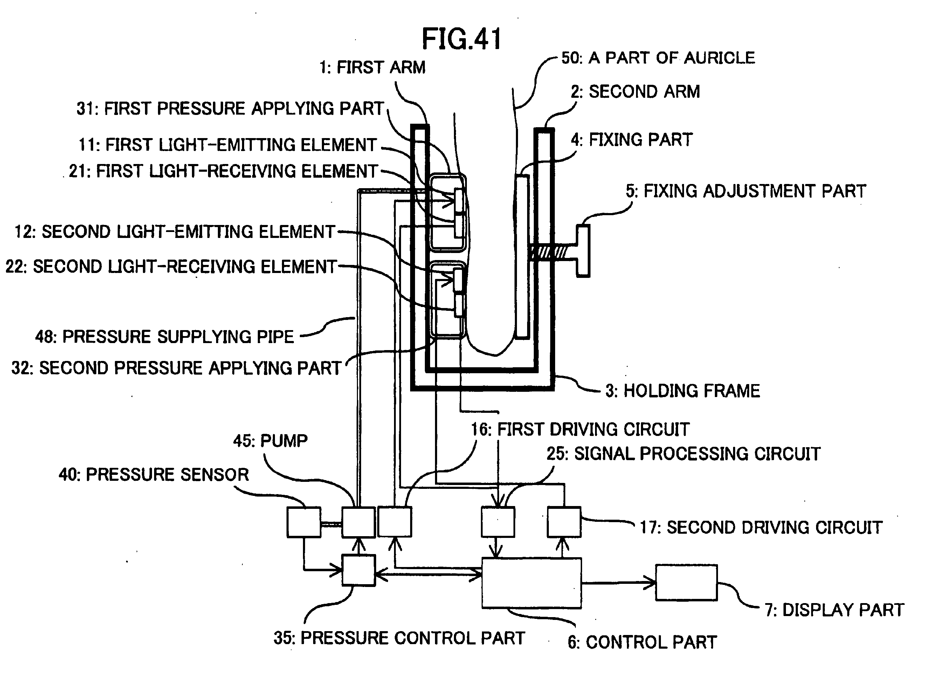

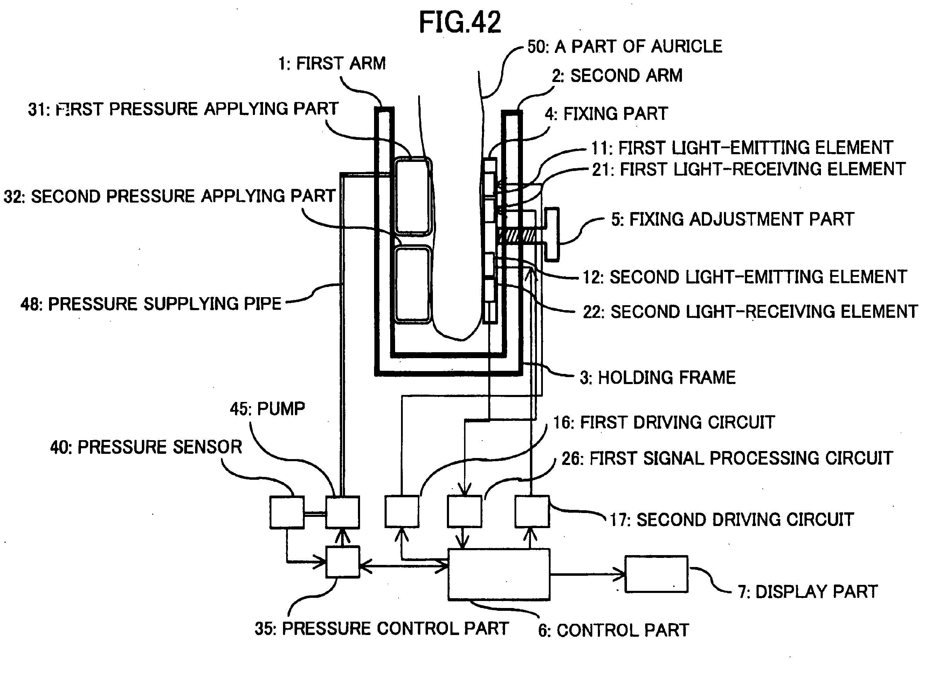

Next, the embodiment 2-11 of the present invention is described with reference to Fig.41 and 42.

The blood-pressure meter of the embodiment 2-11 is one obtained by adding a fixing part 4 and a fixing adjustment part 5 to the blood-pressure meter of the embodiment 2-10. Since the fixing adjustment part 5 adjusts the interval of the pressure applying parts 31 and 32, and the fixing part 4 according to individual variation of thickness of the part 50 of the auricle. Therefore, useless operation of the pump 45 can be eliminated so that there is a merit that the capacity of the pump 45 can be decreased, compared with the configurations of Fig.39 and Fig.40.

### [Embodiment 2-12]

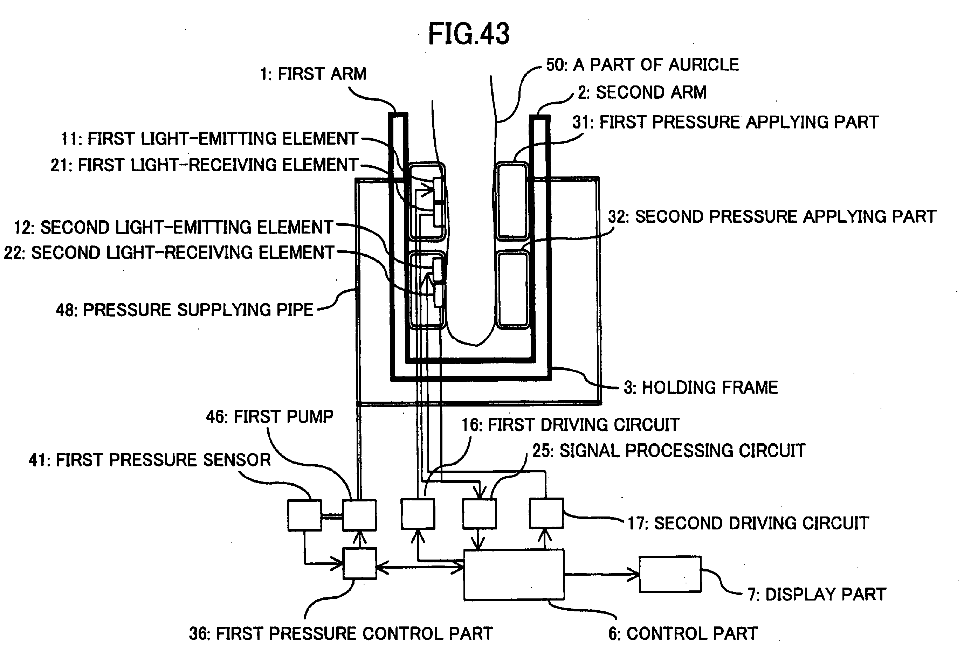

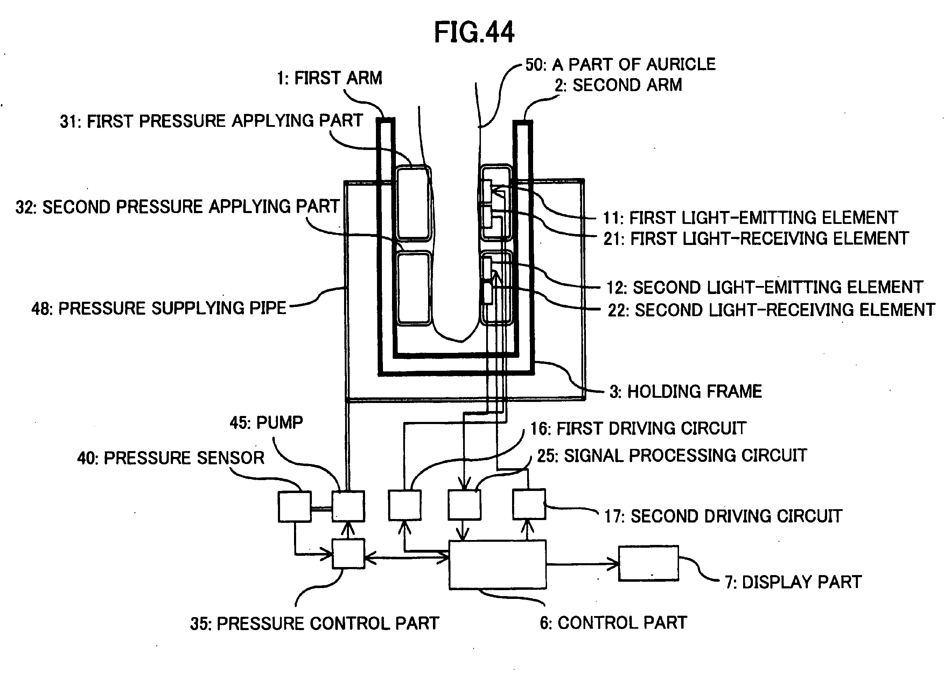

Next, the embodiment 2-12 of the present invention is described with reference to Fig.43 and Fig.44.

The blood-pressure meter of the embodiment 2-12 is different from the configuration shown in Figs.39 and 40 in that the pressure applying part 31 is placed in the inside of each of the arm 1 and the arm 2. Each of the light-receiving element 21 and the light-emitting element 11 is provided in the inside of the pressure applying part 31. The pressure applying part 32, the light-receiving element 22 and the light-emitting element 12 are placed similarly. Other configuration and the measurement method of blood pressure are the same as those of the blood-pressure meter of the embodiment 2-10.

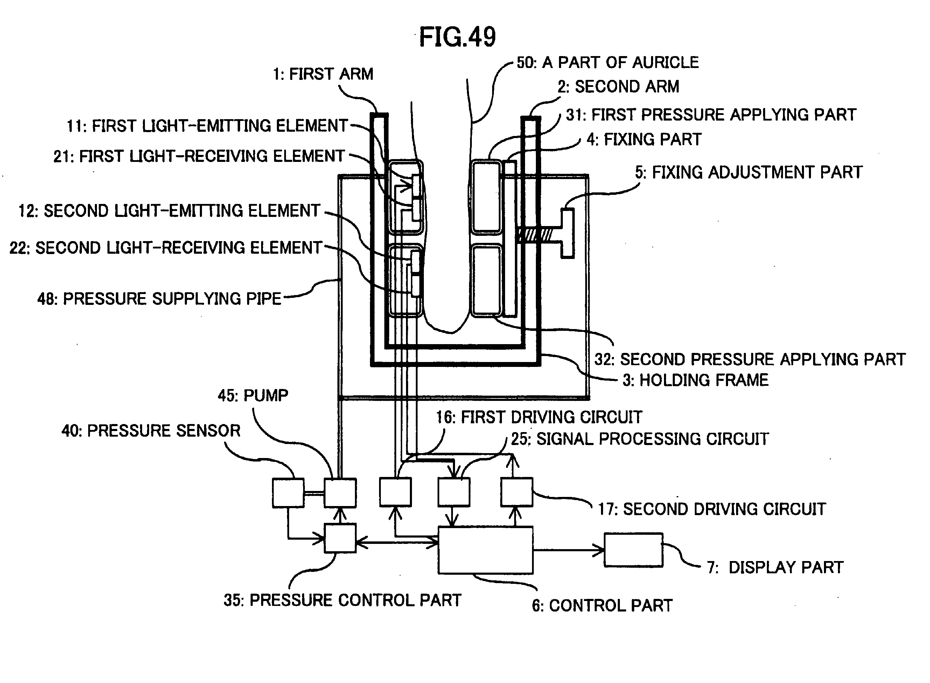

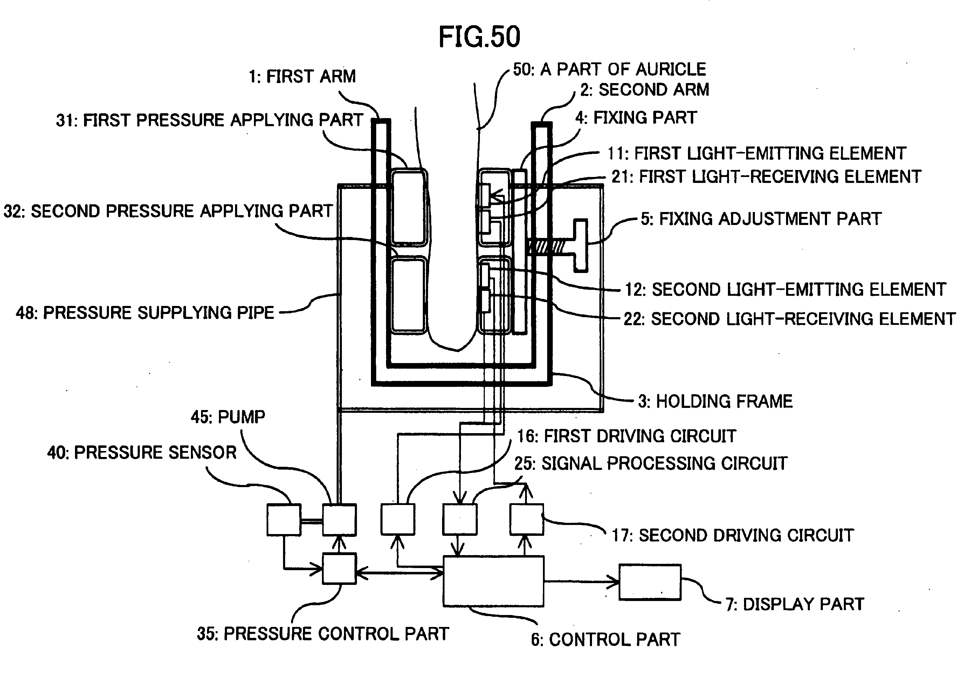

Further, as shown in Fig.49 and Fig.50, a configuration obtained by adding the fixing part 4 and the fixing adjustment part 5 can be applied.

### [Embodiment 2-13]

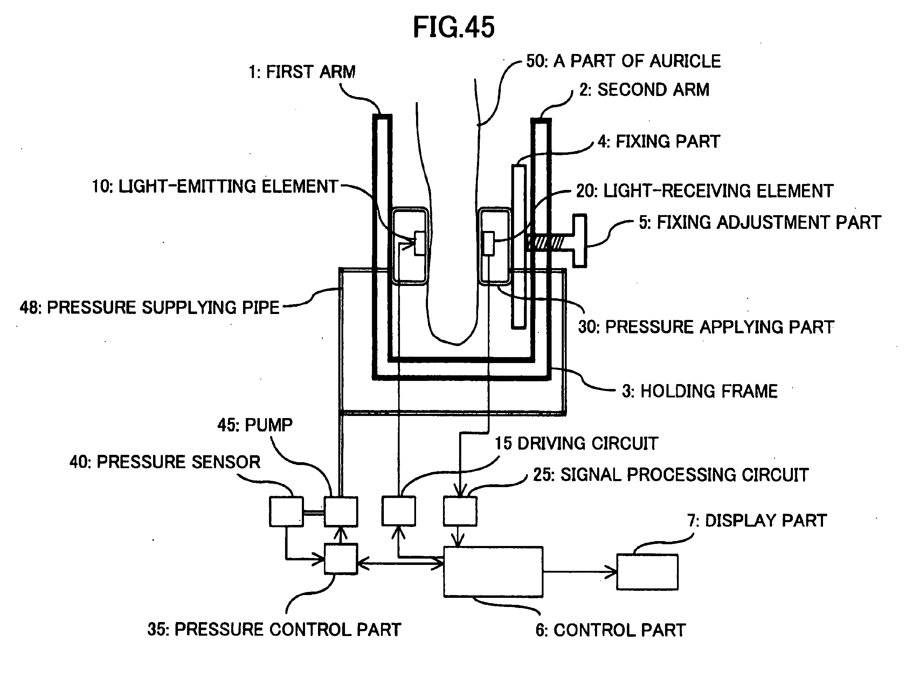

Next, the blood-pressure meter of the embodiment 2-13 of the present invention is described with reference to Figs.45, 46 and 47.

The blood-pressure meter shown in Fig.45 is one obtained by adding the fixing part 4 and the fixing adjustment part 5 between the second arm 2 and the second pressure applying part 32 in the blood-pressure meter of the embodiment 2-3 shown in Fig.29. Configurations other than the addition of the fixing part 4 and the fixing adjustment part 5, and the measurement method for a blood pressure are the same as those of the blood-pressure meter of the embodiment 2-3.

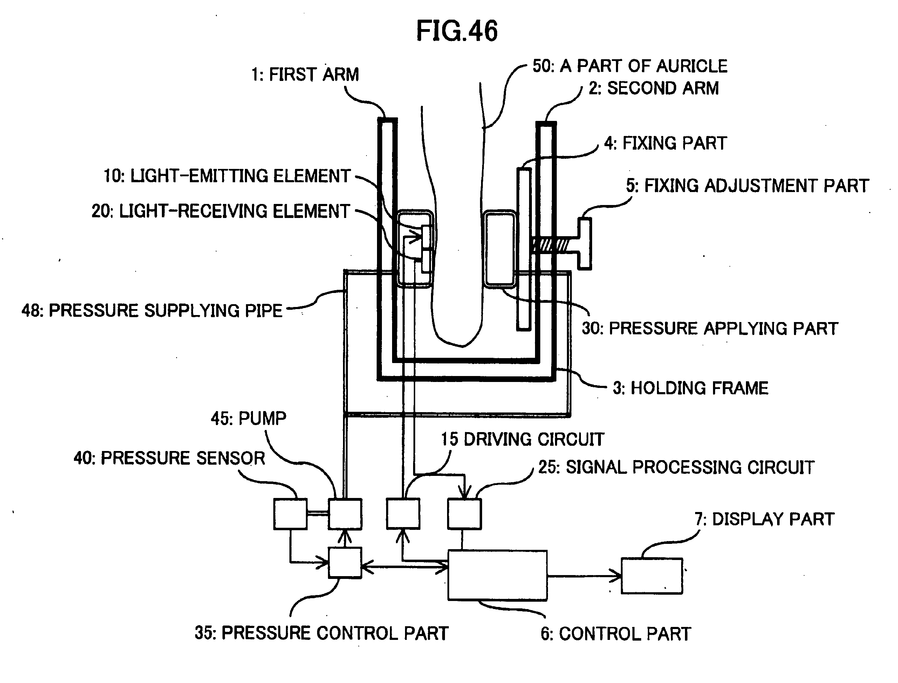

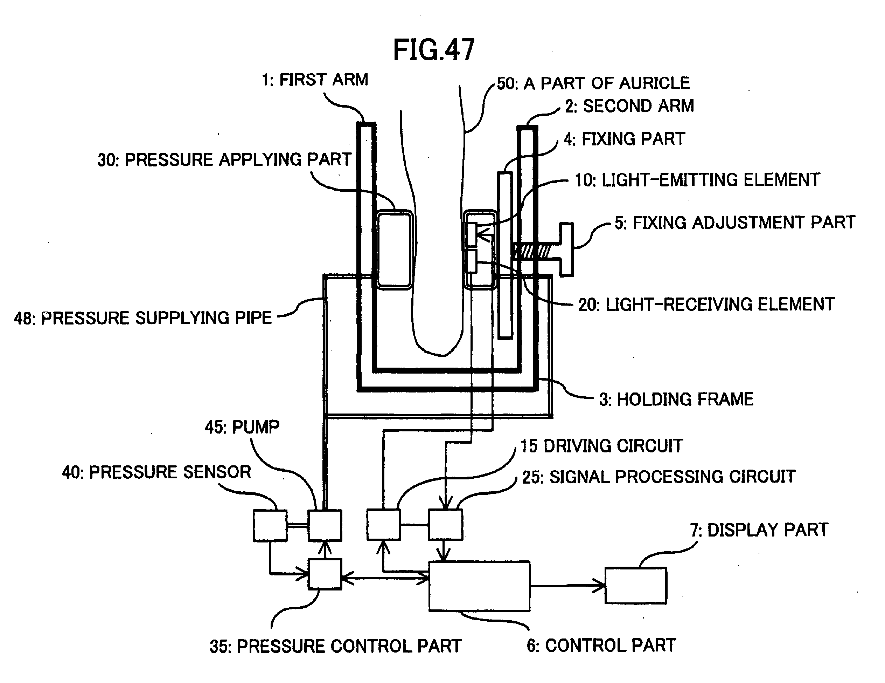

The blood-pressure meter shown in Figs.46 and 47 is one obtained by adding the fixing part 4 and the fixing adjustment part 5 between the second arm 2 and the second pressure applying part 32 in the blood-pressure meter of the embodiment 2-3 shown in Figs.34 and 35 respectively. Configurations other than the addition of the fixing part 4 and the fixing adjustment part 5, and the measurement method for a blood pressure are the same as those of the blood-pressure meter of the embodiment 2-6.

### [Embodiment 2-14 about fixing adjustment part 1]

The fixing adjustment part 5 of the blood-pressure meter in the embodiments described so far includes a screw mechanism for pushing the fixing part to the part 50 of the auricle as shown in Fig.28, for example. That is, in Fig.28, the fixing adjustment part 5 is attached to the second arm 2 by the screw mechanism. Therefore, by rotating the part outside of the second arm 2 of the fixing adjustment part 5, the fixing adjustment part 5 moves the fixing part 4 to a direction for pushing the fixing part 4 to the part 50 of the auricle or moves the fixing part 4 to a direction for separating the fixing part 4 from the part 50 of the auricle. According to the screw mechanism, the individual difference of the thickness of the part 50 of the auricle can be adjusted and the range of movement of the pressure applying part can be minimized. Therefore, the capacity of the pump for supplying the pressure to the pressure applying part can be decreased.

### [Embodiment 2-15 about fixing adjustment part 2]

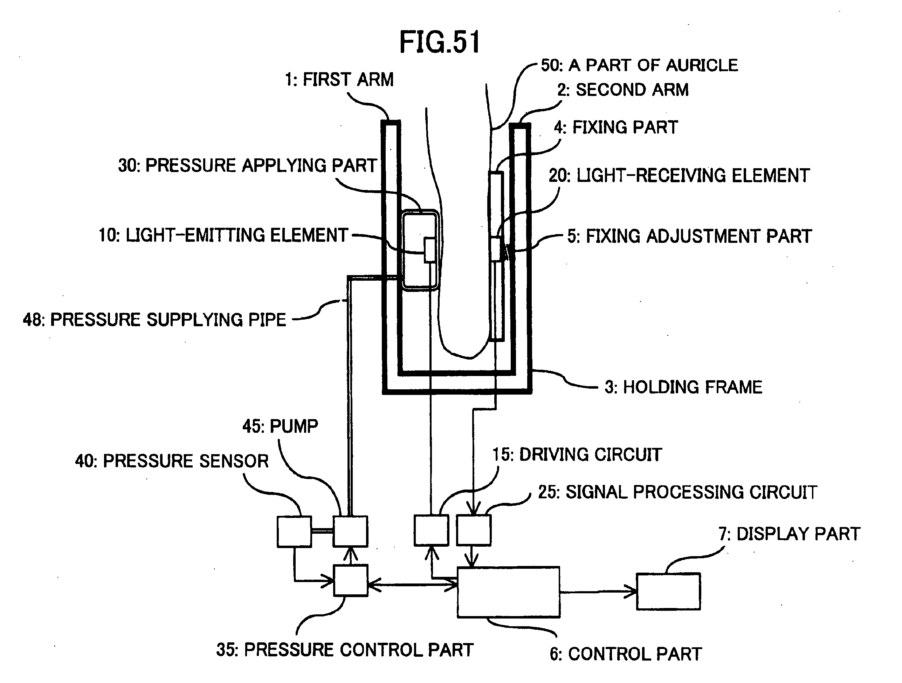

In addition, as shown in Fig.51, the fixing adjustment part 5 can be configured to include a spring fixing mechanism for pushing the fixing part to the part of the auricle. Accordingly, by pushing the fixing part 4 to the part of the auricle by the spring, the blood-pressure meter can be easily put on and taken off from the part 50 of the auricle, and, the individual difference of the thickness of the part 50 of the auricle can be adjusted and the range of movement of the pressure applying part can be minimized. Therefore, the capacity of the pump for supplying the pressure to the pressure applying part can be decreased.

### [Embodiment 2-16]

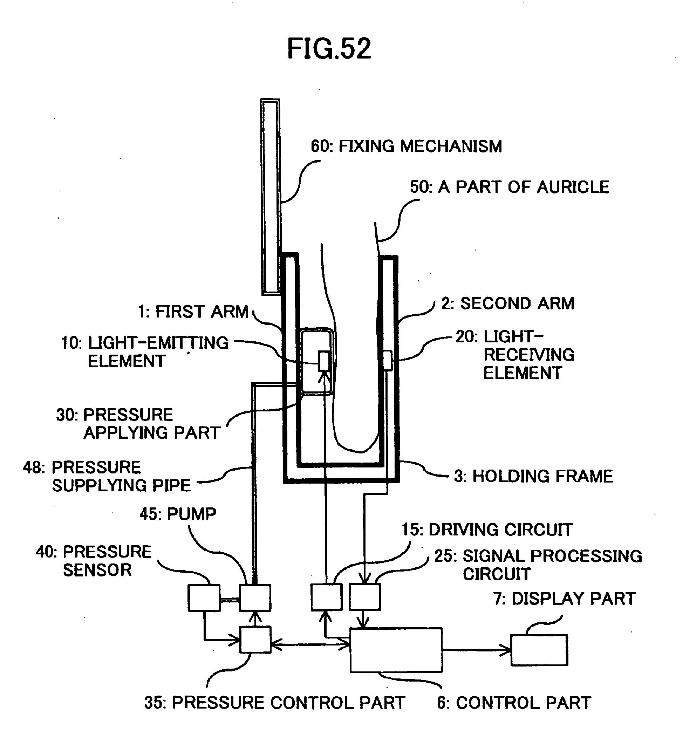

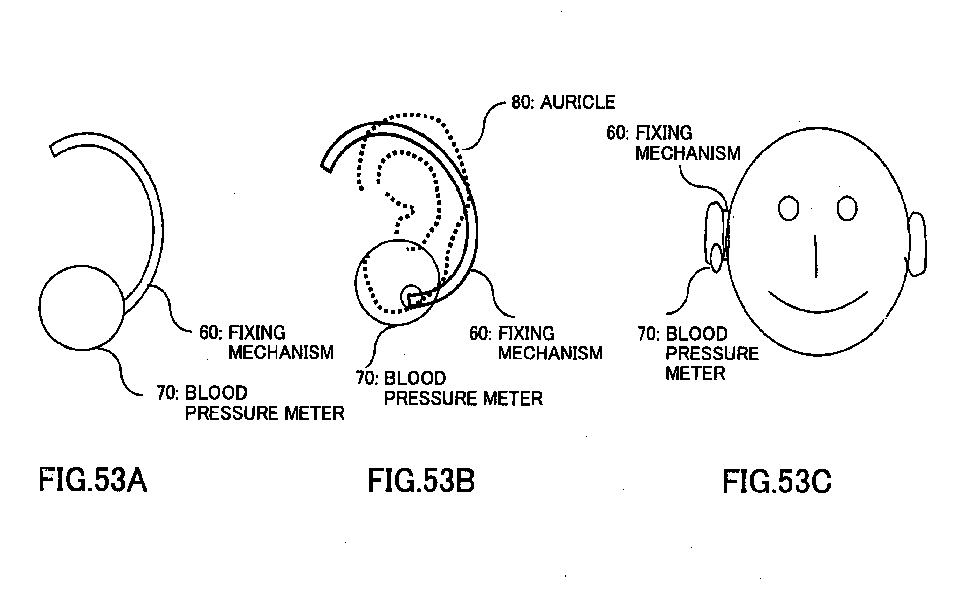

Next, the embodiment 2-16 is described with reference to Figs.52 and 53. The holding frame part 3 of the blood-pressure meter of this embodiment is suspended from a fixing mechanism 60 of a semiellipse shape in which both ends are incurved so as to be worn to the base of the auricle. This mechanism can be applied to all holding frame parts 3 of the blood-pressure meters described so far. Thus, the blood-pressure meter of the embodiment 2-1 is taken as an example for describing this embodiment.

Fig.52 shows an example in which the above-mentioned mechanism is applied to the blood-pressure meter of the embodiment 2-1, and shows a side view of the blood-pressure meter in which the fixing mechanism 60 is attached to the holding frame part 3. Fig.53 shows a state in which the blood-pressure meter is attached to the ear. In Fig.53, Fig.53A shows an example in which the fixing mechanism 60 is attached to the blood-pressure meter 70. Although the outer shape of the blood-pressure meter 70 is circle as an example, this does not mean that the outer shape of the blood-pressure meter is necessarily circle. Fig.53B shows an example of a state in which the blood-pressure meter 70 is worn to the auricle 80. The auricle is shown by a dotted line. As shown in Fig.53B, the fixing mechanism 60 has a shape obtained by cutting an ellipse in half in the major axis direction and further incurving the both ends. It is adequate that the fixing mechanism 60 has a shape that follows a shape of the base of the auricle on the face. Therefore, it does not mean that the fixing mechanism 60 strictly has a shape obtained by cutting an ellipse in half in the major axis direction and further incurving the both ends.

Although the fixing mechanism 60 may be attached on the back side of the blood-pressure meter 70, the figure is drawn such that the fixing mechanism that may be on the back side of the blood-pressure meter 70 can be viewed to show the shape of the fixing mechanism 60. Fig.53C shows an example in which the blood-pressure meter is worn to the auricle. As mentioned above, according to this embodiment, since the holding frame part 3 has the fixing mechanism 60 having a semiellipse shape in which both ends are incurved so as to be worn at the base of the auricle, it becomes easy to wear the blood-pressure meter to the auricle.

### [Embodiment 2-17]

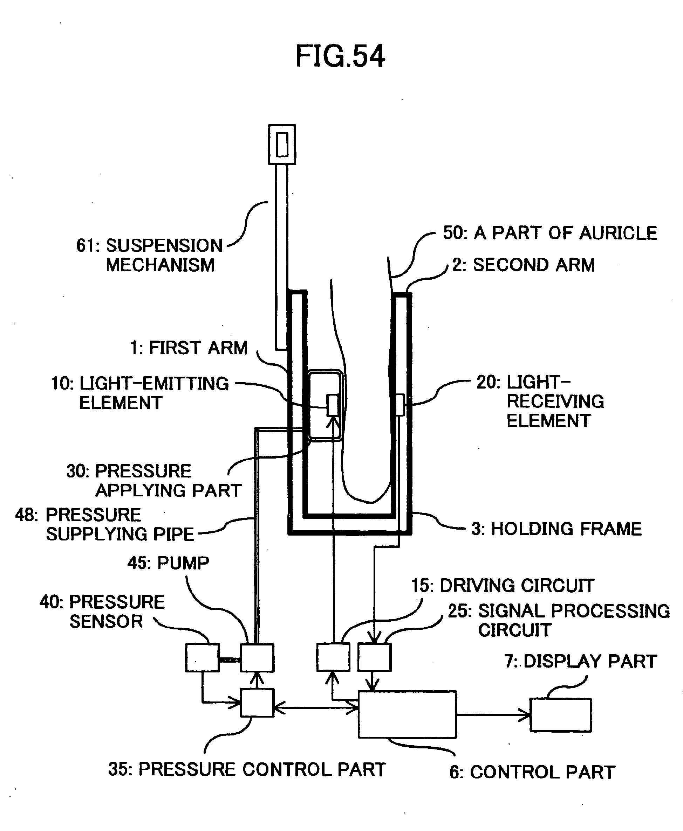

Next, the embodiment 2-17 is described with reference to Figs.54-56. The holding frame part 3 of the blood-pressure meter of this embodiment includes a suspension mechanism 61 such that the holding frame part 3 is suspended from a temple of eyeglasses. This mechanism can be applied to all holding frame parts 3 of the blood-pressure meters described so far. Thus, the blood-pressure meter of the embodiment 2-1 is described as an example.

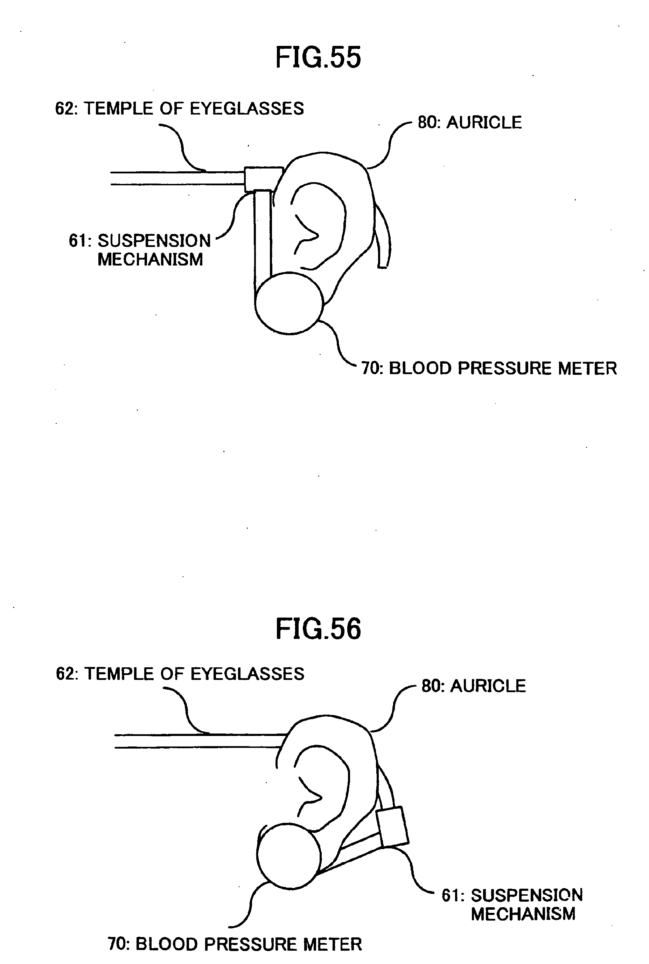

Fig.54 is a section view showing a state in which the suspension mechanism 61 is attached to the holding frame part 3. Fig.55 shows an example in which the suspension mechanism 61 is attached to the temple of the eyeglasses.

As shown in Fig.55, the part for attaching the suspension mechanism 61 to the temple 62 of eyeglasses includes a function for pinching or enclosing the temple 62 of the eyeglasses. The suspension mechanism 61 includes the part for attaching the suspension mechanism 61 to the temple 62 of eyeglasses and a part for connecting the part to the blood-pressure meter. In Fig.55, the part for attaching the suspension mechanism 61 to the temple 62 of eyeglasses and the part for connecting the blood-pressure meter 70 are depicted linearly. But, this is merely an example, and the part for attaching the suspension mechanism 61 to the temple 62 of eyeglasses and the part for connecting the blood-pressure meter 70 may be curved. In addition, Fig.56 shows a case in which the suspension mechanism 61 to be suspended from the temple 62 of the eyeglasses is attached to an end part of the temple 62 of the eyeglasses.

As described above, by providing the suspension mechanism 61 to be suspended from the temple 62 of the eyeglasses, the blood-pressure meter can be worn to the auricle easily and comfortably.

As described above, according to the second embodiment, although a simple structure is adopted in which the pressure applying part, the light-emitting element and the light-receiving element are provided in the frame part including the first arm and the second arm that pinch the part of the auricle, a blood-pressure meter that can measure a blood pressure easily and accurately can be provided. By the way, mechanisms for holding described in this embodiment can be used for other embodiments.

### (Third embodiment)

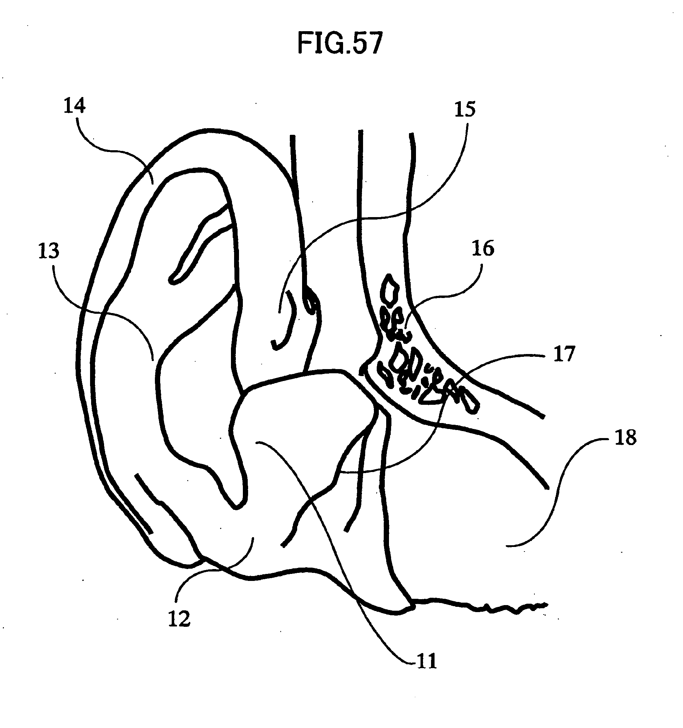

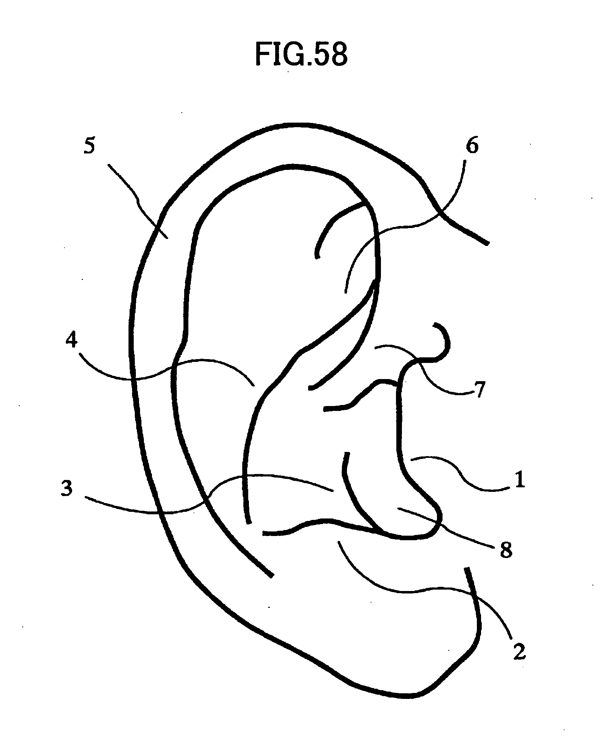

Next, the third embodiment is described. Before describing this embodiment, structures of cartilage of the auricle and structures of each part of the auricle are described with reference to Figs.57-60. Figs.57 and 58 are contained in the non-patent documents 1 and 4. Names of cartilage of the auricle are described with reference to Fig.57, and names of the auricle are described with reference to Fig.58. The names and structures of the ear described in this embodiment are common to the whole of the specification.

In the structure of the cartilage of the auricle shown in Fig.57, 11 is called a lamina of tragus, 12 is called a cartilage of acoustic meatus, 13 is called an antihelix, 14 is called a helix, 15 is called a spina helices, 16 is called a squamous part of temporal bone, 17 is called an incisura cartilaginis meatus acustici externi, and 18 is called a tympanic portion of the temporal bone.

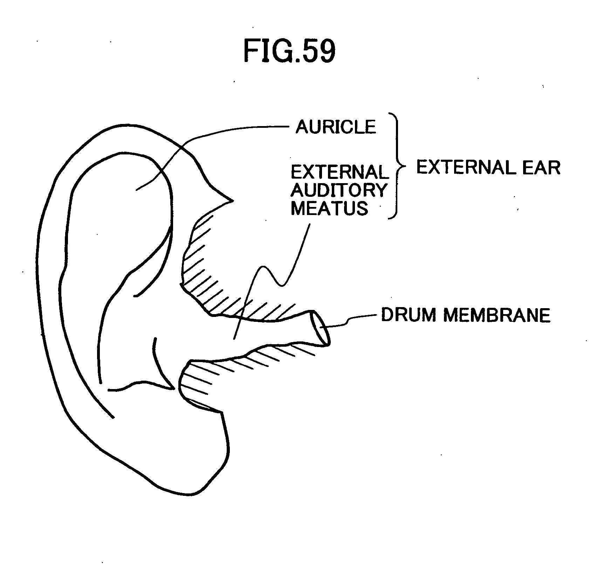

In the structure of the auricle shown in Fig.58, 1 is called a tragus, 2 is called an antitragus, 3 is called a concha auriculae, 4 is called a antihelix, 5 is called a helix, 6 is called a crus anthelicis, 7 is called a crus helicis, and 8 is called a cavum conchae. The auricle is a so-called ear, and is a general term of the whole of the ear shown in Fig.58. In addition, as shown in Fig.59, the external ear consists of the auricle and the external auditory meatus. The part of the external auditory meatus is shown as a section view. The external auditory meatus is an auditory meatus part to the drum membrane leading to the middle ear. The auricle is a part, generally called an ear, jutting out the temporal region.

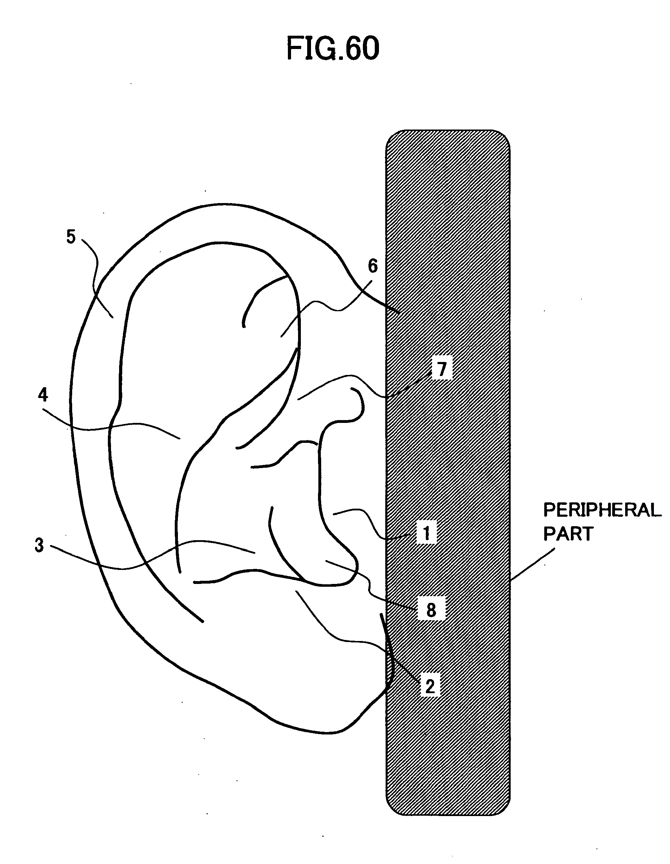

In the present specification and claims, "periphery of external ear" means a periphery of the base of the auricle in the temporal region shown in Fig.60. In addition, "ear part" in the present specification and claims means a part including the external ear and the periphery of the external ear.

A branch artery exists in a subcutaneous part of the auricle and the external auditory meatus. In addition, in the periphery of the base of the auricle in the temporal region, a superficial temporal artery that appears on a surface layer of skin and that extends upward exists. These are useful parts for measuring a pulse wave (measuring a blood pressure).

### [Embodiment 3-1]

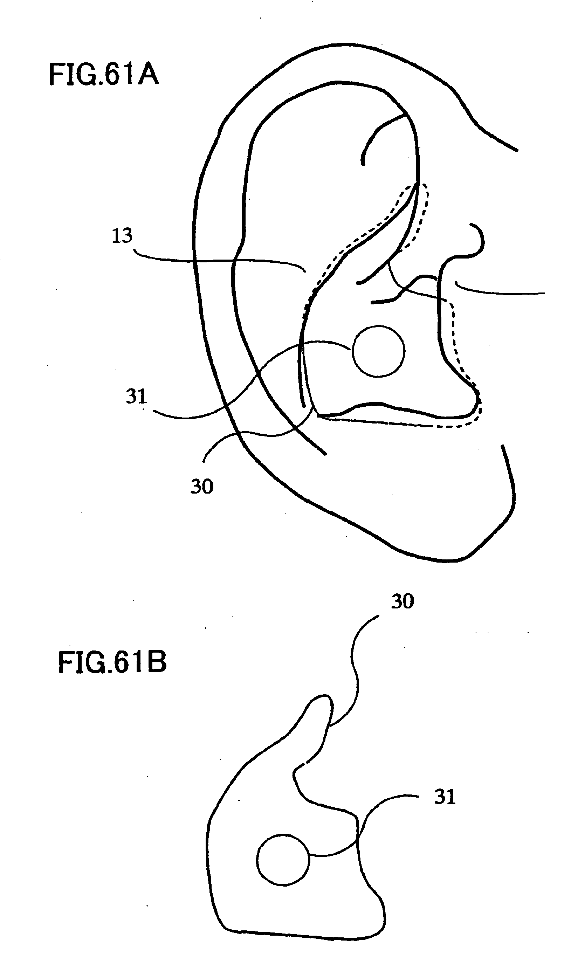

An example of a living body information detection apparatus of this embodiment is shown in Fig.61. A state in which the living body information detection apparatus is attached to the structure of the cartilage of the auricle is described with reference to Fig.61A, and a structure of the living body information detection apparatus is described with reference to Fig.61B. The structure of the living body information detection apparatus having a shape following the cartilage of the auricle is described using the outer appearance of the auricle shown in Fig.58 instead of the cartilage of the auricle shown in Fig.57 since the outer appearance of the auricle appears in Fig.61A. In Figs.61A and 61B, 11 is a lamina of tragus, 13 is an antihelix, 30 is the living body information detection apparatus, and 31 is a hollow provided in the living body information detection apparatus.

The living body information detection apparatus 30 shown in Figs.61A and 61B has a shape that follows the cartilage of the auricle in the periphery of the concha auriculae (refer to Fig.58). By adopting the shape that follows the cartilage of the auricle in the periphery of the concha auriculae, the living body information detection apparatus 30 can be set in a depression of the concha auriculae. Thus, since the living body information detection apparatus 30 can be held by the cartilage of the auricle in the periphery of the concha auriculae, the living body information detection apparatus 30 can be stably worn.

It is desirable that the shape that follows the cartilage of the auricle in the periphery of the concha auriculae is a shape following the antihelix 13. By adopting the shape following the antihelix 13, the living body information detection apparatus 30 set in the depression of the concha auriculae can be held so as to press the living body information detection apparatus 30 against the antihelix 13. Therefore, the living body information detection apparatus 30 can be attached stably. It is preferable that the living body information detection apparatus 30 shown in Figs.61A and 61B has a shape curving around to the inside of the antihelix 13. Since the part curving around to the inside of the antihelix 13 shown in Fig.61A (a part depicted by a dotted line in a shadow of the antihelix 13) is brought into intimate contact with the auricle, the living body information detection apparatus 30 can be worn more stably.

It is preferable that the shape that follows the cartilage of the auricle in the periphery of the concha auriculae is a shape following the antihelix 13 and the lamina of tragus 11. By adopting the shape following the antihelix 13 and the lamina of tragus 11, the living body information detection apparatus 30 set in the depression of the concha auriculae can be held so as to press the living body information detection apparatus 30 against the antihelix 13 or the lamina of tragus 11. Therefore, the living body information detection apparatus 30 can be worn stably. It is preferable that the living body information detection apparatus 30 shown in Figs.61A and 61B has a shape curving around to the inside of the antihelix 13 or the lamina of tragus 11. Since the part curving around to the inside of the antihelix 13 or the lamina of tragus 11 shown in Fig.61A (a part depicted by a dotted line in a shadow of the antihelix 13, or a part depicted by a dotted line in a shadow of the lamina of tragus 11 in Fig.61A) is brought into intimate contact with the auricle, the living body information detection apparatus 30 can be worn more stably.

The living body information detection apparatus 30 includes the hollow 31 for sound transmission. By the hollow 31, even when the living body information detection apparatus 30 is worn, external sound can be heard easily.

### [Embodiment 3-2]

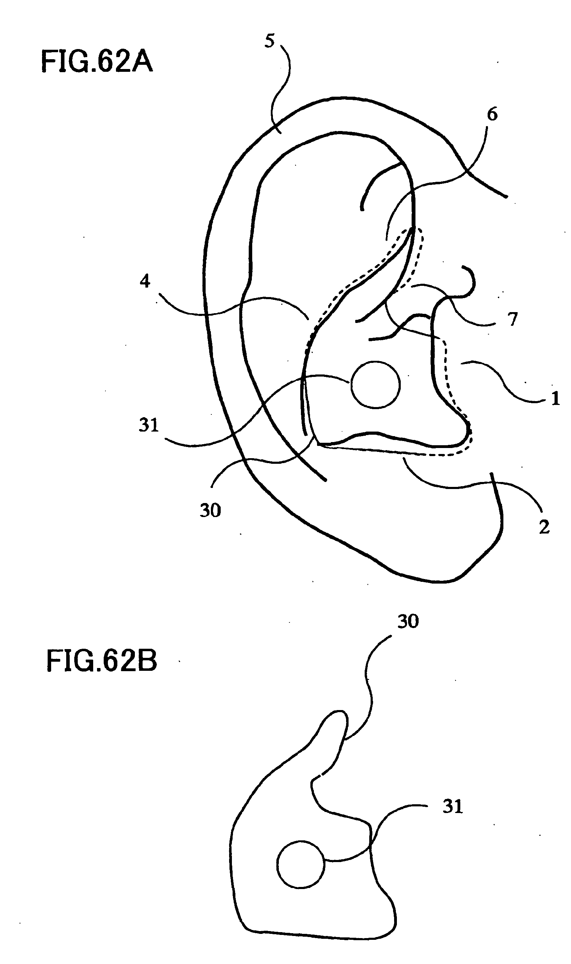

A living body information detection apparatus of this embodiment is shown in Fig.62. A state in which the living body information detection apparatus is worn in the structure of the auricle shown in Fig.58 is described with reference to Fig.62A, and a structure of the living body information detection apparatus is described with reference to Fig.62B. In Figs.62A and 62B, 1 indicates the tragus, 2 indicates the antitragus, 4 indicates the antihelix, 5 indicates the helix, 6 indicates the crus anthelicis, 7 indicates the crus helicis, 30 indicates the living body information detection apparatus, 31 indicates the hollow provided in the living body information detection apparatus 30.

The living body information detection apparatus 30 shown in Figs.62A and 62B has a shape that follows the auricle in the periphery of the concha auriculae (refer to Fig.58). By adopting the shape that follows the auricle in the periphery of the concha auriculae, the living body information detection apparatus 30 can be set in a depression of the concha auriculae. Thus, since the living body information detection apparatus 30 can be held by the auricle in the periphery of the concha auriculae, the living body information detection apparatus 30 can be stably worn.

It is desirable that the shape that follows the auricle in the periphery of the concha auriculae is a shape that follows the concha auriculae 3 (refer to Fig.58) and the antihelix 4. By adopting the shape that follows the concha auriculae 3 and the antihelix 4, the living body information detection apparatus 30 set in the depression of the concha auriculae 3 can be held so as to press the living body information detection apparatus 30 against the antihelix 4. Therefore, the living body information detection apparatus 30 can be worn stably. It is preferable that the living body information detection apparatus 30 shown in Figs.62A and 62B has a shape curving around to the inside of the antihelix 4. Since the part curving around to the inside of the antihelix 4 shown in Fig.62A (a part depicted by a dotted line hidden behind the antihelix 4) is brought into intimate contact with the auricle, the living body information detection apparatus 30 can be worn more stably.

It is desirable that the shape that follows the auricle in the periphery of the concha auriculae is a shape that follows the concha auriculae 3, the tragus 1, the antitragus 2 and the antihelix 4. By adopting the shape that follows the concha auriculae 3, the tragus 1, the antitragus 2 and the antihelix 4, the living body information detection apparatus 30 set in the depression of the concha auriculae 3 can be held by pressing the living body information detection apparatus 30 with the tragus 1, the antitragus 2 or the antihelix 4. Therefore, the living body information detection apparatus 30 can be worn stably. It is preferable that the living body information detection apparatus 30 shown in Figs.62A and 62B has a shape curving around to the inside of the tragus 1, the antitragus 2 or the antihelix 4. Since the part curving around to the inside of the tragus 1, the antitragus 2 or the antihelix 4 shown in Fig.62A (a part depicted by a dotted line hidden behind the tragus 1, a part depicted by a dotted line hidden behind the antitragus 2, and a part depicted by a dotted line hidden behind the antihelix 4 in Fig.62A) is brought into intimate contact with the auricle, the living body information detection apparatus 30 can be worn more stably.

It is desirable that the shape that follows the auricle in the periphery of the concha auriculae is a shape that follows the concha auriculae 3, the tragus 1, the crus helicis 7, the crus anthelicis 6, the antihelix 4, the antitragus 2 and the cavum conchae 8 (refer to Fig.58). By adopting the shape that follows the concha auriculae 3, the tragus 1, the crus helicis 7, the crus anthelicis 6, the antihelix 4, the antitragus 2 and the cavum conchae 8, the living body information detection apparatus 30 set in the depression of the concha auriculae 3 can be held so as to press the living body information detection apparatus 30 against the tragus 1, the crus anthelicis 6, the antihelix 4, or the antitragus 2. Therefore, the living body information detection apparatus 30 can be worn stably. It is preferable that the living body information detection apparatus 30 shown in Figs.62A and 62B has a shape curving around to the inside of the tragus 1, the crus anthelicis 6, the antihelix 4 or the antitragus 2. Since the part curving around to the inside of the tragus 1, the crus anthelicis 6, the antihelix 4, or the antitragus 2 shown in Fig.62A (a part depicted by a dotted line hidden behind the tragus 1, a part depicted by a dotted line hidden behind the crus anthelicis 6, a part depicted by a dotted line hidden behind the antihelix 4, or a part depicted by a dotted line hidden behind the antitragus 2 in Fig.62A) is brought into intimate contact with the auricle, the living body information detection apparatus 30 can be worn more stably.

The living body information detection apparatus 30 includes the hollow 31 for sound transmission. By the hollow 31, even when the living body information detection apparatus 30 is worn, external sound can be heard easily.

### [Embodiment 3-3]

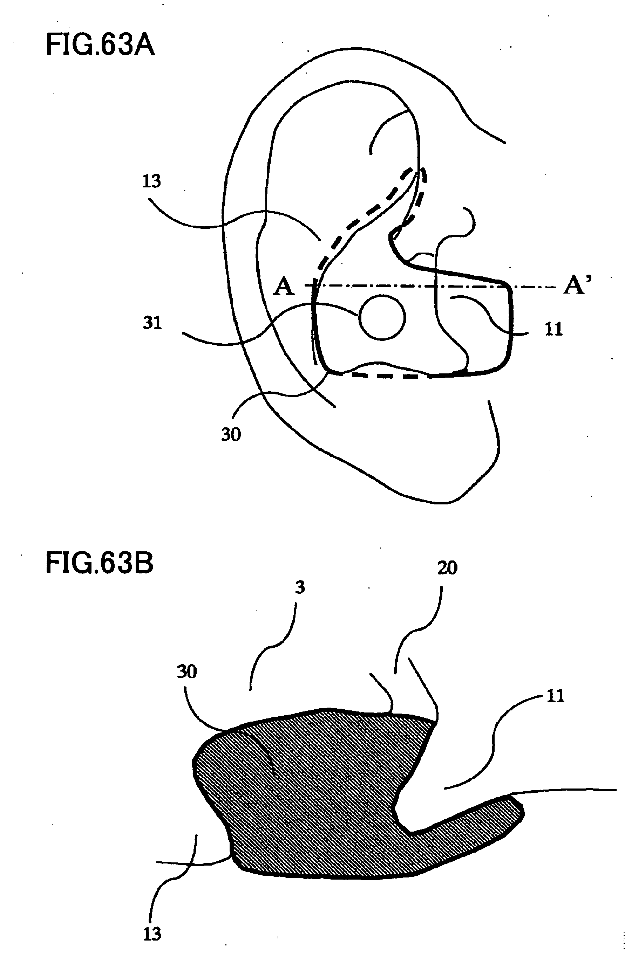

A living body information detection apparatus of this embodiment is shown in Fig.63. A state in which the living body information detection apparatus is worn in the structure of the cartilage of the auricle shown in Fig.57 is described with reference to Fig.63A, and a structure of the living body information detection apparatus is described with reference to Fig.63B. Fig.63B shows a structure of a section at a A-A' line of Fig.63A. The structure of the living body information detection apparatus having a shape following the cartilage of the auricle is described using the outer appearance of the auricle shown in Fig.58 instead of the cartilage of the auricle shown in Fig.57 since the outer appearance of the auricle appears in Fig.63A. In Figs.63A and 63B, 3 is a concha auriculae, 11 is a lamina of tragus, 13 is an antihelix, 30 is the living body information detection apparatus, and 31 is a hollow provided in the living body information detection apparatus.

The living body information detection apparatus 30 shown in Figs.63A and 63B has a shape that follows the cartilage of the auricle in the periphery of the concha auriculae. By adopting the shape that follows the cartilage of the auricle in the periphery of the concha auriculae, the living body information detection apparatus 30 can be set in a depression of the concha auriculae. Thus, since the living body information detection apparatus 30 can be held by the cartilage of the auricle in the periphery of the concha auriculae, the living body information detection apparatus 30 can be stably worn.

The shape described in the embodiment 3-1 can be applied to the shape that follows the cartilage of the auricle in the periphery of the concha auriculae. As shown in the section view of A-A' line of Fig.63B, the shape of the living body information detection apparatus 30 also follows the outer surface of the lamina of tragus 11, and the shape has an inside part and an outside part that covers the tragus wherein the inside part touches the tragus from the inside of the lamina of tragus 11 and wherein the outside part touches the tragus from the outer side of the lamina of tragus 11. By adopting such shape, the living body information detection apparatus 30 set in the depression of the concha auriculae can be held so as to press the living body information detection apparatus 30 against the antihelix 13 or the lamina of tragus 11. Therefore, the living body information detection apparatus 30 can be attached stably.

### [Embodiment 3-4]

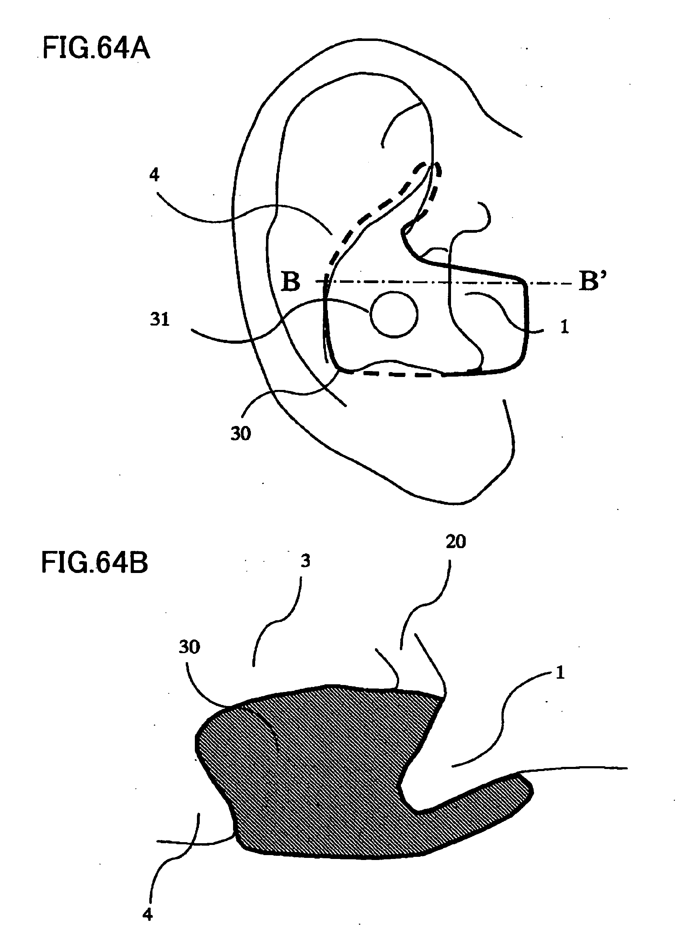

A living body information detection apparatus of this embodiment is shown in Fig.64. A state in which the living body information detection apparatus is worn in the structure of the cartilage of the auricle shown in Fig.58 is described with reference to Fig.64A, and a structure of the living body information detection apparatus is described with reference to Fig.64B. Fig.64B shows a structure of a section at a B-B' line shown in Fig.64A. In Figs.64A and 64B, 1 indicates the tragus, 3 indicates the concha auriculae, 4 indicates the antihelix, 20 indicates the external auditory meatus, 30 indicates the living body information detection apparatus, and 31 indicates a hollow provided in the living body information detection apparatus.

The living body information detection apparatus 30 shown in Figs.64A and 64B has a shape that follows the auricle in the periphery of the concha auriculae. By adopting the shape that follows the auricle in the periphery of the concha auriculae, the living body information detection apparatus 30 can be set in a depression of the concha auriculae. Thus, since the living body information detection apparatus 30 can be held by the auricle in the periphery of the concha auriculae, the living body information detection apparatus 30 can be stably worn.

The shape described in the embodiment 3-2 can be applied to the shape that follows the auricle in the periphery of the concha auriculae. As shown in the section view of B-B' line of Fig.64B, the shape of the living body information detection apparatus 30 also follows the outer surface of the tragus 1, and the shape has an inside part and an outside part that cover the tragus wherein the inside part touches the tragus from the inside of the tragus 1 and wherein the outside part touches the tragus from the outer side of the tragus 1. By adopting such shape, the living body information detection apparatus 30 set in the depression of the concha auriculae can be held so as to press the living body information detection apparatus 30 against the antihelix 4 or the tragus 1. Therefore, the living body information detection apparatus 30 can be attached stably.

### [Embodiment 3-5]

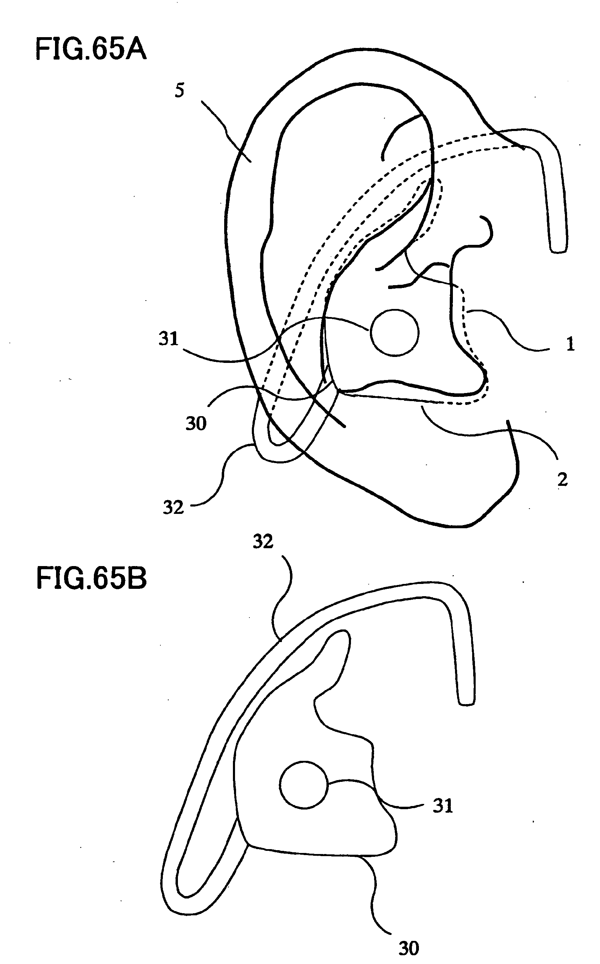

An example of a living body information detection apparatus of this embodiment is shown in Fig.65. A state in which the living body information detection apparatus is worn in the auricle is described with reference to Fig.65A, and a structure of the living body information detection In Figs.65A and 65B, 1 indicates the tragus, 2 indicates the antitragus, 5 indicates the helix, 30 indicates the living body information detection apparatus, 31 indicates a hollow provided in the living body information detection apparatus, and 32 indicates a holding mechanism.

The living body information detection apparatus 30 shown in Fig.65A and Fig.65B is formed by adding the holding mechanism 32 to a living body information detection apparatus described in any one of embodiments 3-1 - 3-4 for fixing the living body information detection apparatus to the auricle. The holding mechanism 32 has a mechanism to curve around from an anterior inferior part of the auricle to a base of the auricle, that is, to a base of the helix 5 so as to fix the main body part of the living body information detection apparatus 30 to the auricle. By using the holding mechanism 32, the living body information detection apparatus can be held on the auricle with reliability.

### [Embodiment 3-6]

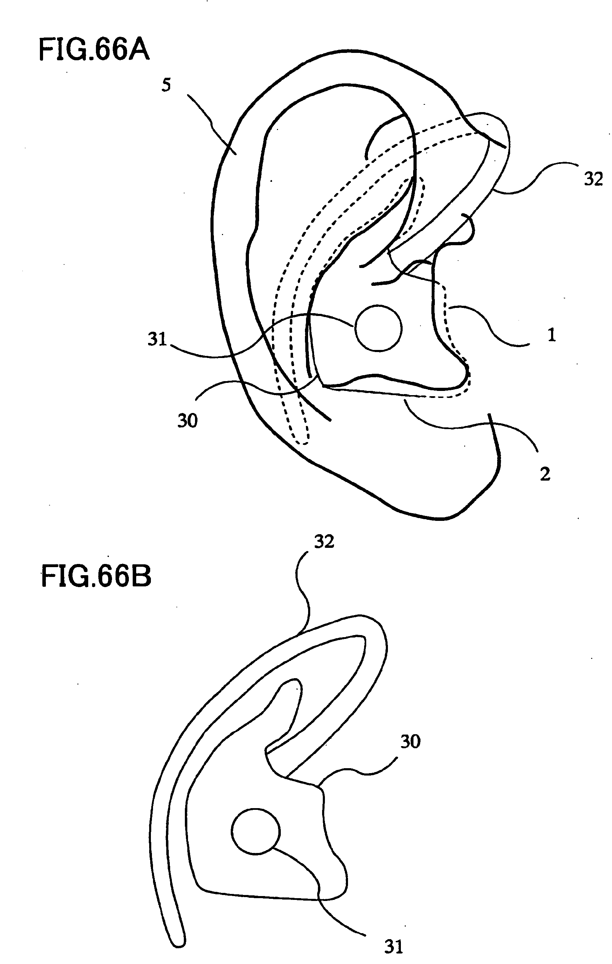

An example of a living body information detection apparatus of this embodiment is shown in Fig.66. A state in which the living body information detection apparatus is worn in the auricle is shown in Fig.66A, and a structure of the living body information detection is shown in Fig.66B. In Figs.66A and 66B, 1 indicates the tragus, 2 indicates the antitragus, 5 indicates the helix, 30 indicates the living body information detection apparatus, 31 indicates a hollow provided in the living body information detection apparatus, and 32 indicates a holding mechanism.

The living body information detection apparatus 30 shown in Fig.66A and Fig.66B is formed by adding the holding mechanism 32 to a living body information detection apparatus described in any one of embodiments 3-1 - 3-4 for fixing the living body information detection apparatus to the auricle. The holding mechanism 32 has a mechanism to curve around from an anterior upper part of the auricle to a base of the auricle, that is, to a base of the helix 5 so as to fix the main body part of the living body information detection apparatus 30 to the auricle. By using the holding mechanism 32, the living body information detection apparatus can be held on the auricle with reliability.

As mentioned above, since the living body information detection apparatus described in the embodiments 1-6 can be stably worn in the auricle of a human body, the measurement result is almost insensitive to vibration and the like and living body information can be stably detected at the auricle. In addition, the living body information detection apparatus can be downsized and weight reduction can be realized.

The living body information detection apparatus described in the embodiments 1-6 may include a light-emitting element and a light-receiving element so that light from the light-emitting element enters a living body tissue, the light-receiving element receives scattered light from the living body tissue, and a pulse wave can be detected from an optical/electrical converted signal. The living body information detection apparatus may have functions for detecting not only the pulse wave but also living body information such as body temperature by a thermistor and after-mentioned blood pressure.

As a concrete example for manufacturing the living body information detection apparatus described in the embodiments 1-6, there is a method for modeling the auricle of each person in resin or clay and manufacturing the apparatus for each person based on it. In addition, it is effective to manufacture the apparatus based on an average shape of auricles of many persons. Further, it is more effective to manufacture plural types such as large, medium, small and the like according to a physique. These manufacturing methods also apply to living body information detection apparatuses described in the following.

Configuration examples and functions of the living body information detection apparatus described in embodiments 1-6 for detecting living body information are described in the following.

### [Embodiment 3-7]

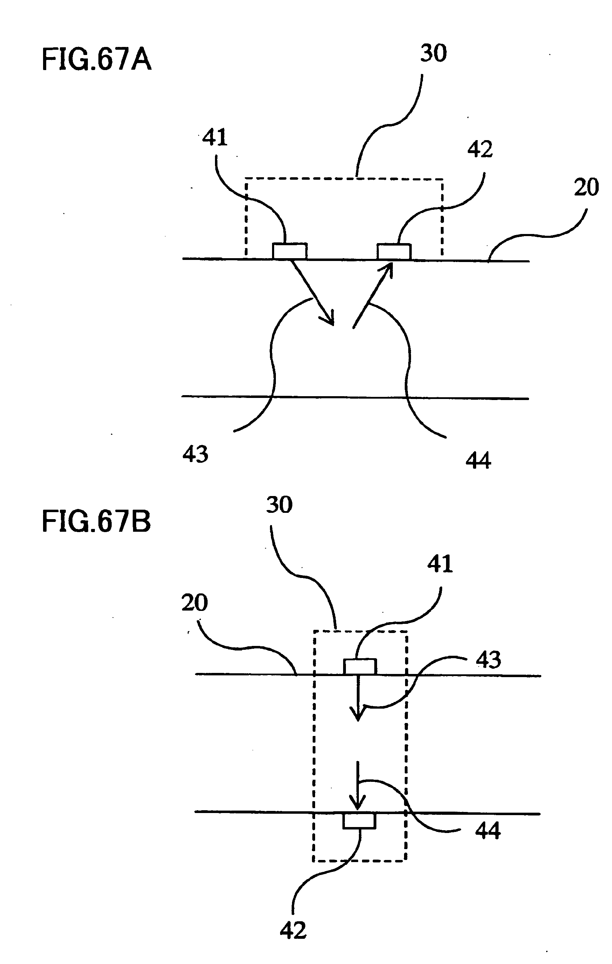

A configuration and function for detecting a pulse wave using a light-emitting element and a light-receiving element are described with reference to Fig.67. In Fig.67, 20 indicates a living body tissue, 30 is any one of the living body information detection apparatus described in embodiments 1-6, 41 indicates the light-emitting element, 42 indicates the light-receiving element, 43 indicates incident light, and 44 indicates scattered light.

Fig.67A shows a state in which the light-emitting element 41 and the light-receiving element 42 are placed on a surface of the living body information detection apparatus 30 that contacts the living body tissue 20 that is a part of the auricle, light emitted from the light-emitting element 41 enters the living body tissue 20, the incident light 43 is scattered by a blood vessel or blood cells in the blood vessel in the living body tissue 20, and the scattered light 44 is received by the light-receiving element 42.

The blood vessel in the living body tissue 20 or the blood cells in the blood vessel pulse according to heartbeat, so that the scattered light 44 receives a change of strength according to the pulsation or a change of optical frequency due to the Doppler effect. The incident light 43 entering the living body tissue 20 from the light-emitting element 41 is scattered in the living body tissue 20 so that the scattered light 44 is generated, and the light-receiving element 42 is placed at a position such that the light-receiving element 42 receives the scattered light 44. The scattered light 44 is received by the receiving-light element 42, and optical/electrical conversion is performed on the scattered light 44, so that the pulse wave corresponding to the pulsation of the blood vessel is detected.

Fig.67B shows a state in which the living body information detection apparatus 30 pinches the living body tissue 20 that is a part of the auricle in which the light-emitting element 41 and the light-receiving element 42 pinches the living body tissue 20 and contacts the living body tissue 20, and they are placed opposite to each other, wherein the incident light 43 entering the living body tissue 20 from the light-emitting element 41 is scattered in the living body tissue 20, and the scattered light 44 is received by the receiving-light element 42. The incident light 43 entering the living body tissue 20 from the light-emitting element 41 is scattered in the living body tissue 20 so that the scattered light 44 is generated, and the light-receiving element 42 is placed opposite to the light-emitting element so as to receive the scattered light 44. In the configuration in Fig.67B, the pulse wave can be detected based on the change of the scattered light 44 received by the light-receiving element 42 in the same way as the configuration of Fig.67A.

As mentioned above, in both cases that are a reflection type shown in Fig.67A and a transmission type shown in Fig.67B, the living body information detection apparatus 30 can detect the pulse wave, and can detect the pulse wave more accurately compared with a conventional case where the pulse wave is detected using sound. As to the living body information detection apparatus described in the embodiment 3-1 or 3-2, the reflection type shown in Fig.67A can be applied. As to the living body information detection apparatus described in the embodiments 3-3 or 3-4, either of the reflection type shown in Fig.67A and the transmission type shown in Fig.67B can be applied. In any case, the living body information detection apparatus worn in the auricle can stably detect the pulse wave at the auricle.

### [Embodiment 3-8]

A cuff for applying a pressure on the tragus may be placed in the inside part that covers the tragus in the living body information detection apparatus described in embodiments 3-3 and 3-4, in which a light-emitting element and a light-receiving element are placed in the cuff. In the configuration, light from the light-emitting element is entered into the living body tissue, the light-receiving element receives the scatted light from the living body tissue, so that the pulse wave can be detected and the blood pressure can be measured based on an pressure applied to the tragus by the cuff and the pulse wave at the time.

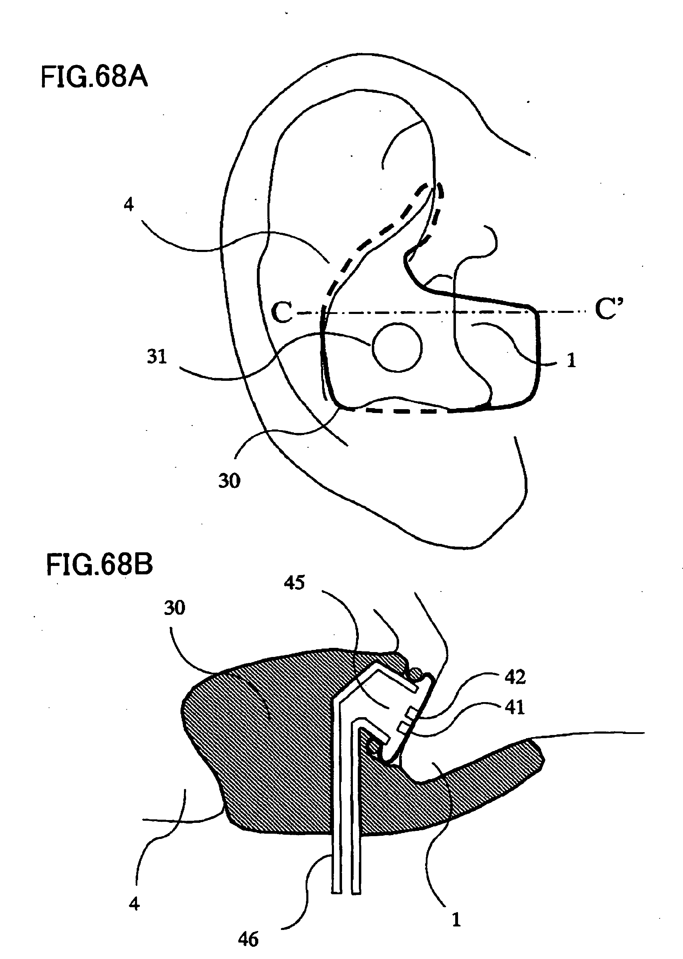

A configuration example and a function of the living body information detection apparatus of this embodiment is described with reference to Fig.68. In Fig.68, 1 indicates the tragus, 4 indicates the antihelix, 30 indicates the living body information detection apparatus, 31 indicates a hollow of the living body information detection apparatus, 41 indicates the light-emitting element, 42 indicates the light-receiving element, 45 indicates the cuff and 46 indicates an air pipe. Fig.68A shows a state in which the living body information detection apparatus 30 is worn in the auricle. Fig.68B indicates a section view at a C-C' line of the Fig.68A. Diagonally shaded areas show the section view of the living body information detection apparatus 30.

In Fig.68B, the living body information detection apparatus 30 is worn so as to cover the tragus 1. The cuff 45 is placed on a surface at which the living body information detection apparatus 30 contacts the tragus 1. Further, the light-emitting element 41 and the light-receiving element 42 are placed on a surface, in the cuff 45, that contacts the tragus 1, and the air pipe 46 is connected to the cuff 45.

In Fig.68B, the light-emitting element 41 and the light-receiving element 42 are placed to keep a position relationship in which light from the light-emitting element 41 enters the tragus 1 and the light-receiving element 42 receives the scattered light obtained by scattering the incident light. By adopting such configuration, the living body information detection apparatus 30 can detect the pulse wave based on the before-mentioned principle.

The principle for measuring a blood pressure is the same as one described with reference to Fig.14 and the like. That is, the pulse wave 120 changes in the process for decreasing the pressure 114 of the cuff from a high pressure that stops bloodstream in the blood vessel, and shows a unique shape. Therefore, by storing shapes of the pulse wave 120 corresponding to a blood pressure of each time, for example, based on a pulse wave 120 measured at an arbitrary time, it can be measured which position the blood pressure at the time of measurement corresponds to between the maximum blood pressure and the minimum blood pressure.

In addition, since the pulse wave 120 especially shows remarkable waveform change at the position A 121 corresponding to the maximum blood pressure 111, the point B 122 corresponding to the average blood pressure 112, and the point C 123 corresponding to the minimum blood pressure 113, the blood pressure can be also measured based on features of the waveforms.

For example, by controlling the pressure 114 of the cuff such that the pulse wave 120 always becomes the maximum vale, at the time when the B point 122 corresponding to the average blood pressure 122 at which the amplitude of the pulse wave 120 is the maximum is measured, the average blood pressure 112 can be measured continuously. In the same principle, continuous measurement is possible also for the maximum blood pressure 111 and the average blood pressure 113.

Further, two kinds of the principles for detecting the pulse wave were described in Figs.67A and 67B, the blood pressure can be measured by either of pulse waves detected in these methods base on the above-mentioned principle.

The living body information detection apparatus 30 of this embodiment of the present invention shown in Fig.68, an air pressure is applied to the cuff 45 via the air pipe 46 so as to press the tragus 1, and the pulse wave is measured by the light-emitting element 41 and the light-receiving element 42 provided in the inside of the cuff. Accordingly, the blood pressure can be measured based on the above-mentioned principle of blood pressure measurement.

Therefore, according to the living body information detection apparatus of this embodiment, the blood pressure can be measured easily and stably at the tragus of the human body.

### [Embodiment 3-9]

A cuff for applying a pressure on the tragus may be placed in the outside part that covers the tragus in the living body information detection apparatus described in embodiments 3-3 and 3-4, in which a light-emitting element and a light-receiving element are placed in the cuff. In the configuration, light from the light-emitting element is entered into the living body tissue, the light-receiving element receives the scatted light from the living body tissue, so that the pulse wave can be detected and the blood pressure can be measured based on an pressure applied to the tragus by the cuff and the pulse wave at the time.

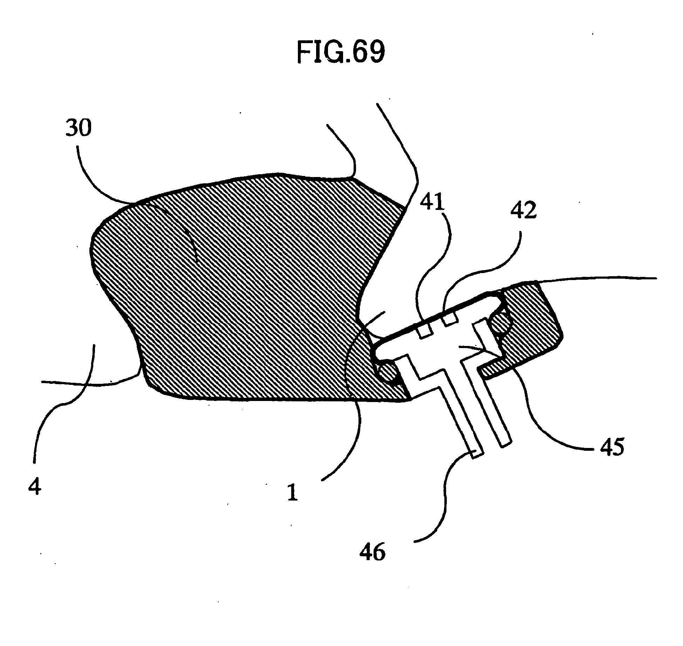

A configuration example and a function of the living body information detection apparatus of this embodiment is described with reference to Fig.69. In Fig.69, 1 indicates the tragus, 4 indicates the antihelix, 30 indicates the living body information detection apparatus, 41 indicates the light-emitting element, 42 indicates the light-receiving element, 45 indicates the cuff and 46 indicates an air pipe. In the living body information detection apparatus 30 shown in Fig.69, the cuff 45 is placed to contact the outside of the tragus 1, and the light-emitting element 41 and the light-receiving element 42 are placed on a surface, in the cuff 45, contacting the tragus 1. By the way, in Fig.69 and figures described hereinafter, the configuration example of the living body information detection apparatus is shown as a section view similar to the section view at C-C' line in Fig.68A.

According to the living body information detection apparatus 30, based on a principle similar to the principle described in the embodiment 3-8, the blood pressure can be measured by adjusting the pressure of the cuff 45 and detecting the pulse wave using the light-emitting element 41 and the light-receiving element 42.

Therefore, according to the living body information detection apparatus of this embodiment, the blood pressure can be measured easily and stable at the tragus of the human body.

### [Embodiment 3-10]

In the living body information detection apparatus described in embodiments 3-3 and 3-4, a cuff for applying a pressure on the tragus may be placed in the inside part that covers the tragus, and the light-emitting element and the light-receiving-element are placed in the outside part that covers the tragus. In the configuration, light from the light-emitting element is entered into the living body tissue, the light-receiving element receives the scatted light from the living body tissue, so that the pulse wave can be detected and the blood pressure can be measured based on an pressure applied to the tragus by the cuff and the pulse wave at the time.

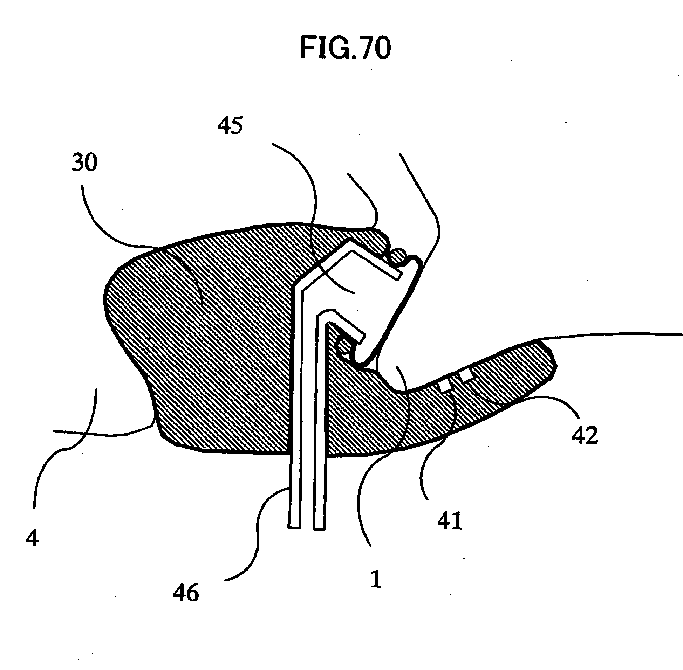

A configuration example and a function of the living body information detection apparatus of this embodiment are described with reference to Fig.70. In Fig.70, 1 indicates the tragus, 4 indicates the antihelix, 30 indicates the living body information detection apparatus, 41 indicates the light-emitting element, 42 indicates the light-receiving element, 45 indicates the cuff and 46 indicates an air pipe. In the living body information detection apparatus 30 shown in Fig.70, the cuff 45 is placed to contact the inside of the tragus 1, and the light-emitting element 41 and the light-receiving element 42 are placed to contact the outside of the tragus 1.

According to the living body information detection apparatus 30 of this embodiment, the cuff 45 presses the inside of the tragus 1 so that a pressure is applied to the tragus in the same way as the embodiment 8, and the pulse wave can be detected by the light-emitting element 41 and the light-receiving element 42. Thus, based on a principle similar to the principle described in the embodiment 8, the blood pressure can be measured.

Therefore, according to the living body information detection apparatus of this embodiment, the blood pressure can be measured easily and stably at the tragus of the human body.

### [Embodiment 3-11]

In the living body information detection apparatus described in embodiments 3-3 and 3-4, a cuff for applying a pressure on the tragus is placed in the outside part that covers the tragus, and the light-emitting element and the light-receiving element are placed in the inside part that covers the tragus. In the configuration, light from the light-emitting element is entered into the living body tissue, the light-receiving element receives the scatted light from the living body tissue, so that the pulse wave can be detected and the blood pressure can be measured based on an pressure applied to the tragus by the cuff and the pulse wave at the time.

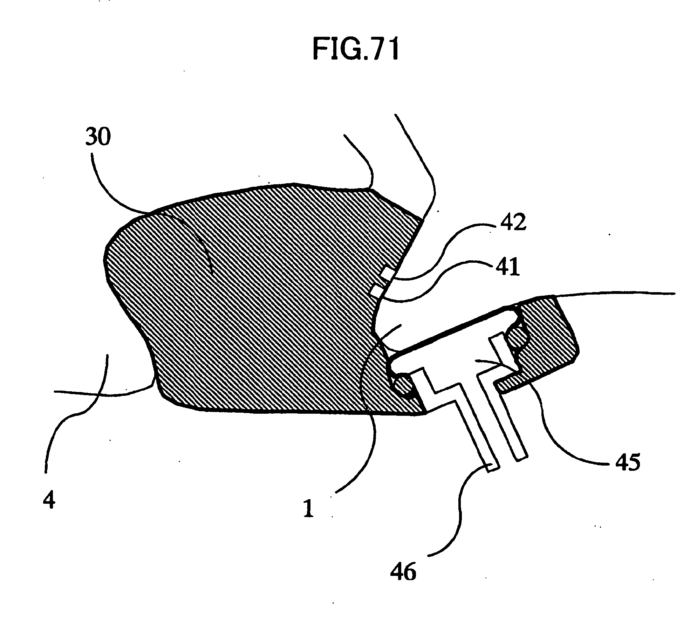

A configuration example and a function of the living body information detection apparatus of this embodiment are described with reference to Fig.71. In Fig.71, 1 indicates the tragus, 4 indicates the antihelix, 30 indicates the living body information detection apparatus, 41 indicates the light-emitting element, 42 indicates the light-receiving element, 45 indicates the cuff and 46 indicates an air pipe. In the living body information detection apparatus 30 shown in Fig.71, the cuff 45 is placed to contact the outside of the tragus 1, and the light-emitting element 41 and the light-receiving element 42 are placed to contact the inside of the tragus 1.

According to the living body information detection apparatus 30, the cuff 45 presses the outside of the tragus 1 so that a pressure is applied to the tragus in the same way as the embodiment 8, and the pulse wave can be detected by the light-emitting element 41 and the light-receiving element 42 that are placed on the inside of the tragus 1. Thus, based on a principle similar to the principle described in the embodiment 8, the blood pressure can be measured.

Therefore, according to the living body information detection apparatus of this embodiment, the blood pressure can be measured easily and stable at the tragus of the human body.

### [Embodiment 3-12]

In the living body information detection apparatus described in embodiments 3-3 and 3-4, a cuff for applying a pressure on the tragus may be placed in the inside part that covers the tragus, the light-emitting element is placed in the cuff, and the light-receiving element is placed in the outside part that covers the tragus. In the configuration, light from the light-emitting element is entered into the living body tissue, the light-receiving element receives the scatted light from the living body tissue, so that the pulse wave can be detected and the blood pressure can be measured based on an pressure applied to the tragus by the cuff and the pulse wave at the time.

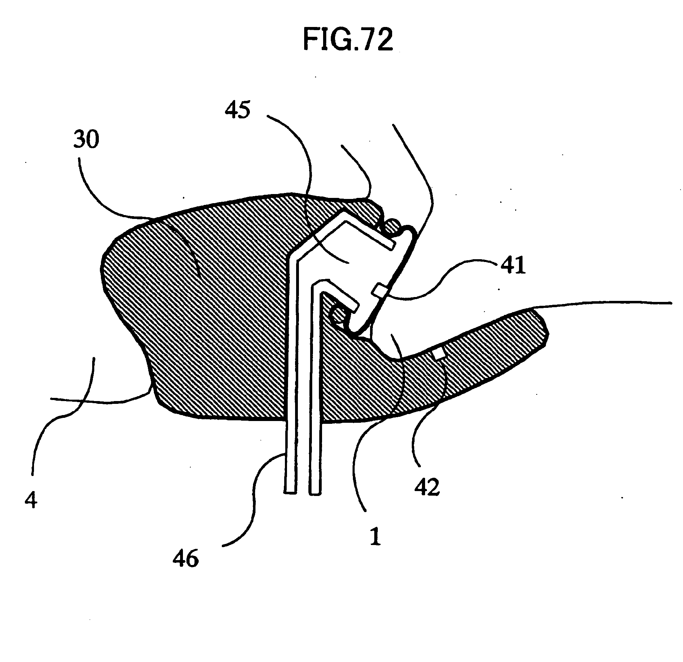

A configuration example and a function of the living body information detection apparatus of this embodiment are described with reference to Fig.72. In Fig.72, 1 indicates the tragus, 4 indicates the antihelix, 30 indicates the living body information detection apparatus, 41 indicates the light-emitting element, 42 indicates the light-receiving element, 45 indicates the cuff and 46 indicates an air pipe. In the living body information detection apparatus 30 shown in Fig.72, the cuff 45 is placed to contact the inside of the tragus 1, and the light-emitting element 41 is placed on a surface, of the cuff 45, contacting the tragus 1, and the light-receiving element 42 is placed to contact the outside of the tragus 1.

According to the living body information detection apparatus 30, the cuff 45 presses the tragus 1 from the inside of the tragus 1 so that a pressure is applied to the tragus 1 in the same way as the embodiment 8, and the pulse wave can be detected by the light-emitting element 41 placed in the inside of the tragus 1 and the light-receiving element 42 placed in the outside of the tragus 1. Thus, based on a principle similar to the principle described in the embodiment 8, the blood pressure can be measured.

Therefore, according to the living body information detection apparatus of this embodiment, the blood pressure can be measured easily and stably at the tragus of the human body.

### [Embodiment 3-13]

In the living body information detection apparatus described in embodiments 3-3 and 3-4, a cuff for applying a pressure on the tragus may be placed in the inside part that covers the tragus, the light-receiving element may be placed in the cuff, and the light-emitting element may be placed in the outside part that covers the tragus. In the configuration, light from the light-emitting element is entered into the living body tissue, the light-receiving element receives the scatted light from the living body tissue, so that the pulse wave can be detected and the blood pressure can be measured based on an pressure applied to the tragus by the cuff and the pulse wave at the time.

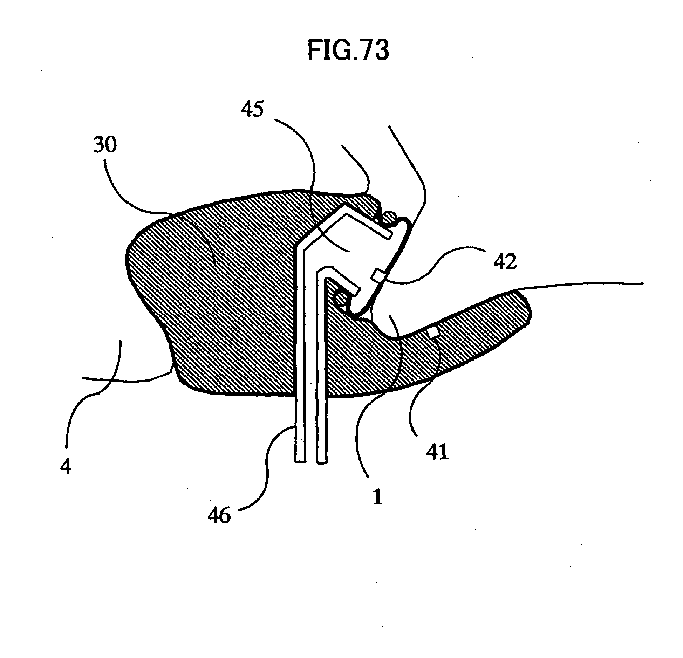

A configuration example and a function of the living body information detection apparatus of this embodiment are described with reference to Fig.73. In Fig.73, 1 indicates the tragus, 4 indicates the antihelix, 30 indicates the living body information detection apparatus, 41 indicates the light-emitting element, 42 indicates the light-receiving element, 45 indicates the cuff and 46 indicates an air pipe. In the living body information detection apparatus 30 shown in Fig.73, the cuff 45 is placed to contact the inside of the tragus 1, and the light-receiving element 42 is placed on a surface, of the cuff 45, contacting the tragus 1, and the light-emitting element 41 is placed to contact the outside of the tragus 1.

According to the living body information detection apparatus 30, the cuff 45 presses the tragus 1 from the inside of the tragus 1 so that a pressure is applied to the tragus 1 in the same way as the embodiment 8, and the pulse wave can be detected by the light-receiving element 42 placed in the inside of the tragus 1 and the light-emitting element 41 placed in the outside of the tragus 1. Thus, based on a principle similar to the principle described in the embodiment 8, the blood pressure can be measured.

Therefore, according to the living body information detection apparatus of this embodiment, the blood pressure can be measured easily and stably at the tragus of the human body.

### [Embodiment 3-14]

In the living body information detection apparatus described in embodiments 3-3 and 3-4, a cuff for applying a pressure on the tragus may be placed in the outside part that covers the tragus, the light-emitting element may be placed in the cuff, and the light-receiving element may be placed in the inside part that covers the tragus. In the configuration, light from the light-emitting element is entered into the living body tissue, the light-receiving element receives the scatted light from the living body tissue, so that the pulse wave can be detected and the blood pressure can be measured based on an pressure applied to the tragus by the cuff and the pulse wave at the time.

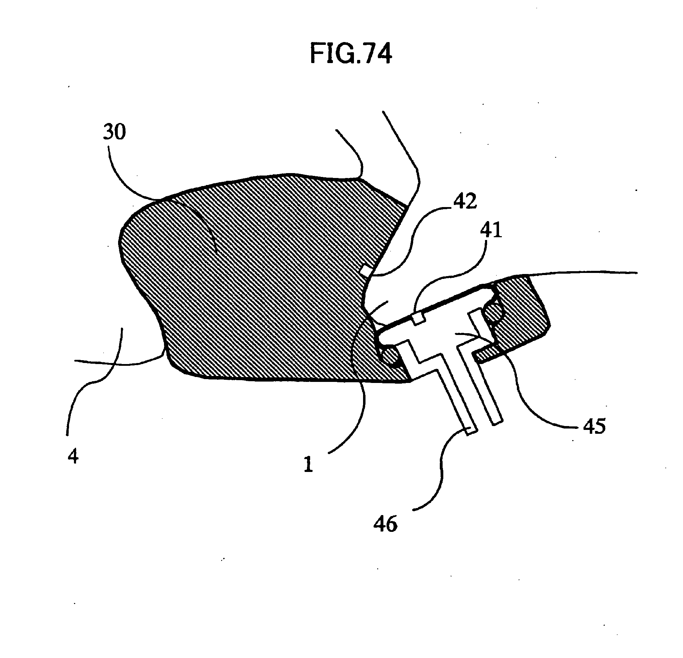

A configuration example and a function of the living body information detection apparatus of this embodiment are described with reference to Fig.74. In Fig.74, 1 indicates the tragus, 4 indicates the antihelix, 30 indicates the living body information detection apparatus, 41 indicates the light-emitting element, 42 indicates the light-receiving element, 45 indicates the cuff and 46 indicates an air pipe. In the living body information detection apparatus 30 shown in Fig.74, the cuff 45 is placed to contact the outside of the tragus 1, and the light-emitting element 41 is placed on a surface, of the cuff 45, contacting the tragus 1, and the light-receiving element 42 is placed to contact the inside of the tragus 1.

According to the living body information detection apparatus 30, the cuff 45 presses the tragus 1 from the outside of the tragus 1 so that a pressure is applied to the tragus 1 in the same way as the embodiment 8, and the pulse wave can be detected by the light-receiving element 42 placed in the inside of the tragus 1 and the light-emitting element 41 placed in the outside of the tragus 1. Thus, based on a principle similar to the principle described in the embodiment 8, the blood pressure can be measured.

Therefore, according to the living body information detection apparatus of this embodiment, the blood pressure can be measured easily and stably at the tragus of the human body.

### [Embodiment 3-15]

In the living body information detection apparatus described in embodiments 3-3 and 3-4, a cuff for applying a pressure on the tragus may be placed in the outside part that covers the tragus, the light-receiving element may be placed in the cuff, and the light-emitting element may be placed in the inside part that covers the tragus. In the configuration, light from the light-emitting element is entered into the living body tissue, the light-receiving element receives the scatted light from the living body tissue, so that the pulse wave can be detected and the blood pressure can be measured based on an pressure applied to the tragus by the cuff and the pulse wave at the time.

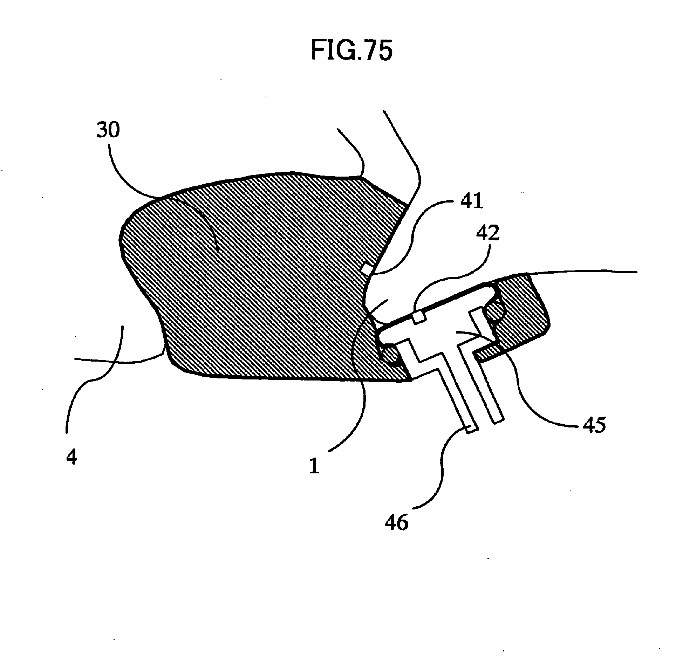

A configuration example and a function of the living body information detection apparatus of this embodiment are described with reference to Fig.75. In Fig.75, 1 indicates the tragus, 4 indicates the antihelix, 30 indicates the living body information detection apparatus, 41 indicates the light-emitting element, 42 indicates the light-receiving element, 45 indicates the cuff and 46 indicates an air pipe. In the living body information detection apparatus 30 shown in Fig.75, the cuff 45 is placed to contact the outside of the tragus 1, and the light-receiving element 42 is placed on a surface, of the cuff 45, contacting the tragus 1, and the light-emitting element 41 is placed to contact the inside of the tragus 1.

According to the living body information detection apparatus 30, the cuff 45 presses the tragus 1 from the outside of the tragus 1 so that a pressure is applied to the tragus 1 in the same way as the embodiment 8, and the pulse wave can be detected by the light-receiving element 42 placed in the outside of the tragus 1 and the light-emitting element 41 placed in the inside of the tragus 1. Thus, based on a principle similar to the principle described in the embodiment 8, the blood pressure can be measured.

Therefore, according to the living body information detection apparatus of this embodiment, the blood pressure can be measured easily and stably at the tragus of the human body.

### [Embodiment 3-16]

In the living body information detection apparatus described in embodiments 3-3 and 3-4, two cuffs, for applying a pressure on the tragus, that are a first cuff and a second cuff may be placed in the inside part and the outside part respectively that cover the tragus, and the light-emitting element and the light-receiving element may be placed in the first cuff in the inside part. In the configuration, light from the light-emitting element is entered into the living body tissue, the light-receiving element receives the scatted light from the living body tissue, so that the pulse wave can be detected, and the blood pressure can be measured based on a pressure applied to the tragus by the first cuff and the second cuff and the pulse wave at the time.

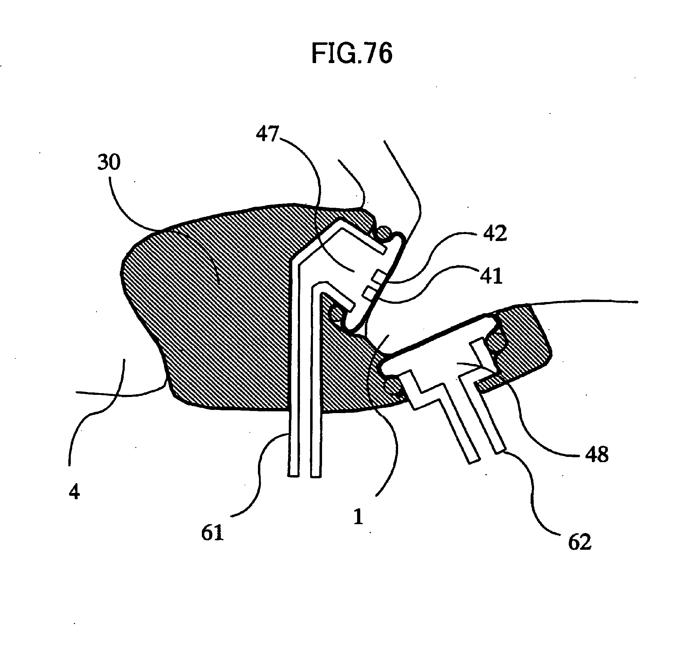

A configuration example and a function of the living body information detection apparatus of this embodiment are described with reference to Fig.76. In Fig.76, 1 indicates the tragus, 4 indicates the antihelix, 30 indicates the living body information detection apparatus, 41 indicates the light-emitting element, 42 indicates the light-receiving element, 47 indicates a cuff as the first cuff, 48 indicates a cuff as the second cuff, 61 indicates an air pipe, and 62 indicates an air pipe. In the living body information detection apparatus 30 shown in Fig.76, the cuff 47 is placed to contact the inside of the tragus 1, and the light-emitting element 41 and the light-receiving element 42 are placed on a surface, in the cuff 47, contacting the tragus 1, the cuff 48 is placed to contact the outside of the tragus 1, the air pipe is connected to the cuff 47, and the air pipe 62 is connected to the cuff 48.

According to the living body information detection apparatus 30, the cuff 47 is supplied with air via the air pipe 61 and the cuff 48 is supplied with air via the air pipe 62 so that the tragus 1 is pressed from both sides, and the pulse wave can be detected by the light-emitting element 41 and the light-receiving element 42. Thus, in the same way as the embodiment 8, the blood pressure can be measured based on the pressure applied to the tragus 1 by the cuff 47 and the cuff 48 and the pulse wave at the time.

Therefore, according to the living body information detection apparatus of this embodiment, the blood pressure can be measured easily and stably at the tragus of the human body.

### [Embodiment 3-17]

In the living body information detection apparatus described in embodiments 3-3 and 3-4, two cuffs, for applying a pressure on the tragus, that are a first cuff and a second cuff may be placed in the inside part and the outside part respectively that covers the tragus, and the light-emitting element and the light-receiving element may be placed in the second cuff in the outside part. In the configuration, light from the light-emitting element is entered into the living body tissue, the light-receiving element receives the scatted light from the living body tissue, so that the pulse wave can be detected, and the blood pressure can be measured based on a pressure applied to the tragus by the first cuff and the second cuff and the pulse wave at the time.

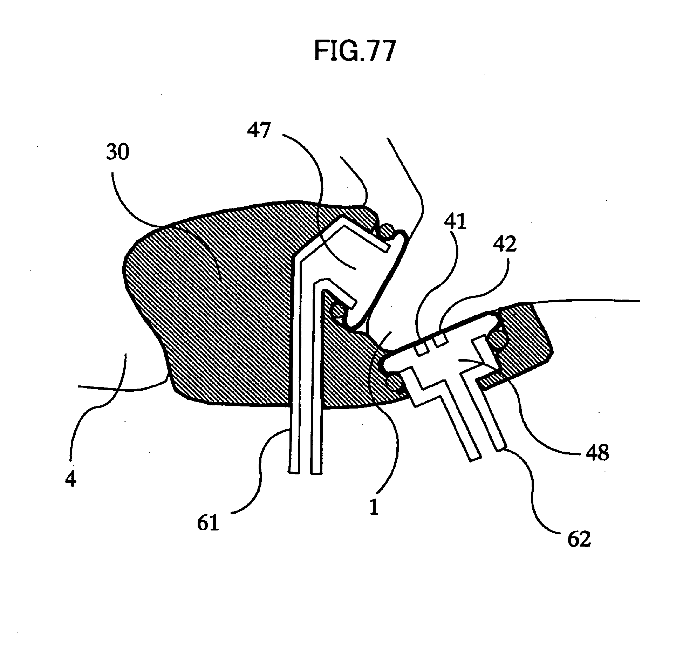

A configuration example and a function of the living body information detection apparatus of this embodiment are described with reference to Fig.77. In Fig.77, 1 indicates the tragus, 4 indicates the antihelix, 30 indicates the living body information detection apparatus, 41 indicates the light-emitting element, 42 indicates the light-receiving element, 47 indicates a cuff as the first cuff, 48 indicates a cuff as the second cuff, 61 indicates an air pipe, and 62 indicates an air pipe. In the living body information detection apparatus 30 shown in Fig.77, the cuff 47 is placed to contact the inside of the tragus 1, the cuff 48 is placed to contact the outside of the tragus 1 and the light-emitting element 41 and the light-receiving element 42 are placed on a surface, in the cuff 48, contacting the tragus 1, and, the air pipe 61 is connected to the cuff 47, and the air pipe 62 is connected to the cuff 48.

According to the living body information detection apparatus 30, the cuff 47 is supplied with air via the air pipe 61 and the cuff 48 is supplied with air via the air pipe 62 so that the tragus 1 is pressed from both sides, and the pulse wave can be detected by the light-emitting element 41 and the light-receiving element 42 in the cuff 48. Thus, in the same way as the embodiment 8, the blood pressure can be measured based on the pressure applied to the tragus 1 by the cuff 47 and the cuff 48 and the pulse wave at the time.

Therefore, according to the living body information detection apparatus of this embodiment, the blood pressure can be measured easily and stably at the tragus of the human body.

### [Embodiment 3-18]

In the living body information detection apparatus described in embodiments 3-3 and 3-4, two cuffs, for applying a pressure on the tragus, that are a first cuff and a second cuff may be placed in the inside part and the outside part respectively that cover the tragus, the light-emitting element may be placed in the first cuff in the inside part and the light-receiving element may be placed in the second cuff in the outside part. In the configuration, light from the light-emitting element is entered into the living body tissue, the light-receiving element receives the scatted light from the living body tissue, so that the pulse wave can be detected, and the blood pressure can be measured based on a pressure applied to the tragus by the first cuff or the second cuff and the pulse wave at the time.

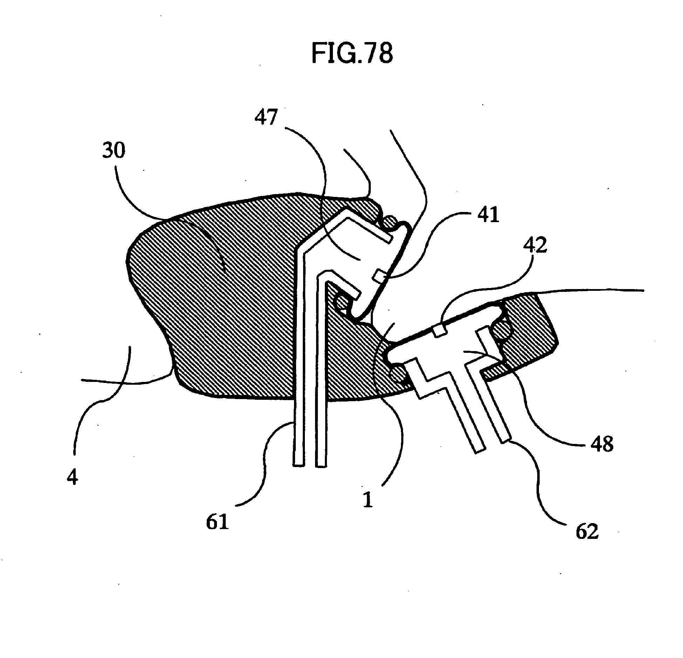

A configuration example and a function of the living body information detection apparatus of this embodiment are described with reference to Fig.78. In Fig.78, 1 indicates the tragus, 4 indicates the antihelix, 30 indicates the living body information detection apparatus, 41 indicates the light-emitting element, 42 indicates the light-receiving element, 47 indicates a cuff as the first cuff, 48 indicates a cuff as the second cuff, 61 indicates an air pipe, and 62 indicates an air pipe. In the living body information detection apparatus 30 shown in Fig.78, the cuff 47 is placed to contact the inside of the tragus 1, the cuff 48 is placed to contact the outside of the tragus 1, the light-emitting element 41 is placed on a surface, in the cuff 47, contacting the tragus 1, the light-receiving element 42 is placed on a surface, in the cuff 48, contacting the tragus 1, and, the air pipe 61 is connected to the cuff 47, and the air pipe 62 is connected to the cuff 48.

According to the living body information detection apparatus 30, the cuff 47 is supplied with air via the air pipe 61 and the cuff 48 is supplied with air via the air pipe 62 so that the tragus 1 is pressed from both sides, and the pulse wave can be detected by the light-emitting element 41 and the light-receiving element 42 in the cuff 48. Thus, in the same way as the embodiment 8, the blood pressure can be measured based on the pressure applied to the tragus 1 by the cuff 47 and the cuff 48 and the pulse wave at the time.

Therefore, according to the living body information detection apparatus of this embodiment, the blood pressure can be measured easily and stably at the tragus of the human body.

### [Embodiment 3-19]

In the living body information detection apparatus described in embodiments 3-3 and 3-4, two cuffs, for applying a pressure on the tragus, that are a first cuff and a second cuff may be placed in the inside part and the outside part respectively that cover the tragus, the light-receiving element may be placed in the first cuff in the inside part and the light-emitting element may be placed in the second cuff in the outside part. In the configuration, light from the light-emitting element is entered into the living body tissue, the light-receiving element receives the scatted light from the living body tissue, so that the pulse wave can be detected, and the blood pressure can be measured based on a pressure applied to the tragus by the first cuff or the second cuff and the pulse wave at the time.

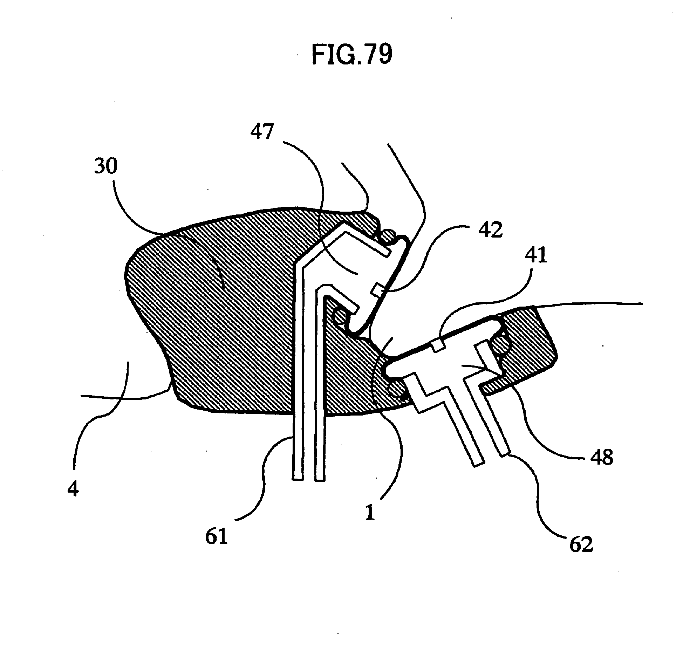

A configuration example and a function of the living body information detection apparatus of this embodiment are described with reference to Fig.79. In Fig.79, 1 indicates the tragus, 4 indicates the antihelix, 30 indicates the living body information detection apparatus, 41 indicates the light-emitting element, 42 indicates the light-receiving element, 47 indicates a cuff as the first cuff, 48 indicates a cuff as the second cuff, 61 indicates an air pipe, and 62 indicates an air pipe. In the living body information detection apparatus 30 shown in Fig.78, the cuff 47 is placed to contact the inside of the tragus 1, the cuff 48 is placed to contact the outside of the tragus 1, the light-receiving element 42 is placed on a surface, in the cuff 47, contacting the tragus 1, the light-emitting element 42 is placed on a surface, in the cuff 48, contacting the tragus 1, and, the air pipe 61 is connected to the cuff 47, and the air pipe 62 is connected to the cuff 48.

According to the living body information detection apparatus 30, the cuff 47 is supplied with air via the air pipe 61 and the cuff 48 is supplied with air via the.air pipe 62 so that the tragus 1 is pressed from both sides, and the pulse wave can be detected by the light-receiving element 42 in the cuff 47 and the light-emitting element 41 in the cuff 48. Thus, in the same way as the embodiment 8, the blood pressure can be measured based on the pressure applied to the tragus 1 by the cuff 47 and the cuff 48 and the pulse wave at the time.

Therefore, according to the living body information detection apparatus of this embodiment, the blood pressure can be measured easily and stably at the tragus of the human body.

### [Embodiment 3-20]

In the living body information detection apparatus described in the embodiments 3-8, 3-9 and 3-12 - 3-19, when one or two cuffs are placed and the light-emitting element or the light-receiving element is placed in at least one of cuffs, it is preferable that the light-emitting element or the light-receiving element is fixed to the cuff for applying a pressure so as to move the light-emitting element or the light-receiving element with the cuff when applying or reducing the pressure, to obtain the blood pressure based on the pressure to the tragus applied by the cuff and the pulse wave at the time.

In the living body information detection apparatus in this embodiment, the light-emitting element or the light-receiving element is placed on an inner surface or an outer surface, of the cuff, at which the cuff contacts the tragus, and the light-emitting element or the light-receiving element is fixed to the cuff.

Therefore, according to the living body information detection apparatus of this embodiment, since the light-emitting element or the light-receiving element is fixed to the cuff, the light-emitting element or the light-receiving element moves in the same way as the cuff. Since the light-emitting element or the light-receiving element surely contacts the tragus, the blood pressure can be measured stably.

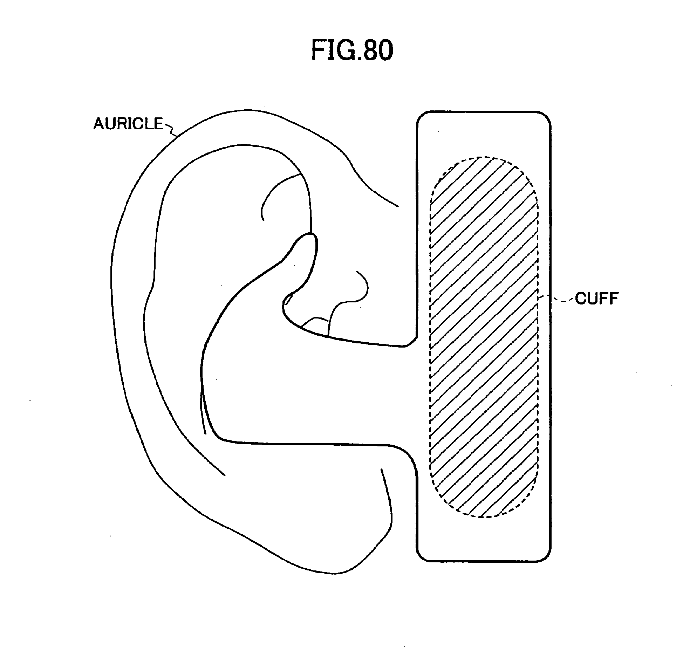

The cuff in the outside part described in embodiments 3-9, 3-11, 3-14, 3-15, 3-16, 3-17, 3-18, 3-19 and 3-20 can be placed in or expanded to the periphery part of the external ear shown in Fig.60. An example of the living body information detection apparatus of this case is shown in Fig.80.

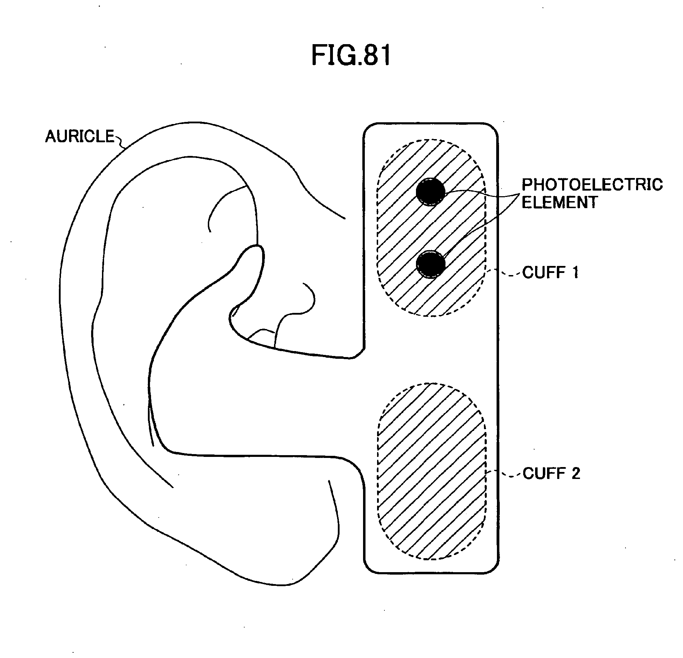

In addition, in this case, it is preferable that the photoelectric elements are placed in a center part of the cuff or in a part where a cuff pressure is evenly applied so as to be opposite to the part. The outside cuff may be divided into a plurality of outside cuffs. In this case, as shown in Fig.81, it is preferable that the photoelectric elements are placed in a cuff in the lower side (peripheral side) of bloodstream.

As described above, according to the living body information detection apparatus of this embodiment, the living body information detection apparatus that can be worn in the auricle using the depression in the periphery of the concha auriculae is provided. Since the living body information detection apparatus is held by the depression in the periphery of the concha auriculae, living body information can be stably detected at the auricle. Especially, by adopting a shape that covers the tragus as the shape of the living body information detection apparatus, and by providing a part that contacts the tragus with a living body information detection sensor, the pulse wave can be detected and the blood pressure can be measured at the tragus. Therefore, the measurement result is almost insensitive to vibration and the like, so that the blood pressure can be measured easily and stably.

In addition, the living body information detection apparatus of this embodiment can be applied to health appliances for detecting living body information for the purpose of health keeping or health checkup.

### (Fourth embodiment)

Next, the living body information detection apparatus of the fourth embodiment is described. The structure and names of each part of the auricle are as shows in Figs.56-60. By the way, in this embodiment, "inside of tragus" means a side of the cavity of the concha 8 of the tragus 1, and "outside of tragus" means an opposite side of the cavity of the concha 8 of the tragus 1.

In the following, the living body information detection apparatus of this embodiment is described. The living body information detection apparatus of this embodiment includes a pair of opposed arms, a spindle connecting the pair of the arms at one end of each of the arms, a distance variable mechanism that is provided in the spindle and that adjusts an interval between other ends of the pair of arms, and a sensor, for detecting living body information, that is attached to the other end of at least one of the arms of the pair in an opposed side of the pair of arms.

In addition, the living body information detection apparatus may further include a rotation mechanism for rotating at least one of the arms of the pair using the spindle as a center axis.

In the living body information detection apparatus of this embodiment, each end of the pair of the opposed arms is connected to the spindle so as to almost form a so-called U-shape. The distance variable mechanism is provided for changing the interval between the opposed surfaces of the arms of the pair by changing an angle between an arm of the pair and the spindle or by sliding one of the arms of the pair in an axis direction of the spindle. Further, the rotation mechanism is provided for moving at least one of the arms of the pair in a rotation direction using the spindle as a center axis so as to change an angle between orientations of the arms of the pair being viewed from an axis direction of the spindle, and the sensor is provided on an opposed surface of at least one arm of the arms of the pair.

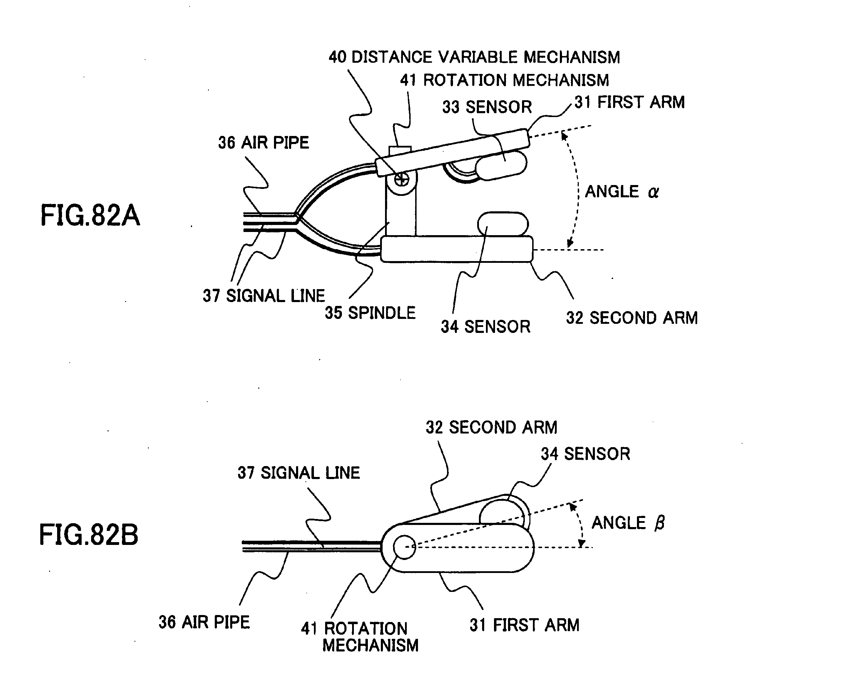

Fig.82 shows a configuration example of the living body information detection apparatus in this embodiment. Figs.82A and 82B are figures showing the configuration example of the living body information detection apparatus 30. In the following description, a view of the living body information detection apparatus 30 viewed from a direction shown in Fig.82A is called a front view, and a view of the living body information detection apparatus 30 viewed from a direction shown in Fig.82B is called a plan view.

In Figs.82A and 82B, 31 indicates a first arm, 32 indicates a second arm, 33 indicates a sensor, 34 indicates a sensor, 35 indicates a spindle, 36 indicates an air pipe, 37 indicates signal lines, 40 indicates a distance variable mechanism, and 41 indicates a rotation mechanism. Also in the following figures in the fourth embodiment, the same numbers indicate the same meaning.

As shown in Fig.82A, the living body information detection apparatus 30 includes the first arm 31, the second arm 32, and the spindle 35, in which one end of each of the first arm 31 and the second arm 32 is connected to the spindle 35.

The living body information detection apparatus 30 of this embodiment includes the distance variable mechanism, for adjusting an interval between other ends of the first arm 31 and the second arm that are opposed to each other, at a part at which each of the first arm 31 and the second arm 32 is connected to the spindle 35, or at the spindle. In the configuration example of the living body information detection apparatus shown in Fig.82A, the distance variable mechanism is provided at a part at which the first arm 31 is connected to the spindle 35 as a variable mechanism for changing a distance between surfaces on which the first arm 31 and the second arm are opposed to each other. The distance variable mechanism 40 has a function for adjusting the interval between the surfaces on which the first arm 31 and the second arm 32 are opposed to each other by changing the angle between the spindle 35 and the first arm 31 so as to change the angle α shown in Fig.82A.

As a mechanism for making the angle adjustable in the distance variable mechanism 40, any mechanism can be adopted such as a mechanism for adjusting the angle between the spindle 35 and the first arm 31 using a screw, a mechanism for using friction together with screw fixing. Further, a mechanism for expanding and contracting the length of the spindle 35 can be used as a mechanism for adjusting the interval of the other ends at which the first arm 31 and the second arm 32 are opposed to each other.

In addition, the living body information detection apparatus 30 shown in Fig.82A includes the rotation mechanism 41, for rotating the first arm 31 using the spindle 35 as an axis, at a part at which the first arm 31 is connected to the spindle 35. The rotation mechanism 41 includes a function for changing an angle β between orientation of the first arm 31 and orientation of the second arm 32 being viewed from an axial direction of the spindle 35 shown in Fig.82B. By the way, it is optional to provide the rotation mechanism 41.

At least one of the first arm 31 and the second arm 32 is provided with a sensor on an surface opposed to other arm. In the configuration example of the living body information detection apparatus 30 shown in Fig.82A, a sensor 33 and a sensor 34 are provided for the first arm 31 and the second arm 32.

Each of the sensor 33 and the sensor 34 shown in Fig.82A may be one of various sensors such as a blood pressure sensor including a cuff for applying a pressure, a temperature sensor and a pulse sensor. The example of the living body information detection apparatus 30 shown in Fig.82A shows a case in which a blood pressure sensor including cuffs for applying a pressure is provided as the sensor 33 and the sensor 34. The air pile and the signal line 37 are connected to each of the sensor 33 and the sensor 34. Each of the air pipe 36 and the signal line 37 passes through the inside of the first arm 31 and the second arm 32, and pulled out to the outside at other end of each of the first arm 31 and the second arm 32. The air pipe 36 and the signal line 37 connected to the sensor 33, and the air pipe 36 and the signal line 37 connected to the sensor 34 are connected respectively and further extended.

Fig.82 and figures hereinafter do not show a storing part for strong detection results of living body information related to the living body information detection apparatus 30, a display part, a power supply part and other parts that can be realized by general technology.

The living body information detection apparatus 30 has a function for detecting the living body information by bringing the sensor 33 and the sensor 34 into contact with a protruding portion in the auricle of the human body, for example, contact with both sides of the tragus 1 of the auricle. When bringing the sensor 33 and the sensor 34 into contact with the both sides of the tragus 1, the interval between the sensor 33 and the sensor 34 is adjusted into a proper contacting state by changing the interval between the opposed surfaces of the first arm 31 and the second arm 32 by the distance variable mechanism 40. In addition, the positions which the sensor 33 and the sensor 34 contact are properly adjusted by changing the angle β shown in Fig.82B by the rotation mechanism 41.

The following embodiments are described in which the tragus of the auricle is adopted as the protruding portion of the auricle of the human body.

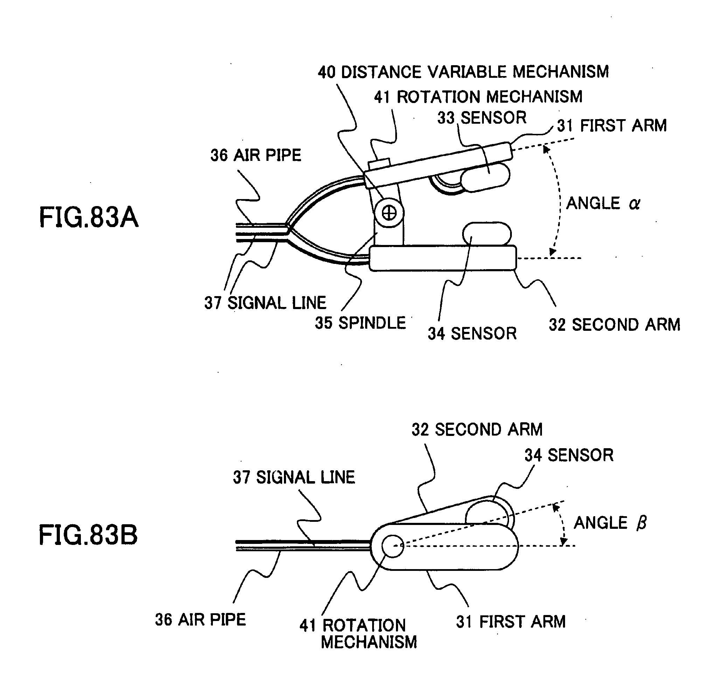

Fig.83 shows another configuration example of the living body information detection apparatus in this embodiment. Figs.83A and 83B are figures showing the configuration example of the living body information detection apparatus 30. In Figs.83A and 83B, 31 indicates a first arm, 32 indicates a second arm, 33 indicates a sensor, 34 indicates a sensor, 35 indicates a spindle, 36 indicates an air pipe, 37 indicates signal lines, 40 indicates a distance variable mechanism, and 41 indicates a rotation mechanism.

As shown in Fig.83A, the living body information detection apparatus 30 includes the first arm 31, the second arm 32, and the spindle 35, in which one end of each of the first arm 31 and the second arm 32 is connected to the spindle 35.

The differences from the living body information detection apparatus described in Figs.82A and 82B are the spindle 35 and the distance variable mechanism 40. Namely, the spindle 35 is divided into two parts that are connected by the distance variable mechanism 40. The distance variable mechanism 40 has a function for adjusting the interval between the surfaces on which the first arm 31 and the second arm 32 are opposed to each other by changing the angle α between the first arm 31 and the second arm 32 shown in Fig.83A. The spindle 35 may be fixed by friction as shown in Fig.83A, or may be fixed by a screw, or both of them may be used together. By the way, it is optional to provide the rotation mechanism 41.

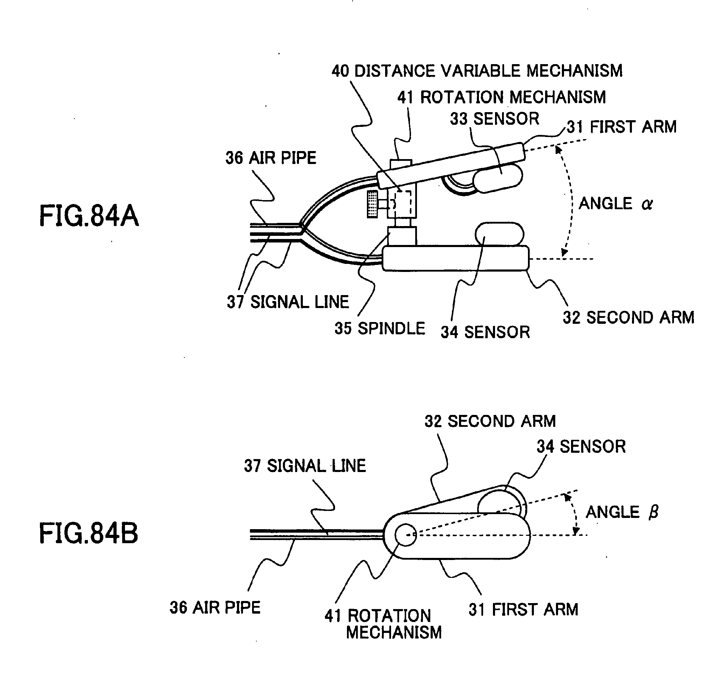

Fig.84 shows another configuration example of the living body information detection apparatus in this embodiment. Figs.84A and 84B are figures showing the configuration example of the living body information detection apparatus 30. In Figs.84A and 84B, 31 indicates a first arm, 32 indicates a second arm, 33 indicates a sensor, 34 indicates a sensor, 35 indicates a spindle, 36 indicates an air pipe, 37 indicates signal lines, 40 indicates a distance variable mechanism, and 41 indicates a rotation mechanism.

As shown in Fig.84A, the living body information detection apparatus 30 includes the first arm 31, the second arm 32, and the spindle 35, in which one end of each of the first arm 31 and the second arm 32 is connected to the spindle 35.

The differences from the living body information detection apparatus described in Figs.82A and 82B are the spindle 35 and the distance variable mechanism 40. Namely, the spindle 35 is divided into two parts that are connected by the distance variable mechanism 40. The distance variable mechanism 40 extends or contracts the length of the spindle 35 so as to adjust the interval of the other ends at which the first arm 31 and the second arm32 are opposed to each other. The spindle 35 may be fixed by a screw as shown in Fig.84A, or may be fixed by friction, or both of them may be used together. By the way, it is optional to provide the rotation mechanism 41.

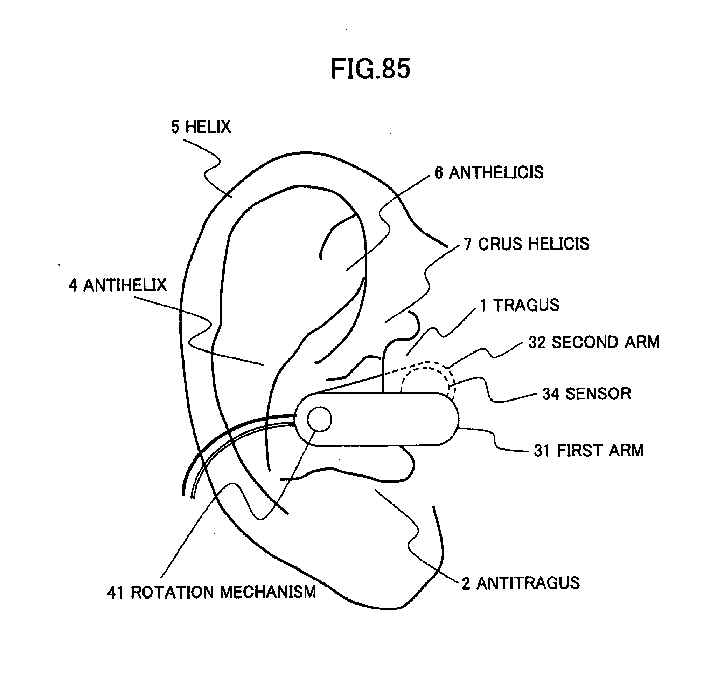

Fig.85 shows an example for placing the living body information detection apparatus 30 in the auricle. As shown in Fig.85, the living body information detection apparatus 30 is worn so as to be brought into contact with the tragus 1 from both sides. The living body information detection apparatus 30 is worn so that the sensor 33 of the first arm 31 contacts the outside of the tragus 1 and the sensor 34 of the second arm 32 contacts the inside of the tragus 1. A part of the second arm 32 and the sensor are drawn with dotted lines since they exists in the inside of the tragus 1.

It is assumed that the sensor 33 and the sensor 34 of the living body information detection apparatus 30 shown in Figs.82A, 83A and 84A are a blood pressure sensor including cuffs for applying a pressure. A method for measuring a blood pressure using the blood pressure sensor including the cuffs that apply a pressure is described later.

As mentioned above, when the living body information detection apparatus 30 of this embodiment is worn on a part of the living body, for example, on both sides of the tragus 1 of the auricle so as to detect the living body information, since positions of the sensor 33 and the sensor 34 are adjusted by the distance variable mechanism or the rotation mechanism 41 according to individual difference of the shape of the tragus 1, the sensor 33 and the sensor 34 can be placed at proper positions in the tragus 1 and in a proper contacting state. By the way, it is optional to provide the rotation mechanism 41.

As described above, the living body information detection apparatus of the present invention is small and light, and can detect living body information stably.

In the living body information detection apparatus of the present invention, the sensor may be mounted on a top of an adjustment screw that is attached to a screw hole passing through the other end of the arm. By the adjustment screw, the interval between the arm opposed to another arm to which the adjustment screw is attached and the sensor can be adjusted. The adjustment screw for adjusting the interval between the arm opposed to another arm to which the adjustment screw is attached and the sensor is further provided.

The living body information detection apparatus of this embodiment is provided with an adjustment screw in one of the first arm and the second arm, or in each of the first arm and the second arm, wherein a sensor is mounted on the adjustment screw, and the adjustment screw has a function for adjusting one of an interval between the sensor and the surface of the first arm and an interval between the sensor and the surface of the second arm, or adjusting both intervals.

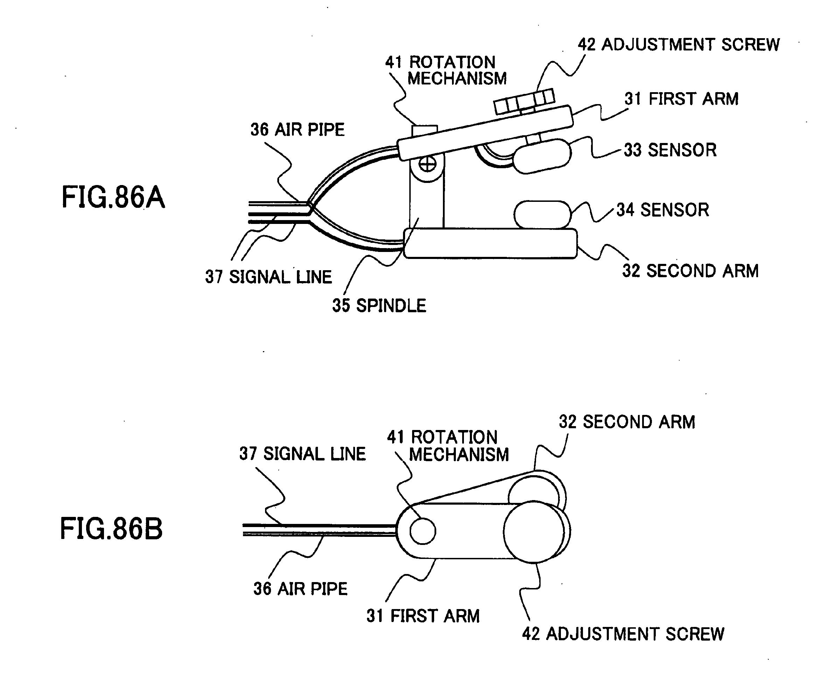

Fig.86A shows a front view of a configuration example of the living body information detection apparatus 30 of this embodiment, and Fig. 86B shows a plan view of a configuration example of the living body information detection apparatus 30 of this embodiment. In Fig.86 and following figures, a part of names are not shown to avoid complexity of the figures. In the configuration example of the living body information detection apparatus 30 shown in Fig.86A and 86B, the first arm 31 of the living body information detection apparatus 30 is provided with an adjustment screw 42, a sensor 33 is mounted on the adjustment screw 42, so that the interval between the sensor 33 and the sensor 34 provided in the second arm 32 is adjusted by the adjustment screw 42. By the way, it is optional to provide the rotation mechanism 41.

The mechanism of the adjustment screw 42 may be a mechanism for adjusting the interval between the sensor 33 and the sensor 34 by adjusting the position of the sensor 33 by rotating the screw, or a mechanism for adjusting the interval between the sensor 33 and the sensor 34 by using a mechanism for fixing with a fixing screw after adjusting the position of the sensor 33 by friction.

As mentioned above, when the living body information detection apparatus 30 of this embodiment is worn in the tragus 1 of the auricle, for example, the interval between the sensor 33 and the sensor 34 can be finely adjusted by the adjustment screw 42 according to individual difference of the shape of the tragus 1, so that the sensor 33 and the sensor 34 can be worn on the tragus 1 with a proper contacting pressure.

As described above, the living body information detection apparatus of the present invention is small and light, and can be worn with a proper contacting pressure according to individual body shape difference so that living body information can be detected stably.

The living body information detection apparatus of the present invention may be configured such that at least one arm of the arms of the pair can change its length from the spindle to the other end.

The living body information detection apparatus of this embodiment is further provided with a mechanism for changing the length of the first arm or the second arm, or both of them, compared with the before-mentioned living body information detection apparatus.

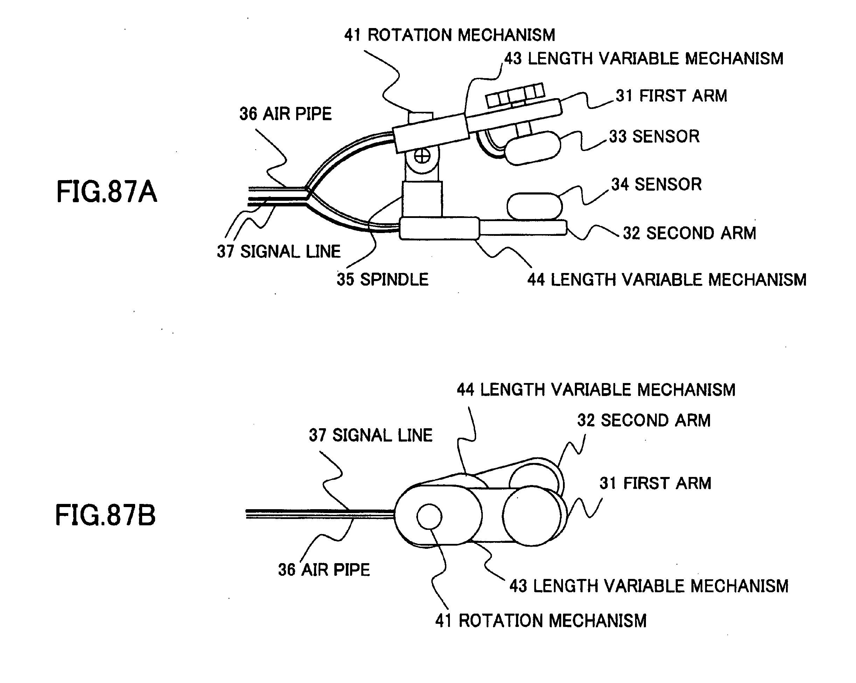

Fig.87A shows a front view of a configuration example of the living body information detection apparatus 30 of this embodiment, and Fig.87B shows a plan view of a configuration example of the living body information detection apparatus 30 of this embodiment. In the case of the configuration example of the living body information detection apparatus 30 shown in Figs.87A and 87B, a length variable mechanism 43 and a length variable mechanism 44 are provided in the first arm 31 and the second arm 32 respectively.

In the case of the length variable mechanism 43 and the length variable mechanism shown in Fig.87A, each of the first arm 31 and the second arm 32 has a two layer structure, so that an arm having a small outer shape is accommodated in an arm having a large outer shape by sliding using a screw mechanism and the like, so that the length of the arm can be variable. The fixing method may be screw fixing or friction. By the way, it is optional to provide the rotation mechanism 41. In the following embodiments, although the rotation mechanism 41 is not referred to, it is optional to provide the rotation mechanism 41.

As described above, the first arm 31 and the second arm 32 are provided with the length variable mechanism 43 and the length variable mechanism 44 respectively, so that the distances between the spindle 35 and the sensor 33 and between the spindle 35 and the sensor 34 are variable. Therefore, for example, when the sensor 33 and the sensor 34 are worn in the tragus 1 of the auricle, the sensor 33 and the sensor 34 can be worn at proper positions according to individual difference of the shape of the tragus 1.

As described above, the living body information detection apparatus of the present invention is small and light, and can be worn with a proper contacting pressure at proper positions according to individual body shape difference so that living body information can be detected stably.

The living body information detection apparatus may be configured such that an arm placed in the inside of the tragus of the human body and an arm placed in the outside of the tragus of the human body in the pair of arms pinch the tragus.

In the living body information detection apparatus of this embodiment, the first arm and the second arm are configured such that an arm placed in the inside of the tragus of the human body and an arm placed in the outside of the tragus of the human body in the pair of arms pinch the tragus.

The first arm and second arm of the living body information detection apparatus of this embodiment are structured as shown in Figs.87A and 87B, for example. Each of the first arm and second arm of the living body information detection apparatus 30 of this embodiment has a shape for pinching a proper part in the inside or the outside of the tragus 1 like the case shown in Fig.85, for example.

As mentioned above, since the first arm 31 and the second arm 32 of the living body information detection apparatus 30 of the present invention are shaped to pinch proper parts of the inside and the outside of the tragus 1, the sensor 33 and the sensor 34 respectively provided in the first arm 31 and the second arm 32 can be worn such that the sensor 33 and the sensor 34 contact proper positions in the inside and the outside of the tragus 1.

As described above, the living body information detection apparatus of the present invention is small and light, and can be worn at proper positions in the tragus of the living body with a proper contacting pressure according to individual body shape difference so that living body information can be detected stably.

In the living body information detection apparatus of the present invention, the arm may include a cushion on the side opposite to the side that is opposed to another arm.

In the living body information detection apparatus of this embodiment, the arm placed in the inside of the tragus of the human body is provided with a cushion, that contacts the auricle, on the side opposite to the side that is opposed to the arm placed in the outside of the tragus of the human body.

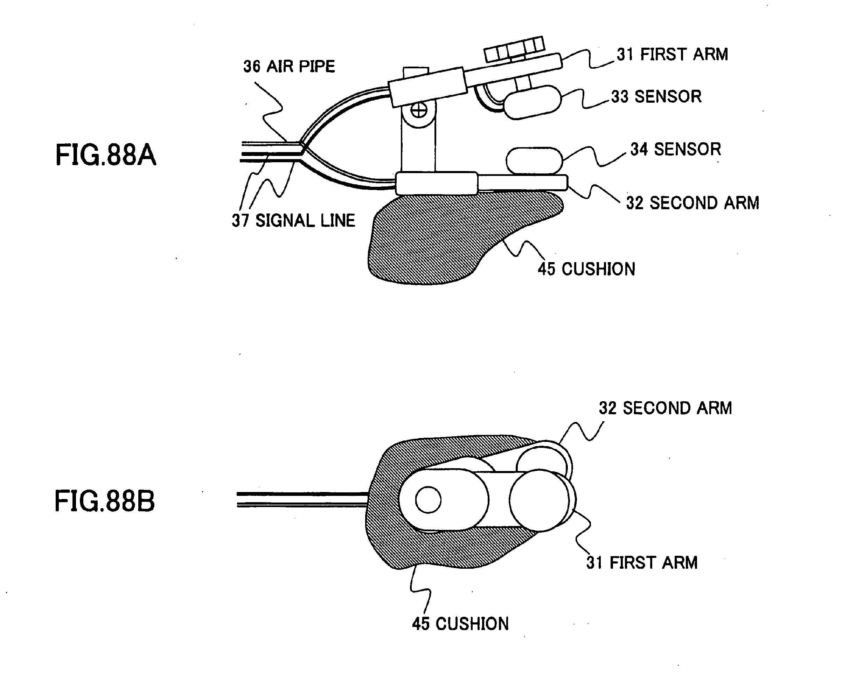

Fig.88A shows a front view of a configuration example of the living body information detection apparatus 30 of this embodiment, and Fig.88B shows a plan view of the configuration example of the living body information detection apparatus 30 of this embodiment. In the configuration example of the living body information detection apparatus 30 of this embodiment shown in Fig.88A, the second arm 32 is provided with a cushion 45 on the side opposite to the side that is opposed to the first arm 31, that is, on the side opposite to the side on which the sensor 34 is placed. In the case when the sensor 34 placed on the second arm 32 is worn so as to contact the inside of the tragus 1, the cushion 45 shown in Figs.88A and 88B is shaped to almost fill a space between the second arm 32 and the concha auriculae 3.

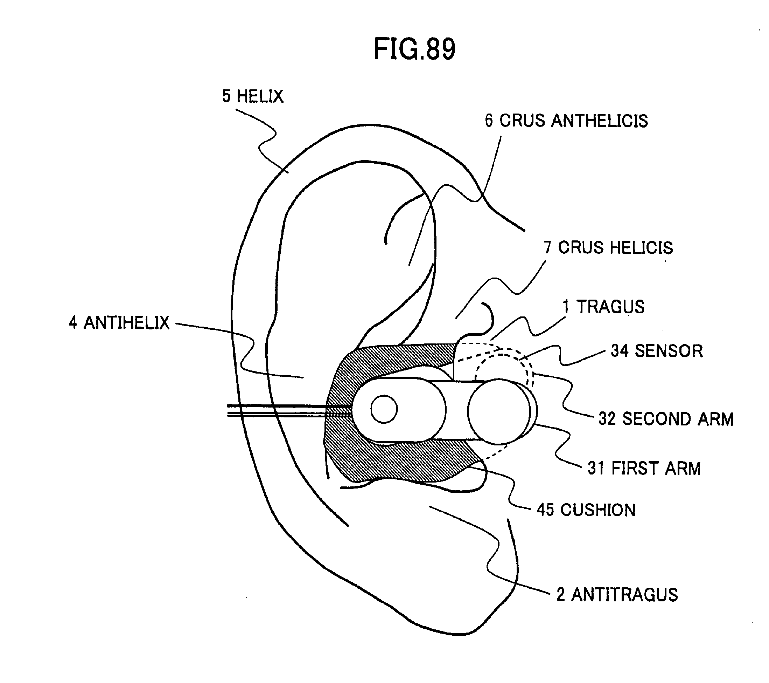

Fig.89 shows a state in which the living body information detection apparatus 30 of this embodiment is worn in the tragus 1 of the auricle. As shown in Fig.89, the first arm 31 of the living body information detection apparatus 30 exists in the outside of the tragus 1, and the second arm 32 exists in the inside of the tragus 1, and the sensor 34 contacts the inside of the tragus 1. The cushion 45 contacts a part in the vicinity of the concha auriculae 3 and has a function for enabling the living body information detection apparatus 30 to be comfortably worn to the auricle. In Fig.89, parts that are a part of the second arm 32, the sensor 34 and a part of the cushion 45 existing in the back side of the tragus 1 are shown with dotted lines.

As mentioned above, in the living body information detection apparatus 30 of this embodiment, the second arm 32 placed in the inside of the tragus of the human body is provided with the cushion 45 that contacts the auricle on the side opposite to the side that is opposed to the first arm 31 placed in the outside of the tragus of the human body. Since the cushion 45 contacts to a part in the vicinity of the concha auriculae 3, the apparatus can be worn comfortably to the living body.

As described above, the living body information detection apparatus of the present invention is small and light, and can be worn with a proper contacting pressure at proper positions of the tragus of the living body according to individual body shape difference so that living body information can be detected stably.

In the living body information detection apparatus of the present invention, the arm placed in the inside of the tragus of the human body may have a shape that follows the concha auriculae or the cavity of the concha of the human body.

In the living body information detection apparatus of this embodiment, the arm placed in the inside of the tragus of the human body has a shape that follows the concha auriculae or the cavity of the concha of the human body.

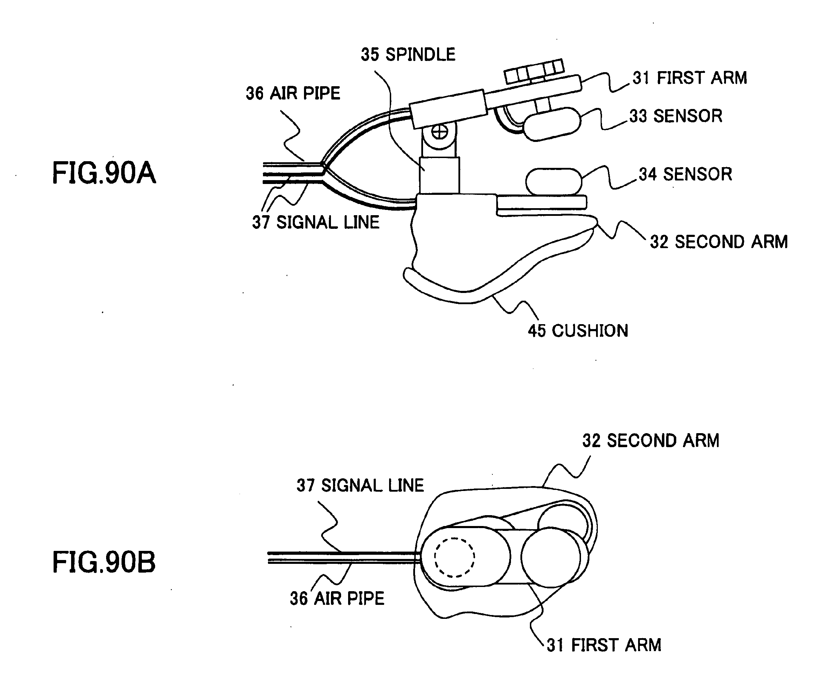

Fig.90A shows a front view of a configuration example of the living body information detection apparatus of this embodiment, and Fig.90B shows a plan view of the configuration example of the living body information detection apparatus 30 of this embodiment. In the configuration example of the living body information detection apparatus 30 shown in Fig.90A, the second arm 32 has a shape that follows the concha auriculae 3 or the cavity of the concha 8 of the human body, and is covered with a cushion 45. In Figs.90A and 90B, although the second arm 32 has a shape that follows the concha auriculae 3 or the cavity of the concha 8 of the human body, and is covered with the cushion 45, the second arm 32 may not be covered with the cushion 45.

As mentioned above, the second arm 32 of the living body information detection apparatus 30 of this embodiment has a shape that follows the concha auriculae 3 and the cavity of the concha 8 of the human body and is possibly covered with the cushion 45. Thus, when the living body information detection apparatus 30 is worn on the tragus 1, the living body information detection apparatus 30 contacts the concha auriculae 3 and the cavity of the concha 8 more stably so that the living body information detection apparatus 30 can detect living body information more stably.

As described above, the living body information detection apparatus of the present invention is small and light, and can be worn comfortably with a proper contacting pressure at proper positions of the tragus of the living body according to individual body shape difference so that living body information can be detected stably and continuously.

The living body information detection apparatus of the present invention may be further provided with an ear suspension for suspending the apparatus from a base of the auricle of the human body.

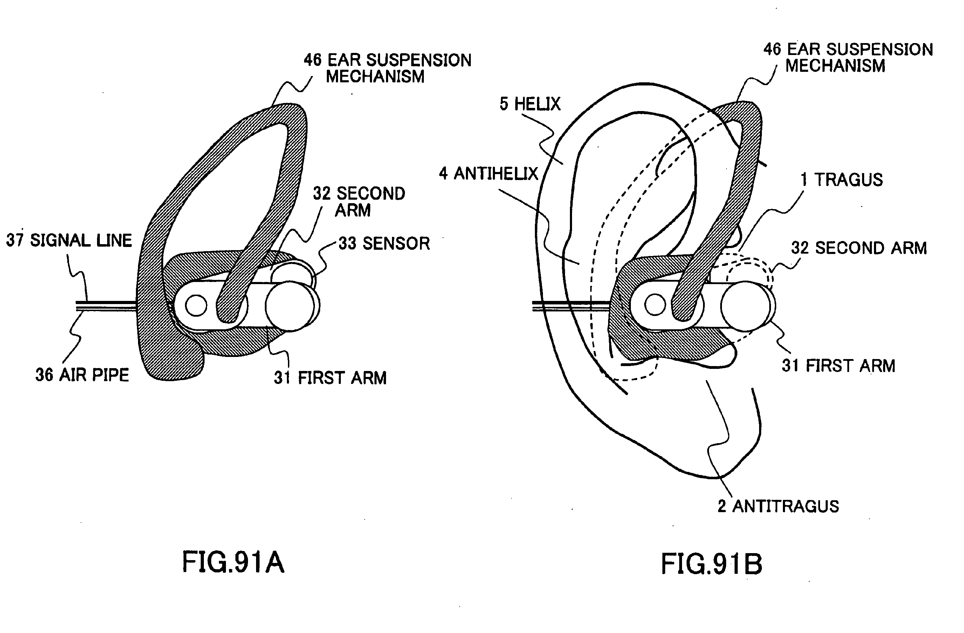

The living body information detection apparatus of this embodiment corresponds to a case in which the before-mentioned living body information detection apparatus is further provided with an ear suspension for suspending the apparatus from the base of the auricle. Fig.91A shows a configuration example of the living body information detection apparatus 30 of this embodiment. Fig.91B shows a state in which the configuration example of the living body information detection apparatus 30 is worn on the auricle. In the configuration example of the living body information detection apparatus 30 shown in Fig.91A, the first arm 31 is provided with an ear suspension mechanism 46. The ear suspension mechanism 46 has a function to curve around from the base of the auricle to the back side of the helix 5 as shown in Fig.91B to fix the living body information detection apparatus 30 to the auricle.

The material of the ear suspension may be metal having plasticity, solder alloy, zinc alloy, brass, copper base alloy, aluminum base alloy, stainless steel, Ni base alloy, tin base alloy, or shape memory alloy. The material of the ear suspension may be resin base that may be plastic, vinyl chloride resin, acrylic resin, ABS resin, MC nylon, fluoroplastics (PTFE), polycarbonate, polypropylene, polyethylene silicone resin, polyurethane resin, or natural rubber. By selecting such material, individual difference of the size of the auricle of the subject can be absorbed.

In addition, the ear suspension mechanism may be structured to be detachable from the living body information apparatus body, so that a ear suspension mechanism having a size suitable for the subject can be selected.

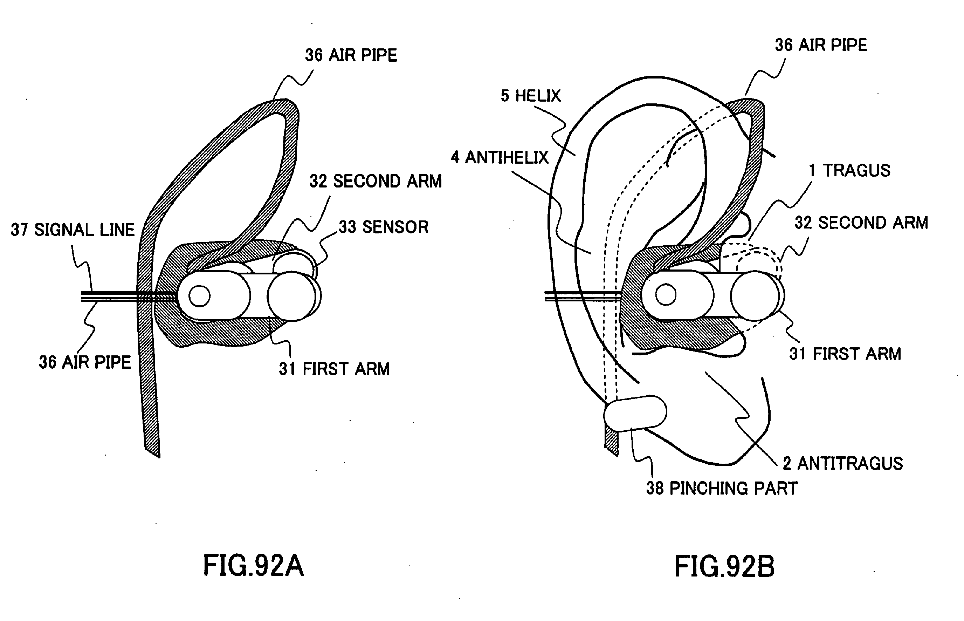

Further, as shown in Figs.92A and 92B, the air pipe 36 or the signal line 37 can be used as the ear suspension mechanism 46 by manufacturing the air pipe 36 or the signal line 37 into a shape of the ear suspension mechanism 46.

By fixing the air pipe 36 to the auricle, the living body information detection apparatus 30 can be stably fixed to the auricle so as to detect living body information more stably, and vibration of the air pile due to body movement of the subject can be reduced so that factors of noise can be reduced.

A pinching part 38 for fixing the air pipe 36 to the earlobe can be provided on the air pipe 36. By providing the pinching part 38, the air pipe 36 is fixed. Thus, vibration of the air pile due to body movement of the subject can be reduced so that factors of noise can be reduced.

As mentioned above, since the living body information detection apparatus 30 further includes the ear suspension mechanism 46 for suspending from the auricle, the living body information detection apparatus 30 can be stably fixed to the auricle so that living body information can be detected more stably.

As described above, the living body information detection apparatus of the present invention is small and light, and can be worn comfortably with a proper contacting pressure at proper positions of the tragus of the living body according to individual body shape difference so that living body information can be detected more stably.

The living body information detection apparatus of the present invention may be further provided with magnets, applying magnetic force with each other, on the ear suspension and the cushion.

The living body information detection apparatus of this embodiment corresponds to a case in which the aforementioned living body information detection apparatus further includes the magnets, applying magnetic force with each other, on the side on which the cushion contacts the auricle and on the side on which the ear suspension mechanism contacts the auricle.

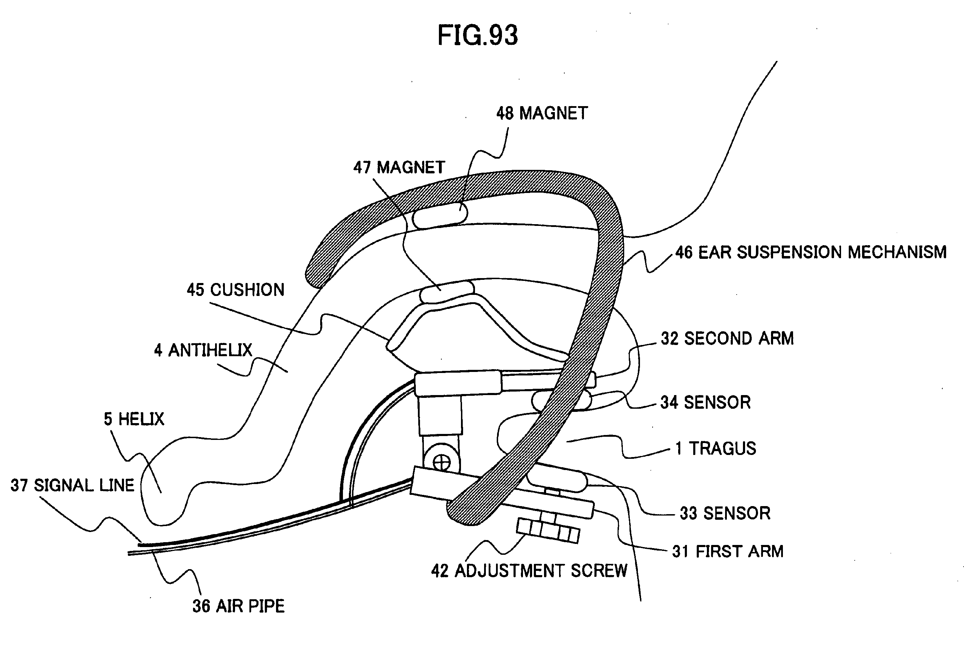

Fig.93 shows the living body information detection apparatus 30 of this embodiment assuming that the living body information detection apparatus 30 is worn in the auricle. The auricle is shown as a section view cut by a horizontal plane near the tragus 1 viewed from an upper side of the head of the living body, and the living body information collecting apparatus 30 is shown as a view of a state in which the apparatus is worn to the human body viewed from an upper side of the head of the living body, and Fig.93 is a schematic diagram showing the both. In Fig.93, the cushion 45 includes a magnet 47 at a position that contacts the auricle, and the ear suspension mechanism 46 includes a magnet at a position that is the back side of the auricle and contacts the auricle.

The magnet 47 and the magnet 48 exist on both sides of the auricle, and are placed in polarity such that magnetic force is applied with each other. The magnet 47 and the magnet 48 are fixed so as to contact the auricle.

As mentioned above, the living body information detection apparatus 30 of this embodiment further includes magnets applying magnetic force with each other on the side on which the cushion contacts the auricle and on the side on which the ear suspension mechanism 46 contacts the auricle. Thus, the living body information detection apparatus 30 can be fixed to the auricle more comfortably so that living body information can be detected more stably.

Although two magnets of the magnet 47 and the magnet 48 are used in Fig.93, alternatively, one may be a magnet and another may be a magnetic material. In addition, each of the magnet 47 and the magnet 48 may be placed in the inside part of the cushion 45 or the ear suspension mechanism 46.

As described above, the living body information detection apparatus of the present invention is small and light, and can be worn more comfortably with a proper contacting pressure at proper positions of the tragus of the living body according to individual body shape difference so that living body information can be detected more stably and continuously.

The living body information detection apparatus of the present invention may include a light shielding cover for shielding at least the sensor from the outside or a light shielding cover for shielding at least the sensor and the tragus of the human body from the outside.

The living body information detection apparatus of this embodiment corresponds to a case in which the aforementioned living body information detection apparatus further includes a light shielding cover for shielding at least each of sensors from the outside, and a light shielding cover for shielding the tragus of the human body from the outside.

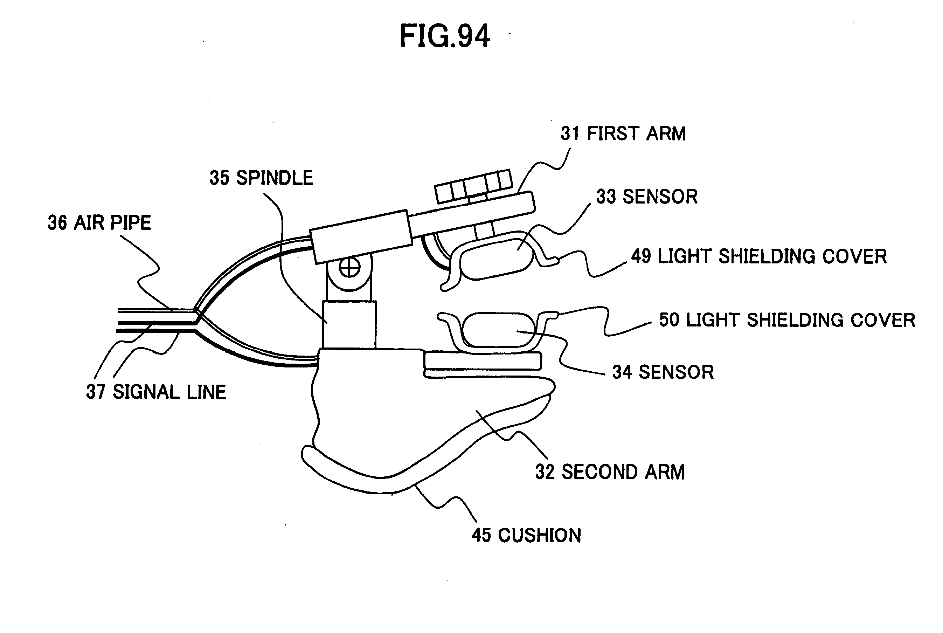

Fig.94 shows a configuration example of the light shielding cover provided for the sensor 33 and the sensor 34 of the living body information detection apparatus 30 of this embodiment. In Fig.94, the sensor 33 and the sensor 34 of the living body information detection apparatus 30 respectively include a light shielding cover 49 and a light shielding cover 50.

The light shielding cover 49 and the light shielding cover 50 are formed of flexible material. When the sensor 33 and the sensor 34 contact the tragus 1 to detect living body information, a periphery of each of the light shielding cover 49 and the light shielding cover 50 contacts the surface of the tragus 1 around each of the sensor 33 and the sensor 34, so that it can be prevented that a surface of each of the sensor 33 and the sensor 34 contacting the surface of the tragus 1 is irradiated with light from the outside. When the sensor 33 and the sensor 34 includes an optical element, the cover prevents a risk of occurrence of error caused by reception of light by the sensor 33 and the sensor 34 from the outside.

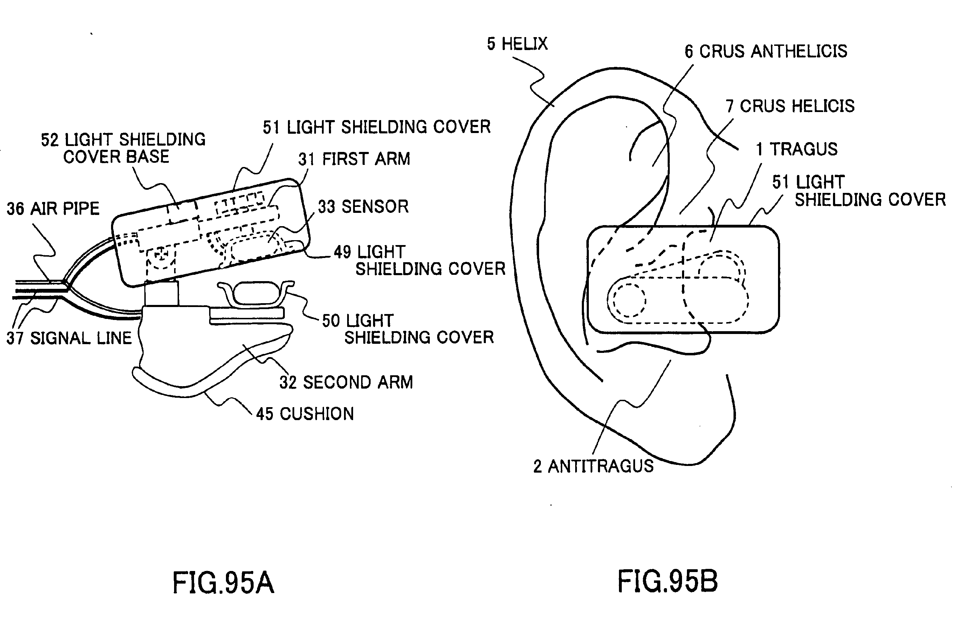

Next, Figs.95A and 95B shows a configuration example of the light shielding cover, for shielding the tragus from the outside, provided in the living body information detection apparatus 30 of this embodiment. As shown in Fig.95A, the light shielding cover 51 has a mechanism for making the cover removable from the first arm 31 using a light shielding cover base 52 provided in the first arm 31. The light shielding cover 51 covers the first arm 31 and the tragus 1 to shield the tragus 1 pinched by the arms from outside light. When the sensor 33 and the sensor 34 includes an optical element, there is a function to prevent the risk of occurrence of error caused by reception of light by the sensor 33 and the sensor 34 from the outside. Fig.95B shows a situation in which the light shielding cover 51 covers the first arm 31 and the tragus 1.

The first arm 31 includes the light shielding cover 49 and the light shielding cover 51 in Fig.95A. But, when the light shielding cover 51 is provided, the function can be realized to prevent the risk of occurrence of error caused by reception of light by the sensor 33 and the sensor 34 from the outside without the light shielding cover 49.

As mentioned above, the living body information detection apparatus 30 of this embodiment includes the light shielding cover 49 and the light shielding cover 50 for shielding at least the sensor 33 and the sensor 34 from the outside. In addition, the light shielding cover 49 and the light shielding cover 50 is provided for shielding at least the sensor 33 and the sensor 34, and the tragus pinched by the arms from the outside. By providing the light shielding cover 51 for shielding the sensor 33, the sensor 34 and the tragus 1 from the outside, interference due to light coming from the outside can be reduced when detecting living body information so that the living body information can be detected with high accuracy.

As described above, the living body information detection apparatus of the present invention is small and light, and can be worn more comfortably with a proper contacting pressure at proper positions of the tragus 1 of the living body according to individual body shape difference so that living body information can be detected more stably and continuously with higher precision.

The living body information detection apparatus of the present invention may further include a speaker for transmitting sound information.

The living body information detection apparatus of this embodiment corresponds to a case in which the aforementioned living body information detection apparatus further includes a speaker for transmitting a sound signal on the second arm having the inside part, for example.

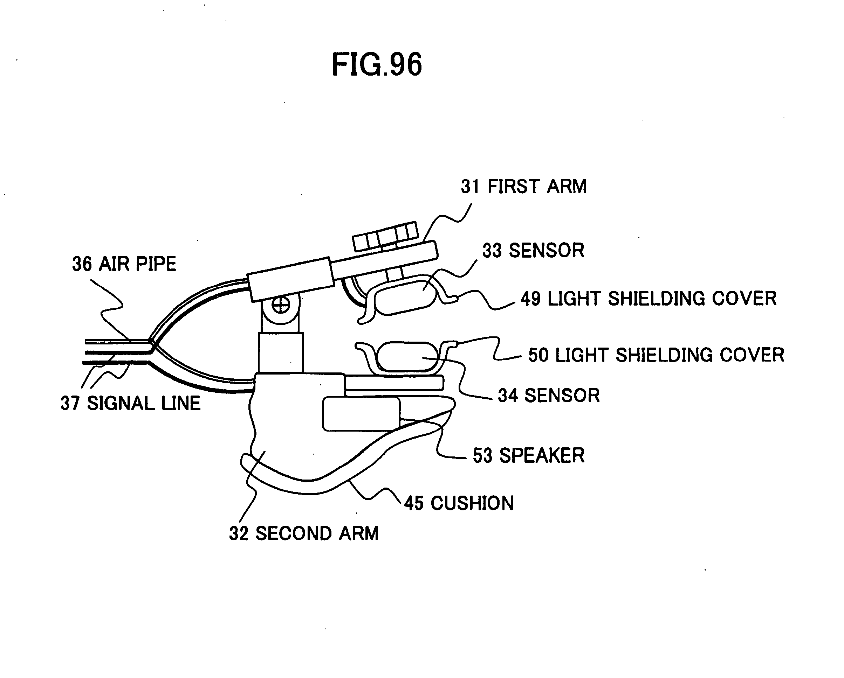

Fig.96 shows a configuration example of the living body information detection apparatus 30 of this embodiment. In Fig.96, the second arm 32 is provided with a speaker 53 for transmitting a sound signal such as voice, music and the like. Fig.96 does not show a signal line of the speaker 53 to avoid complexity of the figure.

The speaker 53 shown in Fig.96 has a function for outputting a sound for informing a person who is not wearing the apparatus of occurrence of emergency and of necessity of an urgent measure when the living body information detection apparatus 30 detects living body information and the detected information shows an abnormal value that requires urgent measure, for example. In addition, based on the obtained living body information, a music suitable for the status of the subject or a music selected by the subject can be output.

As mentioned above, the living body information detection apparatus 30 of this embodiment includes the speaker 53, and when the living body information detection apparatus 30 detects an abnormal state of the living body information, the speaker 30 can inform, by voice, the person who does not wear the apparatus of occurrence of emergency and of necessity of an urgent measure, and can output a music.

As described above, the living body information detection apparatus of the present invention is small and light, and can be worn more comfortably with a proper contacting pressure at proper positions of the tragus of the living body according to individual body shape difference so that living body information can be detected accurately, and more stably, conveniently and continuously.

The sensor of the living body information detection apparatus of the present invention may include a light-emitting element for entering emitted light into a living body tissue of the auricle, and a light-receiving element for receiving scattered light from the living body tissue.

The living body information detection apparatus of this embodiment corresponds to a case in which, in the aforementioned living body information detection apparatus, the sensor includes a light-emitting element for entering emitted light into a living body tissue of the auricle, and a light-receiving element for receiving scattered light from the living body tissue.

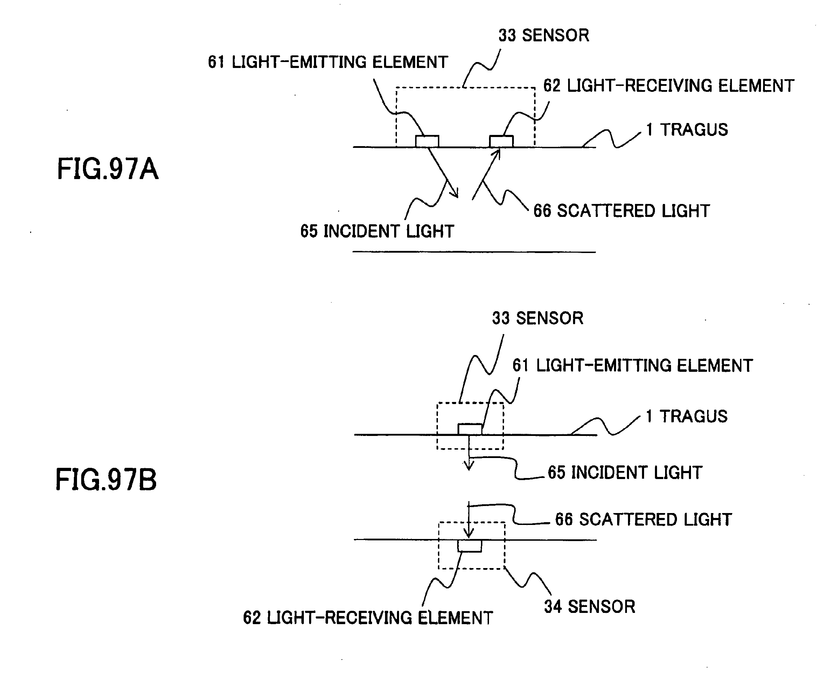

As an example of the sensor of the living body information detection apparatus of this embodiment, the configuration and operation are described with reference to Fig.97 for a case for measuring a pulse wave at the tragus 1.

Figs.97A and 97B show configurations of the sensor 33 and the sensor 34. Fig.97A shows a case where the sensor 33 includes a light-emitting element 61 and a light-receiving element 62. Fig.97B shows a case where the sensor 33 includes the light-emitting element 61 and the sensor 34 includes the light-receiving element 62.

Fig.97A shows, for example, a state in which the light-emitting element 61 and the light-receiving element 62 are placed on a surface on which the sensor 33 contacts the tragus 1 of the auricle, light emitted by the light-emitting element 61 is entered in the tragus 1 as incident light 65 that is scattered by a blood vessel in the tragus 1 or blood corpuscles in the blood vessel, and the scattered light 66 is received by the light-receiving element 62. The incident light 65 enters the tragus 1 from the light-emitting element 61 and the incident light is scattered in the tragus 1, and the light-receiving element 62 is placed at a position so as to receive the scattered light 66.

Fig.97 and following figures do not show circuits that can be realized general technology such as a driving circuit for the light-emitting element 61 and the light-receiving element 62, a signal receiving circuit, a display circuit, and a power supply circuit, and do not show signal lines.

The blood vessel or the blood cells in the blood vessel in the tragus pulsate according to heartbeat, so that the scattered light 66 receives change of strength corresponding to the pulsation or change of optical frequency due to the Doppler effect and is received by the light-receiving element 62. Therefore, by performing photoelectric conversion on the scattered light received by the light-receiving element 62, the pulse wave corresponding to the pulsation of the blood vessel or the blood cells in the blood vessel can be detected. In the following descriptions, the configuration of the light-emitting element 61 and the light-receiving element 62 shown in Fig.97A is called a reflection type pulse wave detection system.

Fig.97B shows, for example, a state in which the light-emitting element 61 is placed on a surface on which the sensor 33 contacts the tragus 1 of the auricle and the light-receiving element 62 is placed on a surface on which the sensor 34 contacts the tragus 1 of the auricle, light emitted by the light-emitting element 61 is entered in the tragus 1 as incident light 65 that is scattered by a blood vessel in the tragus 1 or blood corpuscles the blood vessel, and the scattered light 66 is received by the light-receiving element 62. The incident light 65 enters the tragus 1 from the light-emitting element 61 and the incident light is scattered in the tragus 1, and the light-receiving element 62 is placed at a position opposed to the light-emitting element 61 so as to receive the scattered light 66.

The blood vessel or the blood cells in the blood vessel in the tragus pulsate according to heartbeat, so that the scattered light 66 receives change of strength corresponding to the pulsation or change of optical frequency due to the Doppler effect and is received by the light-receiving element 62. Therefore, by performing photoelectric conversion on the scattered light received by the light-receiving element 62, the pulse wave corresponding to the pulsation of the blood vessel or the blood cells in the blood vessel can be detected. In the following descriptions, the configuration of the light-emitting element 61 and the light-receiving element 62 shown in Fig.97B is called a transmission type pulse wave detection system.

As mentioned above, according to either of the reflection type shown in Fig.97A and the transmission type shown in Fig.97B, the living body information detection apparatus 30 of this embodiment can detect the pulse wave, and can detect the pulse wave more accurately compared with a conventional case for detecting the pulsation of the blood vessel or the blood stream using sound.

As mentioned above, the living body information detection apparatus 30 of this embodiment can detect the pulse wave with high precision by the light-emitting element 62 and the light-receiving element included in the sensor 33 and the sensor 34.

As described above, the living body information detection apparatus of the present invention is small and light, and can be worn more comfortably with a proper contacting pressure at proper positions of the tragus 1 of the living body according to individual body shape difference so that living body information such as the pulse wave can be detected accurately, and more stably, conveniently and continuously.

The living body information detection apparatus of the present invention may include a cuff provided in the inside part for applying a pressure to the tragus, a light-emitting element, provided in the inside of the cuff, for entering output light into a living body tissue of the auricle, a light-receiving element, provided in the inside of the cuff, for receiving scattered light from the living body tissue, and an air pipe for supplying or releasing air in the cuff.

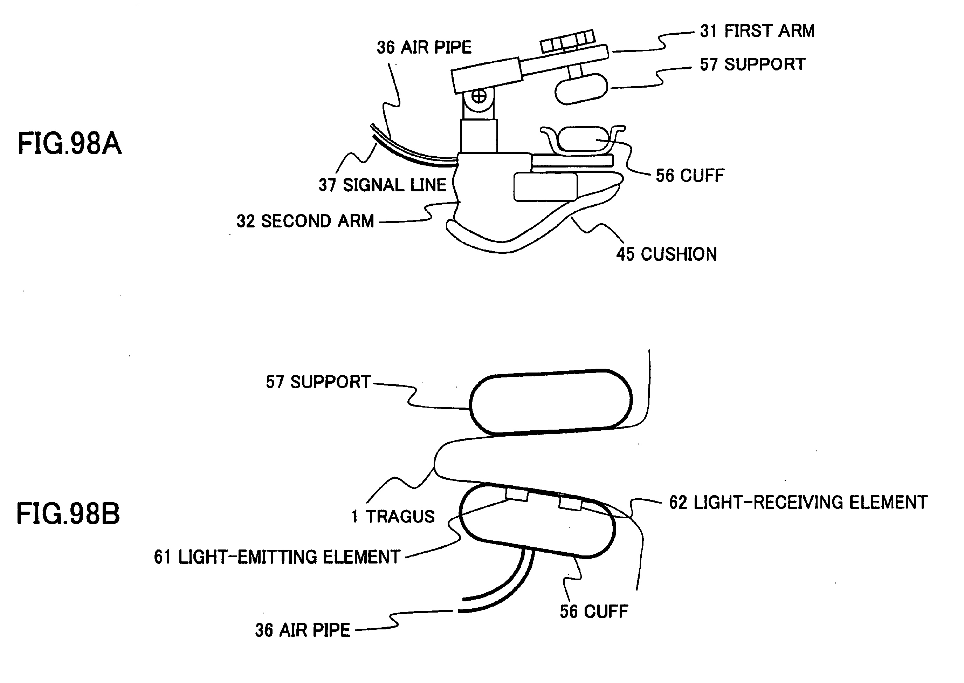

As shown in Figs.98A and 98B, the living body information detection apparatus of this embodiment corresponds to a case in which the aforementioned living body information detection apparatus includes a support 57 instead of the sensor 33 shown in Fig.96, and includes a cuff 56 instead of the sensor 34. The light-emitting element 61 and the light-receiving element 62 are provided in the cuff 56 and the cuff 56 is provided with an air pipe for supplying air. Fig.98B is a magnified view of the part of the support 57 and the cuff 56 in a state in which the living body information detection apparatus 30 shown in Fig.98A is worn at the tragus 1. To avoid complexity of the figure, the light-emitting element 61 and the light-receiving element 62 are not shown in the cuff 56 shown in Fig.98A.

The light-emitting element 61 and the light-receiving element 62 in the cuff 56 shown in Fig.98B forms the reflection type pulse wave detection system described with reference to Fig.97A, and detects the pulse wave. In the process for detecting the pulse wave, by applying a pressure on the tragus 1 by the cuff 56, the blood pressure can be measured by the following method. Any method described so far can be adopted as the method for measuring the blood pressure from the pulse wave.

As described above, the first arm 31 has the support 57 as shown in Fig.98B. But, instead of the support 57, a cuff may be provided so as to apply a pressure from both sides of the tragus 1.

As mentioned above, the living body information detection apparatus 30 of this embodiment includes the cuff 56 provided in the inside part for applying a pressure to the tragus 1, the light-emitting element 61, provided in the inside of the cuff 56, for entering output light in a living body tissue of the auricle, the light-receiving element 62, provided in the inside of the cuff 56, for receiving scattered light from the living body tissue, and the pipe 36 for supplying or releasing air in the cuff 56. The living body information detection apparatus is worn on the tragus 1 so that the cuff 56 applies a pressure on the tragus 1, and the light-emitting element 61 and the light-receiving element 62 that forms the reflection type pulse wave detection system detects the pulse wave, and further, the blood pressure can be measured based on the aforementioned principle from the detected pulse wave.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information detection apparatus of the present invention may include a cuff provided in the outside part for applying a pressure to the tragus, a light-emitting element, provided in the inside of the cuff, for bringing output light to enter a living body tissue of the auricle, a light-receiving element, provided in the inside of the cuff, for receiving scattered light from the living body tissue, and an air pipe for supplying or releasing air in the cuff.

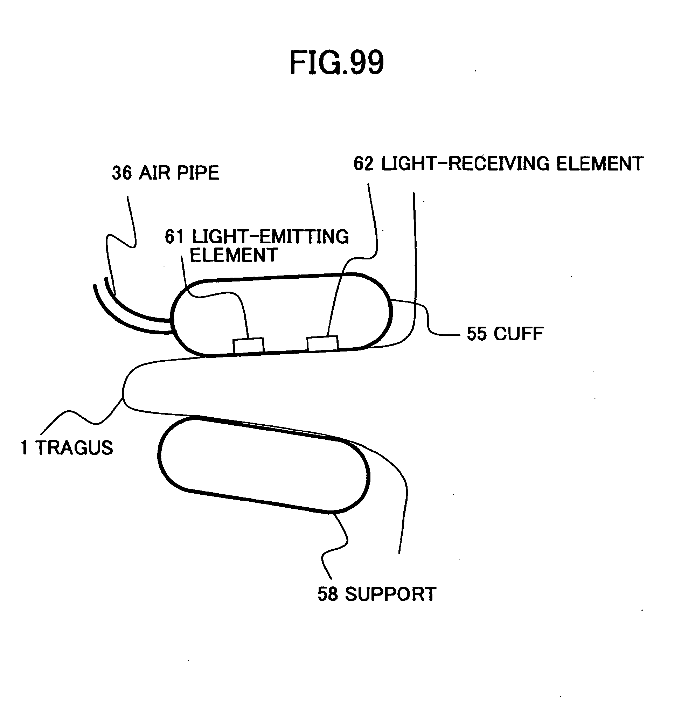

As shown in Fig.99, the living body information detection apparatus of this embodiment corresponds to a case in which the aforementioned living body information detection apparatus includes a cuff 55 instead of the sensor 33 shown in Fig.96, and includes a support 58 instead of the sensor 34. The light-emitting element 61 and the light-receiving element 62 are provided in the cuff 55 and the cuff 55 is provided with an air pipe for supplying air. Fig.99 is a magnified view of the part of the cuff 55 and the support 58.

The cuff shown in Fig.99 applies a pressure on the tragus 1, and the light-emitting element 61 and the light-receiving element 62 in the cuff 55 forms the aforementioned reflection type pulse wave detection system, and detects the pulse wave. The blood pressure can be measured from the detected pulse wave based on the aforementioned principle.

As mentioned above, the living body information detection apparatus 30 of this embodiment includes the cuff 55 provided in the inside part for applying a pressure to the tragus 1, the light-emitting element 61, provided in the inside of the cuff 55, for bringing output light to enter a living body tissue of the auricle, the light-receiving element 62, provided in the inside of the cuff 55, for receiving scattered light from the living body tissue, and the pipe 36 for supplying or releasing air in the cuff 55. The living body information detection apparatus is worn on the tragus 1 so as to detect the pulse wave and measure the blood pressure based on the detected pulse wave.

In the living body information detection apparatus of this embodiment, since relative positions among the light-emitting element 61, the light-receiving element 62, and skin in the inside of the tragus are fixed, it becomes possible to reduce drift of measurement data by the light-receiving element 62 or noise from the surroundings. In addition, by replacing the support on the outside of the tragus with a cuff, since a pulse pressure wave of a smallest artery existing in the outside of the tragus can be efficiently detected by the cuff, it is effective for simultaneously measuring a photoelectric pulse wave on the inside of the tragus and measuring the pulse pressure wave on the outside of the tragus by the cuff at the same time.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information detection apparatus of the present invention may include a cuff provided in the inside part for applying a pressure to the tragus, a light-emitting element, provided in the outside part, for bringing output light to enter a living body tissue of the auricle, a light-receiving element, provided in the outside part, for receiving scattered light from the living body tissue, and an air pipe for supplying or releasing air in the cuff.

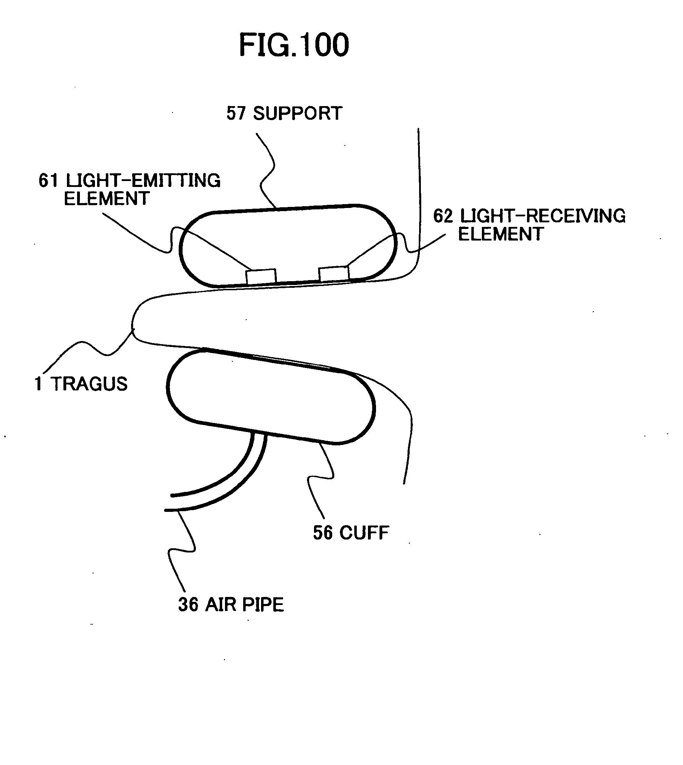

As shown in Fig.100, for example, the living body information detection apparatus of this embodiment corresponds to a case in which the aforementioned living body information detection apparatus includes a support 57 instead of the sensor 33 shown in Fig.96, and includes a cuff 56 instead of the sensor 34. The light-emitting element 61 and the light-receiving element 62 are provided on a surface on which the support 57 contacts the tragus 1, and the cuff 56 is provided with an air pipe 36 for supplying air. Fig.100 is a magnified view of the part of the support 57 and the cuff 56.

The cuff 56 shown in Fig.100 applies a pressure on the tragus 1, and the light-emitting element 61 and the light-receiving element 62 provided on a surface of the support 57 forms the aforementioned reflection type pulse wave detection system, and detects the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information detection apparatus of the present invention may include a cuff, provided in the outside part, for applying a pressure to the tragus, a light-emitting element, provided in the inside part, for bringing output light to enter a living body tissue of the auricle, a light-receiving element, provided in the inside part, for receiving scattered light from the living body tissue, and an air pipe for supplying or releasing air in the cuff.

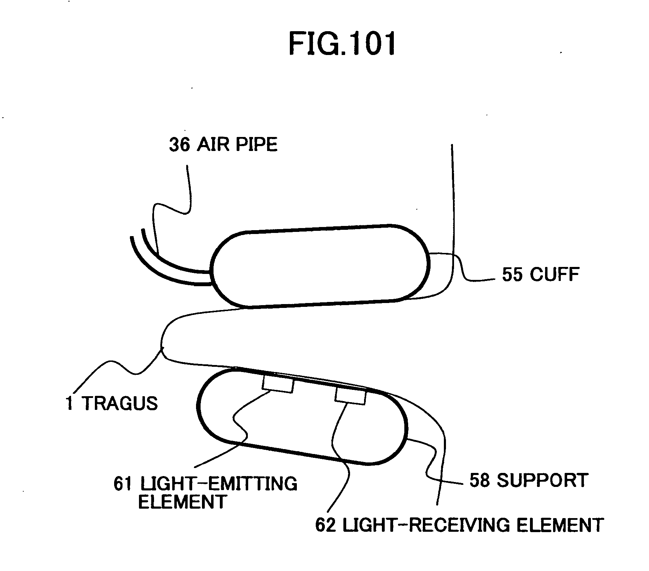

As shown in Fig.101, for example, the living body information detection apparatus of this embodiment corresponds to a case in which the aforementioned living body information detection apparatus includes a cuff 55 instead of the sensor 33 shown in Fig.96, and includes a support 58 instead of the sensor 34. The light-emitting element 61 and the light-receiving element 62 are provided on a surface on which the support 58 contacts the tragus 1, and the cuff 55 is provided with an air pipe 36 for supplying air. Fig.101 is a magnified view of the part of the support 58 and the cuff 55.

The cuff 55 shown in Fig.101 applies a pressure on the tragus 1, and the light-emitting element 61 and the light-receiving element 62 provided on a surface of the support 58 forms the aforementioned reflection type pulse wave detection system, and detects the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus of the living body according to individual body shape difference so that living body information such as the blood pressure, for example, can be detected accurately, and more stably, conveniently and continuously.

The living body information detection apparatus of the present invention may include a cuff, provided in the inside part, for applying a pressure to the tragus, a light-emitting element, provided in the inside of the cuff, for bringing output light to enter a living body tissue of the auricle, a light-receiving element, provided in the outside part, for receiving scattered light from the living body tissue, and an air pipe for supplying or releasing air in the cuff.

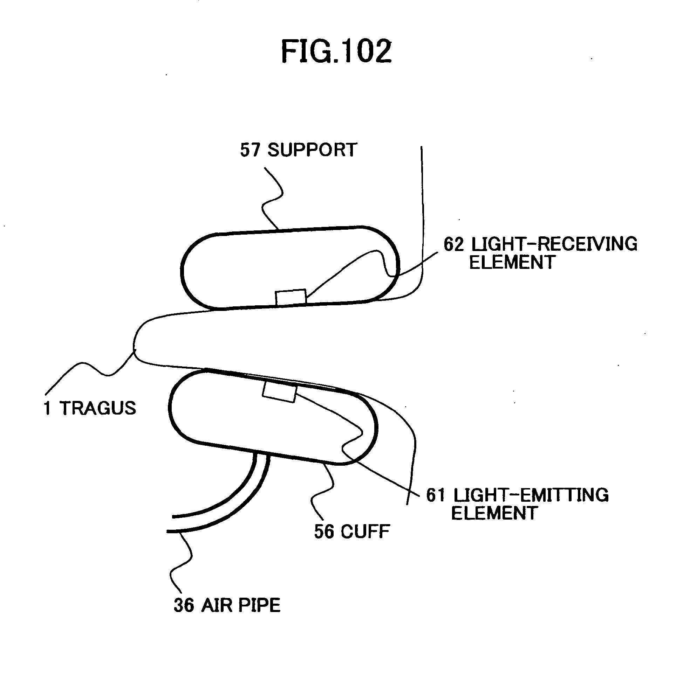

As shown in Fig.102, for example, the living body information detection apparatus of this embodiment corresponds to a case in which the aforementioned living body information detection apparatus includes a support 57 instead of the sensor 33 shown in Fig.96, and includes a cuff 56 instead of the sensor 34. The light-emitting element 61 is provided in the cuff 56, and the light-receiving element 62 is provided on a surface on which the support 57 contacts the tragus 1, and the cuff 56 is provided with an air pipe 36 for supplying air. Fig.102 is a magnified view of the part of the support 57 and the cuff 56.

The cuff 56 shown in Fig.102 applies a pressure on the tragus 1, and the light-emitting element 61 provided in the cuff 56 and the light-receiving element 62 provided on a surface of the support 57 form the aforementioned transmission type pulse wave detection system, and detects the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information detection apparatus of the present invention may include a cuff, provided in the inside part, for applying a pressure to the tragus, a light-emitting element, provided in the outside part, for bringing output light to enter a living body tissue of the auricle, a light-receiving element, provided in the inside of the cuff, for receiving scattered light from the living body tissue, and an air pipe for supplying or releasing air in the cuff.

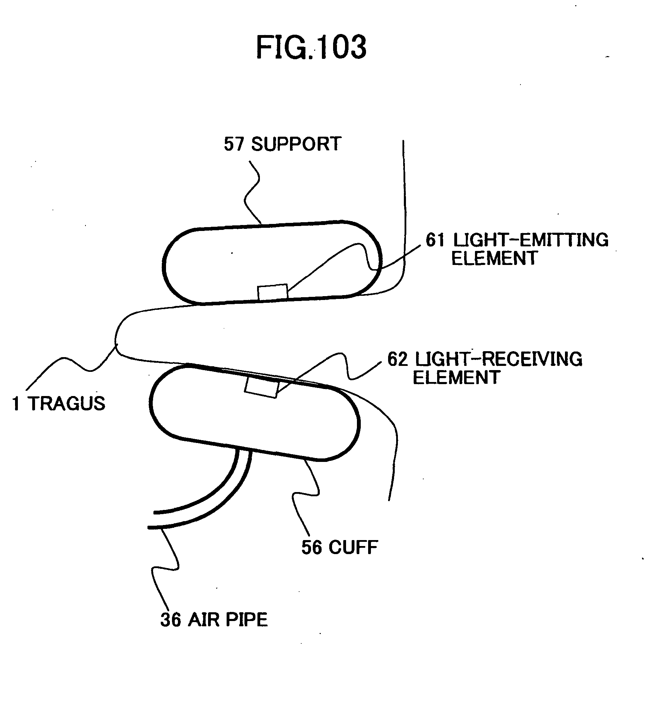

As shown in Fig.103, for example, the living body information detection apparatus of this embodiment corresponds to a case in which the aforementioned living body information detection apparatus includes a support 57 instead of the sensor 33 shown in Fig.96, and includes a cuff 56 instead of the sensor 34. The light-receiving element 62 is provided in the cuff 56, and the light-emitting element 61 is provided on a surface on which the support 57 contacts the tragus 1, and the cuff 56 is provided with an air pipe 36 for supplying air. Fig.103 is a magnified view of the part of the support 57 and the cuff 56.

The cuff 56 shown in Fig.103 applies a pressure on the tragus 1, and the light-emitting element 61 provided in the cuff and the light-receiving element 62 provided on a surface of the support 57 form the aforementioned transmission type pulse wave detection system, and detect the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information detection apparatus of the present invention may include a cuff, provided in the outside part, for applying a pressure to the tragus, a light-emitting element, provided in the inside of the cuff, for bringing output light to enter a living body tissue of the auricle, a light-receiving element, provided in the inside part, for receiving scattered light from the living body tissue, and an air pipe for supplying or releasing air in the cuff.

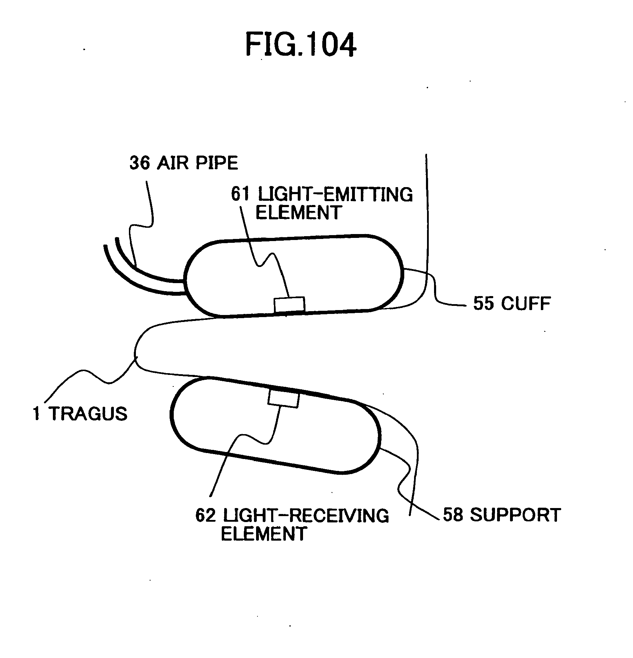

As shown in Fig.104, for example, the living body information detection apparatus of this embodiment corresponds to a case in which the aforementioned living body information detection apparatus includes a cuff 55 instead of the sensor 33 shown in Fig.96, and includes a support 58 instead of the sensor 34. The light-emitting element 61 is provided in the cuff 55, and the light-receiving element 62 is provided on a surface on which the support 58 contacts the tragus 1, and the cuff 55 is provided with an air pipe 36 for supplying air. Fig.104 is a magnified view of the part of the support 58 and the cuff 55.

The cuff 55 shown in Fig.104 applies a pressure on the tragus 1, and the light-emitting element 61 provided in the cuff 55 and the light-receiving element 62 provided on a surface of the support 58 form the aforementioned transmission type pulse wave detection system, and detect the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information detection apparatus of the present invention may include a cuff that is provided in the outside part and applies a pressure to the tragus, a light-emitting element that is provided in the inside part and brings output light to enter a living body tissue of the auricle, a light-receiving element that is provided in the inside of the cuff and receives scattered light from the living body tissue, and an air pipe for supplying or releasing air in the cuff.

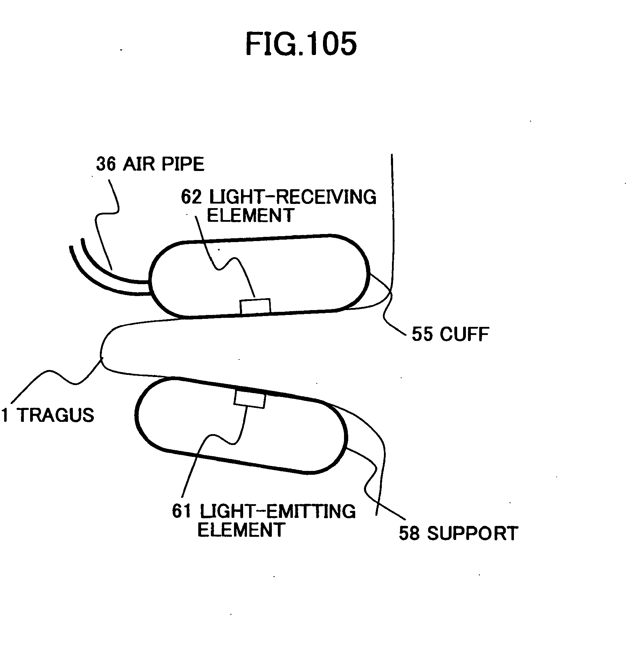

As shown in Fig.105, for example, the living body information detection apparatus of this embodiment corresponds to a case in which the aforementioned living body information detection apparatus includes a cuff 55 instead of the sensor 33 shown in Fig.96, and includes a support 58 instead of the sensor 34. The light-receiving element 62 is provided in the cuff 55, and the light-emitting element 61 is provided on a surface on which the support 58 contacts the tragus 1, and the cuff 55 is provided with an air pipe 36 for supplying air. Fig.105 is a magnified view of the part of the support 58 and the cuff 55.

The cuff 55 shown in Fig.105 applies a pressure on the tragus 1, and the light-receiving element 62 provided in the cuff 55 and the light-emitting element 61 provided on a surface of the support 58 form the aforementioned transmission type pulse wave detection system, and detect the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus of the living body according to individual body shape difference so that living body information such as the blood pressure, for example, can be detected accurately, and more stably, conveniently and continuously.

The living body information detection apparatus of the present invention may include a first cuff that is provided in the outside part and that applies a pressure to the tragus, a second cuff that is provided in the inside part and that applies a pressure to the tragus, a light-emitting element that is provided in the inside of the second cuff in the inside part and that brings output light to enter a living body tissue of the auricle, a light-receiving element that is provided in the inside of the second cuff in the inside part and that receives scattered light from the living body tissue, and air pipes for supplying or releasing air in the first cuff and the second cuff.

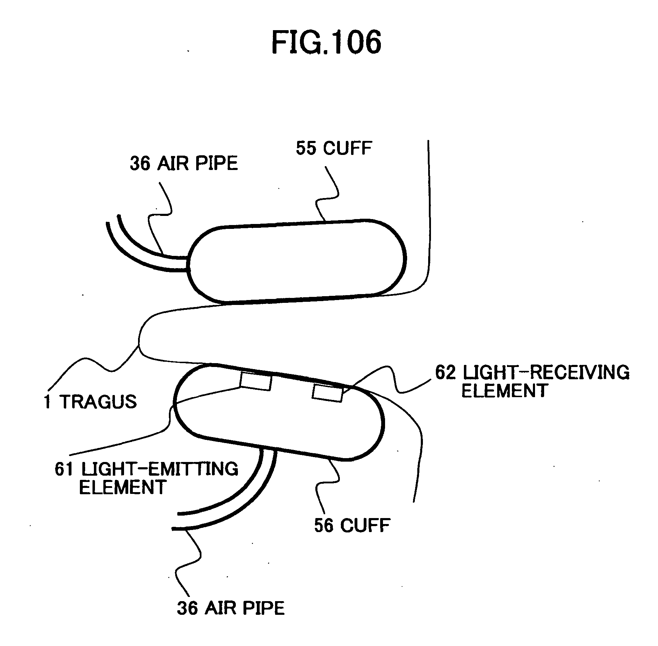

As shown in Fig.106, for example, the living body information detection apparatus of this embodiment, compared with the aforementioned living body information detection apparatus, includes a cuff 55 as the first cuff instead of the sensor 33 shown in Fig.96, and includes a cuff 56 as the second cuff instead of the sensor 34. The light-emitting element 61 and the light-receiving element 62 are provided in the cuff 56, and the cuff 55 and the cuff 56 are provided with air pipes 36 for supplying air. Fig.106 is a magnified view of the part of the cuff 55 and the cuff 56.

The cuff 55 and the cuff 56 shown in Fig.106 apply a pressure on the tragus 1, and the light-emitting element 61 and the light-receiving element 62 provided in the cuff 56 form the aforementioned reflection type pulse wave detection system, and detect the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus of the living body according to individual body shape difference so that living body information such as the blood pressure, for example, can be detected accurately, and more stably, conveniently and continuously.

The living body information detection apparatus of the present invention may include a first cuff that is provided in the outside part and that applies a pressure to the tragus, a second cuff that is provided in the inside part and that applies a pressure to the tragus, a light-emitting element that is provided in the inside of the first cuff in the outside part and that brings output light to enter a living body tissue of the auricle, a light-receiving element that is provided in the inside of the first cuff of the outside part and that receives scattered light from the living body tissue, and air pipes for supplying or releasing air in the first cuff and the second cuff.

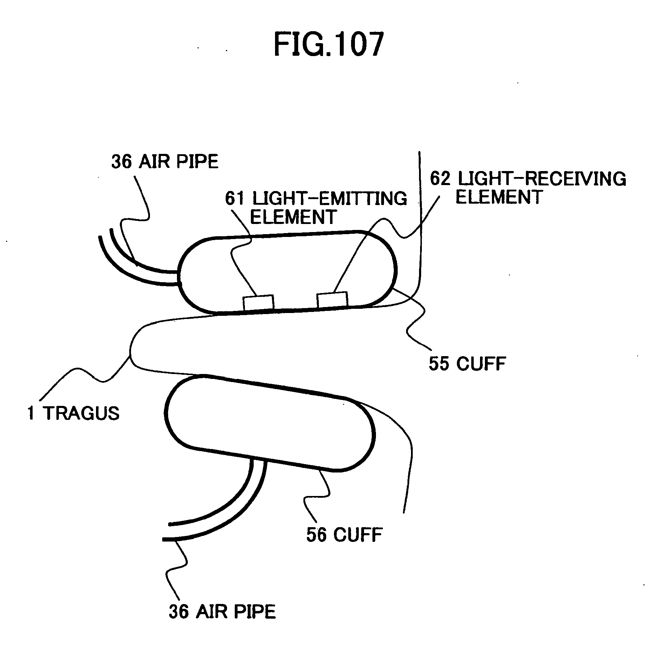

As shown in Fig.107, for example, the living body information detection apparatus of this embodiment corresponds to a case in which the aforementioned living body information detection apparatus includes a cuff 55 as the first cuff instead of the sensor 33 shown in Fig.96, and includes a cuff 56 as the second cuff instead of the sensor 34. The light-emitting element 61 and the light-receiving element 62 are provided in the cuff 55, and the cuff 55 and the cuff 56 are provided with air pipes 36 for supplying air. Fig.106 is a magnified view of the part of the cuff 55 and the cuff 56.

The cuff 55 and the cuff 56 shown in Fig.107 apply a pressure on the tragus 1, and the light-emitting element 61 and the light-receiving element 62 provided in the cuff 55 form the aforementioned transmission type pulse wave detection system, and detect the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information detection apparatus of the present invention may include a first cuff that is provided in the outside part and that applies a pressure to the tragus, a second cuff that is provided in the inside part and that applies a pressure to the tragus, a light-emitting element that is provided in the inside of the second cuff in the inside part and that brings output light to enter a living body tissue of the auricle, a light-receiving element that is provided in the inside of the first cuff in the outside part and that receives scattered light from the living body tissue, and air pipes for supplying or releasing air in the first cuff and the second cuff.

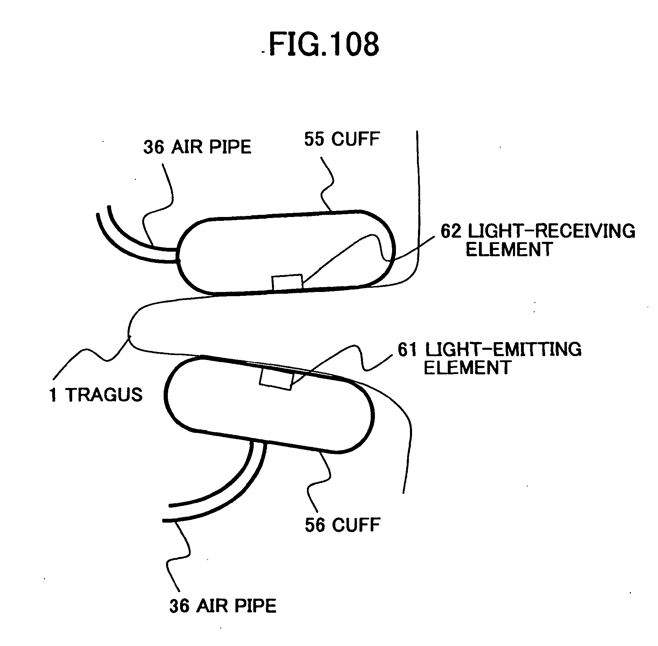

As shown in Fig.108, for example, the living body information detection apparatus of this embodiment corresponds to a case in which the aforementioned living body information detection apparatus includes a cuff 55 as the first cuff instead of the sensor 33 shown in Fig.96, and includes a cuff 56 as the second cuff instead of the sensor 34. The light-emitting element 61 is provided in the cuff 56 and the light-receiving element 62 is provided in the cuff 55, and the cuff 55 and the cuff 56 are provided with air pipes 36 for supplying air. Fig.108 is a magnified view of the part of the cuff 55 and the cuff 56.

The cuff 55 and the cuff 56 shown in Fig.108 apply a pressure on the tragus 1, and the light-emitting element 61 provided in the cuff 56 and the light-receiving element 62 provided in the cuff 55 form the aforementioned transmission type pulse wave detection system, and detect the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information detection apparatus of the present invention may include a first cuff that is provided in the outside part and that applies a pressure to the tragus, a second cuff that is provided in the inside part and that applies a pressure to the tragus, a light-emitting element that is provided in the inside of the first cuff in the outside part and that brings output light to enter a living body tissue of the auricle, a light-receiving element that is provided in the inside of the second cuff in the inside part and that receives scattered light from the living body tissue, and air pipes for supplying or releasing air in the first cuff and the second cuff.

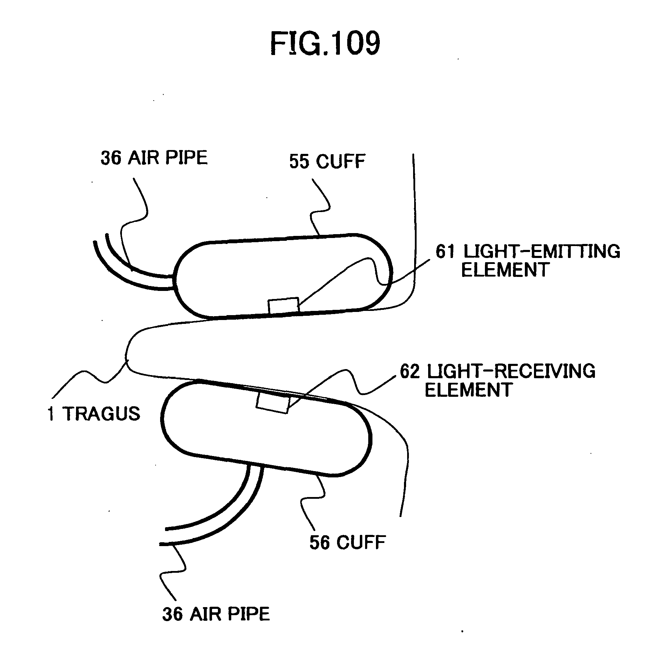

As shown in Fig.109, for example, the living body information detection apparatus of this embodiment corresponds to a case in which the aforementioned living body information detection apparatus includes a cuff 55 as the first cuff instead of the sensor 33 shown in Fig.96, and includes a cuff 56 as the second cuff instead of the sensor 34. The light-emitting element 61 is provided in the cuff 55, and the light-receiving element 62 is provided in the cuff 56, and the cuff 56 and the cuff 56 are provided with air pipes 36 for supplying air. Fig.109 is a magnified view of the part of the cuff 55 and the cuff 56.

The cuff 55 and the cuff 56 shown in Fig.108 apply a pressure on the tragus 1, and the light-emitting element 61 provided in the cuff 55 and the light-receiving element 62 provided in the cuff 56 form the aforementioned transmission type pulse wave detection system, and detect the pulse wave. The blood pressure can be measured from the detected pulse wave based on the aforementioned principle.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

In the living body information detection apparatus of the present invention, it is preferable that a projected shape obtained by projecting the cuff, the first cuff or the second cuff for applying a pressure toward the tragus is round or elliptic, and that the diameter or the minor axis is equal to or less than 11mm.

In the living body information detection apparatus of this embodiment, for example, the shape of the cuff 56 shown in Fig.98B in the aforementioned living body information detection apparatus is round or elliptic, and the diameter or the minor axis of the cuff is equal to or less than 11mm. Similarly, in the living body information detection apparatus 30 of the present invention, also in the examples in which cuffs are provided in both sides of the tragus as shown in Figs.108 and 109, the shape of each of the cuff 55 and the cuff 56 is round or elliptic, and the diameter or the minor axis is equal to or less than 11mm in this embodiment.

According to the non-patent document 2, since the average internal diameter of the cavity of the concha 8 is 8 mm, it is convenient to prepare a plurality of cuffs 56 each having the diameter or the minor axis of equal to or less than 11 mm so as to select one having an optimum size according to an individual body shape. However, when the diameter or the minor axis of the cuff 56 is equal to or less than 6 mm, an area on which the cuff 56 presses becomes small so that a bloodstream intercepted region in a blood vessel of an artery that is necessary for blood pressure measurement becomes too narrow. Thus, a signal from the blood vessel of the artery in which the bloodstream is not adequately intercepted may be mixed into a signal detected by the light-receiving element 62 so that there is a case in which detection accuracy is degraded.

As mentioned above, a projected shape obtained by projecting the cuff, the first cuff or the second cuff for applying a pressure toward the tragus is round or elliptic, and the diameter or the minor axis is equal to or less than 11mm. Accordingly, many people can be supported, the pulse wave can be detected accurately, and the blood pressure can be accurately measured from the detected pulse wave.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

In the living body information detection apparatus of the present invention, the cuff, the first cuff or the second cuff for applying a pressure includes the light-emitting element and the light-receiving element such that a light-emitting part of the light-emitting element and a light-receiving part of the light-receiving element contact the inside of a surface of the cuff contacting the tragus, and the part which the light-emitting part and the light-receiving part contact may be composed of a transparent material, and other parts may be composed of a light shielding or light extinction material.

The living body information detection apparatus of this embodiment corresponds to a case in which, in the cuff 56 in the aforementioned living body information detection apparatus 30 shown in Figs.98A and 98B, the light-emitting element 61 is provided on the inside of a surface by which the cuff 56 contacts the tragus 1 and the light-emitting part of the light-emitting element 61 contacts the cuff 56, the part of the cuff 56 contacting the light-emitting part is composed of a transparent material, the light-receiving element 62 is provided on the inside of the surface by which the cuff 56 contacts the tragus 1, the part of the cuff 56 contacting the light receiving part is composed of a transparent material, and the other parts of the cuff 56 are composed of a light shielding or light extinction material.

By the above-mentioned configuration, light passes well through the part on which the cuff 56 contacts the light-emitting part of the light-emitting element 61 and the light-receiving part of the light-receiving element 62, and light does not pass through other parts of the cuff 56 well, so that outside light such as glare or stray light can be shielded, and further, it can be avoided that emitted light of the light-emitting element 62 spreads and is irradiated onto a blood vessel in which bloodstream is not stopped and that the scattered light or the transmitted light is received by the receiving element 62. Therefore, the light-emitting element 61 and the light-receiving element 62 can detect the pulse wave more accurately according to the aforementioned principle and can measure the blood pressure with reliability from the detected pulse wave.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

In the living body information detection apparatus of the present invention, by fixing the light-emitting element or the light-receiving element to the cuff for applying a pressure, the light-emitting element and the light-receiving element can be moved with the cuff when applying and reducing the pressure.

The living body information detection apparatus of this embodiment corresponds to a case in which, in the aforementioned living body information detection apparatus 30, the light-emitting element 61 and the light-receiving element 62 provided in the cuff 56 shown in Fig.98B, for example, are fixed to the surface that contacts the tragus 1.

As mentioned above, by fixing the light-emitting element 61 and the light-receiving element 62, the light-emitting element 61 and the light-receiving element 62 moves together with the cuff 56 when supplying air into the cuff 56 to apply a pressure on the tragus and when releasing the air from the cuff 56 to release the pressure on the tragus 1, so that position relationship among the cuff, the light-emitting element 61 and the light-receiving element 62 becomes stable. Thus, the pulse wave can be detected with higher precision, and the blood pressure can be measured from the detected pulse wave with higher precision.

The living body information detection apparatus of this embodiment also corresponds to a case in which, in the aforementioned living body information detection apparatus, the light-emitting element 61 provided in the cuff 56 shown in Fig.108, for example, and the light-receiving element 62 provided in the cuff 55 are fixed to surfaces on which each of the cuff 55 and the cuff 56 contacts the tragus 1.

As mentioned above, by fixing the light-emitting element 61 and the light-receiving element 62 in the cuff 56 and the cuff 55, the light-emitting element 61 moves together with the cuff 56 and the light-receiving element 62 moves together with the cuff 55 when supplying air into the cuff 56 and the cuff 55 to apply a pressure on the tragus 1 and when releasing the air from the cuff 55 and the cuff 56 to release the pressure on the tragus 1, so that position relationship among the cuff, the light-emitting element 61 and the light-receiving element 62 becomes stable. Thus, the pulse wave can be detected with higher precision, and the blood pressure can be measured from the detected pulse wave with higher precision.

As described above, the living body information detection apparatus of the present invention is small and light, and can be worn more comfortably with a proper contacting pressure at proper positions of the tragus 1 of the living body according to individual body shape difference so that living body information such as the blood pressure, for example, can be detected accurately, and more stably, conveniently and continuously.

The living body information apparatus described so far detects the pulse wave using the light-emitting element and the light-receiving element. Alternatively, by providing a cuff for applying a pressure on the tragus, the pulse wave can be also detected by detecting pulsation due to pulse wave on a surface of the living body as pressure change by the cuff. That is, the pulsation obtained from the living body by the cuff that applies the pressure is converted to the change of the pressure in the cuff, so that a pressure detection apparatus detects pressure change in the cuff. Also according to such configuration, the pulse wave in the living body can be detected. In addition, a small microphone may be placed at the cuff that contacts the living body so as to detect Korotkoff sounds generated when the cuff presses a part of the living body and measure the blood pressure based on occurrence or disappearance of the Korotkoff sounds that are equal to or greater than a predetermined level. Further, after applying a pressure to the cuff, by reducing the pressure of the cuff while detecting pressure change of the cuff, the blood pressure can be measured based on the before-mentioned principle. In addition, a vibration sensor may be provided so as to detect the pulse wave by detecting vibration of the cuff using the vibration sensor. Therefore, by using the cuff as a sensor of the living body information detection apparatus, effects the same as those of the living body information detection apparatuses described so far can be obtained.

The living body information detection apparatus cannot always press the living body for detecting the living body information by always wearing the living body information detection apparatus on the living body. As described so far, since the living body information detection apparatus of the present invention is fixed to the living body using the pair of the opposed arms almost formed like U-shape, the living body is not always be pressed. Especially, by accommodating the living body information detection apparatus having the shape that covers the tragus into the concha auriculae or the cavity of the concha, the living body information can be detected stably.

The living body information detection apparatus of the present invention can be applied as a living body information detection apparatus for continuously measuring pulse, blood pressure, bloodstream and the like according to the type of the sensor. Therefore, the living body information detection apparatus of the present invention can be applied to a use as a means for safety management for workers working under dangerous environment such as aquaspacemen.

In addition, the part of the ear at which living body information is measured is not limited to the above-mentioned parts, and may be any part in the external ear or the periphery of the external ear. For measuring at the periphery of the external ear, length or shape of one arm is formed according to measurement of the periphery of the external ear.

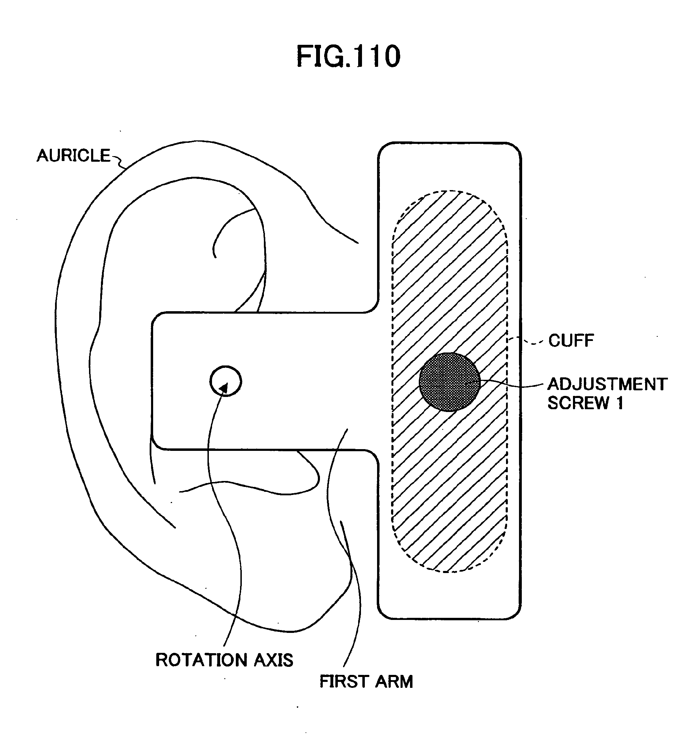

That is, as describe in the last part of the third embodiment, also in the fourth embodiment, the part of the cuff in the outside of the living body information detection apparatus can be placed in or expanded to the external ear periphery part shown in Fig.60. An example of the living body information detection apparatus in this case is shown in Fig.110

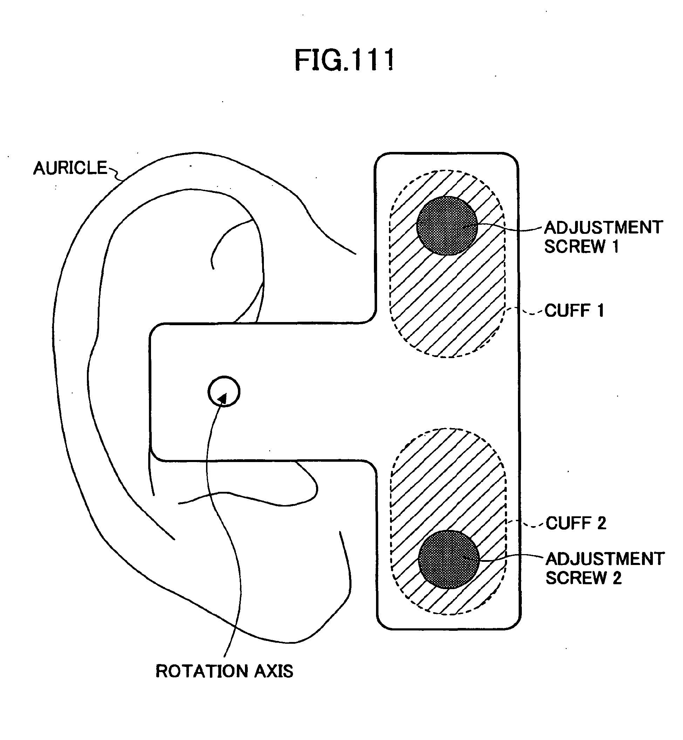

In addition, in this case, it is preferable that the photoelectric elements are placed in a center part of the cuff or in a part where a cuff pressure is evenly applied so as to be opposite to the part. The outside cuff may be divided into a plurality of outside cuffs, as shown in Fig.111. In this case, as described in the third embodiment, it is preferable that the photoelectric elements are placed in a cuff in the lower side (peripheral side) of bloodstream.

By the way, a configuration can be adopted in which each of both arms in this embodiment includes a blood-pressure meter having a cuff, a light-emitting element and a light-receiving element. That is, a blood pressure is measured in one arm side, and another blood pressure is measured in another arm side. Then, for example, one blood-pressure meter is configured to measure a blood pressure in the inside of the tragus and another blood-pressure meter is configured to measure a blood pressure in the outside of the tragus. Accordingly, since a thin blood vessel (arteriola) exists in the inside of the tragus, and a thick blood vessel (superficial temporal artery) exists in the outside of the tragus, a blood pressure of the thick blood vessel and a blood pressure of the thin blood vessel can be measured.

By measuring the blood pressure of the thick blood vessel and the blood pressure of the thin blood vessel, information on arteriosclerosis can be obtained (for example, if difference between them are large, arteriosclerosis is developing). Thus, by adopting the above-mentioned configuration, effects can be obtained in which not only the blood pressure is measured but also information on arteriosclerosis can be obtained. By the way, the part of the thick blood vessel and the part of the thin blood vessel are not limited to the inside and the outride of the tragus.

As described above, according to the fourth embodiment, the living body information detection apparatus is provided in which the living body information detection apparatus is configured to be U-shaped so that the sensor for detecting living body information can be worn on a salient part of the human body, and the living body information detection apparatus includes a mechanism for changing distance between tops of the U-shape and for shifting the two tops of the U-shape such that the sensor is brought into intimate contact with the salient part even if the salient part has individuality. Accordingly, the living body information detection apparatus that can be easily worn and that can detect living body information stably can be provided.

In addition, by mounting the sensor on a top of an adjustment screw attached in a screw hole passing through the other end of the arm, the distance between the arms can be finely adjusted. Thus, the living body information detection apparatus that can be easily worn and that can detect living body information stably can be provided.

In addition, since the length of at least one arm in the arms of the pair can be changed, living body information can be detected even when the living body between the arms of the pair does not have an even thickness.

By devising the shape of the arm or by providing the cushion, the sensor can be stabilized. Thus, the living body information detection apparatus that can be easily worn and that can detect living body information stably can be provided. In addition, by configuring the ear suspension and the cushion to pull against each other via the auricle using magnetic force, the living body information detection apparatus that can detect living body information stably can be provided.

In addition, by providing the light shielding cover for shielding the sensor or the tragus of the human body from the outside, external disturbance due to light from the outside can be reduced so that the sensor can detect living body information stably.

In addition, by further providing a speaker on the arm for transmitting sound signal, information can be transmitted to the subject via the speaker.

As described above, the living body information detection apparatus of this embodiment is small and light, and is easy to be worn to the living body. Thus, it can be worn for a long time to measure living body information stably. Especially, in the blood pressure measurement, since the sensor can press a narrow area in the living body to measure a blood pressure, the measurement can be performed at arbitrary time.

### (Fifth embodiment)

In apparatuses (including a blood-pressure meter) described so far for measuring living body information, following problems can be considered when developing a cuff that is used for applying pressure.

First, it is necessary to keep airtightness for preventing air leakage. As a result of downsizing for enabling the apparatus to be worn on the ear, capacity of the cuff is extremely small. Thus, slight air leakage causes cuff pressure decrease and exerts a bad influence on pressure releasing control. Second, since arteriolas are distributed in the peripheral part, evenness of pressure on a measured part is important. That is, at least for arteriolas existing in a range irradiated with probe light, it is necessary to make pressure distribution on the measured part uniform so that stop and release of blood stream becomes uniform. Third, it is necessary that pressure applying energy is transmitted to the living body effectively via the cuff. When the pressure applying energy is consumed for expanding the cuff, a pressure applied to the living body is reduced by that. This causes increase of output of an air supply pump.

Thus, in the following embodiments, a cuff is described that solves the above-mentioned problems and that is applicable for a blood pressure measurement apparatus and the like for measuring the blood pressure continuously with high precision on the periphery part of the living body such as the auricle.

In the following, the embodiment of the present invention is described with reference to attached figures. The embodiment described below is a configuration example of the present invention, and the present invention is not limited to the following embodiment.

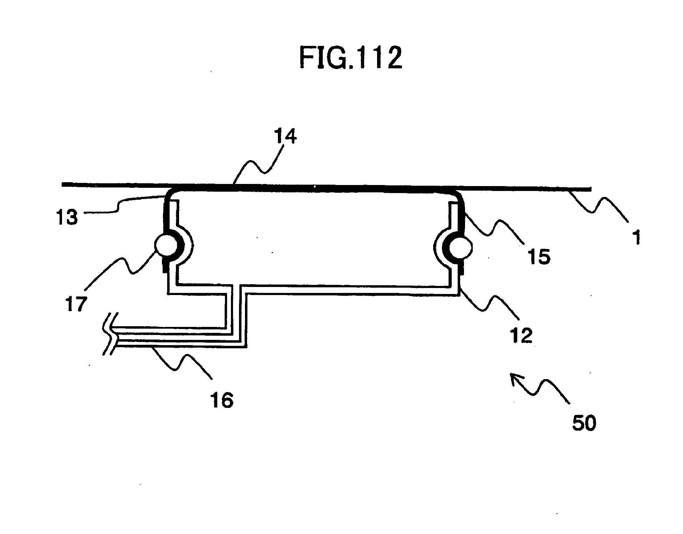

Fig.112 is a schematic section view showing a configuration of the cuff of this embodiment.

The cuff 50 of this embodiment includes a case 12 in which a face is open, an elastic member 13 for covering the face that is open, and an air supplying pipe 16 provided in the case 12, in which a pressing surface 14 of the elastic member 13 swells by supplying air into the cuff surrounded by the case 12 and the elastic member from the air supplying pipe. The swelled pressing surface 14 presses a part of the living body 1.

In Fig.112, the case 12 has a function for holding the elastic member 13. The material of the case 12 may be metal, plastic, glass, wood, paper, ceramics, porcelain, cloth, or complex of these, whose expansion and contraction ratio is smaller than that of the elastic member 13.

The elastic member 13 covers the open face of the case 12 so as to form the pressing surface 14 for pressing the living body 1 in the side of the face. Accordingly, by covering the open face of the case 12 with the elastic member 13, a part of the living body 1 can be pressed at a pinpoint efficiently and evenly. Therefore, blood pressure and the like can be measured with high precision even at a relatively small part of the living body 1 such as the auricle and the tragus, for example.

The material of the elastic member 13 may be material having elasticity such as silicone resin, natural rubber and butyl rubber, general plastic material such as polyethylene, polypropylene, polyvinyl chloride, polyvinyl acetate and copolymer of these, or airtight cloth or paper obtained by coating nonwoven fabric with polymer, in which the material passes light.

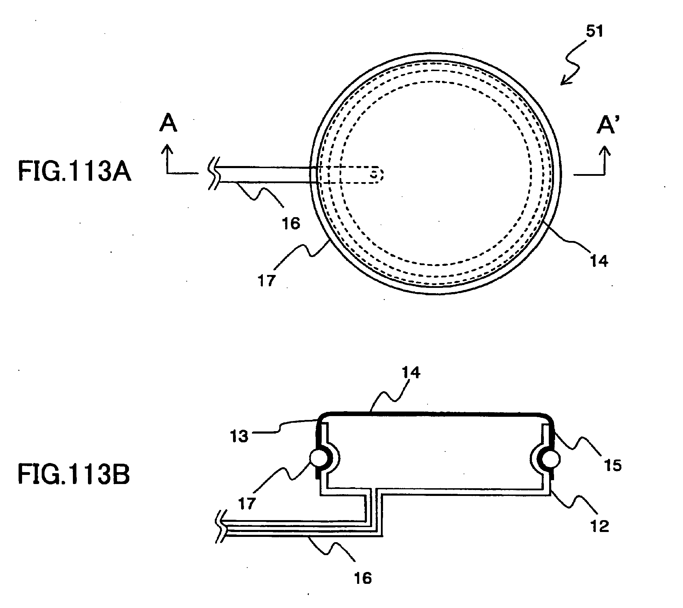

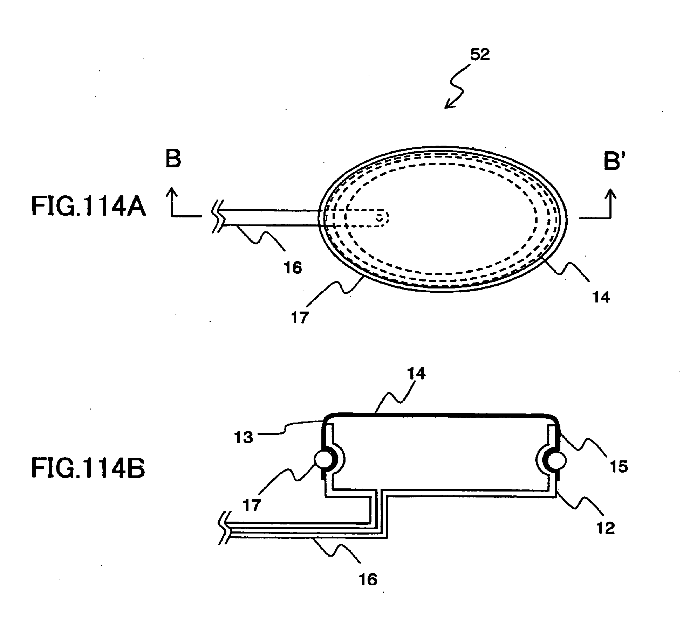

It is desirable that the shape of the pressing surface 14 is round or elliptic. Figs.113 and 114 show schematic views of the configuration of the cuff of this embodiment. Fig.113 shows a case where the shape of the pressing surface 14 is round, and Fig.114 shows a case where the shape of the pressing surface 14 is elliptic. In Fig.113, Fig.113A is a top view, Fig.113B is a section view at A-A' in the top view of Fig.113A. In Fig.114, Fig.114A is a top view, Fig.114B is a section view at B-B' in the top view of Fig.113A.

By forming the shape of the pressing surface 14 to be round as shown in Fig.113 or to be elliptic as shown in Fig.114, compared with polygon such as a quadrangle, (1)airtightness in the inside of the cuff 51 and the cuff 52 can be easily increased, (2) the pressure applied by the pressing surface 14 on the living body is uniform, (3) allowance of position shifts with respect to artery of the living body on which the pressing surface 14 presses is large, (4) when an after-mentioned light-emitting element irradiates the living body with light passing through the pressing surface 14, the irradiated light is scatted by the living body to form scattered light, and a pulse wave and the like is measured by receiving the scattered light by the light-receiving element, since a section of emission pattern of light from the light-emitting element is round or elliptic, it is easy to match the section with the uniform-pressure distribution so that measurement accuracy of the scattered light can be easily increased, (5) since the shape does not have a corner, damage of the elastic member 13 due to repetition of expanding and contracting can be prevented. In addition, by using a rounded quadrangle instead of using the elliptic shape for the pressing surface 14, the same effects described in (1)-(5) obtained by the elliptic shape for the pressing surface 14 can be obtained also by the pressing surface of the rounded quadrangle.

A side part 15 of the elastic member 13 shown in Fig.112 exists between the elastic member and the case 12, and has a function for supporting the pressing surface 14 and keeps airtightness between the elastic member 13 and the case 12. A fixing part 17 has a function for keeping airtightness between the side part 15 of the elastic member 13 and the case 12 to fix the side part 15 of the elastic member 13 to the case 12.

The air supply pipe 16 has a function for supplying air into the cuff 50, and has a function for swelling the pressing surface 14 by the pressure of the air supplied in the inside of the cuff 50 that is surrounded by the elastic member 13 and the case 12. Then, the swelled pressing surface 14 presses the living body 1. The air supply pipe 16 may have a function for releasing the supplied air. Except for the air supply pipe 16, the airtightness in the inside of the cuff 50 is kept by the case 12 and the elastic member 13.

Operation of the cuff of this embodiment is described in a case when the cuff 50 of this embodiment is used as a blood pressure measurement apparatus. Air is supplied to the case 12 of the cuff 50 via the air supply pipe 16 to move the pressing surface 14 toward the living body 1 so that the pressing surface 14 presses the living body 1. The pulse wave of an artery in the inside of the living body 1 in the process of pressing the living body 1 by the pressing surface 14 is detected by a predetermined means that is not shown in the figure.

More specifically, by supplying air into the cuff 50 via the air supply pipe 16 to increase the pressure in the inside of the cuff 50 surrounded by the elastic member 13 and the case 12, the pressing surface 14 is swelled to press the living body 1. Then, bloodstream of the artery of the living body 1 stops by the pressure of the pressing surface 14 to the living body 1. In the state in which the pulse wave is disappeared, air in the inside of the cuff 50 is released via the air supply pipe 16. In the process in which the pressure applied to the living body 1 by the pressing surface 14 decreases, the pulse wave of the artery appears again, and changing state is detected, so that the blood pressure is measured based on a predetermined method from the change of the pulse wave of the artery and the pressure in the inside of the cuff 50.

By configuring the shape of the pressing surface 14 to be round or elliptic, airtightness in the inside of the cuff 50 can be increased and the pressure of the pressing surface 14 can be applied evenly. In addition, allowance for position shifts with respect to the artery to be pressed by the pressing surface 14 is large. In addition, measurement accuracy for measuring the pulse wave by the scattered light can be easily increased. Therefore, by the cuff 50 of this embodiment enables to measure blood pressure with high precision in the periphery part of the living body such as the auricle. In addition, when the shape of the pressing surface 14 is round or elliptic, since there is no corner, damages of the elastic member 13 due to repetition of expanding and contracting can be reduced. Therefore, the cuff 50 of this embodiment can be used many times continuously for long time.

The cuff of this embodiment corresponds to a case in which the shape of the pressing surface 14 of the elastic member 13 is concave in relation to the outside.

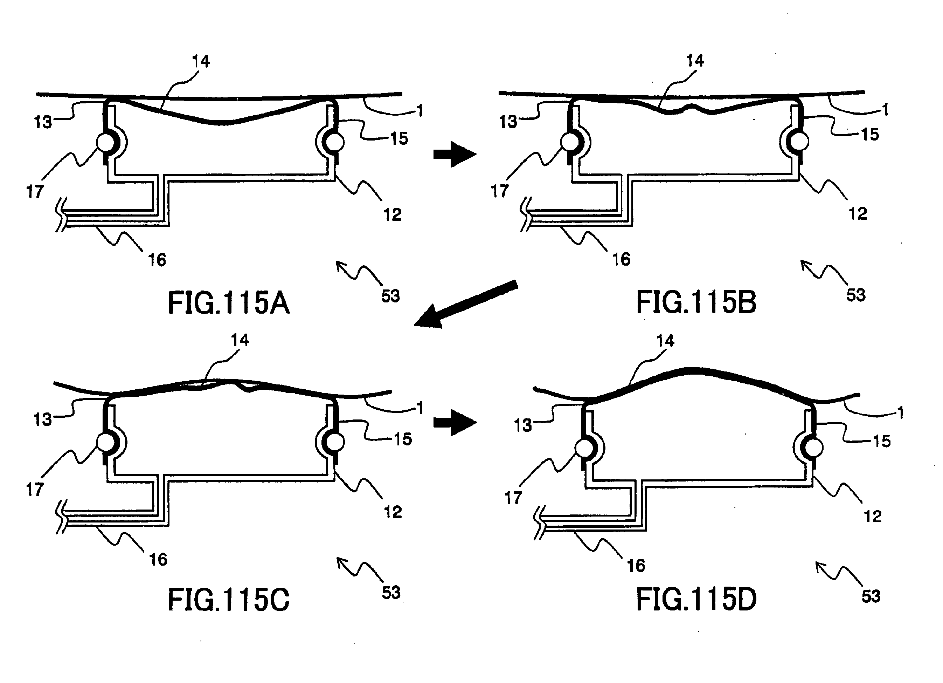

The cuff of this embodiment is described with reference to attached figures. Fig.115A is a schematic section view showing a configuration of the cuff in this embodiment. In Fig.115A, the cuff 53 of this embodiment has a configuration similar to the cuff 50 shown in Fig.112, and functions of each part forming the cuff 53 of this embodiment are similar to those of the cuff 50 shown in Fig.112. However, the cuff 53 is different from the cuff 50 shown in Fig.112 in that the pressing surface 114 is concave in relation to the outside of the cuff 53.

Basic operation of the cuff of this embodiment is the same as that of the cuff 50 described with reference to Fig.112. Figs.115B, 115C and 115D show, in order, processes for contacting the pressing surface 14 of the cuff 53 of this embodiment to the living body 1, supplying air into the cuff 53 via the air supply pipe 16 and pressing the living body 1 by the pressing surface 14.

Fig.115B shows a state in which the pressing surface expands so that contact area contacting the living body 1 increases, but a warp remains in the pressing surface 14 contacting the living body 1. Fig.115C shows a state in which air pressure in the cuff 53 further increases so that the pressing surface 14 further expands and the contact area to the living body 1 increases, and the warp of the pressing surface 14 contacting the living body 1 reduces. Fig.115D shows a state in which air pressure in the cuff 53 further increases so that the pressing surface 14 further expands, the warp of the pressing surface 14 contacting the living body 1 disappears to press the living body 1.

Since the pressing surface 14 of the cuff 53 of this embodiment is concave in relation to the outside, in the process for pressing the living body 1 by the pressing surface 14, the warp exists on the pressing surface 14 contacting the living body 1 so that force for expanding the pressing surface 14 against resilience of the pressing surface is not necessary. Thus, the cuff 53 of this embodiment can press the living body 1 with a small pressure. Therefore, blood pressure measurement can be available even on a small part of the living body 1 such as the auricle, the tragus and the like.

The cuff of this embodiment corresponds to a case in which the shape of the pressing surface 14 of the elastic member 13 is convex toward the outside.

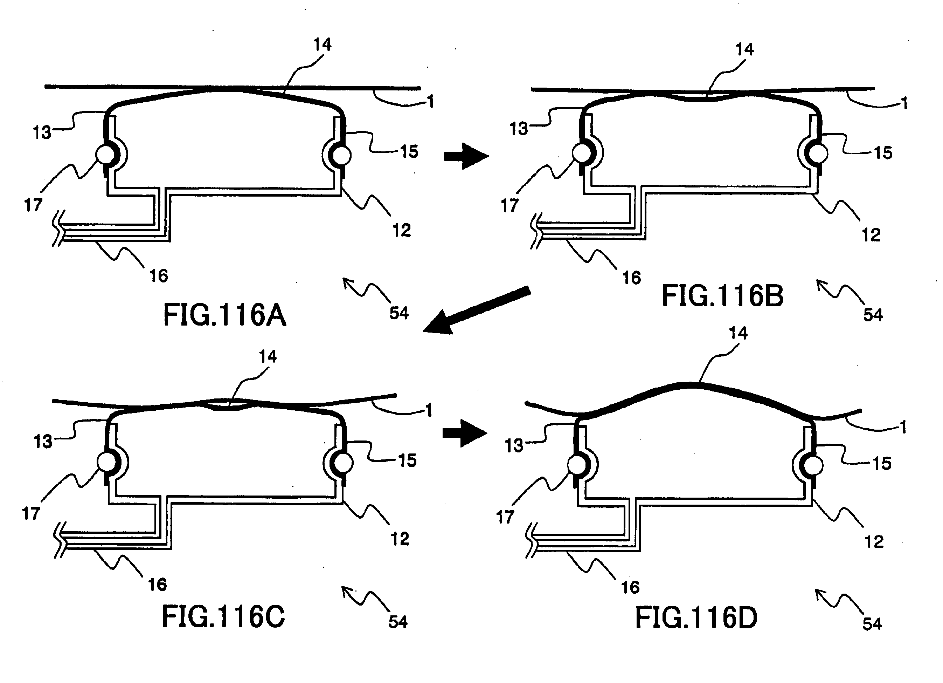

The cuff of this embodiment is described with reference to attached figures. Fig.116A is a schematic section view showing a configuration of the cuff in this embodiment. In Fig.116A, the cuff 54 of this embodiment has a configuration similar to the cuff 50 shown in Fig.112, and functions of each part forming the cuff 54 of this embodiment are similar to those of the cuff 50 shown in Fig.112. However, the cuff 54 is different from the cuff 50 shown in Fig.112 in that the pressing surface 114 is convex to the outside of the cuff 54.

Basic operation of the cuff 54 of this embodiment is the same as that of the cuff 50 described with reference to Fig.112. Figs.116B, 116C and 116D show, in order, processes for contacting the pressing surface 14 of the cuff 56 of this embodiment to the living body 1, supplying air into the cuff 56 via the air supply pipe 16 and pressing the living body 1 by the pressing surface 14.

Fig.116B shows a state in which the pressing surface expands so that contact area contacting the living body 1 increases, but a warp remains in the pressing surface 14 contacting the living body 1. Figf.116C shows a state in which air pressure in the cuff 56 further increases so that the pressing surface 14 further expands and the contact area to the living body 1 increases, and the warp of the pressing surface 14 contacting the living body 1 reduces. Fig.116D shows a state in which air pressure in the cuff 56 further increases so that the pressing surface 14 further expands, the warp of the pressing surface 14 contacting the living body 1 disappears to press the living body 1. 0516

Since the pressing surface 14 of the cuff 54 of this embodiment is convex to the outside, in the process for pressing the living body 1 by the pressing surface 14, the warp exists on the pressing surface 14 contacting the living body 1 so that force for expanding the pressing surface 14 against resilience of the pressing surface is not necessary. Thus, the cuff 53 of this embodiment can press the living body 1 with a small pressure. Therefore, blood pressure measurement can be available even on a small part of the living body 1 such as the auricle, the tragus and the like.

The cuff of this embodiment corresponds to a case in which the shape of the pressing surface 14 of the elastic member 13 is flat.

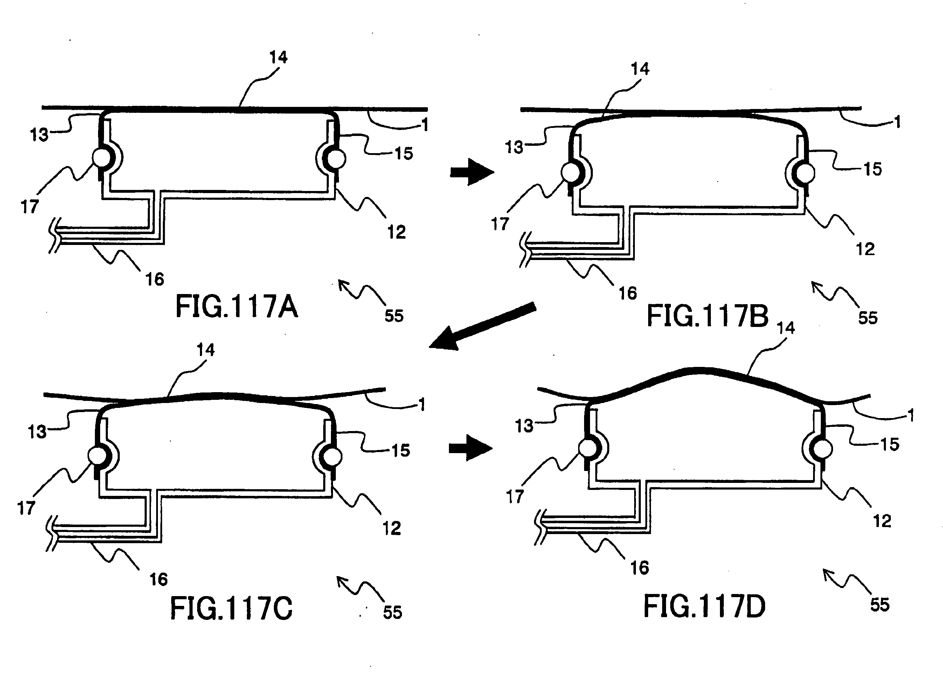

The cuff of this embodiment is described with reference to attached figures. Fig.117A is a schematic section view showing a configuration of the cuff in this embodiment. In Fig.117A, the cuff 55 of this embodiment has a configuration similar to the cuff 50 shown in Fig.112, and functions of each part forming the cuff 55 of this embodiment are similar to those of the cuff 50 shown in Fig.112. However, the cuff 55 is characterized in that the pressing surface 114 is flat.

Basic operation of the cuff 55 of this embodiment is the same as that of the cuff 50 described with reference to Fig.112. Figs.117B, 117C and 117D show, in order, processes for contacting the pressing surface 14 of the cuff 55 of this embodiment to the living body 1, supplying air into the cuff 55 via the air supply pipe 16 and pressing the living body 1 by the pressing surface 14.

Fig.117B shows a state in which the pressing surface 14 expands so as to become convex to the living body 1 to press the living body 1. Fig.117C shows a state in which air pressure in the cuff 55 further increases so that the pressing surface 14 further expands and presses. Fig.117D shows a state in which air pressure in the cuff 55 further increases so that the pressing surface 14 further expands and presses.

Since the pressing surface 14 of the cuff 55 of this embodiment is flat, in the process for pressing the living body 1 by the pressing surface 14, no warp exists on the pressing surface 14 as shown in Figs.117B and 117C. Thus, the living body 1 can be pressed without occurrence of noise due to vanishing of the warp. In addition, since the pressing surface 14 of the cuff 55 of this embodiment is flat, when air in the cuff 55 is released to decrease the pressure, no warp exists on the pressing surface 14 in the pressure decreasing process. Thus, the living body 1 can be pressed without occurrence of noise due to appearance of the warp. Therefor, blood pressure measurement can be available even on a small part of the living body 1 such as the auricle, the tragus and the like.

The cuff of this embodiment can be configured to have slack, on the side part 15 of the elastic member 13 shown in Fig.112, which expands or contracts in the movement direction of the pressing surface 14, that is, expands or contracts toward the living body 1 to move the pressing surface 13.

The cuff of this embodiment corresponds to a case in which the cuff described in Figs.112 and 115-117 includes, on the side 15 of the elastic member 13, the slack for expanding or contracting toward the living body 1 to move the pressing surface 14.

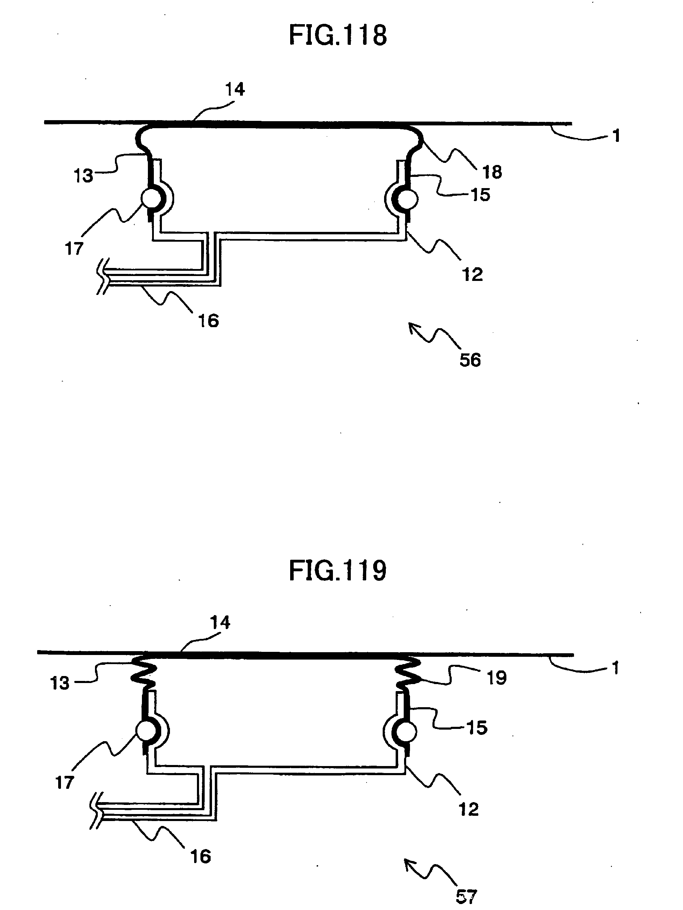

The cuff of this embodiment is described with reference to attached figures. Figs.118 and 119 are schematic section views showing the configuration of the cuff of thus embodiment. In Fig.118, the cuff 56 of this embodiment has the same configuration as the cuff 50 shown in Fig.112 except for including slack 16 in the side part 15 of the elastic member 13. In addition, in Fig.119, the cuff 57 of this embodiment has the same configuration as the cuff 50 shown in Fig.112 except for including slack 19 in the side part 15 of the elastic member 13.

The slack 18 shown in Fig.118 is a case where the slack has a single swelling shape, and the slack 19 shown in Fig.119 is a case where the slack is bellows including a plurality of warps. The shape of the slack may be any of the slacks 18 and 19 shown in Figs.118 and 119.

The slack 18 of the cuff 56 (Fig.118) of this embodiment has a function for expanding and contracting to move the pressing surface 14 to the living body 1 when the pressing surface 14 presses the living body 1 by air supply from the air supply pipe 16 to the cuff 56. Parts other than the slack 18 forming the cuff 56 of this embodiment has functions the same as the cuff 50 shown in Fig.112.

Operation of the cuff 56 of this embodiment is the same as the operation of the cuff 50 described with reference to Fig.112. The cuff 56 of this embodiment includes the slack 18 on the side part 15 having the function for supporting the pressing surface 14 and fixing the pressing surface 14 to the case 12 wherein the slack 18 expands and contracts to move the pressing surface 14 to the living body 1. Accordingly, since the pressing surface 14 can be easily moved to the living body 1, the living body 1 can be pressed with small pressure. Therefore, for example, blood pressure measurement can be performed on a small part of the living body 1 such as the auricle and the tragus and the like. The function and effects of the bellows-like slack 19 shown in Fig.19 are the same as those described for the slack 18 shown in Fig.118.

In the cuff (not shown in the figure) of this embodiment, an expansion ratio of the pressing surface in the swelling direction of the pressing surface 14 shown in Figs.118 and 119 can be set to be less than an expansion ratio of the slack 18, 19 in the swelling direction of the pressing surface 14.

The cuff of this embodiment corresponds to a case in which, in the cuff 56, 57 described in Figs.118 and 119, the expansion ratio of the pressing surface 14 in the swelling direction of the pressing surface 14 is less than the expansion ratio of the slack 18, 19 in the swelling direction of the pressing surface 14. The cuff of this embodiment has the same configuration as the aforementioned cuffs 56 and 57 described with reference to Figs.118 and 119, and is characterized in that the expansion ratio of the pressing surface 14 in the swelling direction of the pressing surface 14 is less than the expansion ratio of the slack 18, 19 in the swelling direction of the pressing surface 14.

Each function forming the cuff of this embodiment is the same as that of the aforementioned cuffs 56 and 57 described with reference to Figs.118 and 119. That is, operation of the cuff of this embodiment is the same as the operation of the cuff 50 described with reference to Fig.112.

The expansion ratio of the pressing surface 14 in the swelling direction of the pressing surface 14 is a swelling amount of the pressing surface 14 with respect to pressure in the cuff when the pressure in the cuff surrounded by the elastic member 13 and the case 12 increases by air supply from the air supply pipe 16 so that the pressing surface 14 swells according to the amount of the pressure. The expansion ratio of the pressing surface 14 may change according to thickness of the elastic member 13 in the part for forming the pressing surface 14. For example, when the thickness of the elastic member 13 in the part for forming the pressing surface 14 is doubled, the expansion ratio of the pressing surface 14 becomes about half. The reason is that internal stress per a unit area decreases.

The expansion ratio of the slack 18, 19 in the swelling direction of the pressing surface 14 is an expanding amount of the slack with respect to pressure in the cuff when the pressure in the cuff surrounded by the elastic member 13 and the case 12 increases by air supply from the air supply pipe 16 so that the pressing surface 14 swells according to the amount of the pressure and the slack expands in the swelling direction of the pressing surface 14. The expansion ratio of the slack 18, 19 may change according to thickness of the elastic member 13 in the part for forming the slack 18, 19. For example, when the thickness of the elastic member 13 in the part for forming the slack 18, 19 is doubled, the expansion ratio of the slack 18, 19 becomes about half. The reason is that internal stress per a unit area decreases.

In the cuff of this embodiment, the expansion ratio of the pressing surface 14 in the swelling direction of the pressing surface 14 shown in Figs.118 and 119 is less than the expansion ratio of the slack 18, 19 on the side part 15 in the swelling direction of the pressing surface 14. Accordingly, since shape change of the pressing surface 14 is small even when pressure is applied, occurrence of noise is small and the living body 1 which the pressing surface 14 contacts can be pressed evenly. Therefore, for example, blood pressure measurement can be performed with high precision.

The cuff of this embodiment can be configured such that thickness of the part forming the pressing surface 14 of the elastic member 13 shown in Figs.118 and 119 is greater than thickness of the part for forming the slack 18, 19 of the side part 15 of the elastic member 13.

The cuff of this embodiment corresponds to a case in which, in the cuffs 56 and 57 described in Figs.118 and 119, thickness of the part forming the pressing surface 14 of the elastic member 13 shown in Figs.118 and 119 is greater than thickness of the part for forming the slack 18, 19 of the side part 15 of the elastic member 13.

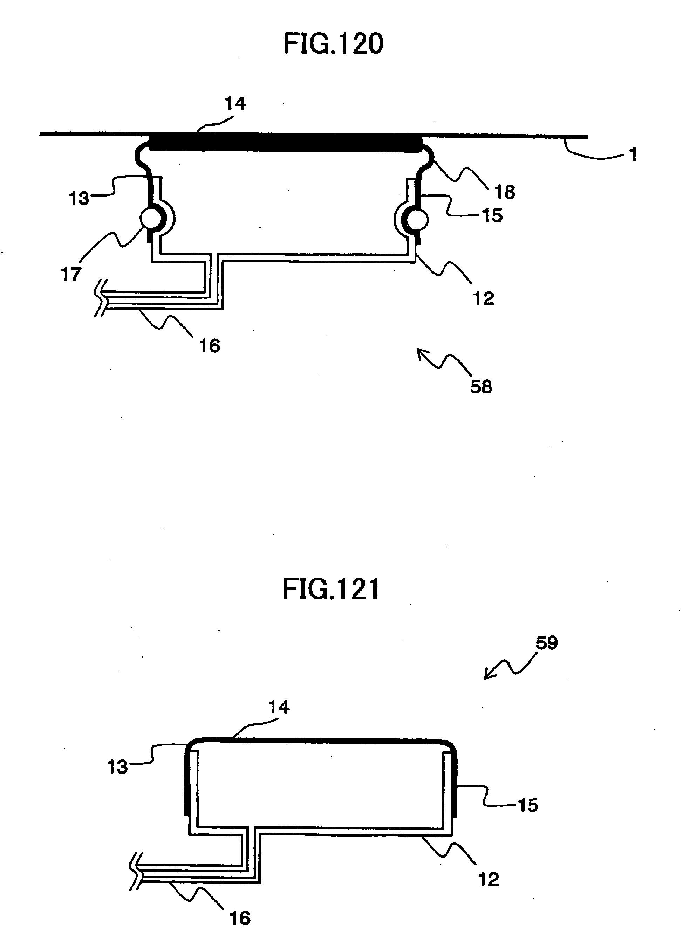

The cuff of this embodiment is described with reference to the attached figure. Fig.120 is a schematic section view showing the configuration of the cuff of this embodiment. In Fig.120, the cuff 58 of this embodiment has the same configuration as the cuffs 56 and 57 shown in Figs.118 and 119, but is characterized in that the thickness of the part forming the pressing surface 14 in the elastic member 13 is greater than the thickness of the part for forming the slack 18, 19 of the side part 15.

Each function forming the cuff 58 of this embodiment is the same as that of the aforementioned cuffs 56 and 57 described with reference to Figs.118 and 119. That is, operation of the cuff 58 of this embodiment is the same as the operation of the cuff described with reference to Fig.112.

In the cuff 58 of this embodiment, the thickness of the part forming the pressing surface 14 in the elastic member 13 is greater than the thickness of the part for forming the slack 18, 19 of the side part 15. Accordingly, since shape change of the pressing surface 14 is small even when pressure is applied, occurrence of noise is small and the living body 1 which the pressing surface 14 contacts can be pressed evenly. Therefore, for example, blood pressure measurement can be performed with high precision.

In the cuff (not shown in the figure) of this embodiment, elasticity of material of the part forming the pressing surface 14 in the elastic member 13 shown in Figs.118 and 119 can be configured to be less than elasticity of material for forming the slack 18, 19 on the side part 15 of the elastic member 13.

The cuff of this embodiment corresponds to a case in which, in the cuffs 56 and 57 described in Figs.118 and 119, elasticity of material of the part forming the pressing surface 14 in the elastic member 13 shown in Figs.118 and 119 is less than elasticity of material for forming the slack 18, 19 on the side part 15 of the elastic member 13. The cuff of this embodiment has the same configuration as the aforementioned cuffs 56 and 57 described witch reference to Figs.118 and 119, but is characterized in that elasticity of material of the part forming the pressing surface 14 in the elastic member 13 shown in Figs.118 and 119 is less than elasticity of material for forming the slack 18, 19 on the side part 15 of the elastic member 13.

Each function forming the cuff of this embodiment is the same as that of the aforementioned cuffs 56 and 57 described with reference to Figs.118 and 119. That is, operation of the cuff of this embodiment is the same as the operation of the cuff 50 described with reference to Fig.112.

The elasticity of the material of the part forming the pressing surface 14 is the Young's modulus of the material of the part forming the pressing surface 14. For example, when the material of the part forming the pressing surface 14 is rubber, the elasticity of the material of the part forming the pressing surface 14 is high, and when the material of the part forming the pressing surface 14 is paper that does not expand, the elasticity of the material of the part forming the pressing surface 14 is low.

The elasticity of the material of the part forming the slack 18, 19 is the Young's modulus of the material of the part forming the slack 18, 19. For example, when the material of the part forming the slack 18, 19 is rubber, the elasticity of the material of the part forming the pressing surface 14 is high, and when the material of the part forming the slack 18, 19 is paper that does not expand, the elasticity of the material of the part forming the slack 18, 19 is low.

In the cuff of this embodiment, elasticity of material of the part forming the pressing surface 14 in the elastic member 13 shown in Figs.118 and 119 is less than elasticity of material for forming the slack 18, 19 on the side part 15 of the elastic member 13. Accordingly, since shape change of the pressing surface 14 is small even when pressure is applied, occurrence of noise is small and the living body 1 which the pressing surface 14 contacts can be pressed evenly. Therefore, for example, blood pressure measurement can be performed with high precision.

The cuff (not shown in the figure) of this embodiment can be configured such that the side part of the elastic member 13 shown in Fig.112 is fixed to an outer wall of the case 12 by an elastic body.

The cuff of this embodiment corresponds to a case in which, in the cuffs described in Figs.112-115 and 120, the side part 15 of the elastic member 13 is fixed to an outer wall of the case 12 by an elastic body. The cuff of this embodiment has the same configuration as the aforementioned cuff described with reference to Figs.112-115 and 120, and each part forming the cuff is also the same as the cuff 30 shown in Fig.112, but the cuff of this embodiment is characterized in that the fixing part 17 shown in Fig.112 is the elastic body. Operation of the cuff of this embodiment is the same as the operation of the cuff described with reference to Fig.112.

In the cuff of this embodiment, by fixing the side part 15 shown in Fig. 112 to the case 12 using an elastic body such as an O ring, for example, when the pressing surface 14 or the side part 15 of the elastic member 13 becomes deteriorated due to long time use, the elastic member can be easily exchanged while keeping airtightness. Therefore, maintenance becomes easy.

The cuff of this embodiment can be configured such that the side part 15 of the elastic member 13 shown in Fig.112 may be fixed to an outer wall of the case 12 by elasticity of the side part 15 of the elastic member 13.

The cuff of this embodiment corresponds to a case in which, in the cuffs described in Figs.112-115 and 120, the side part 15 of the elastic member 13 is fixed to an outer wall of the case 12 by elasticity of the side part 15 of the elastic member 13.

The cuff of this embodiment is described with reference to the attached figure. Fig.121 is a schematic section view showing the configuration of the cuff of this embodiment. In Fig.121, the cuff 59 includes the case 12, the elastic member 13 and the air supply pipe 16. The elastic member 13 includes the pressing surface 14 and the side part 15.

The case 12 includes a function for holding the elastic member 13, and the air supply pipe 16 includes a function for supplying air into the inside of the case 12 and may include a function for releasing the supplied air.

The pressing surface 14 of the elastic member 13 contacts the living body 1, and has a function for pressing the living body 1 by pressure of air supplied into the cuff 59 surrounded by the elastic member 13 and the case 12. The side part 15 of the elastic member 13 has a function for holding the pressing surface 4 and keeping airtightness between the elastic member 13 and the 12 using elasticity.

Operation of the cuff 59 of this embodiment is the same as the cuff described with reference to Fig.112.

In the cuff of this embodiment, by keeping airtightness in the cuff 59 surrounded by the elastic member 13 and the case 12 by using elasticity of the side part 15 of the elastic member 13, for example, when the side part 15 of the elastic member 13 becomes deteriorated due to long time use, the elastic member can be easily exchanged while keeping airtightness without requiring excessive parts. Therefore, maintenance becomes easy.

The cuff (not shown in the figure) of this embodiment can be configured such that the side part 15 of the elastic member 13 shown in Fig.112 may be fixed to an outer wall of the case 12 by thermocompression bonding.

The cuff of this embodiment corresponds to a case in which, in the cuffs described in Figs.112- 115 and 120, the side part 15 of the elastic member 13 shown in Fig.112 is fixed to an outer wall of the case 12 by thermocompression bonding. The cuff of this embodiment has the same configuration of the cuff 59 described with reference to Fig.121.

Functions of the case 12, the air supply pipe 16 and the pressing surface 14 of the elastic member 13 are the same as those of the cuff 59 described with reference to Fig.121, but the side part 15 of the elastic member 13 in the cuff of the embodiment is fixed to the wall of the case 13 by thermocompression bonding.

Operation of the cuff of this embodiment is the same as the cuff described with reference to Fig.112.

In the cuff of this embodiment, by fixing the side part 15 of the elastic member 13 to the case 12 by thermocompression bonding, airtightness in the cuff can be kept without requiring excessive parts. Therefore, the cuff of this embodiment is economical.

The cuff of this embodiment can be configured such that a light-emitting element for emitting light to the outside from the inside of the cuff through the pressing surface 14 is provided in the inside of the case 12 shown in Fig.112, and the pressing surface 14 of the elastic member 13 is transparent or semitransparent for light emitted by the light-emitting element.

The cuff of this embodiment corresponds to a case in which, in the cuffs described in Figs.112 115 and 121, a light-emitting element for emitting light to the outside from the inside of the cuff through the pressing surface 14 is provided in the inside of the case 12, and the pressing surface 14 of the elastic member 13 is transparent or semitransparent for light emitted by the light-emitting element.

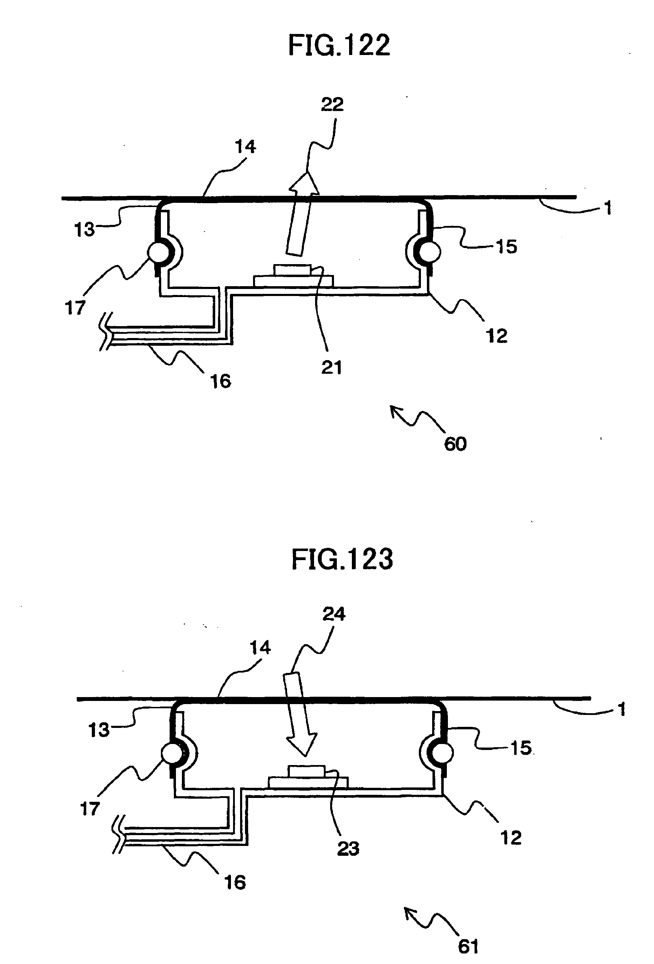

The cuff of this embodiment is described with reference to the attached figure. Fig.122 is a schematic section view showing the configuration of the cuff of this embodiment. In Fig.122, the cuff 60 includes the case 12, the elastic member 13, the air supply pipe 16, the fixing part 17 and the light-emitting element 21. The elastic member 13 includes the pressing surface 14 and the side part 15. In Fig.122, parts such as a driving circuit of the light-emitting element 21 that can be realized by general technology are not shown.

Functions of the case 12, the air supply pipe 16 and the fixing part 17 forming the cuff 60 are the same as those of the cuff 50 described with reference to Fig.112. The light-emitting element 21 is placed in the case 12, and has a function for emitting light to the outside from the inside of the cuff 60 through the pressing surface 14. That is, the light-emitting element 21 emits irradiation light 22 to the living body on which the pressing surface 14 presses. The pressing surface 14 of the elastic member 13 contacts the living body 1, and has a function for pressing the living body by the air pressure supplied in the inside of the cuff 60 surrounded by the elastic member 13 and the case. In addition, the elastic member 13 is transparent or semitransparent for the irradiation light emitted by the light-emitting element 21. In addition, the side part 15 of the elastic member 13 holds the pressing surface 13 and has a function for keeping airtightness between the elastic member 13 and the case 12.

Operation of the cuff of this embodiment is described in a case when the cuff 60 of this embodiment is used as a blood pressure measurement apparatus. By supplying air into the cuff 60 via the air supply pipe 16, the pressing surface 14 is swelled to press the living body 1. Then, bloodstream of the artery of the living body 1 stops by the pressure of the pressing surface 14 to the living body 1. In the state in which the pulse wave is disappeared, air in the inside of the cuff 60 is released via the air supply pipe 16 to decrease the pressure for pressing the living body by the pressing surface.14.

In the process in which the pressure applied to the living body 1 by the pressing surface 14 is increased and decreased after that, the light-emitting element 21 emits the irradiation light to the living body 1 on which the pressing surface presses. The irradiation light 22 is scattered by the artery of the living body 1. By receiving the scattered light by a light-receiving element (not shown in the figure) placed at a position opposed to the living body 1 in the cuff 60, change of the pulse wave of the artery of the living body 1 can be detected. Based on the change of the pulse wave of the artery and the pressure in the cuff 60, blood pressure, bloodstream amount and speed of the blood flow can be measured according to a predetermined method.

In the aforementioned blood pressure measurement, since the pressing surface 14 is transparent or semitransparent for the light emitted by the light-emitting element 21, the irradiation light of the light-emitting element can be efficiently irradiated on the living body 1. Therefore, the cuff 60 of this embodiment can measure the blood pressure, for example, with high precision.

The cuff of this embodiment can be configured such that a light-receiving element for receiving scattered light scattered in the outside of the cuff through the pressing surface is provided in the inside of the case 12 shown in Fig.112, and the pressing surface 14 of the elastic member 13 is transparent or semitransparent for the scattered light received by the light-receiving element.

The cuff of this embodiment corresponds to a case in which, in the cuffs described in Figs.112-115 and 121, a light-receiving element for receiving scattered light scattered in the outside of the cuff through the pressing surface is provided in the inside of the case 12 shown in Fig.112, and the pressing surface 14 of the elastic member 13 is transparent or semitransparent for the scattered light received by the light-receiving element.

Fig. 123 is a schematic section view showing the configuration of the cuff of this embodiment. The cuff 61 of this embodiment is configured to be provided with a light-receiving element 23 shown in Fig.123 instead of the light-emitting element 21 shown in Fig.21. That is, functions of the case 12, the elastic member 13, the air supply pipe 16 and the fixing part 17 forming the cuff of this embodiment are the same as those of the cuff described with reference to Fig.122. The light-receiving element 23 shown in Fig.123 can be placed in the inside of the same case 12 in the cuff 60 shown in Fig.122 in addition to the light-emitting element 21.

The pressing surface 14 of the elastic member 13 shown in Fig.123 contacts the living body 1, and has a function for pressing the living body 1 by the air pressure supplied in the inside of the cuff 61 surrounded by the elastic member 13 and the case 12. The light-receiving element 23 has a function for receiving the scattered light 24 scattered in the outside of the cuff through the pressing surface 14. In addition, the elastic member 13 is transparent or semitransparent for the scattered light 24 scattered in the outside of the cuff 61. In addition, the side part 15 of the elastic member 13 holds the pressing surface 14 and has a function for keeping airtightness between the elastic member 13 and the case 12.

Operation of the cuff 61 of this embodiment is described taking, as an example, a case in which the cuff 61 of this embodiment is used as a blood pressure measurement apparatus. By supplying air into the cuff 61 by the air supply pipe 16, the pressing surface 14 is swelled to press the living body 1. Then, bloodstream of the artery of the living body 1 stops by the pressure of the pressing surface 14 on the living body 1. In the state in which the pulse wave is disappeared, air in the inside of the cuff 60 is released via the air supply pipe 16 to decrease the pressure for pressing the living body 1 by the pressing surface 14.

In the process in which the pressure applied to the living body 1 by the pressing surface 14 is increased and decreased after that, a light-emitting element (not shown in the figure) provided at a position opposite to the cuff 61 with respect to the living body 1 emits irradiation light to the living body 1 on which the pressing surface presses. The irradiation light is scattered by the artery of the living body 1. By receiving the scattered light 24 by the light-receiving element 23, change of the pulse wave of the artery of the living body 1 can be detected. Based on the detected change of the pulse wave of the artery and the pressure in the cuff 61, blood pressure, bloodstream amount and speed of the blood can be measured according to a predetermined method.

In the aforementioned blood pressure measurement, since the pressing surface 14 is transparent or semitransparent for the scattered light scattered by the living body 1, the light-receiving element 23 can receive the scattered light 24 efficiently. Therefore, the cuff 61 of this embodiment can measure the blood pressure, for example, with high precision.

The cuff of this embodiment includes, in the inside of the case 12 shown in Fig.112, a light-emitting element for emitting light from the inside to the outside of the cuff through the pressing surface and a light-receiving element for receiving scattered light scattered in the outside of the cuff 50 through the pressing surface, and the pressing surface 14 is transparent or semitransparent for the light emitted by the light-emitting element and the scattered light received by the light-receiving element.

The cuff of this embodiment corresponds to a case in which, in the cuffs described in Figs.112-115 and 121, the cuff includes, in the inside of the case 12, a light-emitting element for emitting light from the inside to the outside of the cuff through the pressing surface and a light-receiving element for receiving scattered light scattered in the outside of the cuff 50 through the pressing surface, and the pressing surface 14 is transparent or semitransparent for the light emitted by the light-emitting element and the scattered light received by the light-receiving element.

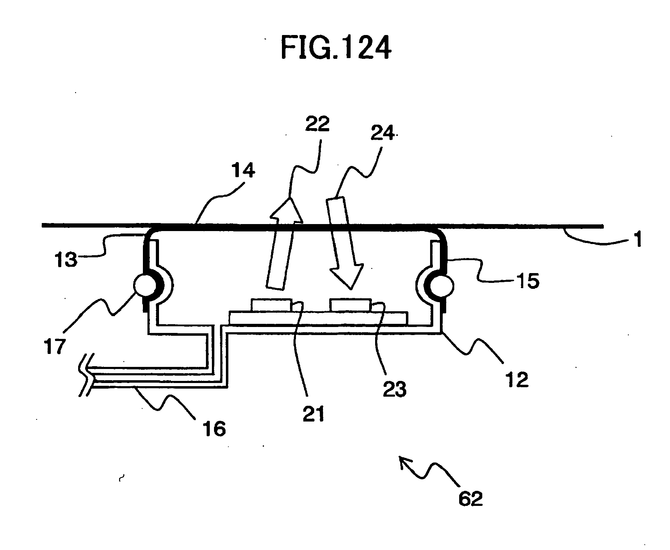

Fig.124 is a schematic section view showing the configuration of the cuff of this embodiment. The cuff 62 of this embodiment is configured to be provided with the light-receiving element 23 shown in Fig.123 in addition to the light-emitting element shown in Fig.122 in the same case 12. That is, functions of the case 12, the elastic member 13, the air supply pipe 16, the fixing part 17, the light-emitting element 21 and the light-receiving element 23 forming the cuff 62 of this embodiment are the same as those of the cuffs 60 and 61 described with reference to Figs.122 and 123.

The pressing surface 14 of the elastic member 13 contacts the living body 1, and has a function for pressing the living body 1 by the air pressure supplied into the inside of the cuff 62 surrounded by the elastic member 13 and the case 12. In addition, the side part 15 of the elastic member 13 holds the pressing surface 14 and has a function for keeping airtightness between the elastic member 13 and the case 12. The elastic member 13 is transparent or semitransparent for the irradiation light 22 emitted by the light-emitting element 21 and the scattered light 23 received by the light-receiving element 23.

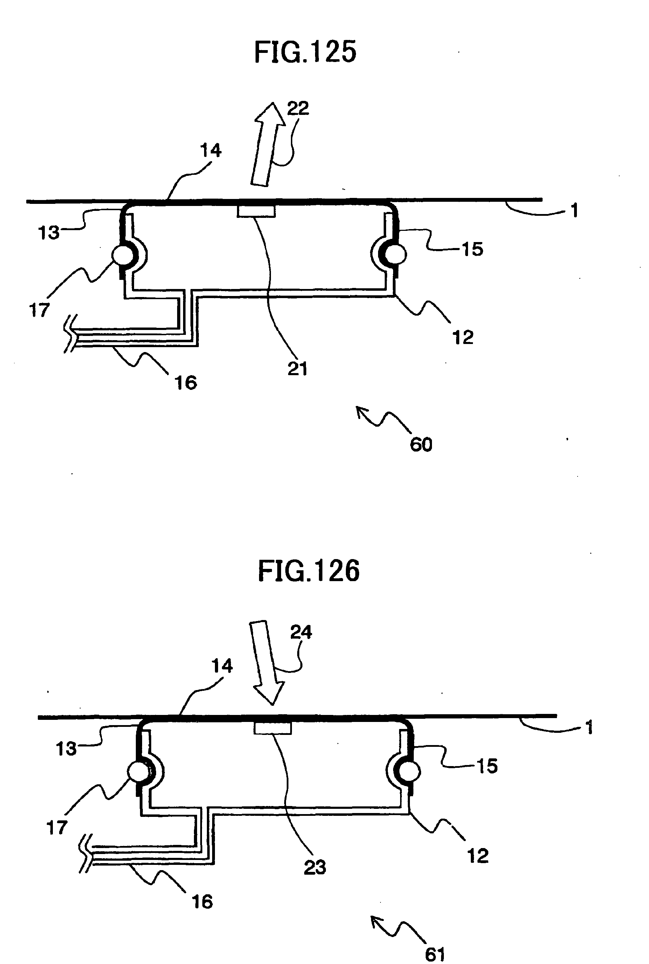

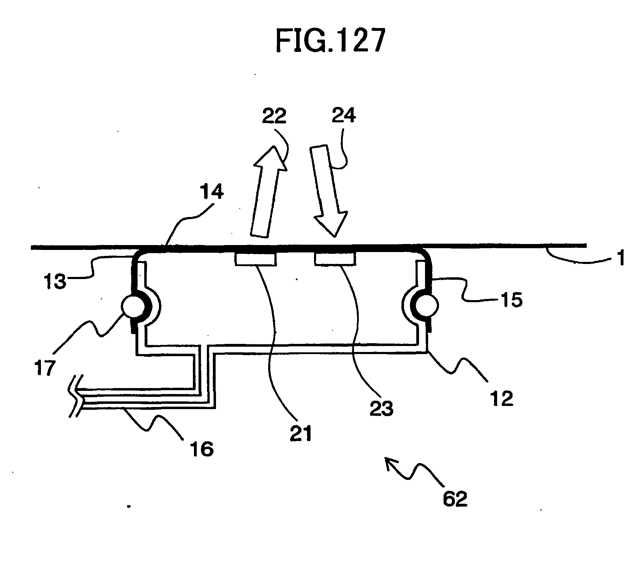

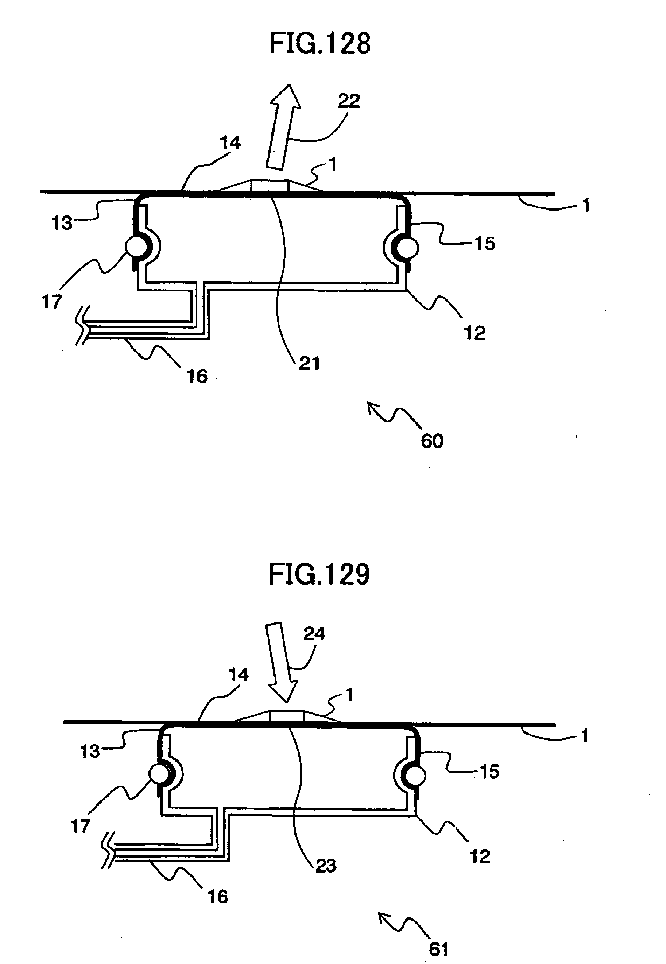

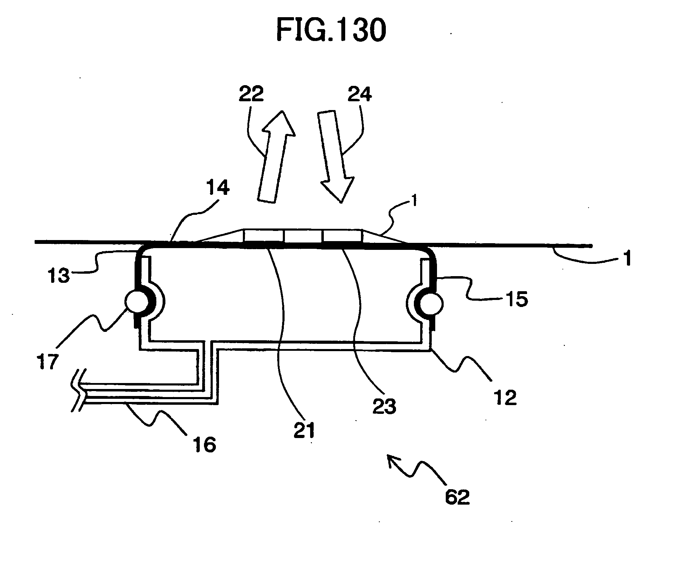

By the way, Figs.122, 123 and 124 show examples in which the light-emitting element 21 and the light-receiving element 23 are placed on the case in the inside of the cuff. But, the light-emitting element and the light-receiving element may be stuck to an inner surface (back surface) of the cuff as shown in Figs.125, 126 and 127. Or, they may be stuck to an outer surface of the cuff as shown in Fig.128, 129 and 130. Both cases have a merit that they are not susceptible to body movement noise due to change of distance between the living body 1 and the light-emitting element 21 or the light-receiving element 23 caused by body movement. In addition, as to the latter case, since light does not pass through the cuff, there is a merit that even a cuff composed of a material that absorbs light can be used.

Operation of the cuff 62 of this embodiment is described taking a case as an example in which the cuff 62 of this embodiment is used as a blood pressure measurement apparatus. By supplying air into the cuff 62 by the air supply pipe 16, the pressing surface 14 is swelled to press the living body 1. Then, bloodstream of the artery of the living body 1 stops by the pressure of the pressing surface 14 on the living body 1. In the state in which the pulse wave is disappeared, air in the inside of the cuff 62 is released via the air supply pipe 16 to decrease the pressure for pressing the living body 1 by the pressing surface 14.

In the process in which the pressure applied to the living body 1 by the pressing surface 14 is increased, and decreased after that, the light-emitting element 21 emits irradiation light 22 to the living body 1 on which the pressing surface presses. The irradiation light is scattered by the artery of the living body 1. By receiving the scattered light 24 by the light-receiving element 23, change of the pulse wave of the artery of the living body 1 can be detected. Based on the detected change of the pulse wave of the artery and the pressure in the cuff 62, blood pressure, bloodstream amount and speed of the blood flow can be measured according to a predetermined method.

In the aforementioned blood pressure measurement, since the pressing surface 14 is transparent or semitransparent for the light emitted to the living body 1 by the light-emitting element 21 and the scattered light 24 scattered by the living body 1, the light emitting 21 light can irradiate the pressed part of the living body 1 with the irradiation light 22 efficiently and the light-receiving element 23 can receive the scattered light 24 efficiently. In addition, by providing the light-emitting element 21 and the light-receiving element 23 in the same case 12, optical path length from light emission by the light-emitting element 21 to light reception by the light-receiving element 23 can be decreased. Thus, attenuation of light strength is small. Therefore, the cuff 62 of this embodiment can measure the blood pressure, for example, with high precision.

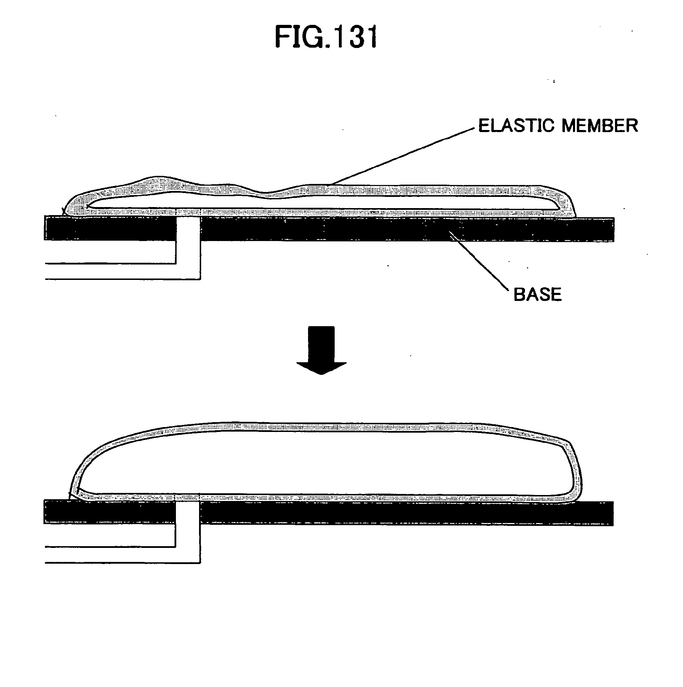

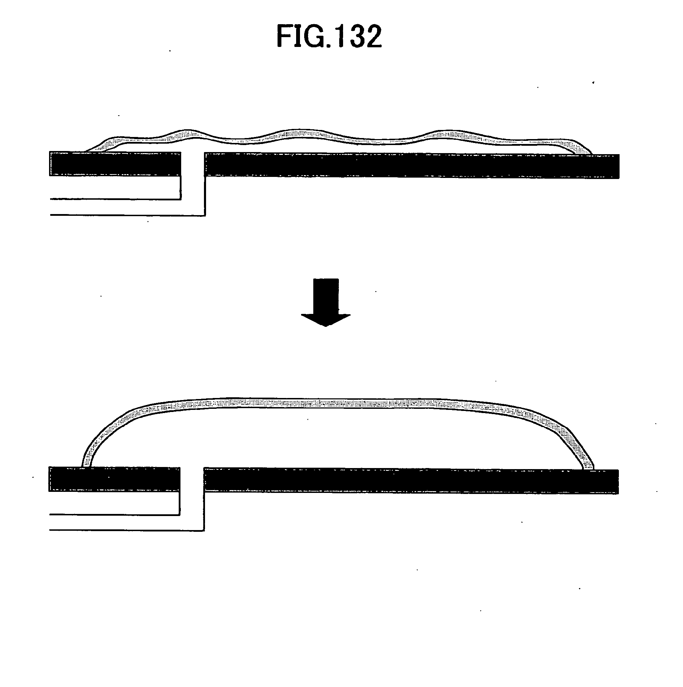

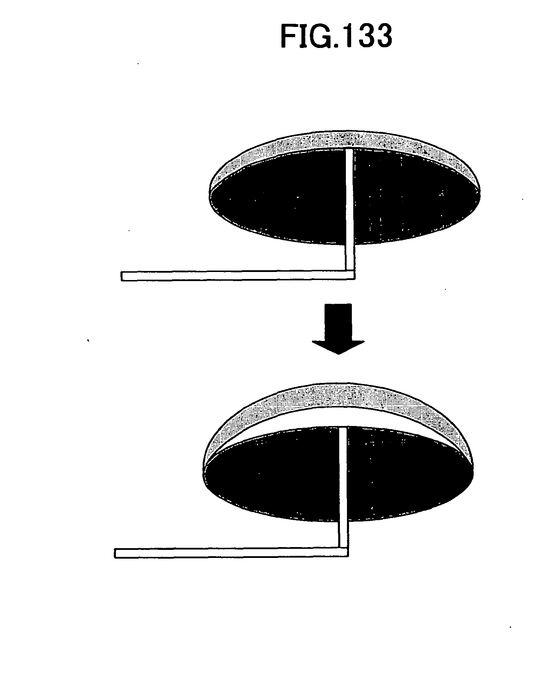

By the way, the case of the cuff of this embodiment forms a base for supporting the elastic member. The shape of the base is not necessarily a case-like shape as long as the base is composed of non-elastic member. For example, it can be plane-like as shown in Figs.131 and 132. Fig.131 shows an example in which an elastic member that is a bag keeping airtightness by itself is fixed on the base. Fig.132 shows an example in which an end part of a sheet-like member is fixed on the base by bonding, fusing and the like to keep airtightness. In addition, as shown in Fig.133, the shape of the base can be like a curved plane.

In such cuffs, expansion of the cuff to the base side is restricted by the base composed of the non-elastic material as shown in Fig.131-133. As a result, pressure can be efficiently applied to the living body. It may be difficult to apply this structure of the cuff to a conventional blood-pressure meter for winding around the upper arm, but this structure is effective for applying pressure on a narrow part such as the ear.

By the way, the cuff described in the fifth embodiment can be applied to apparatuses (including blood-pressure meters) for measuring living body information in every embodiment in this specification.

As described above, the cuff of this embodiment is configured to place the elastic member on an open face of the case so as to be able to press a periphery part of a living body, that is the tragus for example, by supplying air into the cuff. Thus, a part of the living body can be pressed at a pinpoint efficiently and evenly. Therefore, the blood pressure and the like can be measured with high precision even in a relatively small part of the living body such as the auricle and the tragus, for example.

By forming the outer shape of the pressing surface of the cuff that contacts and presses the living body to be round or elliptic, compared with polygon such as quadrangle, (1)airtightness in the inside of the case can be easily increased, (2) the pressure applied by the pressing surface becomes uniform, (3) allowance of position shifts with respect to artery of the living body on which the pressing surface presses is large, (4) when an after-mentioned light-emitting element irradiates the living body with light passing through the pressing surface, since a section of an emission pattern of light emitted from the light-emitting element is round or elliptic, it is easy to match the section with the uniform-pressure distribution of the pressing surface so that measurement accuracy can be increased, (5) since the shape does not have a corner, damage of the elastic member due to repetition of expanding and contracting can be prevented.

In addition, by shaping the pressing surface of the elastic member to be concave in relation to the outside of the cuff so as to provide a warp, the pressure for expanding the pressing surface can be small in the process for pressing the living body by the pressing surface. Therefore, the pressing surface can press the living body with a small air pressure.

In addition, by shaping the pressing surface of the elastic member to be convex in relation to the outside of the cuff so as to provide a warp, the pressure for expanding the pressing surface can be small in the process for pressing the living body by the pressing surface. Therefore, the pressing surface can press the living body with small air pressure.

In addition, by shaping the pressing surface of the elastic member to be plane, even when applying a pressure or releasing a pressure in the inside of the cuff surrounded by the case and the elastic member, any warp does not appear on the pressing surface. Therefore, since noise due to appearance and disappearance of warps does not occur, blood pressure, for example, can be measured with high precision.

In addition, by providing a warp for expanding and contracting toward the living body to move the pressing surface on the side part of the elastic member having functions for supporting the pressing surface of the cuff and keeping airtightness between the elastic member and the case, the pressing surface can be easily moved to the living body. Therefore, the pressing surface can press the living body with small pressure.

By setting the expansion ratio of the pressing surface of the elastic member to be smaller than the expansion ratio of the warp of the side part of the elastic member, shape change becomes small when applying pressure or releasing pressure in the inside of the cuff surrounded by the case and the elastic member. Thus, noise does not occur very much, and the living body which the pressing surface contacts can be pressed evenly. Therefore, for example, blood pressure and the like can be measured with high precision.

In addition, by forming the elastic member such that the thickness of the part forming the pressing surface is greater than the thickness of the part forming the warping part, shape change becomes small when applying pressure or releasing pressure in the inside of the cuff surrounded by the case and the elastic member. Thus, noise does not occur very much, and the living body which the pressing surface contacts can be pressed evenly. Therefore, for example, blood pressure and the like can be measured with high precision.

In addition, by forming the elastic member such that the expansion ratio of the part forming the pressing surface is smaller than the expansion ratio of the part forming the warping part, shape change of the pressing surface becomes small when applying pressure or releasing pressure in the inside of the cuff surrounded by the case and the elastic member. Thus, noise does not occur very much, and the living body which the pressing surface contacts can be pressed evenly. Therefore, for example, the blood pressure and the like can be measured with high precision.

In addition, by fixing the side part of the elastic member to the outer wall of the case by an elastic body such as an O ring, for example, airtightness can be kept, and on the other hand, the elastic member can be easily exchanged. Therefore, maintenance becomes easy.

In addition, by fixing the side part of the elastic member to the outer wall of the case by using elasticity of the side part of the elastic member, airtightness can be kept without requiring redundant parts, and on the other hand, the elastic member can be easily exchanged. Therefore, maintenance becomes easy.

In addition, by fixing the side part of the elastic member to the outer wall of the case by thermocompression bonding, airtightness can be kept without requiring redundant parts, so that an economical cuff can be provided.

In addition, by providing, in the inside of the case, a light-emitting element for emitting light to the outside from the inside of the cuff through the pressing surface, that is, for irradiating a part of the living body which the pressing surface of the elastic member contacts with light, and by forming the pressing surface of the elastic member to be transparent or semitransparent for the light emitted by the light-emitting element, the pressed part of the living body can be efficiently irradiated with light. Therefore, by receiving the scattered light scattered in the artery, for example, among the light emitted to the pressed part of the living body, the pulse wave of the artery when the living body is pressed, speed of blood or blood flow amount can be measured with high precision, so that blood pressure and the like can be measured with high precision.

In addition, by providing, in the inside of the case, a light-receiving element for receiving scattered light scattered in the outside of the cuff through the pressing surface, that is, for receiving scattered light scattered in a part of the living body, and by forming the pressing surface of the elastic member to be transparent or semitransparent for the scattered light scattered in the part of the living body, the scattered light scattered in the pressed part of the living body, that is the artery for example, can be efficiently received. Therefore, the pulse wave of the artery when the living body is pressed, speed of blood or blood flow amount can be measured with high precision, so that blood pressure and the like can be measured with high precision.

In addition, by providing, in the inside of the case, a light-emitting element for emitting light to the outside from the inside of the cuff through the pressing surface, that is, for irradiating a part of the living body which the pressing surface of the elastic member contacts with light, and a light-receiving element for receiving scattered light scattered in the outside of the cuff through the pressing surface, that is, for receiving scattered light scattered in a part of the living body, and by forming the pressing surface of the elastic member to be transparent or semitransparent for light emitted by the light-emitting element and for the scattered light scattered in the part of the living body, the pressed part of the living body can be efficiently irradiated with light, and the scattered light scattered in the pressed part of the living body that is the artery, for example, can be efficiently received. In addition, by providing the light emitting element and the light-receiving element in the same case, since the optical path length from light emission by the light-emitting element to the light reception by the light-receiving element can be decreased, attenuation of the light strength is small. Therefore, the pulse wave of the artery when the living body is pressed, speed of blood or blood flow amount can be measured with high precision, so that blood pressure and the like can be measured with high precision.

As mentioned above, while the cuff of this embodiment is easy to perform maintenance, the part of the living body can be pressed uniformly and efficiently with a small pressure while keeping airtightness in the inside of the cuff. In addition, according to the cuff of the present invention, the pressure in the cuff changes continuously so that noise due to rapid change of the pressure in the cuff does not occur very much. Therefore, the blood pressure and the like can be measured with high precision, for example. Further, the cuff of the present invention including the light-emitting element or the light-receiving element can measure the pulse wave of the artery, speed of blood flow or blood flow amount when pressing the living body, for example.

### (Sixth embodiment)

In the living body information detection apparatus described so far, when using the light-receiving element and the light-emitting element, it is a problem to receive scattered light from a target position of the living body with high precision. In the following, embodiments of living body information detection circuit that can receive the scattered light from the living body with high precision and that includes the light-receiving element and the light-emitting element are described.

The living body information detection circuit of this embodiment includes a light-emitting element for irradiating a part of the living body with light and a light-receiving element for receiving scattered light of the irradiating light scattered in the part of the living body to detect a pulsation waveform, wherein the light-receiving element includes a light shielding structure for limiting an angle of light entering the light-receiving element in front of the light-receiving element. By the way, "front" means an external side of the light-receiving element with respect to a plane including a light-receiving surface of the light-receiving element. When reciting "in front of the light-emitting element", the "front" means an external side of the light-emitting element with respect to a plane including a light-emitting surface of the light-emitting element.

The living body information detection circuit of this embodiment is described with reference to attached figures taking a case, as an example, for applying the circuit to blood pressure measurement for a living body.

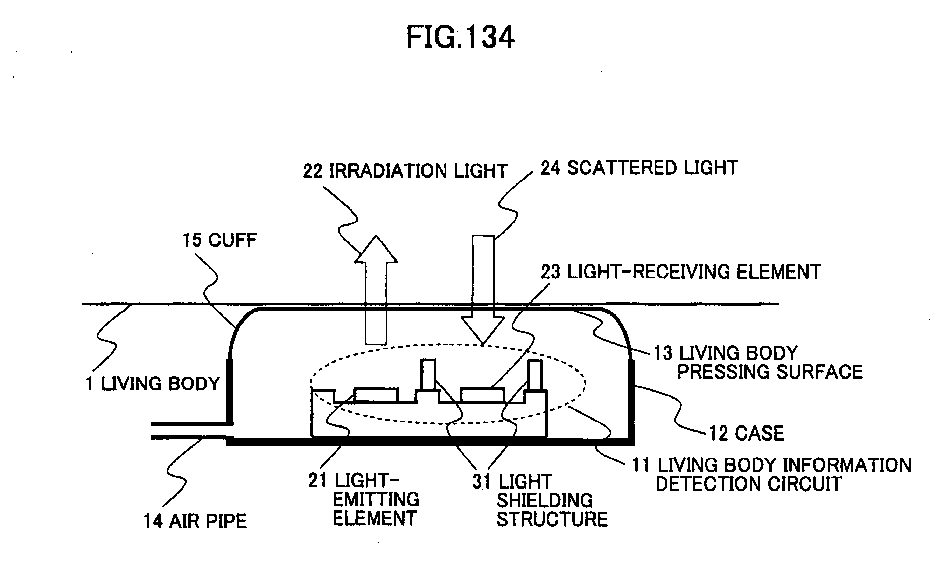

Fig.134 is a figure showing a configuration of the living body information detection circuit of this embodiment. In Fig.134, the living body information detection circuit 11 includes a light-emitting element 21, the light-receiving element 23 and a light shielding structure 31. The living body information detection circuit 11 is embedded in the inside of the cuff 15 that includes a case 12, a living body pressing surface 13 and an air pipe 13. Parts that can be realized by general technology such as a driving circuit of the light-emitting element 21, an amplifying circuit of the light-receiving element 23 and a power supply are not shown.

In the cuff 15 shown in Fig.134, the case 12 holds the living body information detection circuit 11 and the living body pressing surface 13, and the living body pressing surface 13 contacts the living body 1. The light-emitting surface of the light-emitting element 21 is directed to the living body 1 to be irradiated with the irradiating light 22, and the light-receiving surface of the light-receiving element 23 is directed to receive the scattered light of the irradiating light 22 scattered by the living body 1.

The light-shielding structure 31 is provided ahead of both sides of the light-receiving element 23 so as to sandwich the light-receiving element 23, or is provided ahead of the perimeter of the light-receiving element 23 so as to surround the light-receiving element 23. But, in Fig.134, to avoid complexity of the figure, a case in which the light shielding structure 31 is provided ahead of both sides of the light-receiving element 23 so as to sandwich the light-receiving element 23 is shown. It is adequate that the light shielding structure exists between the light-receiving element 23 and the living body 1.

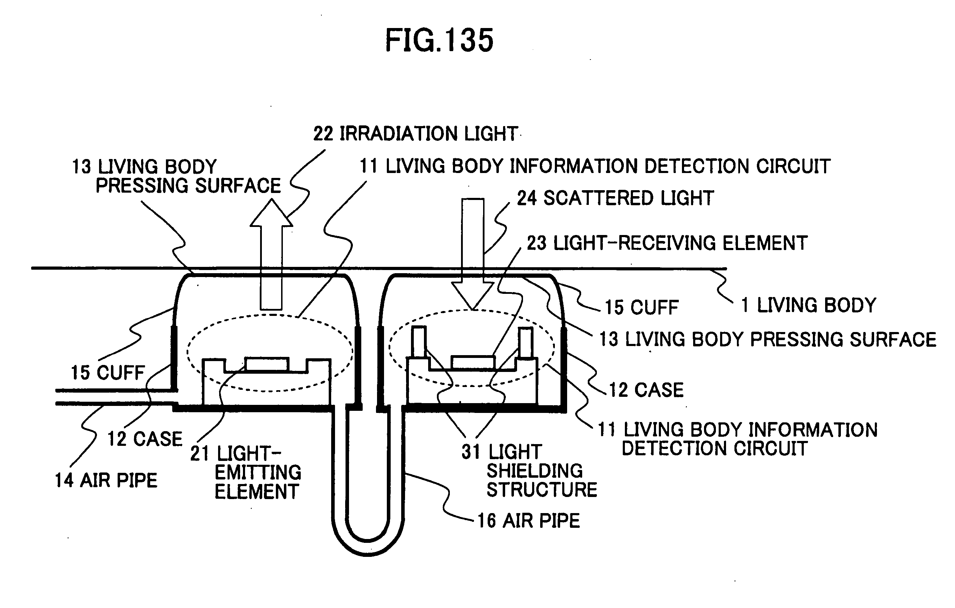

The living body information detection circuit of this embodiment can be also configured as shown in Fig.135. In the living body information detection circuit 11 of this embodiment shown in Fig.135, the cuff 15 formed by the case 12 and the living body pressing surface 13 is divided into two parts. The light-emitting element 21 of the living body information detection circuit 11 is provided in the inside of one cuff 15, and the light receiving element 23 and the light shielding structure 31 of the living body information detection circuit 11 are provided in the inside of another cuff 15. Cases 12 of the cuffs 15 are connected by the air pipe 16 so that air pressures in the cuffs 12 are kept equal.

As to the living body information detection circuit 11 and the cuff 15 of this embodiment shown in Fig.135, configuration, functions of each part, and operation are the same as those of the living body information detection circuit 11 and the cuff 15 of this embodiment shown in Fig.134 except that the cuff 15 is divided into two parts, the light-emitting element 21 and the light-receiving element 23 are provided in each cuff 15, and that the cuffs 15 are connected by the air pipe 16. Thus, in the following, descriptions are given in accordance with the living body information detection circuit 11 and the cuff 15 of this embodiment shown in Fig.134. Also for living body information detection circuits and cuffs in after-mentioned embodiments, both of the single cuff configuration such as one shown in Fig.134 and the double cuff configuration such as one shown in Fig.135 are available. But, since both are the same in functions, descriptions are given for the case in which the cuff 15 is single as shown in Fig.134.

The light-emitting element 21 has a function for irradiating the living body 1 with irradiating light. The light-receiving element 23 has a function for receiving scattered light 24 of the irradiating light 22 scattered in the living body to detect a pulsation waveform.

The light shielding structure has a function for limiting an angle of light entering the light-receiving element, and shields light, in the scattered light 24, entering the light-receiving element 23 at an angle out of a predetermined angle range so that the light-receiving element 23 receives only scattered light 24 entering the light-receiving element within the predetermined angle range from a target position in the living body, that is, from a position at which the center and the vicinity of the center of the living body pressing surface 13 of the cuff 15 adequately presses the living body. The light-shielding structure 31 may be like partitions provided in both sides of the light-receiving element 23, or may be like a tube surrounding the light-receiving element 23.

The case 12 has functions for holding the living body pressing surface 13, keeping airtightness between the case 12 and the living body pressing surface 13, and embedding the light-emitting element 21, the light-receiving element 23 and the light-shielding structure 31. The living body pressing surface 13 is made of a flexible material, contacts the living body 1 and has a function for pressing the living body 1 by the air pressure supplied to the case 12 through the air pipe 13. The air pipe has a function for supplying air into the case 12, and may further has a function for releasing air from the case 12.

Operation of the living body information detection circuit 11 and the cuff 15 of this embodiment is described. The living body pressing surface 13 of the cuff 15 presses the living body 1 by a pressure of air supplied into the case 12 by the air pipe 14 so that bloodstream in the living body stops. After that, the air in the cuff 12 is gradually released through the air pipe 13 to decrease the pressure for pressing the living body 1.

In the process for decreasing the pressure for pressing the living body 13 by the living body pressing surface 13, the light-emitting element 21 of the living body information detection circuit 11 irradiates the living body with the irradiation light 22, the irradiation light 22 is scattered in the living body 1 to become the scattered light 24. The light-shielding structure 31 limits an angle of the scattered light 24 entering the light-receiving element 23 to shield light in the scattered light 24 entering the light-receiving element 23 from a position that is not a target position in the living body 1, that is, a position other than a position at which the center and the vicinity of the center of the living body pressing surface 13 of the cuff 15 adequately presses the living body. The light-receiving element receives the scattered light 24 entering within a predetermined angle range from a position that is a target position in the living body 1, that is, a position at which the center and the vicinity of the center of the living body pressing surface 13 of the cuff 15 adequately presses the living body 1 to detect the pulsation waveform.

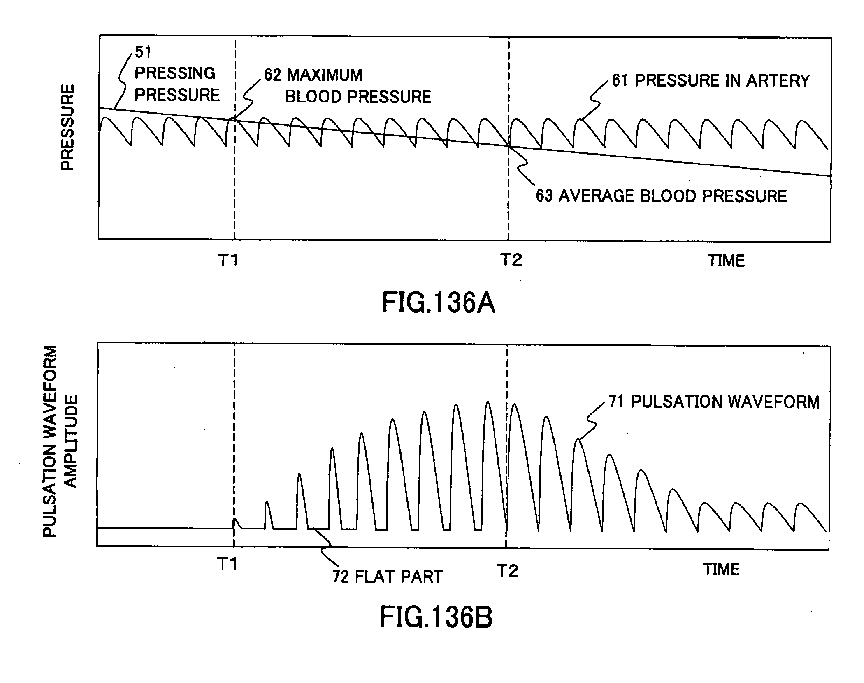

In the process for decreasing the pressure for pressing the living body 1, relationship between the pressure for pressing the living body 1 by the living body pressing surface 13 and the pulsation waveform detected by the light-receiving element 23 is described with reference to Figs.136A and 136B.

In Fig.136A, the vertical axis indicates pressure, and the horizontal axis indicates time. Fig.136A shows the relationship between the pressure 51 for pressing the living body 1 by the living body pressing surface 13 and the pressure in the artery, that is, a pressure in the inside of the artery of the living body 1.

In Fig.136B, the vertical axis indicates the amplitude of the pulsation waveform, and the horizontal axis is the same time as the horizontal axis of the Fig.136A. Fig.136B shows change of the pulsation waveform 71 of the artery of the living body 1.

In Figs.136A and 136B, the pressure 51 for pressing the living body 1 by the living body pressing surface 13 decreases over time from a high pressure to such an extent as to stop bloodstream of the artery, and at the time T1 when the pressure 51 becomes equal to the highest value of the pressure 61 in the artery that pulses due to heartbeat, the blood starts to flow and a pulsation waveform 71 appears. The pressure 51 at the time T1 is the maximum blood pressure 62. Further, an average blood pressure 63 is a value of the pressing pressure 51 at time T2 when the pressure decreases to be equal to the lowest value of the pressure 61 in the artery. Between the time T1 and the time T2, in periods when the pressure 61 in the artery is greater than the pressing pressure 51, the artery expands so that the pulse waveform 71 is detected. In periods when the pressure 61 in the artery is smaller than the pressing pressure 51, since the blood vessel cannot expand, a flat part 72 exists in the vicinity of the lowest value of the pulsation waveform 71 in which the pulsation waveform 71 is not detected. When the pressing pressure 51 further decreases to become equal to or less than the average blood pressure 63, a pulsation waveform 71 in which the artery repeats expansion and contraction is detected so that the flat part 2 disappears.

As mentioned above, the maximum blood pressure can be measured based on the value of the pressing pressure 51 at the time T1 when the pulsation waveform 71 starts to appear, and the average blood pressure can be measured based on the value of the pressing pressure 51 at the time T2 when the flat part 72 in the pulsation waveform 71 disappears. Therefore, for measuring the blood pressure with high precision, it is important to detect the pulsation waveform 71 accurately.

On the other hand, when the above-mentioned measurement is performed in a periphery part of the living body such as the auricle, it is reported that a cuff pressure 131 at a time (indicated as T2) when the amplitude of the pulsation waveform 71 becomes maximum can be approximated into the minimum blood pressure (refer to non-patent document 5, for example).

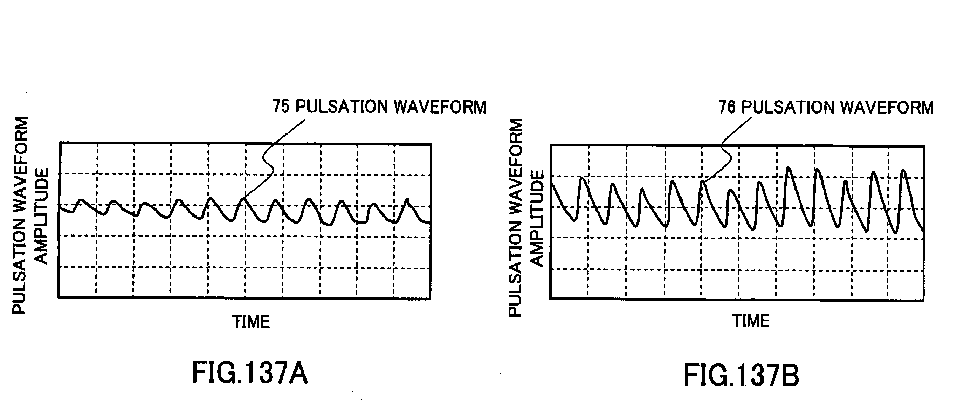

An example for detecting the pulse wave waveform using the living body information detection circuit 11 to which the light shielding structure 31 is provided in this embodiment is shown in Fig. 137A, and an example for detecting the pulse wave waveform using a conventional living body information detection circuit to which the light shielding structure 31 is not provided is shown in Fig.137B. In Figs.137A and 137B, the vertical axis indicates a pulsation waveform amplitude and the horizontal axis indicates time. Each of the pulsation waveform 75 shown in Fig.137A and the pulsation waveform 76 shown in Fig.137B is a pulsation waveform corresponding to the pulsation waveform 71 between the time T1 and the time T2 shown in Fig.136.

In the pulsation waveform 76 shown in Fig.137B, a flat part corresponding to the flat part 72 in the pulsation waveform 71 shown in Fig.136B is not clear. The reason is that, since the light shielding structure 31 is not provided, the light-receiving element 23 receives the scattered light 24 in a state in which the scattered light 24 from the artery at a position adequately pressed by the center and the vicinity of the center of the living body pressing surface 13 and the scattered light 24 from the artery at a position that is not adequately pressed by an end part of the living body pressing surface 13 are mixed. That is, even when pulsation of the artery at the position adequately pressed by the center and the vicinity of the center of the living body pressing surface 13 stops, pulsation of the artery at a position that is not adequately pressed by an end part of the living body pressing surface 13 remains, and the scattered lights 24 of both are mixed. Thus, the flat part of the pulsation waveform corresponding to a time when the pulsation of the artery stops cannot be clearly detected in the pulsation waveform 76.

On the other hand, in the pulsation waveform 75 shown in Fig.137A, a flat part corresponding to the flat part 72 of the pulsation waveform 71 shown in Fig.136 exists. The reason is that, since the light shielding structure 31 is provided, the light-receiving element 23 receives only the scattered light 24 from the artery at a position adequately pressed by the center and the vicinity of the center of the living body pressing surface 13.

As mentioned above, in the living body information detection circuit 11 of this embodiment, the light shielding structure 31 is provided in front of the light-receiving element 23 so that the angle of the light entering the light-receiving element 23 is limited and the scattered light 24 entering the light-receiving element 23 at an angle out of the predetermined range is shielded. Thus, the scattered light 23 from the artery at a position at which the living body 1 is surely pressed by the living body pressing surface 13 can be selectively received, so that the pulsation waveform 71 can be detected with high precision. As a result, since T1 and T2 can be detected accurately, the maximum blood pressure, the average blood pressure or the minimum blood pressure can be accurately measured.

Up to this, the living body information detection circuit of this embodiment has been described taking a case as an example in which the circuit is applied to measurement for blood pressure of the living body. But, the living body information detection circuit of this embodiment, living body information detection circuits in after-mentioned embodiments, and living body information measurement apparatuses in after-mentioned embodiments, can be applied to detection of various living body information and to measurement of various living body information other than the blood pressure measurement.

As described above, according to the present invention, a living body information detection circuit detecting the pulsation waveform with high precision can be provided.

In the living body information detection circuit of this embodiment, the light shielding structure may be a hood provided in front of the light-receiving element.

The living body information detection circuit of this embodiment is described with reference to attached figures taking a case, as an example, for applying the circuit to blood pressure measurement for a living body.

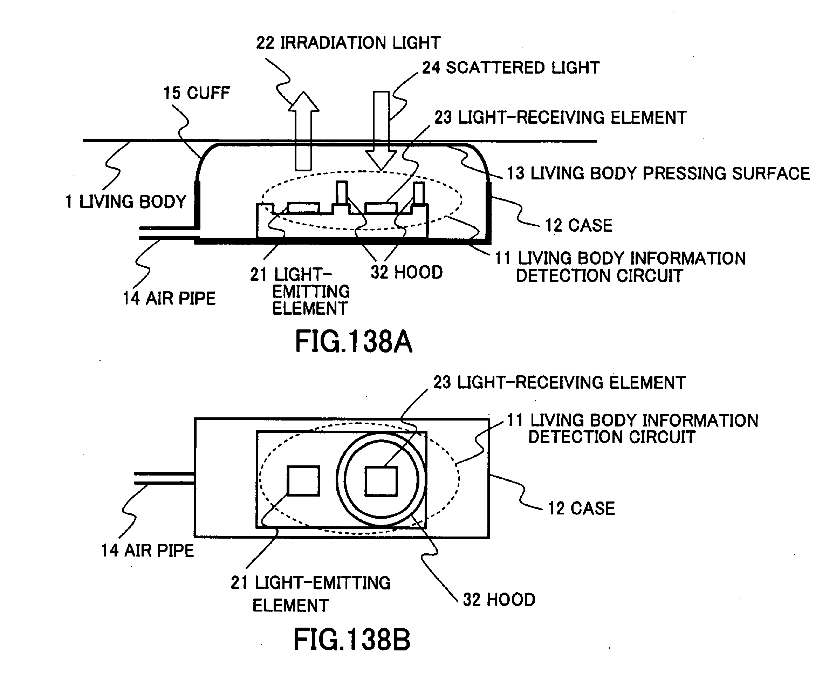

Fig. 138A and 138B are figures showing a configuration of the living body information detection circuit of this embodiment. Fig. 138A shows a state in which the living body information detection circuit of this embodiment is embedded in the cuff 15 and the cuff 15 contacts the living body. Fig.138B is a view, viewed from the living body 1, of the state in which the living body information detection circuit of this embodiment is embedded in the cuff 15 shown in Fig.138A.

The living body information detection circuit of this embodiment shown in Figs.138A and 138B is configured to be provided with the hood 32 instead of the light shielding structure 31 of the living body information detection circuit 11 of this embodiment described with reference to Fig.134. In the configuration of the living body information detection circuit 11 and the cuff 15 of this embodiment shown in Figs.138A and 138B, configurations other than the hood 32 of the living body information detection circuit are the same as those of the living body information detection circuit 11 and the cuff 15 of the embodiment described with reference to Fig.134.

In Figs.138A and 138B, the hood 32 of the living body information detection circuit 11 is shaped like a cylinder and is provided so as to surround the light-receiving element 23. Although the cylinder-like hood 32 is shown as an example, it may be like a square-tube.

In the living body information detection circuit 11 and the cuff 15 of this embodiment, functions of parts other than the hood 32 are the same as those of the living body information detection circuit 11 and the cuff 15 of the embodiment described with reference to Fig.134, and the function of the hood 32 of the living body information detection circuit 11 of this embodiment is the same as the light shielding structure 31 of the living body information detection circuit 11 of the embodiment described with reference to Fig.134.

In the living body information detection circuit 11 and the cuff 15 of this embodiment, operations of parts other than the hood 32 are the same as those of the living body information detection circuit 11 and the cuff 15 of the embodiment described with reference to Fig.134, and the operation of the hood 32 of the living body information detection circuit 11 of this embodiment is the same as the light shielding structure 31 of the living body information detection circuit 11 of the embodiment described with reference to Fig.134.

As mentioned above, in the living body information detection circuit 11 of this embodiment, the hood 32 is provided in front of the light-receiving element 23 so that the angle of the light entering the light-receiving element 23 is limited and the scattered light 24 entering the light-receiving element 23 at an angle out of the predetermined range is shielded. Thus, the scattered light 23 from the artery at a position at which the living body 1 is surely pressed by the living body pressing surface 13 can be selectively received, so that the pulsation waveform 71 can be detected with high precision.

As described above, according to the present invention, a living body information detection circuit detecting the pulsation waveform with high precision can be provided.

In the living body information detection circuit of this embodiment, the light shielding structure may include an aperture in front of the light-receiving element.

The living body information detection circuit of this embodiment is described with reference to attached figures taking a case, as an example, for applying the circuit to blood pressure measurement for a living body.

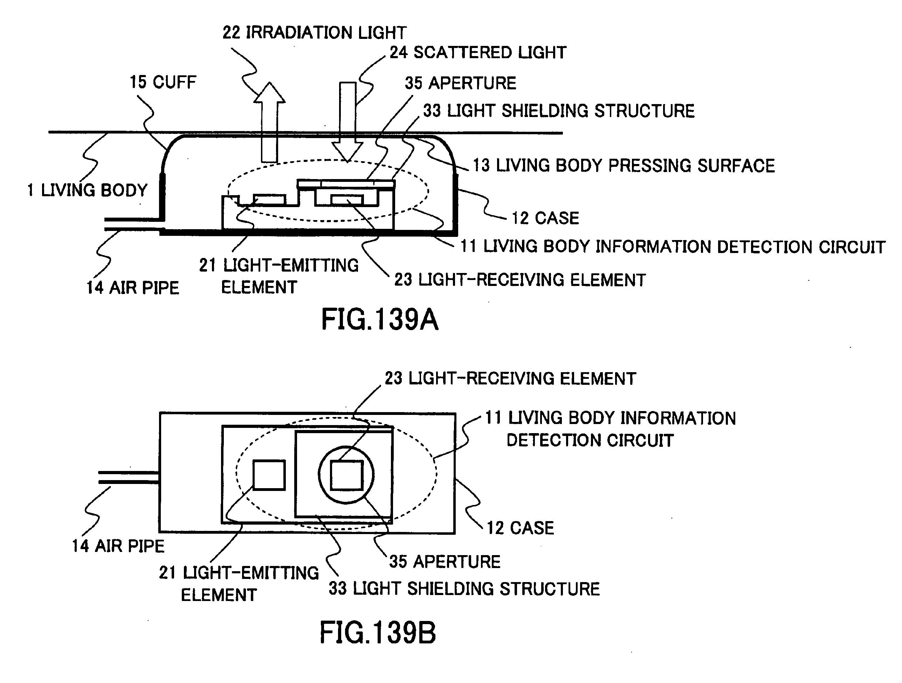

Fig.139A and 139B are figures showing a configuration of the living body information detection circuit of this embodiment. Fig.139A shows a state in which the living body information detection circuit of this embodiment is embedded in the cuff 15 and the cuff 15 contacts the living body. Fig.139B is a view, viewed from the living body 1, of the state in which the living body information detection circuit of this embodiment is embedded in the cuff 15 shown in Fig.139A.

The living body information detection circuit of this embodiment shown in Figs.139A and 139B is configured to be provided with a light shielding structure 33 having an aperture 35 in place of the light shielding structure 31 of the living body information detection circuit 11 of the embodiment described with reference to Fig.134. In the configuration of the living body information detection circuit 11 and the cuff 15 of this embodiment shown in Figs.139A and 139B, configurations other than the light shielding structure having the aperture 35 of the living body information detection circuit 11 are the same as those of the living body information detection circuit 11 and the cuff 15 of the embodiment described with reference to Fig.134.

In Figs.139A and 139B, the light shielding structure including the aperture 35 of the living body information detection circuit 11 includes a round aperture 35, and is provided ahead of the light-receiving element 23. Although the round aperture is shows as an example of the aperture of the light-shielding structure 33, the aperture 35 may be an ellipse, a rectangle, or other shape.

In the living body information detection circuit 11 and the cuff 15 of this embodiment, functions of parts other than the light shielding structure 33 having the aperture 35 are the same as those of the living body information detection circuit 11 and the cuff 15 of the embodiment described with reference to Fig.134, and the function of the light shielding structure 33 having the aperture 35 of the living body information detection circuit 11 of this embodiment is the same as the light shielding structure 31 of the living body information detection circuit 11 of the embodiment described with reference to Fig.134.

In the living body information detection circuit 11 and the cuff 15 of this embodiment, operations of parts other than the light shielding structure 33 having the aperture 35 are the same as those of the living body information detection circuit 11 and the cuff 15 of the embodiment described with reference to Fig.134, and the operation of the light shielding structure 33 having the aperture 35 of the living body information detection circuit 11 of this embodiment is the same as the light shielding structure 31 of the living body information detection circuit 11 of the embodiment described with reference to Fig.134.

As mentioned above, in the living body information detection circuit 11 of this embodiment, the light shielding structure 33 having the aperture 35 is provided in front of the light-receiving element 23 so that the angle of the light entering the light-receiving element 23 is limited and the scattered light 24 entering the light-receiving element 23 at an angle out of the predetermined range is shielded. Thus, the scattered light 23 from the artery at a position at which the living body 1 is surely pressed by the living body pressing surface 13 can be selectively received, so that the pulsation waveform 71 can be detected with high precision.

As described above, according to the present invention, a living body information detection circuit detecting the pulsation waveform with high precision can be provided.

The living body information detection circuit of this embodiment includes a light-emitting element for irradiating a part of the living body with light and a light-receiving element for receiving scattered light of the irradiating light scattered in the part of the living body so as to detect a pulsation waveform, wherein the light-receiving element includes a lens, in front of the light-receiving element, for concentrating scattered light from a particular position of the living body among the scattered light onto a light-receiving surface.

The living body information detection circuit of this embodiment is described with reference to attached figures taking a case, as an example, for applying the circuit to blood pressure measurement for a living body.

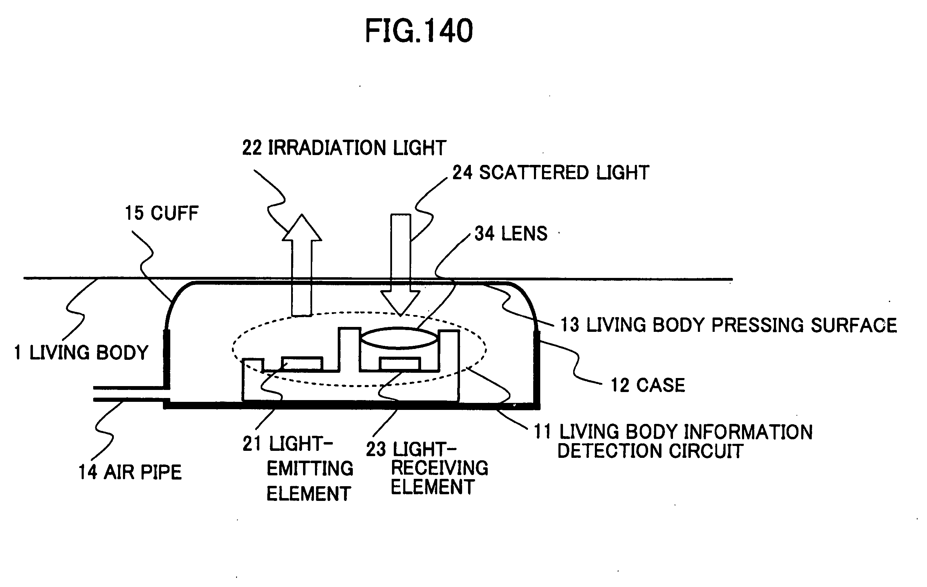

Fig.140 is a figure showing a configuration of the living body information detection circuit of this embodiment. Fig.140 shows a state in which the living body information detection circuit 11 of this embodiment is embedded in the cuff 15 and the cuff 15 contacts the living body.

In the configuration of the living body information detection circuit 11 and the cuff 15 of this embodiment shown in Fig.140, configurations other than the lens 34 of the living body information detection circuit 11 are the same as those of the living body information detection circuit 11 and the cuff 15 of the embodiment described with reference to Fig.134. The lens 34 is provided in front of the light-receiving surface of the light-receiving element 23

In the configuration of the living body information detection circuit 11 and the cuff 15 of this embodiment shown in Fig.140, operations other than the lens 34 of the living body information detection circuit 11 are the same as those of the living body information detection circuit 11 and the cuff 15 of the embodiment described with reference to Fig.134. The lens 34 has a function for concentrating scattered light 24 from a particular point of the living body 1 among the scattered light 24 onto the light-receiving surface of the light-receiving element 23. The lens 34 is set such that the scattered light 24 from the artery at a position at which the living body pressing surface 13 surely presses the living body 1 is concentrated onto the light-receiving surface of the light-receiving element 23.

Operation of the living body information detection circuit 11 of this embodiment is described. In the configuration of the living body information detection circuit 11 and the cuff 15 of this embodiment, operations of parts other than the lens 34 are the same as those of the living body information detection circuit 11 and the cuff 15 of the embodiment described with reference to Fig.134. The lens 34 concentrates only scattered light 24, in all scattered light 24, from the artery at a position at which the living body pressing surface 13 surely presses the living body 1, onto the light-receiving surface of the light-receiving element 23, and the light-receiving element 23 receives the scattered light 24 from the artery at a position at which the living body pressing surface 13 surely presses the living body 1 so as to detect the pulsation waveform.

As mentioned above, in the living body information detection circuit 11 of this embodiment, by providing the lens 34 for concentrating only scattered light 24, in all scattered light, from the artery at a position at which the living body pressing surface 13 surely presses the living body 1 onto the light-receiving surface of the light-receiving element 23, the scattered light 23 from the artery at a position at which the living body 1 is surely pressed by the living body pressing surface 13 can be selectively received, so that the pulsation waveform can be detected with high precision.

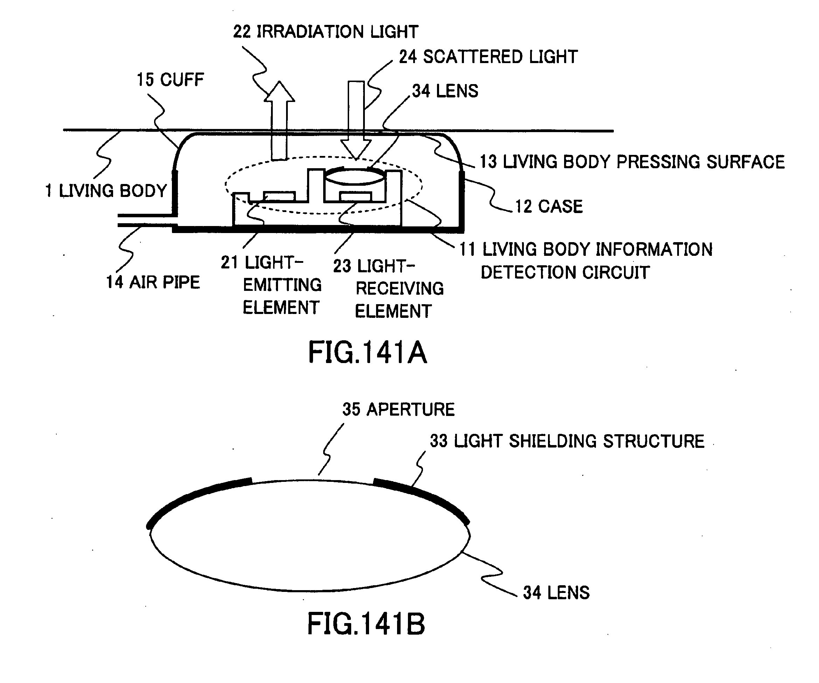

A light shielding structure including an aperture can be provided on the lens of the living body information detection circuit 11 described with reference to Fig.140. Fig.141 shows the living body information detection circuit 11 in which the lens is provided with the light shielding structure including the aperture. Fig.141A shows a configuration of the living body information detection circuit of this embodiment, and Fig.141B shows a section view of the lens including the light shielding structure including the aperture. Difference from the living body information detection circuit shown in Fig.140 is that the surface of the lens 34 is provided with the light shielding structure 33 having the aperture. Only scattered light 24 that travels in straight lines through the aperture concentrates on the light-receiving surface of the light-receiving element 23.

By using the lens 34, only scattered light 24, in all scattered light 24, from the artery at a position at which the living body pressing surface 13 surely presses the living body 1 can be concentrated onto the light-receiving surface of the light-receiving element 23, and by using the light shielding structure 33, light that is scattered in other parts of the living body 1 can be prevented from entering.

As described above, according to the present invention, a living body information detection circuit detecting the pulsation waveform with high precision can be provided.

The living body information detection circuit of this embodiment includes a light-emitting element for irradiating a part of the living body with light and a light-receiving element for receiving scattered light of the irradiating light scattered in the part of the living body so as to detect a pulsation waveform, wherein the light-emitting element is provided with a light shielding structure, in front of the light-emitting element, for limiting an angle of outgoing light from the light-emitting element.

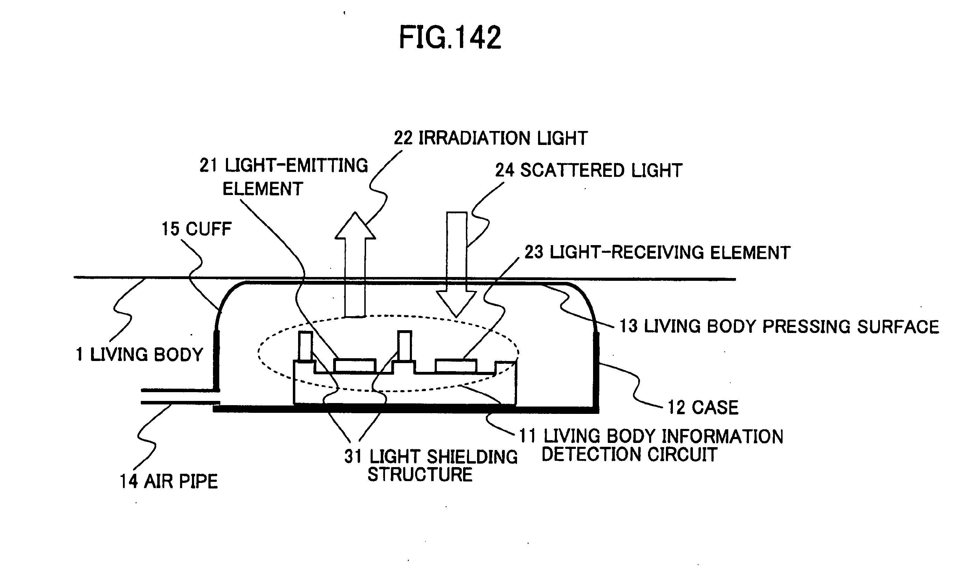

The living body information detection circuit of this embodiment is described with reference to attached figures taking a case, as an example, for applying the circuit to blood pressure measurement for a living body. Fig.142 is a figure showing a configuration of the living body information detection circuit of this embodiment. Fig.142 shows a state in which the living body information detection circuit of this embodiment is embedded in the cuff 15 and the cuff 15 contacts the living body.

The configuration of the living body information detection circuit 11 and the cuff 15 of this embodiment shown in Fig.142 corresponds to a case in which the light shielding structure 31 of the living body information detection circuit 11 in the embodiment described by Fig.134 is removed, and the light shielding structure 31 is provided ahead of both sides of the light-emitting element 21 so as to put the light-emitting element 21 between both sides of the light shielding structure 31. Configurations other than the light shielding structure 31 are the same as the configurations of the living body information detection circuit 11 and the cuff in the embodiment described with reference to Fig.134. It is adequate that the light shielding structure 31 is placed between the light-emitting element 21 and the living body 1.

In the configuration of the living body information detection circuit 11 and the cuff 15 of this embodiment, functions other than the light shielding structure 31 are the same as those of the living body information detection circuit 11 and the cuff 15 of the embodiment described with reference to Fig.134.

In the configuration of the living body information detection circuit 11 shown in Fig.142, the light shielding structure 31 has a function for limiting an angle of the outgoing light emitted by the light-emitting element 21. The light shielding structure 31 shields the outgoing light emitted at an angle out of a predetermined angle range, so that only the outgoing light emitted at an angle within the predetermined angle range becomes the irradiation light 22 irradiating the living body 1. The light shielding structure 31 may be like partitions provided in both sides of the light-emitting element 21, or may be like a tube surrounding the light-emitting element 21.

Operation of the living body information detection circuit 11 of this embodiment is described. In the living body information detection circuit 11 and the cuff 15 of this embodiment, operations of parts other than the light shielding structure 31 are the same as those of the living body information detection circuit 11 and the cuff 15 of the embodiment described with reference to Fig.134.

The light shielding structure 31 of the living body information detection circuit 11 in this embodiment limits an angle of the outgoing light emitted by the light-emitting element 21 so as to shield the outgoing light emitted at an angle out of a predetermined angle range, that is, at an angle directing to parts other than the artery at a position at which the living body pressing surface 13 surly presses the living body 1, so that only the outgoing light emitted at an angle within the predetermined angle range, that is, at an angle directing to the artery at a position at which the living body pressing surface 13 surly presses the living body 1 becomes the irradiation light 22 irradiating the living body 1.

As mentioned above, since the living body information detection circuit 11 in this embodiment includes the light shielding structure 31 for limiting the angle of the outgoing light emitted by the light-emitting element 21 in front of the light-emitting element 21, the living body information detection circuit 11 shields the outgoing light emitted at an angle directing to parts other than the artery at a position at which the living body pressing surface 13 surly presses the living body 1. Thus, by using the scattered light 24 from the artery at a position at which the living body pressing surface 13 surly presses the living body 1, the pulsation waveform can be detected with high precision.

As described above, according to the present invention, a living body information detection circuit detecting the pulsation waveform with high precision can be provided.

In the living body information detection circuit of this embodiment, the light shielding structure may be a hood provided in front of the light-emitting element.

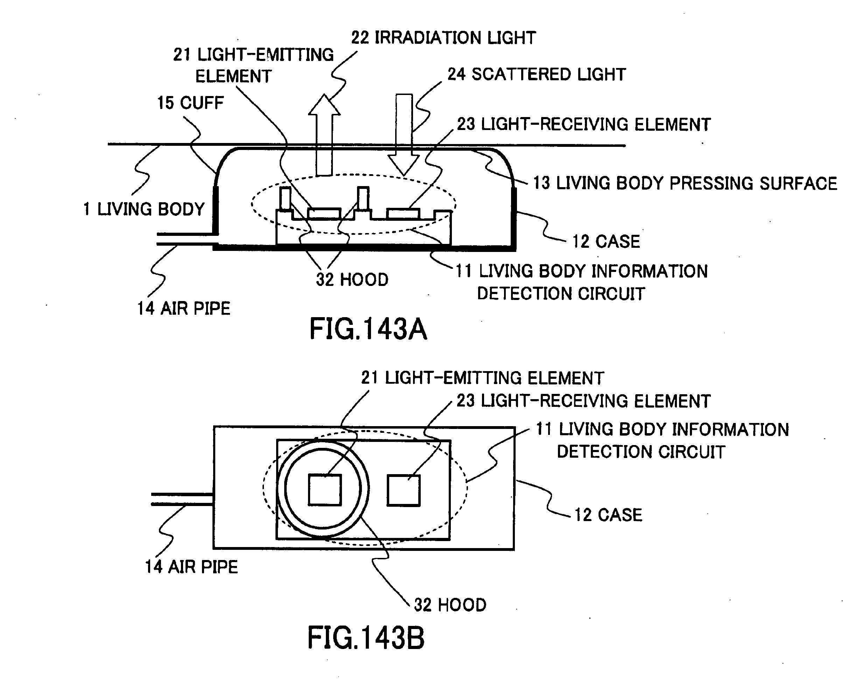

The living body information detection circuit of this embodiment is described with reference to attached figures taking a case, as an example, for applying the circuit to blood pressure measurement for a living body. Fig.143A and 143B are figures showing a configuration of the living body information detection circuit of this embodiment. Fig.143A shows a state in which the living body information detection circuit of this embodiment is embedded in the cuff 15 and the cuff 15 contacts the living body. Fig.143B is a view, viewed from the living body 1, of the state in which the living body information detection circuit of this embodiment is embedded in the cuff 15 shown in Fig. 138A.

The living body information detection circuit of this embodiment shown in Figs.143A and 143B is configured to be provided with the hood 32 instead of the light shielding structure 31 of the living body information detection circuit 11 of this embodiment described with reference to Fig.142. In the configuration of the living body information detection circuit 11 and the cuff 15 of this embodiment shown in Figs.143A and 143B, configurations other than the hood 32 of the living body information detection circuit are the same as those of the living body information detection circuit 11 and the cuff 15 of the embodiment described with reference to Fig.142.

In Figs.143A and 143B, the hood 32 of the living body information detection circuit 11 is shaped like a cylinder and is provided so as to surround the light-emitting element 21. Although the cylinder-like hood 32 is shown as an example, it may be like a square-tube.

In the living body information detection circuit 11 and the cuff 15 of this embodiment, functions of parts other than the hood 32 are the same as those of the living body information detection circuit 11 and the cuff 15 of the embodiment described with reference to Fig.142, and the function of the hood 32 of the living body information detection circuit 11 of this embodiment is the same as the light shielding structure 31 of the living body information detection circuit 11 of the embodiment described with reference to Fig.142.

Operation of the living body information detection circuit 11 of this embodiment is described. In the living body information detection circuit 11 and the cuff 15 of this embodiment, operations of parts other than the hood 32 are the same as those of the living body information detection circuit 11 and the cuff 15 of the embodiment described with reference to Fig.142, and the operation of the hood 32 of the living body information detection circuit 11 of this embodiment is the same as the light shielding structure 31 of the living body information detection circuit 11 of the embodiment described with reference to Fig.142.

As mentioned above, since the living body information detection circuit 11 in this embodiment includes the hood 32 for limiting the angle of the outgoing light emitted by the light-emitting element 21 in front of the light-emitting element 21, the living body information detection circuit 11 shields the outgoing light emitted at an angle directing to parts other than the artery at a position at which the living body pressing surface 13 surly presses the living body 1. Thus, by the scattered light 24 from the artery at a position at which the living body pressing surface 13 surly presses the living body 1, the pulsation waveform can be detected with high precision.

As described above, according to the present invention, a living body information detection circuit detecting the pulsation waveform with high precision can be provided.

In the living body information detection circuit of this embodiment, the light shielding structure may include an aperture in front of the light-emitting element.

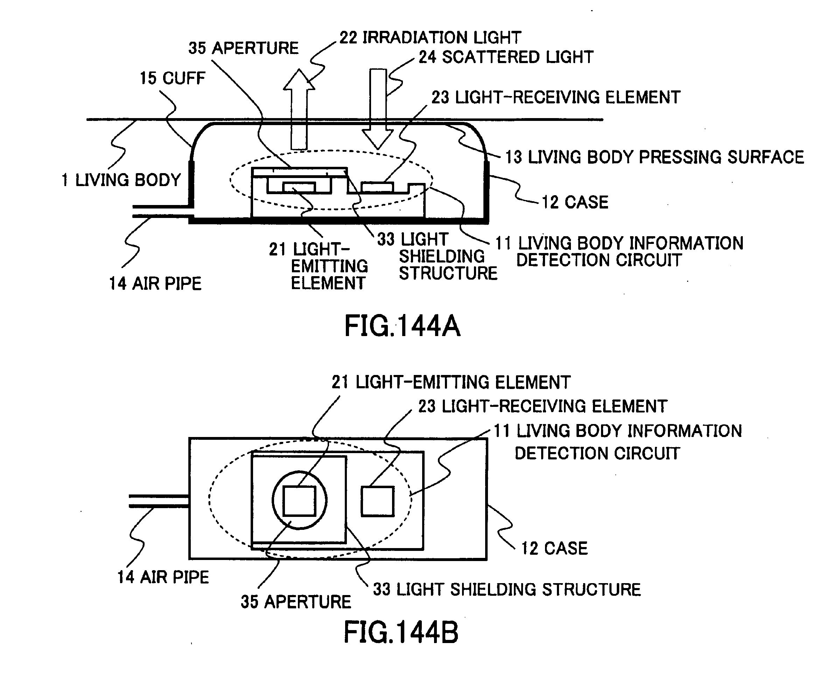

The living body information detection circuit of this embodiment is described with reference to attached figures taking a case, as an example, for applying the circuit to blood pressure measurement for a living body. Fig.144A and 144B are figures showing a configuration of the living body information detection circuit of this embodiment. Fig.144A shows a state in which the living body information detection circuit of this embodiment is embedded in the cuff 15 and the cuff 15 contacts the living body. Fig.144B is a view, viewed from the living body 1, of the state in which the living body information detection circuit of this embodiment is embedded in the cuff 15 shown in Fig.144A.

The living body information detection circuit of this embodiment shown in Figs.144A and 144B is configured to be provided with a light shielding structure 33 having an aperture 35 in place of the light shielding structure 31 of the living body information detection circuit 11 of the embodiment described with reference to Fig.142. In the configuration of the living body information detection circuit 11 and the cuff 15 of this embodiment shown in Figs.144A and 144B, configurations other than the light shielding structure having the aperture 35 of the living body information detection circuit 11 are the same as those of the living body information detection circuit 11 and the cuff 15 of the embodiment described with reference to Fig.142.

In Figs.144A and 144B, the light shielding structure 33 including the aperture 35 of the living body information detection circuit 11 includes a round aperture 35, and is provided ahead of the light-emitting element 21. Although the round aperture is shows as an example of the aperture of the light-shielding structure 33, the aperture 35 may be an ellipse, a rectangle, or other shape.

In the living body information detection circuit 11 and the cuff 15 of this embodiment, functions of parts other than the light shielding structure 33 having the aperture 35 are the same as those of the living body information detection circuit 11 and the cuff 15 of the embodiment described with reference to Fig.142, and the function of the light shielding structure 33 having the aperture 35 of the living body information detection circuit 11 of this embodiment is the same as the light shielding structure 31 of the living body information detection circuit 11 of the embodiment described with reference to Fig.142.

In the living body information detection circuit 11 and the cuff 15 of this embodiment, operations of parts other than the light shielding structure 33 having the aperture 35 are the same as those of the living body information detection circuit 11 and the cuff 15 of the embodiment described with reference to Fig.142, and the operation of the light shielding structure 33 having the aperture 35 of the living body information detection circuit 11 of this embodiment is the same as the light shielding structure 31 of the living body information detection circuit 11 of the embodiment described with reference to Fig.142.

As mentioned above, since the living body information detection circuit 11 in this embodiment includes the light shielding structure 33 having the aperture 35 for limiting the angle of the outgoing light emitted by the light-emitting element 21 in front of the light-emitting element 21, the living body information detection circuit 11 shields the outgoing light emitted at an angle directing to parts other than the artery at a position at which the living body pressing surface 13 surly presses the living body 1. Thus, by the scattered light 24 from the artery at a position at which the living body pressing surface 13 surly presses the living body 1, the pulsation waveform can be detected with high precision.

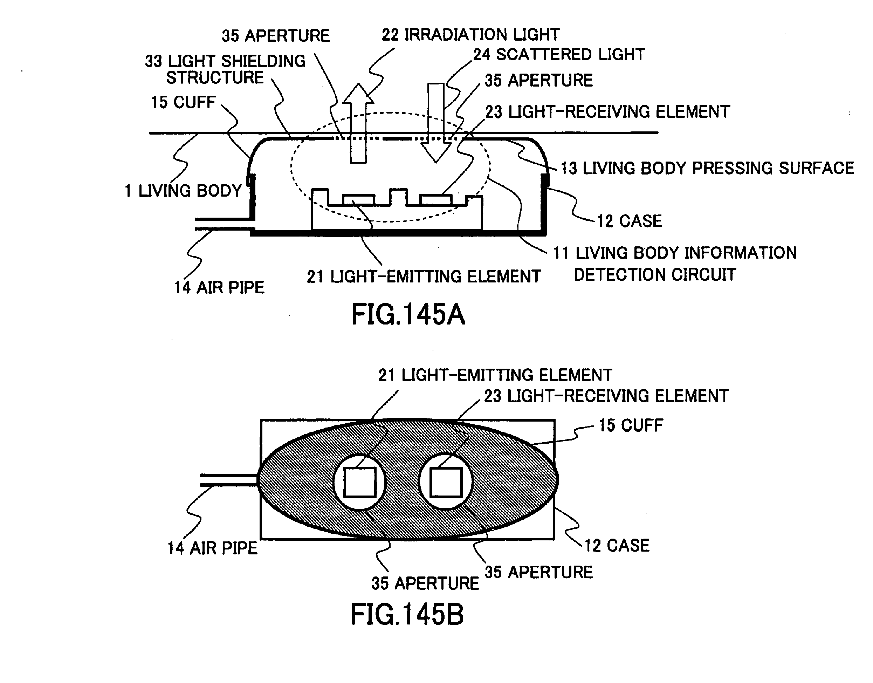

In addition, the cuff itself can be formed as a light shielding structure. Fig.145 shows the living body information detection circuit including a cuff having apertures. Difference from Fig.144 is that the cuff 15 includes the light shielding structure 33 having apertures 35 in place of the light shielding structure having an aperture provided above the light-emitting element. A shielding agent may be mixed to the cuff 15 or a shielding agent may be applied to the surface of the cuff 15. The apertures are provided in a part through which the irradiation light 22 emitted from the light-emitting element 21 passes and in a part through which the scattered light 24 scattered from the living body 1 passes.

By adopting this structure, the light shielding structure can be provided without adding new parts. Although the round aperture 35 is shown as an example in Fig.145, the aperture 35 may be an ellipse, a rectangle, or other shape.

As described above, according to the present invention, a living body information detection circuit detecting the pulsation waveform with high precision can be provided.

The living body information detection circuit of this embodiment includes a light-emitting element for irradiating a part of the living body with light and a light-receiving element for receiving scattered light of the irradiating light scattered in the part of the living body so as to detect a pulsation waveform, wherein the light-emitting element includes a lens, in front of the light-receiving element, for concentrating the outgoing light from the light-emitting element onto a particular position.

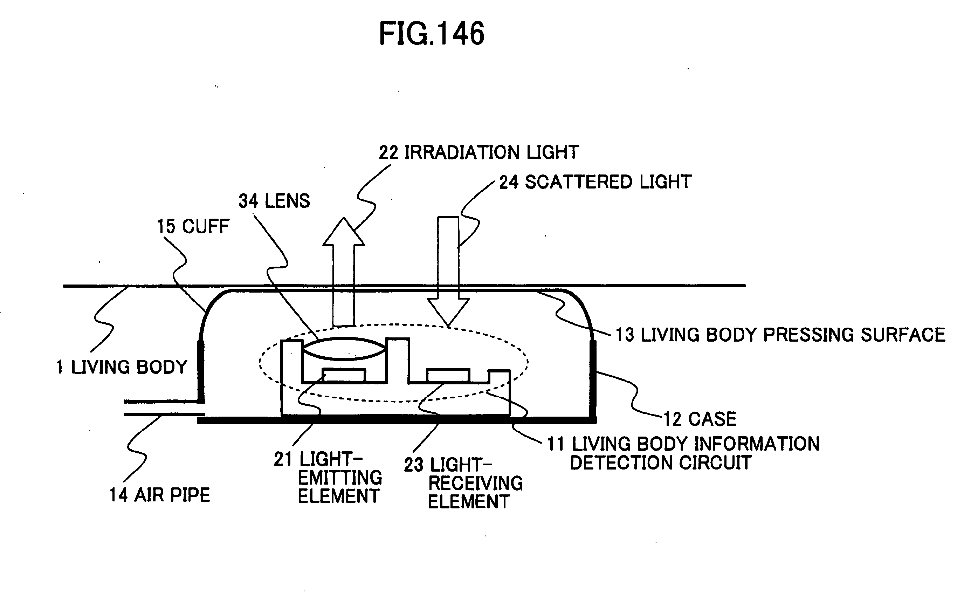

The living body information detection circuit of this embodiment is described with reference to attached figures taking a case, as an example, for applying the circuit to blood pressure measurement for a living body. Fig.146 is a figure showing a configuration of the living body information detection circuit of this embodiment. Fig.146 shows a state in which the living body information detection circuit of this embodiment is embedded in the cuff 15 and the cuff 15 contacts the living body.

The configuration of the living body information detection circuit 11 and the cuff 15 of this embodiment shown in Fig.146 corresponds to a configuration in which the light shielding structure 31 of the living body information detection circuit 11 in the embodiment described by Fig.142 is removed and the lens is provided ahead of the light-emitting element 21. Configurations other than the lens 34 of the living body information detection circuit 11 shown in Fig.146 are the same as those of the living body information detection circuit 11 and the cuff 15 of the embodiment described with reference to Fig.142 The lens 34 is provided in front of the light-emitting element 21.

In the configuration of the living body information detection circuit 11 and the cuff 15 of this embodiment, operations other than the lens 34 of the living body information detection circuit 11 are the same as those of the living body information detection circuit 11 and the cuff 15 of the embodiment described with reference to Fig.142.

In the living body information detection circuit 11 shown in Fig.146, the lens 34 has a function for concentrating the outgoing light from the light-emitting element 21 onto a particular position of the living body 1. The lens 34 is set such that the outgoing light from the light-emitting element 21 concentrates onto the artery at a position at which the living body pressing surface 13 surely presses the living body 1.

Operation of the living body information detection circuit 11 of this embodiment is described. In the configuration of the living body information detection circuit 11 and the cuff 15 of this embodiment, operations of parts other than the lens 34 are the same as those of the living body information detection circuit 11 and the cuff 15 of the embodiment described with reference to Fig.142.

The lens 34 of the living body information detection circuit 11 in this embodiment concentrates the outgoing light from the light-emitting element 21 onto the artery at a position at which the center part of the living body pressing surface 13 surely presses the living body 1 so as to irradiate the artery at the position, and the light-receiving element 23 receives the scattered light 24 from the artery at the position at which the living body pressing surface 13 surely presses the living body 1, so as to detect the pulsation waveform.

As mentioned above, in the living body information detection circuit 11 of this embodiment, by providing the lens 34, in front of the light-emitting element 21, for concentrating the outgoing light from the light-emitting element 21 onto the artery at a position at which the living body pressing surface 13 surely presses the living body 1, the light-receiving element 23 selectively receives the scattered light 24 from the artery at the position at which the living body pressing surface 13 surely presses the living body 1, so that the pulsation waveform can be detected with high precision.

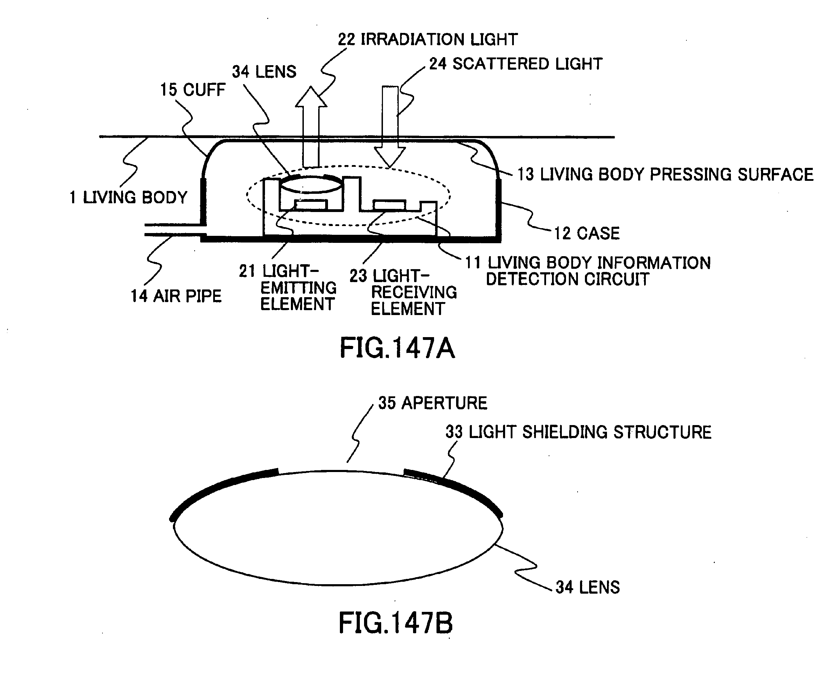

A light shielding structure including an aperture can be provided on the lens of the living body information detection circuit 11 described with reference to Fig.146. Fig.147 shows the living body information detection circuit 11 in which the lens is provided with the light shielding structure including the aperture. Fig.147A shows a configuration of the living body information detection circuit of this embodiment, and Fig.147B shows a section view of the lens including the light shielding structure including the aperture. Difference from the living body information detection circuit shown in Fig.147 is that the light shielding structure 33 including the aperture 35 is provided on a surface of the lens 34.

By the lens 34, the outgoing light from the light-emitting element 21 is concentrated onto the artery at a position at which the living body pressing surface 13 surely presses the living body 1, and, by the light shielding structure 33, irradiation of light on other parts of the living body 1 can be prevented.

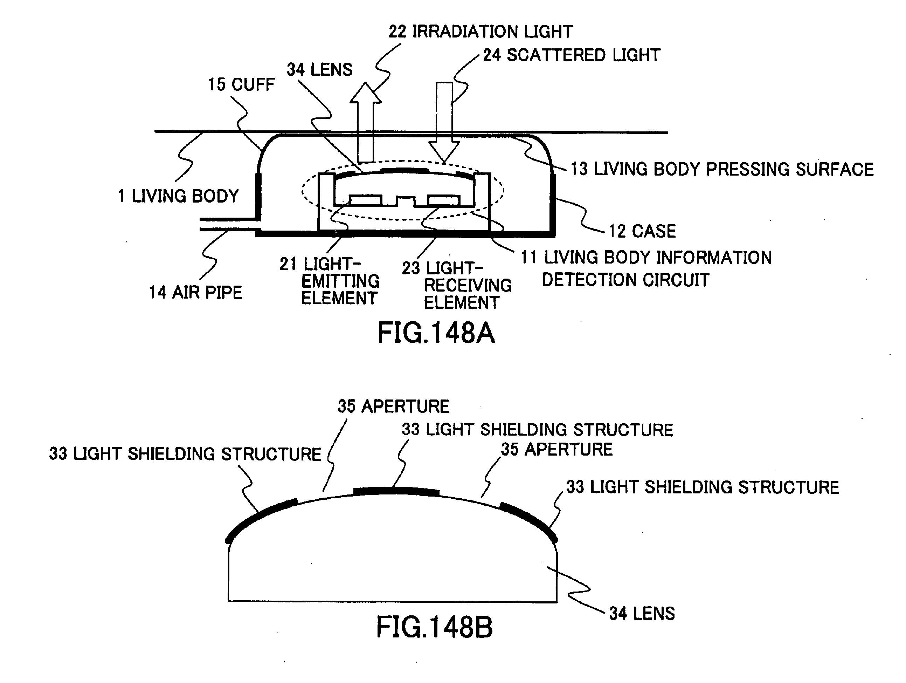

In addition, a common lens can be used for the light-emitting element and the light-receiving element. In this case, it is more effective to provide a light shielding structure including apertures for the light-emitting element and the light-receiving element respectively. Fig.148 shows the living body information detection circuit in which the light shielding structure having the apertures on the lens. Fig.148A is a figure for showing a configuration of the living body information detection circuit of this embodiment, and Fig.148B shows a section view of the lens provided with the light shielding structure having the apertures. Difference from the living body information detection circuit shown in Fig.147 is that the lens 34 is common for the light-emitting element and the light-receiving element, and two apertures 35 are provided on the surface of the lens 34 for the light shielding structure 33.

The lens 34 can be composed of resin and the like. The light shielding structure 33 can be formed by applying a light shielding agent. Effects by the lens and effects by the light shielding structure are the same as those of the aforementioned living body information detection circuit.

As described above, according to the present invention, a living body information detection circuit detecting the pulsation waveform with high precision can be provided.

The living body information detection circuit of this embodiment includes an edge emitting laser or a vertical cavity surface emitting laser for irradiating a part of the living body with light and a light-receiving element for receiving scattered light of the irradiating light scattered in the part of the living body so as to detect a pulsation waveform.

The living body information detection circuit of this embodiment is described with reference to attached figures taking a case, as an example, for applying the circuit to blood pressure measurement for a living body.

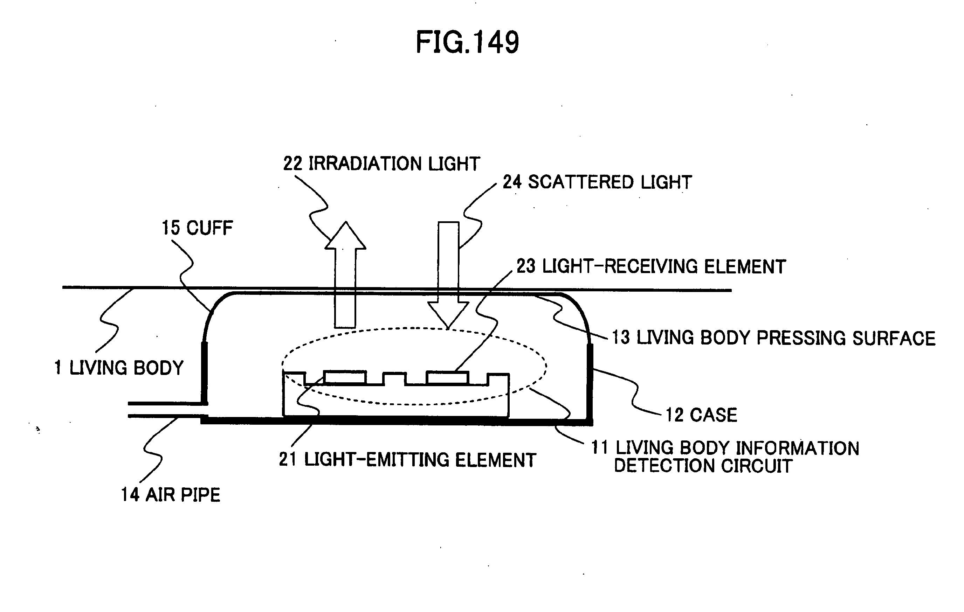

Fig.149 is a figure showing a configuration of the living body information detection circuit of this embodiment. Fig.149 shows a state in which the living body information detection circuit of this embodiment is embedded in the cuff 15 and the cuff 15 contacts the living body.

The configuration of the living body information detection circuit 11 and the cuff 15 of this embodiment shown in Fig.149 is the same as a configuration in which the light shielding structure 31 is removed from the living body information detection circuit 11 in the embodiment described by Fig.134. Configurations other than the light shielding structure 31 of the living body information detection circuit 11 are the same as those of the living body information detection circuit 11 and the cuff 15 of the embodiment described with reference to Fig.134

In the living body information detection circuit 11 in this embodiment shown in Fig.149, the light emitting element 21 is the edge emitting laser or the vertical cavity surface emitting laser. The edge emitting laser or the vertical cavity surface emitting laser is small and is characterized in that it can efficiently emit the irradiation light 22 with low power consumption.

Functions of each part in the living body information detection circuit 11 and the cuff 15 of this embodiment shown in Fig.149 are the same as functions of corresponding parts of the living body information detection circuit 11 in the embodiment described by Fig.134 except for the light shielding structure 31.

Operations of each part in the living body information detection circuit 11 and the cuff 15 of this embodiment shown in Fig.149 are the same as functions of corresponding parts of the living body information detection circuit 11 in the embodiment described by Fig.134 except for the light shielding structure 31. In the living body information detection circuit 11 in this embodiment, since the light emitting element 21 is the edge emitting laser or the vertical cavity surface emitting laser, the living body information detection circuit 11 is small and is characterized in that it can efficiently emit the irradiation light 22 with low power consumption.

As mentioned above, in the living body information detection circuit 11 of this embodiment, by using the edge emitting laser or the vertical cavity surface emitting laser as the light emitting element 21, the living body information detection circuit 11 can be realized to be small and low power consumption, and can detect the pulse wave waveform easily and with high precision.

As described above, according to the present invention, a living body information detection circuit detecting the pulsation waveform with high precision can be provided.

The living body information measurement apparatus of this embodiment includes U-shaped arms that pinch a tragus of a human body, a cuff for applying a pressure on the tragus wherein the cuff is provided in the inside of one of the arms, and any one of the living body information detection circuits described in Figs.134 and 138-149, wherein the living body information detection circuit is embedded in the cuff.

The living body information measurement apparatus of this embodiment is described with reference to attached figures taking a case, as an example, for applying the circuit to blood pressure measurement for a living body. The living body information measurement apparatus of this embodiment includes any one of the living body information detection circuits described in Figs.134 and 138-149. In any case, configuration, function and operation of the living body information detection circuit are the same as those of one of the living body information detection circuits 11 described in Figs.134 and 138-149. Therefore, as a representative example, a case including the living body information detection circuit 11 of the embodiment described with reference to Fig.134 is described.

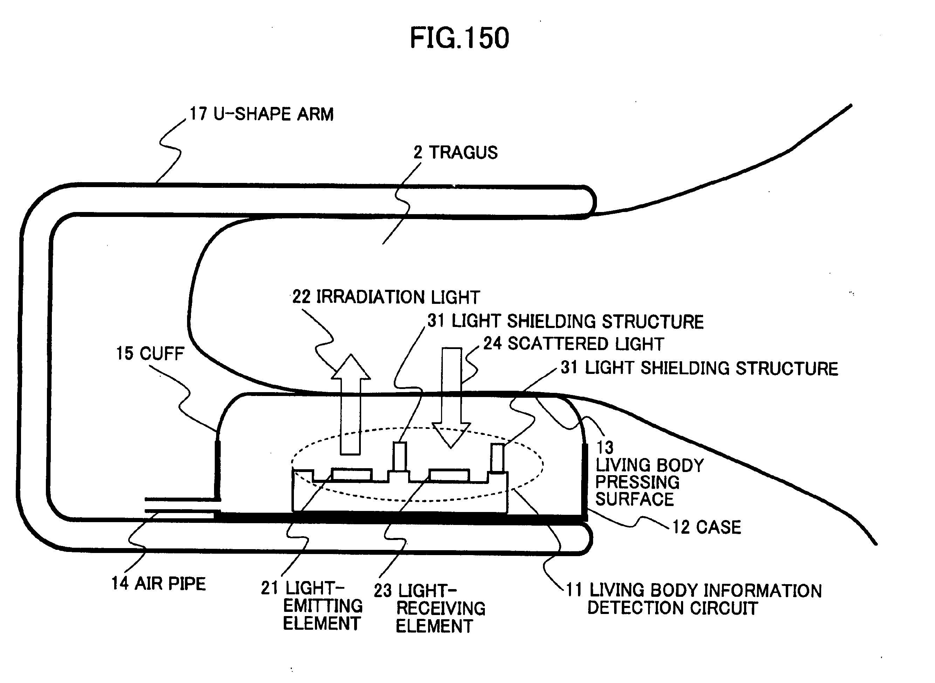

Fig.150 shows a configuration of the living body information measurement apparatus of this embodiment. In Fig.150, the living body information detection circuit 11 and the cuff 15 are similar to the living body information detection circuit 11 and the cuff 15 in the embodiment described by Fig.134, in which the U-shaped arms 17 mount the cuff 15 on the inside of one arm in a state in which the living body pressing surface 13 is directed to the inside. Fig.150 shows a case in which the U-shapes arms 17 are worn so that the surface of the inside of another arm and the living body pressing surface 13 of the cuff 15 pinch the tragus 2 of the human body. The inside of an arm means a side opposed to another arm of the U-shaped arms.

In the living body information measurement apparatus of this embodiment, functions of parts other than the U-shaped arms 17 are the same as the functions of the living body information detection circuit 11 and the cuff 15 in the embodiment described with reference to Fig.134.

The U-shaped arms 17 have a function for contacting and holding the tragus 2 so as to pinch the tragus 2 by the living body pressing surface 13 of the cuff 15 mounted in the inside of one arm in a state in which the living body pressing surface 13 of the cuff is directed to the inside, and a surface of the inside of another arm.

Operation of the living body information measurement apparatus of this embodiment is described. In the living body information measurement apparatus of this embodiment shown in Fig.150, operations of parts other than the U-shaped arms 17 are the same as the operations of the living body information detection circuit 11 and the cuff 15 in the embodiment described with reference to Fig.134.

The living body information measurement apparatus of this embodiment contacts the tragus 2 and is worn at the tragus 2 so as to pinch the tragus 2 by the living body pressing surface 13 of the cuff 15 mounted in the inside of one arm and a surface of the inside of another arm, so that the living body information measurement apparatus detects the pulsation waveform based on the operation similar to the living body information detection circuit 11 and the cuff 15 in the embodiment described with reference to Fig.134.

A case in which the living body information measurement apparatus of this embodiment includes the living body information detection circuit described in Figs.134 is described. But, configuration, function and operation of the living body information detection circuit when any one of the living body information detection circuits 11 described in Figs.138-149 is included are the same as those of the living body information detection circuits 11 described in Figs. 138-149.

As mentioned above, the living body information measurement apparatus of this embodiment mounts the cuff 15, embedding the living body information detection circuit 11, on the inside of one arm of the U-shaped arms 17, and is worn so as to pinch the tragus 2 of the human body by the cuff 15 and another arm, so that living body information can be measured continuously and with high precision.

As described above, according to the present invention, the living body information measurement apparatus for detecting living body information continuously and with high precision can be provided.

The living body information measurement apparatus of this embodiment includes U-shaped arms that pinch a tragus of a human body, cuffs for applying a pressure on the tragus wherein the cuffs are provided on each inside of both arms, and any one of the living body information detection circuits described in Figs.134 and 138-149, wherein the light-emitting element of the living body information detection circuit is embedded in one cuff, and the light-receiving element of the living body information detection circuit is embedded in another cuff.

The living body information measurement apparatus of this embodiment is described with reference to attached figures taking a case, as an example, for applying the circuit to blood pressure measurement for a living body. The living body information measurement apparatus of this embodiment includes one of the living body information detection circuits described in Figs.134 and 138-149 corresponding to a configuration including two cuffs. In any case, configuration, function and operation of the living body information detection circuit are the same as those of one of the living body information detection circuits 11 described in Figs.134 and 138-149. Therefore, as a representative example, a case including the living body information detection circuit 11 of the embodiment described with reference to Fig.135 including two cuffs 15 described in Fig.135 is described.

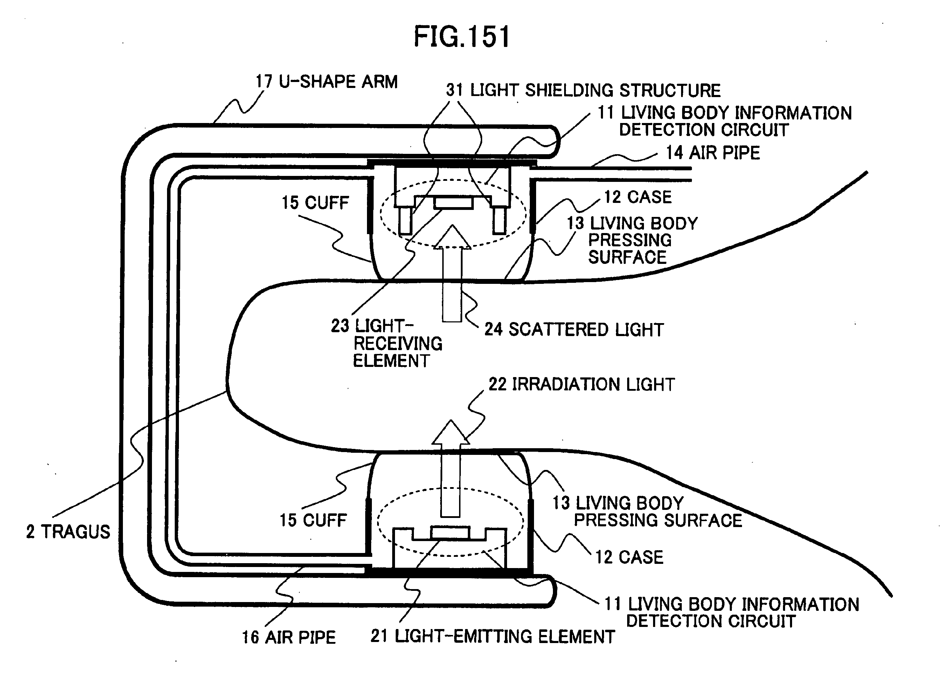

Fig.151 shows a configuration of the living body information measurement apparatus of this embodiment. In Fig.151, the living body information detection circuit 11 and the cuff 15 are similar to the living body information detection circuit 11 and the cuff 15 in the embodiment described by Fig.135, in which the U-shaped arms 17 mount a cuff 15 embedding the light emitting element 21 on the inside of one arm in a state in which the living body pressing surface 13 is directed to the inside, and mount another cuff 15 embedding the light-receiving element 23 on the inside of another arm in a state in which the living body pressing surface 13 is directed to the inside. Fig.151 shows a case in which the U-shapes arms 17 are worn so that the living pressing surfaces 13 of the cuffs 15 provided in both insides of the U-shaped arms 17 pinch and contact the tragus 2 of the human body. The inside of the arm means a side opposed to another arm of the U-shaped arms.

In the living body information measurement apparatus of this embodiment shown in Fig. 151, functions of parts other than the U-shaped arms 17 are the same as the functions of the living body information detection circuit 11 and the cuff 15 in the embodiment described with reference to Fig.135.

The U-shaped arms 17 have a function for contacting and holding the tragus 2 of a human body so as to pinch the tragus 2 by the living body pressing surface 13 of the cuff 15 including the light-emitting element 21 mounted on the inside of one arm and the living body pressing surface 13 of the cuff 15 including the light-receiving element 23 and the light shielding structure 31 mounted in the inside of another arm.

Operation of the living body information measurement apparatus of this embodiment is described. In the living body information measurement apparatus of this embodiment shown in Fig.151, operations of parts other than the U-shaped arms 17 are the same as the operations of the living body information detection circuit 11 and the cuff 15 in the embodiment described with reference to Fig.135.

The living body information measurement apparatus of this embodiment contacts the tragus 2 and is worn at the tragus 2 so as to pinch the tragus 2 by the living body pressing surfaces 13 of the cuffs 15 mounted in each inside of both U-shaped arms 17, so that the living body information measurement apparatus detects the pulsation waveform based on the operation similar to the living body information detection circuit 11 and the cuff 15 in the embodiment described with reference to Fig.135.

As described above, a case in which the living body information measurement apparatus of this embodiment includes the living body information detection circuit described in Figs.135 is described. But, configuration, function and operation of the living body information detection circuit when any one of the living body information detection circuits 11 described in Figs.138-149 having two cuffs is included are the same as those of the living body information detection circuits 11 described in Figs.138-149.

As mentioned above, in the living body information measurement apparatus of this embodiment, the light-emitting element 21 of the living body information detection circuit 11 is embedded in the cuff 15 that is mounted on the inside of one arm of the U-shaped arms 17, and the light-receiving element 23 and the light shielding structure 31 are embedded in the cuff 15 that is mounted on the inside of another arm of the U-shaped arms 17, so that the living body information measurement apparatus is worn so as to pinch the tragus 2 of the human body by both cuffs 15. Thus, living body information can be measured continuously and with high precision.

As described above, according to the present invention, the living body information measurement apparatus for detecting living body information continuously and with high precision can be provided.

Next, the living body information measurement apparatus of this embodiment is described more concretely.

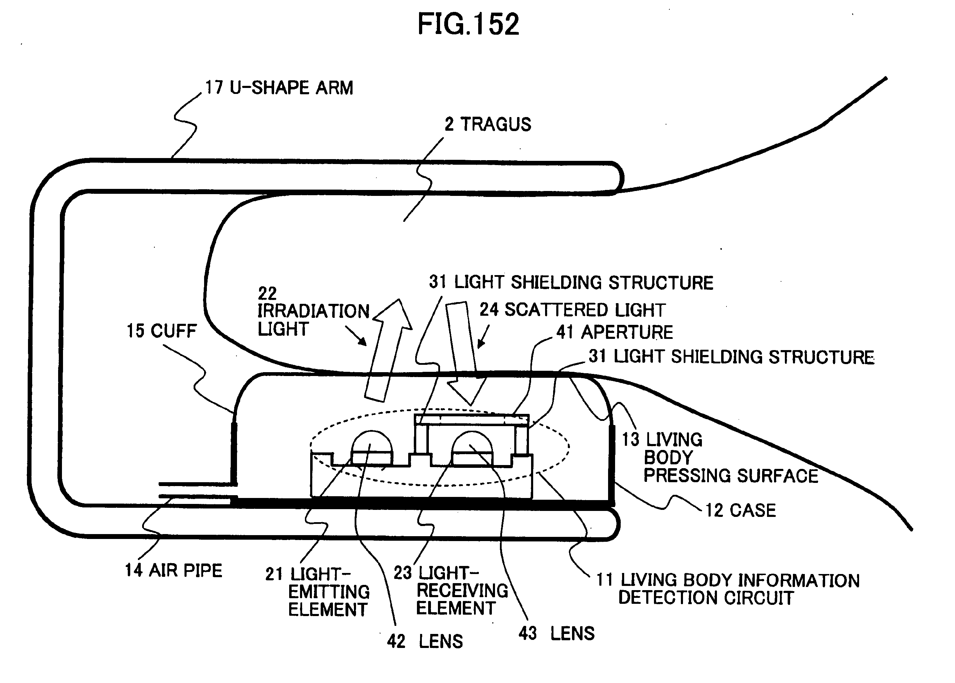

The living body information measurement apparatus shown in Fig.152 is provided with a GaAs infrared-emitting diode as the light-emitting element 21, and is provided with a visible light cut filter and an epoxy resin lens 42 of 1 mm in diameter on the light-emitting element 21. It is assumed that light-emitting wavelength in this case is near-infrared light of 0.9mm.

By using the lens 42, mode field of the outgoing light from the light-emitting diode is about 1 mm just after being emitted from the lens, and the light is emitted to the living body at directivity half-value angle of ± 15 degrees as directivity characteristics, that is a relatively narrow angle. The case 12 is round and 10 mm in diameter. The cuff 15 is composed of a silicone resin that is transparent for near-infrared region light, and is bonded to the case. Alternatively, the cuff 15 can be fixed to the case using an O ring. In this case, there is a merit that the cuff, which is easy to be worn, can be easily replaced. Assuming that distance between the surface of the lens and the surface of the tragus is 2 mm, the mode field of the outgoing light at the surface of the tragus becomes equal to or less than about 1.5 mm, which is adequately less than the diameter of 10 mm of the cuff 15. Thus, when the cuff 15 applies pressure on the tragus, light can be emitted to only a part of the living body on which the pressure is applied evenly.

As the light-receiving element 23, a Si phototransistor is used. In addition, similar to the light-emitting element 21, a visible light cut filter and an epoxy resin lens 43 of 1 mm in diameter is provided above the light-receiving element 23. The spectral sensitivity characteristics of the silicon phototransistor are 0.6mm - 0.97mm. But, due to the effect of the visible light cut resin, wavelength dependence of the spectral sensitivity characteristics is a range of 0.76mm - 0.97mm, and the peak exists at 0.87mm. As a result, spectral sensitivity for visible light is low so that effects of external light and the like can be suppressed to be low. The directivity characteristics of the light-receiving element 23 was measured, and the result was ± 30 degrees in half-value angle. Although the outgoing light to the living body is emitted for a narrow region, there is a possibility that light scattered in the inside of the living body may be received at a wide angle. Therefore, a light shielding structure 31 and an aperture 41 are placed above the light-receiving element 23. By the way, distance between the center of the light-emitting element 21 and the center of the light-receiving element 23 is set to be 2 mm, and the lenses are placed inwardly inclined slightly in order to improve S/N, so that the light emitted from the light-emitting element 21 is directed to the side of the light-receiving element 23.

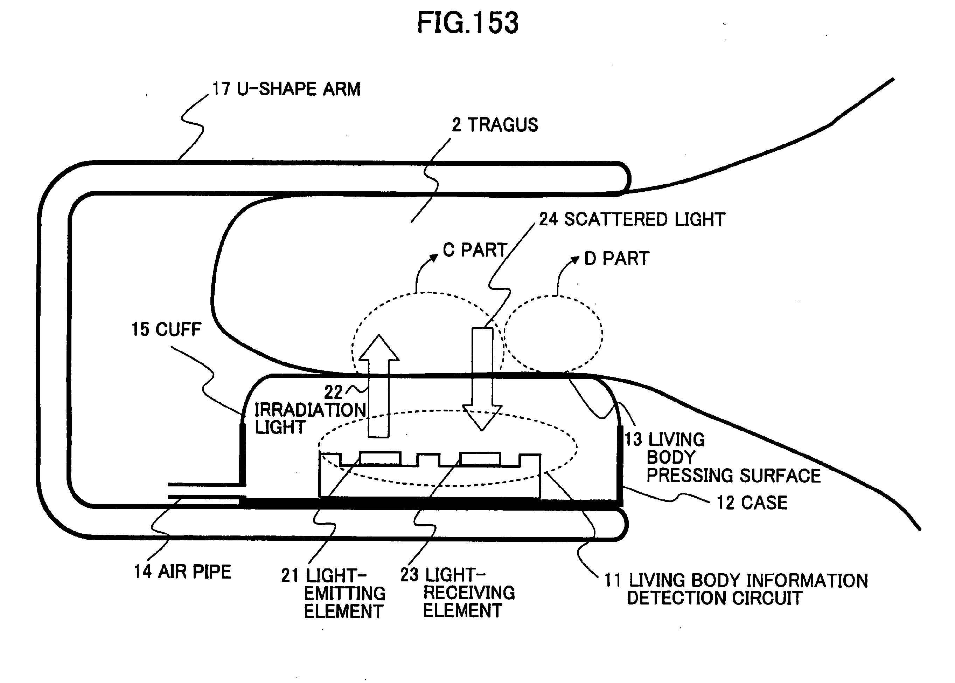

An apparatus shown in Fig.152 except for the lenses, aperture and light shielding structure 31 was manufactured for comparative experiment. The structure is shown in Fig.153.

First, blood pressure measurement was performed using the living body information detection circuit shown in Fig.153. After applying a pressure to the tragus 2 up to 200mmHg by supplying air in the case 12 from the air pipe 14, the pressure was reduced to obtain a pulsation waveform 75 similar to one shown in Fig.136. In the obtained pulsation waveform, both of the rising T1 of the pulsation waveform and the maximum value T2 of the pulsation waveform are not clear. In addition, in a region between T1 and T2, a waveform shown in Fig.137B was obtained. According to the above-mentioned results, it can be considered that the unclearness of the pulsation waveform is caused by a result that light signals from both of a part C and a part D are mixed, wherein the part C contacts a center and the vicinity of the center of the cuff and receives a pressure about the same as the inside pressure of the cuff, and the part D contacts a periphery part of the cuff and receives a pressure lower than the inside pressure of the cuff. As a result of the blood pressure measurement, it can be considered that measurement accuracy of the maximum blood pressure, the average blood pressure or the minimum blood pressure is degraded.

On the other hand, similar blood pressure measurement was performed using the living body information detection circuit having the structure shown in Fig.152. As a result, a pulsation waveform in which the rising T1 and the maximum value T2 are clear as shown in Fig.136A was obtained. In addition, a waveform as shown in Fig.137A was obtained in the region between T1 and T2. It can be considered that the result was obtained since the light signal from the D part was not received by virtue of the aperture 41 and only light signal from the C part was received. As a result of the blood pressure measurement, the maximum blood pressure, the average blood pressure or the minimum blood pressure was obtained with good accuracy.

In the above-mentioned embodiment, an example in which the near-infrared wavelength of about 0.9 mm is used is described. But, the wavelength range to be used is not limited to this. As a semiconductor laser, in a wavelength range of 0.65 mm - 1.00 mm, a semiconductor laser element that is embedded in a CD pickup element can be used. More specifically, a semiconductor laser of AlGaInP series near 0.65 mm or a semiconductor laser of GaAlAs series near 0.78 mm can be used. Alternatively, a laser diode of GaAsP series near 0.65 mm, a laser diode of GaP(Zn,O) series near 0.7 mm or a laser diode of AlGaAs series near 0.75 mm can be used. In a wavelength range of 1.00 mm - 1.70 mm, a semiconductor laser embedded in an optical communication apparatus can be used. More specifically, a semiconductor laser of InGaAsP series and the like can be used.

As a light-receiving element, the above-mentioned Si phototransistor may be used. In addition, a phototransistor may be used. When using visible light, a blue sensitive photodiode and the like can be used.

Concrete examples can be mentioned also for other embodiments. For example, when using the edge emitting laser or the vertical cavity surface emitting laser is used as the light-emitting element 21 as shown in Fig.149, mode field of light at the light outgoing part is narrow, that is, 1mm and 10mm respectively. Since output angle (far-field pattern) of light is ± 13 degrees for either case as a representative value, adequately narrow output angle can be obtained even without the lens. As a result, effects the same as those obtained by the structure of Fig.152 can be obtained.

The living body information detection circuit of the sixth embodiment can be applied to apparatuses (including blood-pressure meter) for measuring living body information in every embodiment in this specification of this application.

As mentioned above, the living body information detection circuit of the sixth embodiment includes a means for narrowing irradiation light irradiating the living body so as to irradiate a target position of the living body, and a means for selectively receiving scattered light from the target position of the living body. Thus, living body information is detected from the scattered light with high precision. In addition, the living body information measurement apparatus of this embodiment includes the living body information detection circuit, and can measure living body information continuously while being worn on the tragus.

In addition, the living body information detection circuit of the sixth embodiment includes a light shielding structure for restricting an angle at which incident light enters the light-receiving element. Therefore, since the scattered light from the target position of the living body can be selectively received and scattered light from a position that is not the target of the living body is not received, the pulsation waveform can be detected from the scattered light with high precision.

In addition, by using a hood provided in front of the light-receiving element as the light shielding structure, since the scattered light from the target position of the living body can be selectively received and scattered light from a position that is not the target of the living body is not received, the pulsation waveform can be detected from the scattered light with high precision.

In addition, by configuring the living body information detection circuit to include a light shielding structure, in front of the light-receiving element, having an aperture for limiting the angle at which the incident light enters the light-receiving element, since the scattered light from the target position of the living body can be selectively received and scattered light from a position that is not the target of the living body is not received, the pulsation waveform can be detected from the scattered light with high precision.

In addition, by configuring the living body information detection circuit to include a lens, in front of the light-receiving element, for concentrating scattered light from a particular position of the living body onto a light-receiving surface of the light-receiving element, since the scattered light from the target position of the living body can be selectively received and scattered light as noise from a position that is not the target of the living body is not received, the pulsation waveform can be detected from the scattered light with high precision.

In addition, by configuring the living body information detection circuit to include a light shielding structure, in front of the light-emitting element, for limiting an angle of outgoing light emitted from the light-emitting element, since outgoing light directed to a position other than the target position of the living body can be shielded and any position other than the target position of the living body is not irradiated, the pulsation waveform can be detected with high precision from the scattered light of the irradiation light scattered from the target position of the living body.

In addition, by configuring the living body information detection circuit to include a hood, in front of the light-emitting element, for limiting an angle of outgoing light emitted from the light-emitting element, since outgoing light directed to a position other than the target position of the living body is shielded, scatter of irradiation light at any position other than the target position of the living body is prevented so that the pulsation waveform can be detected with high precision from only scattered light from the target position of the living body.

In addition, by configuring the living body information detection circuit to include a light shielding structure having an aperture, in front of the light-emitting element, for limiting an angle of outgoing light emitted from the light-emitting element, since outgoing light directed to a position other than the target position of the living body is shielded, scatter of irradiation light at any position other than the target position of the living body is prevented so that the pulsation waveform can be detected with high precision from only scattered light from the target position of the living body.

In addition, by configuring the living body information detection circuit to include a lens, in front of the light-emitting element, for concentrating outgoing light emitted from the light-emitting element onto a particular position of the living body, since the target position of the living body is selectively irradiated with the outgoing light from the light-emitting element, scatter of irradiation light at any position other than the target position of the living body is prevented so that the pulsation waveform can be detected with high precision from only scattered light from the target position of the living body.

In addition, by irradiating a part of the living body with light emitted from the edge emitting laser or the vertical cavity surface emitting laser so that the light-receiving element receives scattered light of the irradiation light scattered in the part of the living body, the pulsation waveform can be easily detected with low power consumption and with high precision.

In addition, by configuring the living body information measurement apparatus to mount the living body information detection circuit in a cuff provided in the inside of one arm of the U-shaped arms that pinch the tragus, the living body information can be measured continuously with high precision. The inside of the U-shape arms is an opposing side of the U-shape arms.

In addition, by configuring the living body information measurement apparatus to mount the light-emitting element and the light-receiving element of the living body information detection circuit in both cuffs provided in each inside of the U-shaped arms that pinch the tragus, the living body information can be measured continuously with high precision.

As mentioned above, according to the present embodiment, by selectively irradiating the target position of the living body using an optical light-emitting element that is the edge emitting laser or the vertical cavity surface emitting laser and by selectively receiving scattered light from the target position of the living body, a living body information detection circuit that is small and consumes low power and can detect living body information with high precision can be provided.

In addition, by mounting the living body information detection circuit in the cuff for pinching the tragus, a living body information measurement apparatus that can measure living body information continuously and easily can be provided.

### (Seventh embodiment)

By the way, as variously described so far, for realizing an apparatus for measuring living body information at the auditory meatus part, since the auditory meatus is filled with a measurement part, there is a problem that the living body information measurement apparatus cannot report, to a subject, information such as a measurement result, start of measurement, under measurement, and the like when measuring the living body information.

Thus, in this embodiment, measured living body information is reported to the subject by using a configuration in which an ear measurement part (one shown in Fig.1, for example) to be worn in the auditory meatus part is provided with a acoustic part (speaker part) as shown in Fig.24, for example.

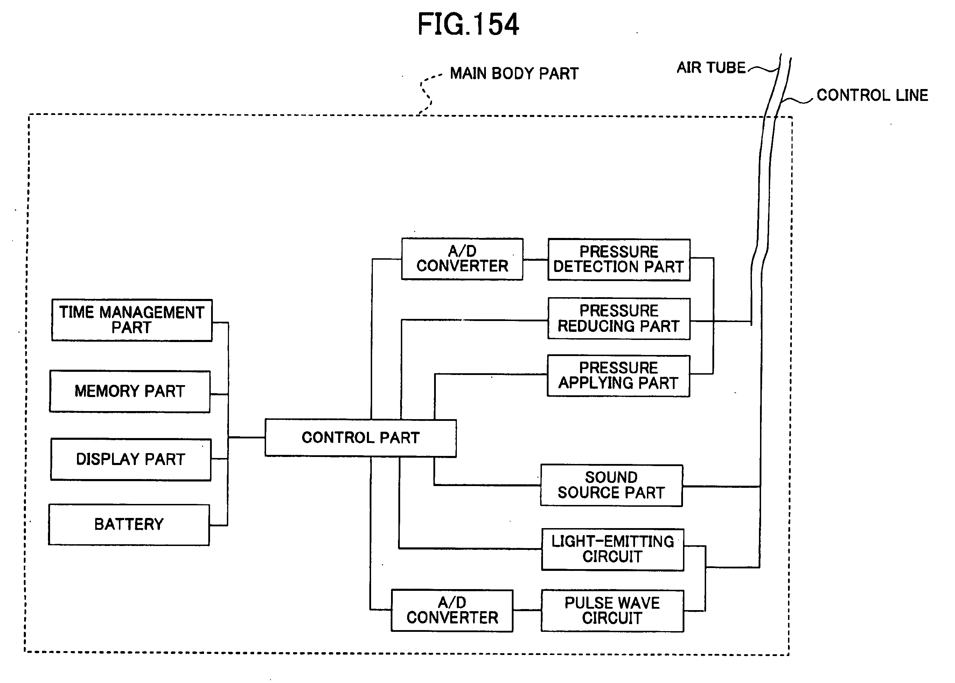

A configuration example of a main body part connected to the ear measurement part when using the ear measurement part as a blood-pressure meter is shown in Fig.154. The main body part shown in Fig.154 includes an air system including a pressure applying part for supplying air to the cuff to expand the cuff, a pressure reducing part for releasing air from the expanded cuff at a constant ratio to reduce the pressure in the cuff and a pressure detection part for detecting the pressure in the cuff, and the main body part further includes a light-emitting circuit for driving a light-emitting element, a pulse wave circuit for detecting a pulse wave signal obtained by irradiating the artery by the light-emitting element, a sound source part for generating a sound signal in this embodiment, and a control part for controlling these, and they are packaged in one case in high density so that the main body part can be put in a breast pocket. The main body part further includes a display part, a memory part, a time management part and a battery and the like. In addition, the main body part can be integrated with the ear measurement part. The sound source part generates various sound signals.

For example, when the control part detects end of measurement of a blood pressure, the result is reported to the sound source part. Then, based on the result, the sound source part generates an electrical signal and sends it to the speaker part wherein the electrical signal is for causing the speaker part to generate sound such as "maximum blood pressure is 120 and minimum blood pressure is 80", for example. Accordingly, the speaker part reports "maximum blood pressure is 120 and minimum blood pressure is 80" to the subject by voice sound.

In addition, by reporting start of measurement or the advance notice of the start to the sound source part by the control part, the subject can be notified of the start of measurement. For example, the report is performed by sound such as "blood pressure measurement starts now", or "pu, pu, pu, peen" like a time signal, or the like.

As mentioned above, by reporting the start of measurement or the advance notice, the subject can take a predetermined posture or a state such as rest, standing, sitting and the like, so that noise due to body movement and the like can be decreased and the blood pressure and the like can be measured more reliably.

Further, information indicating that measurement is in progress can be reported to the subject. For example, the report is performed using sound such as "under measurement", "pu, pu, pu, ..." like a pulse, or the like.

In addition, by storing a music, in the sound source part, for decreasing mental stress caused by measurement (for relaxing the subject), the music may be played for the subject while measurement is in progress. Accordingly, mental stress caused by measurement to the subject can be decreased. In this case, a wireless receiving part may be provided in place of the sound source part so as to play a music received by the wireless receiving part to the subject.

In addition, a configuration may be adopted in which predetermined times are set in the memory part, and the time management part refers to the information to report the control part when it becomes a set time (bedtime, for example), so that the control part controls to prevent generation of sound even when measurement starts. By adopting this configuration, sound can be vanished in the time such as the bedtime and the like.

In addition, a sound level can be changed by providing a volume control for sound. Accordingly, the sound level can be decreased for a case when an external sound level is low in the nighttime or in a quiet place or the like, and the sound level can be increased for a case when the external sound level is high in a crowd or in a noisy place or the like.

In addition, by providing a microphone to the living body information measurement apparatus to measure an external sound level, the sound level can be automatically adjusted according to the external sound level. For example, when the external sound level is higher than a predetermined sound level, the sound level is increased, and when the external sound level is lower than a predetermined sound level, the sound level is decreased.

The configuration for performing notification by sound in this embodiment is not limited to the living body information measurement apparatus including the ear measurement part worn in the auditory meatus part, and can be applied to apparatuses for measuring living body information in every embodiment described so far. For example, as shown in Fig.96, by providing a speaker in the ear measurement part for performing measurement by pinching a part of the external ear, the report of the sound as described in this embodiment can be performed.

By the way, although sound can be generated from the main body part, since it is desirable that information relating to privacy such as the living body information is not heard by other person, it is preferable that the ear measurement part close to the ear generates sound that can be heard only by the subject.

It is needless to say that the mechanism for holding the apparatus for measuring living body information and other distinctive mechanisms described in embodiments in this specification can be properly applied to apparatuses for measuring living body information in other embodiments.

The present invention is not limited to the specifically disclosed embodiments, and variations and modifications may be made without departing from the scope of the present invention.

## Claims

1. A blood-pressure meter comprising:
a frame part (3) including a first arm (1) and a second arm (2);
a pump (45), a pressure supplying pipe (48), a pressure sensor (40), a pressure control part (35), a control part (6) and a pressure applying part (30) for applying pressure to part (50) of an ear part, the pressure applying part (30) being provided on a side of the first arm (1) opposed to the second arm (2), the pressure applying part (30) being arranged to receive pressure from the pump (45) via the pressure supplying pipe (48);
a detection part for detecting a pulse wave at the part (50) of the ear part, the detection part comprising at least a pair of a light-emitting element (10) and a light-receiving element (20) for measuring the transmittance of light transmitted between the pressure applying part (30) and the second arm (2); the light-emitting element and the light-receiving element being placed in the pressure applying part and the fixing part so as to be opposed to each other across the part (50) of the ear part;
the pressure sensor (40) being connected by a pipe to the pump (45) and arranged to provide a pressure signal to the pressure control part (35) which is connected to control the pump (45);
driving and signal processing circuitry (15,25) arranged to drive the light-emitting element (10) and to process a signal from the light-receiving element (20) to provide an output to the control part (6) which is arranged to set the pressure controlled by the pressure control part (35) and to determine blood pressure from the output of the signal processing circuit; **characterised by**
a fixing part (4) provided on the inside of the second arm (2), wherein the pressure applying part (30) and the fixing part (4) are arranged to pinch the part (50) of the ear part; and
a fixing adjustment part (5) attached to the second arm (2) by a screw mechanism and confgured to push the fixing part (4) to allow adjustment of the spacing between the pressure applying part (30) and the fixing part (4) by means of a screw mechanism.

2. The blood-pressure meter as claimed in claim 1, wherein the pressure applying part (30) includes a mechanism using air pressure or an actuator.

## Patentansprüche

1. Blutdruckmessgerät, das aufweist:
ein Rahmenteil (3), das einen ersten Arm (1) und zweiten Arm (2) umfasst;
eine Pumpe (45), ein Drückzuführrohr (48), einen Drucksensor (40), ein Drucksteuerteil (35), ein Steuerteil (6) und ein Druckanwendungsteil (30) für das Anwenden des Druckes am Teil (50) eines Ohrteils, wobei das Druckanwendungsteil (30) auf einer Seite des ersten Arms (1) gegenüberliegend dem zweiten Arm (2) bereitgestellt wird, wobei das Druckanwendungsteil (30) angeordnet ist, um einen Druck von der Pumpe (45) über das Druckzuführrohr (48) zu empfangen;
ein Nachweisteil für das Nachweisen einer Impulswelle am Teil (50) des Ohrteils, wobei das Nachweisteil mindestens ein Paar eines lichtemittierenden Elementes (10) und eines lichtaufnehmenden Elementes (20) für das Messen der Durchlässigkeit des Lichtes, das zwischen dem Druckanwendungsteil (30) und dem zweiten Arm (2) durchgelassen wird, aufweist; wobei das lichtemittierende Element und das lichtaufnehmende Element im Druckanwendungsteil und im Befestigungsteil so angeordnet sind, dass sie über das Teil (50) des Ohrteils einander gegenüberliegend sind;
wobei der Drucksensor (40) mittels eines Rohres mit der Pumpe (45) verbunden und angeordnet ist, um ein Drucksignal zum Drucksteuerteil (35) zu liefern, das angeschlossen ist, um die Pumpe (45) zu steuern;
eine Antriebs- und Signalverarbeitungsschaltung (15, 25), die angeordnet ist, um das lichtemittierende Element (10) anzutreiben, und um ein Signal vom lichtaufnehmenden Element (20) zu verarbeiten, um ein Ausgabesignal zum Steuerteil (6) zu liefern, das angeordnet ist, um den Druck einzustellen, der vom Drucksteuerteil (35) gesteuert wird, und um den Blutdruck vom Ausgang der Signalverarbeitungsschaltung zu ermitteln;
**gekennzeichnet durch**
ein Befestigungsteil (4), das auf der Innenseite des zweiten Arms (2) vorhanden ist, wobei das Druckanwendungsteil (30) und das Befestigungsteil (4) angeordnet sind, um das Teil (50) des Ohrteils zusammenzudrücken; und
ein Befestigungseinstellteil (5), das am zweiten Arm (2) mittels eines Schraubmechanismus befestigt und ausgebildet ist, um das Befestigungsteil (4) zu drücken, um die Einstellung des Abstandes zwischen dem Druckanwendungsteil (30) und dem Befestigungsteil (4) mittels eines Schraubmechanismus zu gestatten.

2. Blutdruckmessgerät nach Anspruch 1, bei dem das Druckanwendungsteil (30) einen Mechanismus umfasst, der einen Luftdruck oder ein Betätigungselement benutzt.

## Revendications

1. Tensiomètre artériel, comprenant :
une partie de cadre (3), englobant un premier bras (1) et un deuxième bras (2) ;
une pompe (45), un tuyau d'alimentation de pression (48), un capteur de pression (40), une partie de contrôle de la pression (35), une partie de commande (6) et une partie d'application de la pression (30), pour appliquer une pression à une partie (50) d'une partie d'oreille, la partie d'application de la pression (30) étant agencée sur un côté du premier bras (1) opposé au deuxième bras (2), la partie d'application de la pression (30) étant agencée de sorte à recevoir la pression respective de la pompe (45) à travers le tuyau d'alimentation de la pression (48) ;
une partie de détection, pour détecter une onde d'impulsion (50) de la partie d'oreille, la partie de détection comprenant au moins une paire comprenant un élément d'émission de lumière (10) et un élément de réception de la lumière (20), pour mesurer la transmission de la lumière transmise entre la partie d'application de la pression (30) et le deuxième bras (2) ; l'élément d'émission de lumière et l'élément de réception de la lumière étant agencés dans la partie d'application de la pression et la partie de fixation, de sorte à être opposés l'un à l'autre à travers la partie (50) de la partie d'oreille ;
le capteur de pression (40) étant connecté par un tuyau à la pompe (45) et servant à transmettre un signal de pression à la partie de contrôle de la pression (35), qui est connectée de sorte à contrôler la pompe (45) ;
un circuit d'entraînement et de traitement de signaux (15, 25), destiné à entraîner l'élément d'émission de lumière (10) et à traiter un signal provenant de l'élément de réception de la lumière (20) pour transmettre un signal de sortie à la partie de commande (6), qui est destinée à régler la pression contrôlée par la partie de contrôle de la pression (35) et à déterminer la tension artérielle sur la base de la sortie du circuit de traitement des signaux ; **caractérisé par**
une partie de fixation (4), agencée sur l'intérieur du deuxième bras (2), la partie d'application de la pression (30) et la partie de fixation (4) étant destinées à serrer la partie (50) de la partie d'oreille ; et
une partie d'ajustement de fixation (5), fixée sur le deuxième bras (2) par un mécanisme à vis et configurée de sorte à exercer une poussée sur la partie de fixation (4), pour permettre l'ajustement de l'espacement entre la partie d'application de la pression (30) et la partie de fixation (4) par l'intermédiaire d'un mécanisme à vis.

2. Tensiomètre artériel selon la revendication 1, dans lequel la partie d'application de la pression (30) englobe un mécanisme utilisant une pression d'air ou un actionneur.
